(19) Europäisches Patentamt
European Patent Office
Office européen des brevets

(11) EP 2 074 086 B1

(12) EUROPEAN PATENT SPECIFICATION

(45) Date of publication and mention of the grant of the patent:
**15.06.2011 Bulletin 2011/24**

(21) Application number: **07852791.8**

(22) Date of filing: **16.10.2007**

(51) Int Cl.:
*C07D 207/06* (2006.01)
*C07D 401/12* (2006.01)
*C07D 403/10* (2006.01)
*C07D 405/12* (2006.01)
*C07D 409/10* (2006.01)
*C07D 417/10* (2006.01)
*A61K 31/506* (2006.01)
*A61P 25/28* (2006.01)

(86) International application number:
**PCT/US2007/022086**

(87) International publication number:
**WO 2008/048609 (24.04.2008 Gazette 2008/17)**

(54) **BIPHENYL SULFONYL AND PHENYL-HETEROARYL SULFONYL MODULATORS OF THE HISTAMINE H3-RECEPTOR USEFUL FOR THE TREATMENT OF DISORDERS RELATED THERETO**

BIPHENYL-SULFONYL- UND PHENYL-HETEROARYL-SULFONYL-MODULATOREN DES HISTAMIN-H3-REZEPTORS ZUR BEHANDLUNG VON DAMIT VERBUNDENEN ERKRANKUNGEN

MODULATEURS BIPHENYLSULFONYLE ET DE PHENYL-HETERORARYLSULFONYLE DU RECEPTEUR H3 DE L'HISTAMINE UTILES POUR LE TRAITEMENT DE TROUBLES ASSOCIES A CELUI-CI

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **17.10.2006 US 852610 P**
**11.06.2007 US 934358 P**

(43) Date of publication of application:
**01.07.2009 Bulletin 2009/27**

(73) Proprietor: **Arena Pharmaceuticals, Inc.**
**San Diego, CA 92121-3223 (US)**

(72) Inventors:
- **SANTORA, Vincent J.**
  **Carlsbad, CA 92008 (US)**
- **HART, Ryan M.**
  **San Diego, CA 92115 (US)**
- **IBARRA, Jason B.**
  **Las Vegas, NV 89139 (US)**
- **PARK, Douglas M.**
  **Valley Center, CA 92082 (US)**
- **REN, Albert S.**
  **San Diego, CA 92130 (US)**
- **SEMPLE, Graeme**
  **San Diego, CA 92127 (US)**
- **SCHULTZ, Jeffrey A.**
  **San Diego, CA 92127 (US)**
- **SMITH, Brian**
  **San Diego, CA 92129 (US)**
- **SMITH, Jeffrey**
  **San Diego, CA 92131 (US)**

(74) Representative: **Wytenburg, Wilhelmus Johannes et al**
**Mewburn Ellis LLP**
**33 Gutter Lane**
**London**
**EC2V 8AS (GB)**

(56) References cited:
**WO-A-2005/097740**
**WO-A-2008/005338**
**US-A1- 2005 256 127**

- **CELANIRE, S. ET AL.: "Keynote review: Histamine H3 receptor antagonists reach out for the clinic" DDT - DRUG DISCOVERY TODAY, ELSEVIER SCIENCE LTD, GB, vol. 10, no. 23-24, December 2005 (2005-12), pages 1613-1627, XP005222014 ISSN: 1359-6446**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF THE INVENTION

The present invention relates to certain compounds of Formula (**Ia**) and pharmaceutical compositions thereof that modulate the activity of the histamine H3-receptor. Compounds of the present invention and pharmaceutical compositions thereof are directed to methods useful in the treatment of histamine H3-associated disorders, such as, cognitive disorders, epilepsy, brain trauma, depression, obesity, disorders of sleep and wakefulness such as narcolepsy, shift-work syndrome, drowsiness as a side effect from a medication, maintenance of vigilance to aid in completion of tasks and the like, cataplexy, hypersomnia, somnolence syndrome, jet lag, sleep apnea and the like, attention deficit hyperactivity disorder (ADHD), schizophrenia, allergies, allergic responses in the upper airway, allergic rhinitis, nasal congestion, dementia, Alzheimer's disease and the like.

WO 2008/005338 A1 (Arena Pharmaceuticals, Inc.) describes certain compounds of the following formula which are modulators of the histamine H3-receptor, and which are useful for the treatment of disorders related thereto. The compounds of the present invention differ, at least, through the group -W-$R^1$.

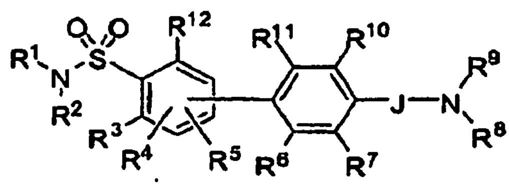

US 2005/0256127 A1 (Ku et al; Abbott Laboratories) describes certain salts of 2-(6-{2-[(2R)-2-methyl-1-pyrrolidin-1-yl]-ethyl}-2-naphthalen-2-yl)-2H-pyridazin-3-one (shown below), which demonstrate histamine-3 receptor ligand activity. The compounds of the present invention differ, at least, through the group -B-A-$SO_2$-W-$R^1$.

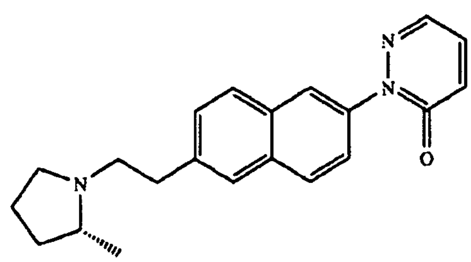

WO 2005/097740 A1 (Eli Lilly and Company) describes certain compounds of the following formula which have histamine-H3 receptor antagonist or inverse agonist activity, and which are useful for the treatment of obesity, cognitive deficiencies, narcolepsy, and other histamine H3 receptor-related diseases. The compounds of the present invention differ, at least, through the group -C($R^6R^7$)-C($R^8R^9$)-.

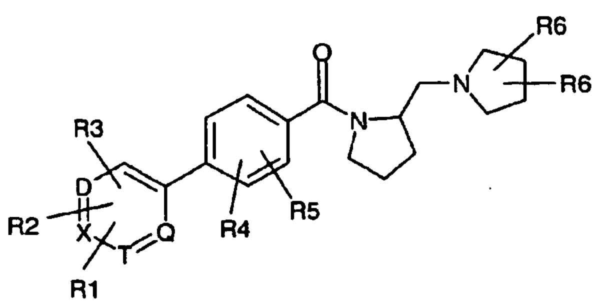

Celanire et al., Drug Discovery Today, December 2005, Vol. 10, No. 23/24, pp. 1613-1627 provides a review of histamine H3 receptor antagonists.

## SUMMARY OF THE INVENTION

One aspect of the present invention pertains to certain compounds as shown in Formula (**Ia**):

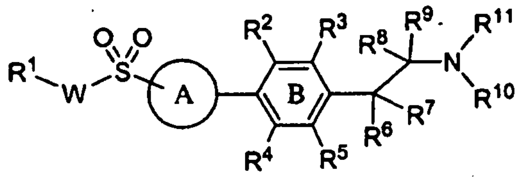

**(Ia)**

or a pharmaceutically acceptable salt, hydrate or solvate thereof; wherein:

$R^1$ is selected from the group consisting of H, $C_1$-$C_6$ acyl, $C_1$-$C_6$ acyloxy, $C_2$-$C_8$ alkenyl, $C_1$-$C_6$ alkoxy, $C_1$-$C_8$ alkyl, $C_1$-$C_8$ alkylcarboxamide, $C_2$-$C_8$ alkynyl, $C_1$-$C_8$ alkylsulfonamide, $C_1$-$C_8$ alkylsulfinyl, $C_1$-$C_8$ alkylsulfonyl, $C_1$-$C_8$ alkylthio, $C_1$-$C_8$ alkylureyl, amino, $C_1$-$C_8$ alkylamino, $C_2$-$C_8$ dialkylamino, carbo-$C_1$-$C_6$-alkoxy, carboxamide, carboxy, cyano, $C_3$-$C_7$ cycloalkyl, $C_2$-$C_8$ dialkylcarboxamide, $C_2$-$C_8$ dialkylsulfonamide, halogen, $C_1$-$C_6$ haloalkoxy, $C_1$-$C_6$ haloalkyl, $C_1$-$C_6$ haloalkylsulfinyl, $C_1$-$C_6$ haloalkylsulfonyl, $C_1$-$C_6$ haloalkylthio, $C_3$-$C_7$ heterocyclyl, hydroxyl, thiol, nitro, phenyl and sulfonamide, and each is optionally substituted with 1, 2, 3, 4 or 5 substituents selected independently from the group consisting of $C_1$-$C_6$ acyl, $C_1$-$C_6$ acyloxy, $C_2$-$C_8$ alkenyl, $C_1$-$C_6$ alkoxy, $C_1$-$C_8$ alkyl, $C_1$-$C_8$ alkylcarboxamide, $C_2$-$C_8$ alkynyl, $C_1$-$C_8$ alkylsulfonamide, $C_1$-$C_8$ alkylsulfinyl, $C_1$-$C_8$ alkylsulfonyl, $C_1$-$C_8$ alkylthio, $C_1$-$C_8$ alkylureyl, amino, $C_1$-$C_8$ alkylamino, $C_2$-$C_8$ dialkylamino, carbo-$C_1$-$C_6$-alkoxy, carboxamide, carboxy, cyano, $C_3$-$C_7$ cycloalkyl, $C_2$-$C_8$ dialkylcarboxamide, $C_2$-$C_8$ dialkylsulfonamide, halogen, $C_1$-$C_6$ haloalkoxy, $C_1$-$C_6$ haloalkyl, $C_1$-$C_6$ haloalkylsulfinyl, $C_1$-$C_6$ haloalkylsulfonyl, $C_1$-$C_6$ haloalkylthio, hydroxyl, thiol, nitro and sulfonamide; or

$R^1$ together with the W-$SO_2$ group and the ring atom to which the W-$SO_2$ group is bonded form a $C_5$-$C_7$ heterocyclic ring with Ring A whereby said $C_5$-$C_7$ heterocyclic ring and Ring A share two adjacent ring atoms, and said $C_5$-$C_7$ heterocyclic ring is optionally substituted with 1, 2, 3 or 4 substituents selected independently from the group consisting of $C_1$-$C_6$ acyl, $C_1$-$C_6$ acyloxy, $C_1$-$C_8$ alkenyl, $C_1$-$C_6$ alkoxy, $C_1$-$C_8$ alkyl, $C_1$-$C_8$ alkylcarboxamide, $C_2$-$C_8$ alkynyl, $C_1$-$C_8$ alkylsulfonamide, $C_1$-$C_8$ alkylsulfinyl, $C_1$-$C_8$ alkylsulfonyl, $C_1$-$C_8$ alkylthio, $C_1$-$C_8$ alkylureyl, amino, $C_1$-$C_8$ alkylamino, $C_2$-$C_8$ dialkylamino, carbo-$C_1$-$C_6$-alkoxy, carboxamide, carboxy, cyano, $C_3$-$C_7$ cycloalkyl, $C_2$-$C_8$ dialkylcarboxamide, $C_2$-$C_8$ dialkylsulfonamide, halogen, $C_1$-$C_6$ haloalkoxy, $C_1$-$C_6$ haloalkyl, $C_1$-$C_6$ haloalkylsulfinyl, $C_1$-$C_6$ haloalkylsulfonyl, $C_1$-$C_6$ haloalkylthio, hydroxyl, thiol, nitro, oxo and sulfonamide;

W is $C_1$-$C_4$ alkylene, $C_2$-$C_4$ alkenylene, $C_3$-$C_7$ cycloalkylene, $C_3$-$C_7$ heterocyclylene or phenylene, each optionally substituted with 1, 2, 3, 4, 5, 6, 7 or 8 substituents selected independently from the group consisting of $C_1$-$C_3$ alkyl, $C_1$-$C_4$ alkoxy, carboxy, cyano, $C_1$-$C_3$ haloalkyl, halogen, hydroxyl and oxo;

Ring A is 1,3-phenylene or 1,4-phenylene, each substituted with $R^{12}$, $R^{13}$, $R^{14}$ and $R^{15}$, wherein $R^{12}$, $R^{13}$, $R^{14}$ and $R^{15}$ are each selected independently from the group consisting of H, $C_1$-$C_6$ acyl, $C_1$-$C_6$ acyloxy, $C_2$-$C_8$ alkenyl, $C_1$-$C_6$ alkoxy, $C_1$-$C_8$ alkyl, $C_1$-$C_8$ alkylcarboxamide, $C_2$-$C_8$ alkynyl, $C_1$-$C_8$ alkylsulfonamide, $C_1$-$C_8$ alkylsulfinyl, $C_1$-$C_8$ alkylsulfonyl, $C_1$-$C_8$ alkylthio, $C_1$-$C_8$ alkylureyl, amino, $C_1$-$C_8$ alkylamino, $C_2$-$C_8$ dialkylamino, carbo-$C_1$-$C_6$-alkoxy, carboxamide, carboxy, cyano, $C_3$-$C_7$ cycloalkyl, $C_2$-$C_8$ dialkylcarboxamide, $C_2$-$C_8$ dialkylsulfonamide, halogen, $C_1$-$C_6$ haloalkoxy, $C_1$-$C_6$ haloalkyl, $C_1$-$C_6$ haloalkylsulfinyl, $C_1$-$C_6$ haloalkylsulfonyl, $C_1$-$C_6$ haloalkylthio, hydroxyl, thiol, nitro and sulfonamide; or

Ring A is a 6-membered heteroarylene or a 5-membered heteroarylene, each optionally substituted with $R^{16}$, $R^{17}$ and $R^{18}$, wherein $R^{16}$, $R^{17}$ and $R^{18}$ are each selected independently from the group consisting of $C_1$-$C_6$ acyl, $C_1$-$C_6$ acyloxy, $C_2$-$C_8$ alkenyl, $C_1$-$C_6$ alkoxy, $C_1$-$C_8$ alkyl, $C_1$-$C_8$ alkylcarboxamide, $C_2$-$C_8$ alkynyl, $C_1$-$C_8$ alkylsulfonamide, $C_1$-$C_8$ alkylsulfinyl, $C_1$-$C_8$ alkylsulfonyl, $C_1$-$C_8$ alkylthio, $C_1$-$C_8$ alkylureyl, amino, $C_1$-$C_8$ alkylamino, $C_2$-$C_8$ dialkylamino, carbo-$C_1$-$C_6$-alkoxy, carboxamide, carboxy, cyano, $C_3$-$C_7$ cycloalkyl, $C_2$-$C_8$ dialkylcarboxamide, $C_2$-$C_8$ dialkylsulfonamide, halogen, $C_1$-$C_6$ haloalkoxy, $C_1$-$C_6$ haloalkyl, $C_1$-$C_6$ haloalkylsulfinyl, $C_1$-$C_6$ haloalkylsulfonyl, $C_1$-$C_6$ haloalkylthio, hydroxyl, thiol, nitro and sulfonamide;

$R^2$, $R^3$, $R^4$ and $R^5$ are each selected independently from the group consisting of H, $C_1$-$C_6$ acyl, $C_1$-$C_6$ acyloxy, $C_2$-$C_8$ alkenyl, $C_1$-$C_6$ alkoxy, $C_1$-$C_8$ alkyl, $C_1$-$C_8$ alkylcarboxamide, $C_2$-$C_8$ alkynyl, $C_1$-$C_8$ alkylsulfonamide, $C_1$-$C_8$ alkylsulfinyl, $C_1$-$C_8$ alkylsulfonyl, $C_1$-$C_8$ alkylthio, $C_1$-$C_8$ alkylureyl, amino, $C_1$-$C_8$ alkylamino, $C_2$-$C_8$ dialkylamino, carbo-$C_1$-$C_6$-alkoxy, carboxamide, carboxy, cyano, $C_3$-$C_7$ cycloalkyl, $C_2$-$C_8$ dialkylcarboxamide, $C_2$-$C_8$ dialkylsulfonamide, halogen, $C_1$-$C_6$ haloalkoxy, $C_1$-$C_6$ haloalkyl, $C_1$-$C_6$ haloalkylsulfinyl, $C_1$-$C_6$ haloalkylsulfonyl, $C_1$-$C_6$ haloalkylthio, hydroxyl, thiol, nitro and sulfonamide;

$R^6$, $R^7$, $R^8$ and $R^9$ are each selected independently from the group consisting of H, $C_1$-$C_3$ alkyl, $C_1$-$C_4$ alkoxy, carboxy, cyano, $C_1$-$C_3$ haloalkyl, halogen and hydroxyl; and
$R^{10}$ and $R^{11}$ together with the nitrogen atom to which they are both bonded form 2-methyl-pyrrolidin-1-yl;
provided:

1) that Ring B and the sulfur of the $R^1$-W-S(O)$_2$- group are not bonded to adjacent ring atoms of Ring A; and
2) if Ring A is 1,3-phenylene or 1,4-phenylene, and W is $C_3$-$C_7$ heterocyclylene, then the ring atom of W that is directly bonded to the sulfur of the $R^1$-W-S(O)$_2$- group is other than nitrogen

One aspect of the present invention pertains to pharmaceutical compositions comprising a compound of the present invention and a pharmaceutically acceptable carrier.
Also described herein are methods for treating histamine H3-receptor associated disorders in an individual comprising administering to the individual in need thereof a therapeutically effective amount of a compound of the present invention or a pharmaceutical composition thereof.
Also described herein are methods for treating histamine H3-receptor associated disorders selected from the group consisting of cognitive disorders, epilepsy, brain trauma, depression, obesity, disorders of sleep and wakefulness such as narcolepsy, cataplexy, hypersomnia, somnolence syndrome, jet lag, sleep apnea and the like, attention deficit hyperactivity disorder (ADHD), schizophrenia, allergies, allergic responses in the upper airway, allergic rhinitis, nasal congestion, dementia and Alzheimer's disease.
Also described herein are methods for treating disorders of sleep and wakefulness in an individual comprising administering to the individual in need thereof a therapeutically effective amount of a compound of the present invention or a pharmaceutical composition thereof.
Also described herein are methods for treating cognitive disorders in an individual comprising administering to the individual in need thereof a therapeutically effective amount of a compound of the present invention or a pharmaceutical composition thereof.
Also described herein are methods for treating cataplexy in an individual comprising administering to the individual in need thereof a therapeutically effective amount of a compound of the present invention or a pharmaceutical composition thereof.
Also described herein are methods for inducing wakefulness in an individual comprising administering to the individual in need thereof a therapeutically effective amount of a compound of the present invention or a pharmaceutical composition thereof.
Also described herein are methods for treating pain in an individual comprising administering to the individual in need thereof a therapeutically effective amount of a compound of the present invention or a pharmaceutical composition thereof.
One aspect of the present invention pertains to the use of compounds of the present invention for production of a medicament for the treatment of a histamine H3-receptor associated disorder.
One aspect of the present invention pertains to the use of compounds of the present invention for production of a medicament for the treatment of a histamine H3-receptor associated disorder selected from the group consisting of cognitive disorders, epilepsy, brain trauma, depression, obesity, disorders of sleep and wakefulness such as narcolepsy, cataplexy, hypersomnia, somnolence syndrome, jet lag, sleep apnea and the like, attention deficit hyperactivity disorder (ADHD), schizophrenia, allergies, allergic responses in the upper airway, allergic rhinitis, nasal congestion, dementia and Alzheimer's disease.
One aspect of the present invention pertains to the use of compounds of the present invention for production of a medicament for the treatment of disorders of sleep and wakefulness.
One aspect of the present invention pertains to the use of compounds of the present invention for production of a medicament for the treatment of cognitive disorders.
One aspect of the present invention pertains to the use of compounds of the present invention for production of a medicament for the treatment of cataplexy.
One aspect of the present invention pertains to the use of compounds of the present invention for production of a medicament for use in inducing wakefulness.
One aspect of the present invention pertains to the use of compounds of the present invention for production of a medicament for treating pain.
One aspect of the present invention pertains to compounds of the present invention for use in a method of treatment of the human or animal body by therapy.
One aspect of the present invention pertains to compounds of the present invention for use in a method for the treatment of a histamine H3-receptor associated disorder in the human or animal body by therapy.
One aspect of the present invention pertains to compounds of the present invention for use in a method for the treatment of a histamine H3-receptor associated disorder selected from the group consisting of cognitive disorders,

epilepsy, brain trauma, depression, obesity, disorders of sleep and wakefulness such as narcolepsy, cataplexy, hypersomnia, somnolence syndrome, jet lag, sleep apnea and the like, attention deficit hyperactivity disorder (ADHD), schizophrenia, allergies, allergic responses in the upper airway, allergic rhinitis, nasal congestion, dementia and Alzheimer's disease in the human or animal body by therapy.

One aspect of the present invention pertains to compounds of the present invention for use in a method for the treatment of a disorder of sleep or wakefulness in the human or animal body by therapy.

One aspect of the present invention pertains to compounds of the present invention for use in a method for the treatment of a cognitive disorder in the human or animal body by therapy.

One aspect of the present invention pertains to compounds of the present invention for use in a method for the treatment of cataplexy in the human or animal body by therapy.

One aspect of the present invention pertains to compounds of the present invention for use in a method of inducing wakefulness in the human or animal body by therapy.

One aspect of the present invention pertains to compounds of the present invention for use in a method of treating pain in the human or animal body by therapy.

One aspect of the present invention pertains to processes for preparing a composition comprising admixing a compound of the present invention and a pharmaceutically acceptable carrier.

These and other aspects of the invention disclosed herein will be set forth in greater detail as the patent disclosure proceeds.

## BRIEF DESCRIPTION OF THE DRAWINGS

**Figure 1** shows a general synthetic scheme for the synthesis of (*R*)-2-methylpyrrolidine via reduction of L-prolinol and for its subsequent conversion into (*R*)-1-(4-bromophenethyl)-2-methylpyrrolidine and (*R*)-4-(2-(2-methylpyrrolidin-1-yl)ethyl)phenylboronic acid.

**Figure 2** shows a general synthetic scheme for the preparation of compounds of the present invention by microwave mediated, palladium catalyzed Suzuki reaction between a phenylboronic acid, and Ring A substituted with a leaving group such as halogen or triflate. The Ring A substituted with a sulfonyl group is prepared by the reaction of a thiol group with $R^1$-W-$LG^4$ and further oxidizing the resulting thioether to the sulfonyl group with an appropriate oxidizing agent. The boronic acid is prepared in two steps from a precursor containing two leaving groups. The first step involves reaction with an amine. The second step involves reaction with a trialkyl borate.

**Figure 3** shows a general synthetic scheme for the preparation of compounds of the present invention by microwave mediated, palladium catalyzed coupling reaction, such as a Suzuki Reaction, between a phenyl halide or triflate or the like and Ring A substituted with a sulfone and a boronic acid.

**Figure 4** shows a general synthetic scheme for the preparation of intermediates used in the preparation of compounds of the present invention.

**Figure 5** shows a general synthetic scheme for the preparation of compounds of the present invention wherein $R^8$ and $R^9$ are both hydrogen. The first step utilizes a compound of formula $R^1$-W-$LG^6$ to introduce the R'-W- group to the intermediate from Figure 4. The second step involves displacement of a leaving group (i.e., $LG^5$) with an amine. This is a particularly useful preparation for introducing a wide range of R'-W- groups by the selection of the appropriate reagent (i.e. $R^1$-W-$LG^6$). This preparation is also useful for introducing a wide range of $R^{10}$ and $R^{11}$ groups by the selection of the appropriate amine.

**Figure 6** shows a general synthetic scheme for the preparation of intermediates used in the preparation of compounds of the present invention.

**Figure 7** shows a general synthetic scheme for the preparation of compounds of the present invention wherein $R^8$ and $R^9$ are both hydrogen. This is a particularly useful preparation for introducing a wide range of $R^1$-W- groups by the selection of the appropriate reagent (i.e. $R^1$-W-$LG^6$).

## DETAILED DESCRIPTION OF THE INVENTION

### DEFINITIONS

For clarity and consistency, the following definitions will be used throughout this patent document.

The term **"agonists"** is intended to mean moieties that interact and activate the receptor, such as the histamine H3-receptor, and initiate a physiological or pharmacological response characteristic of that receptor. For example, when moieties activate the intracellular response upon binding to the receptor, or enhance GTP binding to membranes.

The term **"antagonists"** is intended to mean moieties that competitively bind to the receptor at the same site

as agonists (for example, the endogenous ligand), but which do not activate the intracellular response initiated by the active form of the receptor, and can thereby inhibit the intracellular responses by agonists or partial agonists. Antagonists do not diminish the baseline intracellular response in the absence of an agonist or partial agonist.

The term **"contact or contacting"** is intended to mean bringing the indicated moieties together, whether in an *in vitro* system or an *in vivo* system. Thus, "contacting" a histamine H3-receptor with a compound of the invention includes the administration of a compound of the present invention to an individual, preferably a human, having a histamine H3-receptor, as well as, for example, introducing a compound of the invention into a sample containing a cellular or more purified preparation containing a histamine H3-receptor.

The term **"in need of treatment"** and the term **"in need thereof'** when referring to treatment are used interchangeably to mean a judgment made by a caregiver (e.g. physician, nurse, nurse practitioner, etc. in the case of humans; veterinarian in the case of animals, including non-human mammals) that an individual or animal requires or will benefit from treatment. This judgment is made based on a variety of factors that are in the realm of a caregiver's expertise, but that includes the knowledge that the individual or animal is ill, or will become ill, as the result of a disease, condition or disorder that is treatable by the compounds of the invention. Accordingly, the compounds of the invention can be used in a protective or preventive manner; or compounds of the invention can be used to alleviate, inhibit or ameliorate the disease, condition or disorder.

The term **"individual"** is intended to mean any animal, including mammals, preferably mice, rats, other rodents, rabbits, dogs, cats, swine, cattle, sheep, horses, or primates, and most preferably humans.

The term **"inverse agonists"** is intended to mean moieties that bind to the endogenous form of the receptor or to the constitutively activated form of the receptor, and which inhibit the baseline intracellular response initiated by the active form of the receptor below the normal base level of activity which is observed in the absence of agonists or partial agonists, or decrease GTP binding to membranes. Preferably, the baseline intracellular response is inhibited in the presence of the inverse agonist by at least 30%, more preferably by at least 50%, and most preferably by at least 75%, as compared with the baseline response in the absence of the inverse agonist.

The term **"modulate or modulating"** is intended to mean an increase or decrease in the amount, quality, response or effect of a particular activity, function or molecule.

The term **"pharmaceutical composition"** is intended to mean a composition comprising at least one active ingredient; including but not limited to, salts, solvates and hydrates of compounds of the present invention; whereby the composition is amenable to investigation for a specified, efficacious outcome in a mammal (for example, without limitation, a human). Those of ordinary skill in the art will understand and appreciate the techniques appropriate for determining whether an active ingredient has a desired efficacious outcome based upon the needs of the artisan.

The term **"therapeutically effective amount"** is intended to mean the amount of active compound or pharmaceutical agent that elicits the biological or medicinal response in a tissue, system, animal, individual or human that is being sought by a researcher, veterinarian, medical doctor or other clinician, which includes one or more of the following:

(1) Preventing the disease; for example, preventing a disease, condition or disorder in an individual that may be predisposed to the disease, condition or disorder but does not yet experience or display the pathology or symptomatology of the disease,

(2) Inhibiting the disease; for example, inhibiting a disease, condition or disorder in an individual that is experiencing or displaying the pathology or symptomatology of the disease, condition or disorder (i.e., arresting further development of the pathology and/or symptomatology), and

(3) Ameliorating the disease; for example, ameliorating a disease, condition or disorder in an individual that is experiencing or displaying the pathology or symptomatology of the disease, condition or disorder (i.e., reversing the pathology and/or symptomatology).

## CHEMICAL GROUP, MOIETY OR RADICAL

The term **"$C_1$-$C_6$ acyl"** is intended to mean a $C_1$-$C_6$ alkyl radical attached to the carbon of a carbonyl group wherein the definition of alkyl has the same definition as described herein; some examples include, but are not limited to, acetyl, propionyl, *n*-butanoyl, *iso*-butanoyl, pivaloyl, pentanoyl, and the like.

The term **"$C_1$-$C_6$ acyloxy"** is intended to mean an acyl radical attached to an oxygen atom wherein acyl has the same definition as described herein; some embodiments are when acyloxy is $C_1$-$C_5$ acyloxy, some embodiments are when acyloxy is $C_1$-$C_4$ acyloxy. Some examples include, but are not limited to, acetyloxy, propionyloxy, butanoyloxy, *iso*-butanoyloxy, pentanoyloxy, hexanoyloxy, and the like.

The term **"$C_2$-$C_8$ alkenyl"** is intended to mean a radical containing 2 to 8 carbons wherein at least one carbon-carbon double bond is present, some embodiments are 2 to 7 carbons, some embodiments are 2 to 6 carbons, some embodiments are 2 to 5 carbons, some embodiments are 2 to 4 carbons, some embodiments are 2 to 3 carbons, and some embodiments have 2 carbons. Both *E* and *Z* isomers are embraced by the term **"alkenyl."** Furthermore, the term

**"alkenyl"** includes di- and tri-alkenyls. Accordingly, if more than one double bond is present then the bonds may be all E or all *Z* or a mixture thereof. Examples of an alkenyl include vinyl, allyl, 2-butenyl, 3-butenyl, 2-pentenyl, 3-pentenyl, 4-pentenyl, 2-hexenyl, 3-hexenyl, 4-hexenyl, 5-hexanyl, 2,4-hexadienyl and the like.

The term **"$C_1$-$C_6$ alkoxy"** is intended to mean a $C_1$-$C_6$ alkyl radical, as defined herein, attached directly to an oxygen atom, some embodiments are 1 to 5 carbons, some embodiments are 1 to 4 carbons, some embodiments are 1 to 3 carbons, and some embodiments are I or 2 carbons. Examples include methoxy, ethoxy, *n*-propoxy, *iso*-propoxy, *n*-butoxy, *t*-butoxy, *iso*-butoxy, *sec*-butoxy and the like.

The term **"$C_1$-$C_8$ alkyl"** is intended to mean a straight or branched carbon radical containing 1 to 8 carbons, some embodiments are I to 7 carbons, some embodiments are 1 to 6 carbons, some embodiments are 1 to 5 carbons, some embodiments are 1 to 4 carbons, some embodiments are 1 to 3 carbons, and some embodiments are 1 or 2 carbons. Examples of an alkyl include, but are not limited to, methyl, ethyl, *n*-propyl, *iso*-propyl, *n*-butyl, *sec*-butyl, *iso*butyl, *t*-butyl, pentyl, *iso*-pentyl, *t*-pentyl, *neo*-pentyl, 1-methylbutyl [i.e., $-CH(CH_3)CH_2CH_2CH_3$], 2-methylbutyl [i.e., $-CH_2CH(CH_3)CH_2CH_3$], *n*-hexyl, *n*-heptyl, *n*-octyl and the like.

The term **"$C_1$-$C_8$ alkylcarboxamido"** or **"$C_1$-$C_8$ alkylcarboxamide"** is intended to mean a single $C_1$-$C_8$ alkyl group attached to either the carbon or the nitrogen of an amide group, wherein alkyl has the same definition as found herein. The $C_1$-$C_8$ alkylcarboxamido may be represented by the following:

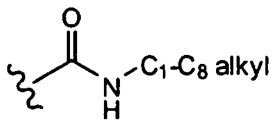

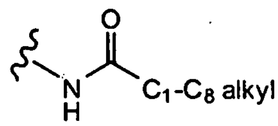

Examples include, but are not limited to, *N*-methylcarboxamide, *N*-ethylcarboxamide, *N*-*n*-propylcarboxamide, *N*-*iso*-propylcarboxamide, *N*-*n*-butylcarboxamide, *N*-*sec*-butylcarboxamide, *N*-*iso*-butylcarboxamide, *N*-*t*-butylcarboxamide and the like.

The term **"$C_1$-$C_4$-alkylene"** is intended to mean a $C_1$-$C_4$ divalent straight carbon group containing I to 4 carbons, some embodiments are 1 to 3 carbons, and some embodiments are 1 to 2 carbons. In some embodiments, alkylenyl refers to, for example, $-CH_2-$, $-CH_2CH_2-$, $-\ CH_2CH_2CH_2-$, and/or $-CH_2CH_2CH_2CH_2-$.

The term **"$C_2$-$C_4$-alkenylene"** is intended to mean a $C_2$-$C_4$ divalent straight carbon group containing 1 to 4 carbons and at least one double bond, some embodiments are 2 to 3 carbons, and some embodiments are 2 carbons. In some embodiments, alkenylene refers to, for example, $-CH=CH-$, $-CH_2CH=CH-$, $-CH=CHCH_2-$, $-CH_2CH=CHCH_2-$, $-CH=CHCH_2CH_2-$, and the like.

The term **"aryl-$C_1$-$C_4$-alkylenyl"** is intended to mean a $C_1$-$C_4$ alkylene group bonded to an aryl group, each as defined herein. In some embodiments aryl-$C_1$-$C_4$ alkylenyl refers to, for example, benzyl ($-CH_2$-phenyl), phenylethyl ($-CH_2CH_2$-phenyl), and the like.

The term **"heteroaryl-$C_1$-$C_4$-alkylenyl"** is intended to mean a $C_1$-$C_4$ alkylene group bonded to a heteroaryl group, each as defined herein. In some embodiments, heteroaryl-$C_1$-$C_4$-alkyleneyl refers to, for example, pyridinylmethyl ($-CH_2$-pyridinyl) and the like.

The term **"$C_1$-$C_8$ alkylsulfinyl"** is intended to mean a $C_1$-$C_8$ alkyl radical attached to the sulfur of a sulfoxide radical having the formula: $-S(O)-$ wherein the alkyl radical has the same definition as described herein. Examples include, but are not limited to, methylsulfinyl, ethylsulfinyl, *n*-propylsulfinyl, *iso*-propylsulfinyl, *n*-butylsulfinyl, *sec*-butylsulfinyl, *iso*butylsulfinyl, *t*-butylsulfinyl, and the like.

The term **"$C_1$-$C_8$ alkylsulfonamide"** is intended to mean the groups shown below:

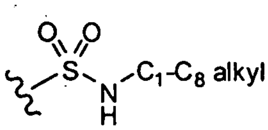

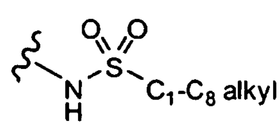

wherein $C_1$-$C_8$ alkyl has the same definition as described herein.

The term **"$C_1$-$C_8$ alkylsulfonyl"** is intended to mean a $C_1$-$C_8$ alkyl radical attached to the sulfur of a sulfone radical having the formula: $-S(O)_2-$ wherein the alkyl radical has the same definition as described herein. Examples include, but are not limited to, methylsulfonyl, ethylsulfonyl, *n*-propylsulfonyl, *iso*-propylsulfonyl, *n*-butylsulfonyl, *sec*-butylsulfonyl, *iso*-butylsulfonyl, *t*-butylsulfonyl, and the like.

The term **"$C_1$-$C_8$ alkylthio"** is intended to mean a $C_1$-$C_8$ alkyl radical attached to a sulfur atom (i.e., -S-) wherein

the alkyl radical has the same definition as described herein. Examples include, but are not limited to, methylsulfanyl (i.e., $CH_3S$-), ethylsulfanyl, *n*-propylsulfanyl, *iso*-propylsulfanyl, *n*-butylsulfanyl, *sec*-butylsulfanyl, *iso*-butylsulfanyl, *t*-butylsulfanyl, and the like.

The term **"$C_1$-$C_8$ alkylureyl"** is intended to mean the group of the formula: -NC(O)N-wherein one are both of the nitrogens are substituted with the same or different $C_1$-$C_8$ alkyl group wherein alkyl has the same definition as described herein. Examples of an alkylureyl include, but are not limited to, $CH_3NHC(O)NH$-, $NH_2C(O)NCH_3$-, $(CH_3)_2NC(O)NH$-, $(CH_3)_2NC(O)NH$-, $(CH_3)_2NC(O)NCH_3$-, $CH_3CH_2NHC(O)NH$-, $CH_3CH_2NHC(O)NCH_3$-, and the like.

The term **"$C_2$-$C_8$ alkynyl"** is intended to mean a radical containing 2 to 8 carbons and at least one carbon-carbon triple bond, some embodiments are 2 to 4 carbons, some embodiments are 2 to 3 carbons, and some embodiments have 2 carbons. Examples of an alkynyl include, but are not limited to, ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and the like. The term **"alkynyl"** includes di- and tri-ynes.

The term **"amino"** is intended to mean the group $-NH_2$.

The term **"$C_1$-$C_8$ alkylamino"** is intended to mean one alkyl radical attached to a -NH-radical wherein the alkyl radical has the same meaning as described herein. Some examples include, but are not limited to, methylamino, ethylamino, *n*-propylamino, *iso*-propylamino, *n*-butylamino, *sec*-butylamino, *iso*-butylamino, *t*-butylamino, and the like. Some embodiments are **"$C_1$-$C_2$ alkylamino."**

The term **"aryl"** is intended to mean an aromatic ring radical containing 6 to 10 ring carbons. Examples include phenyl and naphthyl.

The term **"carbo-$C_1$-$C_6$-alkoxy"** is intended to mean a $C_1$-$C_6$ alkyl ester of a carboxylic acid, wherein the alkyl group is as defined herein. Examples include, but are not limited to, carbomethoxy [$-C(=O)OCH_3$], carboethoxy, carbopropoxy, carbo-*iso*-propoxy, carbobutoxy, carbo-*sec*-butoxy, carbo-*iso*-butoxy, carbo-*t*-butoxy, carbo-*n*-pentoxy, carbo-*iso*-pentoxy, carbo-*t*-pentoxy, carbo-*neo*-pentoxy, carbo-*n*-hexyloxy, and the like.

The term **"carboxamide"** is intended to mean the group $-CONH_2$.

The term **"carboxy"** or **"carboxyl"** is intended to mean the group $-CO_2H$; also referred to as a carboxylic acid group.

The term **"cyano"** is intended to mean the group -CN.

The term **"$C_3$-$C_7$ cycloalkyl"** is intended to mean a saturated ring radical containing 3 to 7 carbons; some embodiments contain 3 to 6 carbons; some embodiments contain 3 to 5 carbons; some embodiments contain 5 to 7 carbons; some embodiments contain 3 to 4 carbons. Examples include cyclopropyl, cyclobutyl, cyclopentyl, cyclohexyl, cycloheptyl and the like.

The term **"$C_3$-$C_7$ cycloalkylene"** is intended to mean a saturated ring di-radical containing 3 to 7 carbons; some embodiments contain 3 to 6 carbons; some embodiments contain 3 to 5 carbons; some embodiments contain 5 to 7 carbons; some embodiments contain 3 to 4 carbons. Examples include cyclopropylenyl, cyclobutylenyl, cyclopentylenyl, cyclohexylenyl, cycloheptylenyl and the like. In some embodiments the $C_3$-$C_7$ cycloalkylenyl di-radical may be 1,2 disubstituted; for example 1,2-cyclopropyl, 1,2-cyclobutyl, 1,2-cyclopentyl, 1,2-cyclohexyl, 1,2-cycloheptyl and the like.

The term **"$C_2$-$C_8$ dialkylamino"** is intended to mean an amino substituted with two of the same or different $C_1$-$C_4$ alkyl radicals wherein alkyl radical has the same definition as described herein. Some examples include, but are not limited to, dimethylamino, methylethylamino, diethylamino, methylpropylamino, methylisopropylamino, ethylpropylamino, ethylisopropylamino, dipropylamino, propylisopropylamino and the like.

Some embodiments are **"$C_2$-$C_4$ dialkylamino."**

The term **"$C_2$-$C_8$ dialkylcarboxamido"** or **"$C_2$-$C_8$ dialkylcarboxamide"** is intended to mean two alkyl radicals, that are the same or different, attached to an amide group, wherein alkyl has the same definition as described herein. A $C_2$-$C_8$ dialkylcarboxamido may be represented by the following groups:

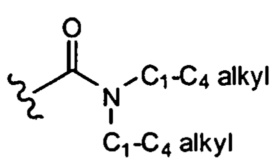

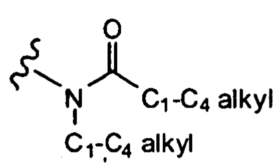

wherein $C_1$-$C_4$ has the same definition as described herein. Examples of a dialkylcarboxamide include, but are not limited to, *N,N*-dimethylcarboxamide, *N*-methyl-*N*-ethylcarboxamide, *N,N*-diethylcarboxamide, *N*-methyl-*N*-isopropylcarboxamide, and the like.

The **term "$C_2$-$C_8$ dialkylsulfonamide"** is intended to mean one of the following groups shown below:

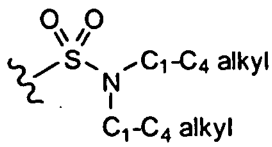

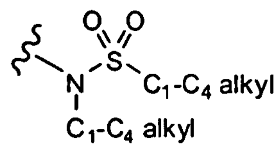

wherein $C_1$-$C_4$ has the same definition as described herein, for example but not limited to, methyl, ethyl, *n*-propyl, isopropyl, and the like.

The term **"$C_1$-$C_6$ haloalkoxy"** is intended to mean a $C_1$-$C_6$ haloalkyl, as defined herein, which is directly attached to an oxygen atom. Examples include, but are not limited to, difluoromethoxy, trifluoromethoxy, 2,2,2-trifluoroethoxy, pentafluoroethoxy and the like.

The term **"$C_1$-$C_6$ haloalkyl"** is intended to mean an $C_1$-$C_6$ alkyl group, defined herein, wherein the alkyl is substituted with one halogen up to fully substituted and a fully substituted $C_1$-$C_6$ haloalkyl can be represented by the formula $C_nL_{2n+1}$ wherein L is a halogen and "n" is 1, 2, 3, 4, 5 or 6; when more than one halogen is present then they may be the same or different and selected from the group consisting of F, Cl, Br and I, preferably F, some embodiments are I to 5 carbons, some embodiments are 1 to 4 carbons, some embodiments are 1 to 3 carbons, and some embodiments are 1 or 2 carbons. Examples of haloalkyl groups include, but are not limited to, fluoromethyl, difluoromethyl, trifluoromethyl, chlorodifluoromethyl, 2,2,2-trifluoroethyl, pentafluoroethyl and the like.

The term **"$C_1$-$C_6$ haloalkylsulfinyl"** is intended to mean a $C_1$-$C_6$ haloalkyl radical attached to the sulfur atom of a sulfoxide group having the formula: -S(O)- wherein the haloalkyl radical has the same definition as described herein. Examples include, but are not limited to, tnfluoromethylsulfinyl, 2,2,2-trifluoroethylsulfinyl, 2,2-difluoroethylsulfinyl and the like.

The term **"$C_1$-$C_6$ haloalkylsulfonyl"** is intended to mean a $C_1$-$C_6$ haloalkyl radical attached to the sulfur atom of a sulfone group having the formula: $-S(O)_2-$ wherein haloalkyl has the same definition as described herein. Examples include, but are not limited to, trifluoromethylsulfonyl, 2,2,2-trifluoroethylsulfonyl, 2,2-difluoroethylsulfonyl and the like.

The term **"$C_1$-$C_6$ haloalkylthio"** is intended to mean a $C_1$-$C_6$ haloalkyl radical directly attached to a sulfur wherein the haloalkyl has the same meaning as described herein. Examples include, but are not limited to, trifluoromethylthio (i.e., $CF_3S$-, also referred to as trifluoromethylsulfanyl), 1,1-difluoroethylthio, 2,2,2-trifluoroethylthio and the like.

The term **"halogen" or "halo"** is intended to mean to a fluoro, chloro, bromo or iodo group.

The term **"heteroaryl"** is intended to mean an aromatic ring system that may be a single ring, two fused nngs or three fused rings wherein at least one ring carbon is replaced with a heteroatom selected from, but not limited to, the group consisting of O, S and N wherein the N can be optionally substituted with H, $C_1$-$C_4$ acyl or $C_1$-$C_4$ alkyl. In some embodiments, heteroaryl is a 6-mcmbered heteroaryl (such as, pyridyl, pyrazinyl, and the like). In some embodiments, heteroaryl is a 5-membered heteroaryl (such as, pyrrolyl, thiazolyl, triazolyl, 1,3,4-thiadiazolyl, 1,2,3-thiadiazolyl, and the like). Examples of heteroaryl groups include, but arc not limited to, pyridyl, benzofuranyl, pyrazinyl, pyridazinyl, pyrimidinyl, triazinyl, quinolinyl, benzoxazolyl, benzothiazolyl, 1*H*-benzimidazolyl, isoquinolinyl, quinazolinyl, quinoxalinyl and the like. In some embodiments, the heteroatom is selected from, but not limited to, the group consisting of O, S and N, wherein N is substituted with H (i.e., NH), examples include, but are not limited to, pyrrolyl, indolyl, 1*H*-benzoimidazol-2-yl, and the like.

The term **"heteroarylene"** is intended to mean a di-radical of a heteroaryl ring wherein heteroaryl is as defined herein. In some embodiments, heteroarylene refers to 6-membered heteroarylene, for example, pyridazine, pyridine; and pyrimidine as shown respectively:

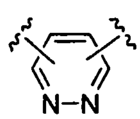

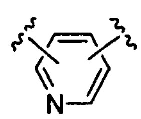

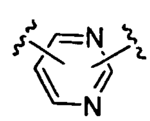
.

In some embodiments, heteroarylene refers to 5-membered heteroarylene, for example, [1,2,4]thiadiazole, 4H-[1,2,4]triazole, and [1,3,4]thiadiazole as shown respectively:

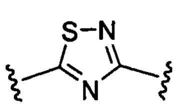

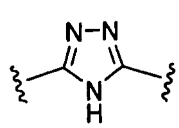

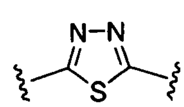
.

The term **$C_3$-$C_7$ heterocyclylene"** is intended to mean a di-radical of a heterocyclic ring wherein heterocyclic ring is as defined herein. In some embodiments, heterocyclylene refers to, for example, tetrahydropyran, tetrahydrofuran, piperidine, pyrrolidine, and the like; these can be represented as shown respectively:

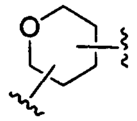 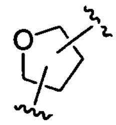 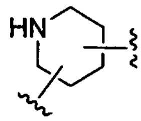 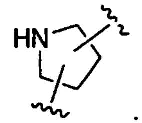 .

The term **"$C_3$-$C_7$ heterocyclic"** or **"$C_3$-$C_7$ heterocyclyl"** is intended to mean a nonaromatic carbon ring (i.e., $C_3$-$C_7$ cycloalkyl or $C_4$-$C_7$ cycloalkenyl as defined herein) wherein one or two ring carbons are replaced by a heteroatom selected from, but not limited to, the group consisting of O, S, S(=O), $S(=O)_2$, NH, wherein the N can be optionally substituted with $C_1$-$C_4$ alkyl or as described herein, in some embodiments, the nitrogen is optionally substituted with $C_1$-$C_4$ acyl or $C_1$-$C_4$ alkyl, and ring carbon atoms are optionally substituted with oxo or a thiooxo thus forming a carbonyl or thiocarbonyl group. The heterocyclic group can be attached/bonded to any available ring atom, for example, ring carbon, ring nitrogen, and the like. The heterocyclic group is a 3-, 4-, 5-, 6- or 7-membered ring. Examples of a heterocyclic group include, but are not limited to, aziridin-1-yl, aziridin-2-yl, azetidin-1-yl, azetidin-2-yl, azetidin-3-yl, piperidin-1-yl, piperidin-2-yl, piperidin-3-yl, piperidin-4-yl, morpholin-2-yl, morpholin-3-yl, morpholin-4-yl, piperzin-1-yl, piperzin-2-yl, piperzin-3-yl, piperzin-4-yl, pyrrolidin-1-yl, pyrrolidin-2-yl, pyrrolidin-3-yl, [1,3]-dioxolan-2-yl, thiomorpholin-4-yl, [1,4]oxazepan-4-yl, 1,1-dioxo-1$\lambda^6$-thiomorpholin-4-yl, azepan-1-yl, azepan-2-yl, azepan-3-yl, azepan-4-yl, tetrahydro-furan-2-yl, tetrahydro-furan-3-yl, tetrahydro-pyran-2-yl, tetrahydro-pyran-3-yl, tetrahydro-pyran-4-yl, and the like.

The term **"hydroxyl"** is intended to mean the group -OH.

The term **"nitro"** is intended to mean the group $-NO_2$.

The term **"oxo"** is intended to mean the substituent =O, accordingly, as a result, when a carbon is substituted by an "oxo" group the new group resulting from the carbon and oxo together is a carbonyl group.

The term **"phenyl"** is intended to mean the group $C_6H_5$-.

The term **"phenylene"** is intended to mean the di-radical of benzene. In some embodiments, phenylene is intended to mean 1,2-phenylene, in some embodiments, phenylene is intended to mean 1,3-phenylene, in some embodiments, phenylene is intended to mean 1,4-phenylene, they can be represented as follows:

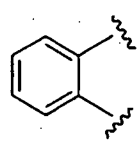
1,2-phenylene

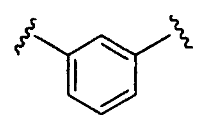
1,3-phenylene

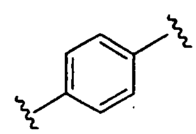
1,4-phenylene.

The term "**sulfonamide**" is intended to mean the group $-SO_2NH_2$.

The term "**thiol**" is intended to mean the group -SH.

**COMPOUNDS OF THE INVENTION:**

One aspect of the present invention pertains to certain compounds as shown in Formula (**Ia**):

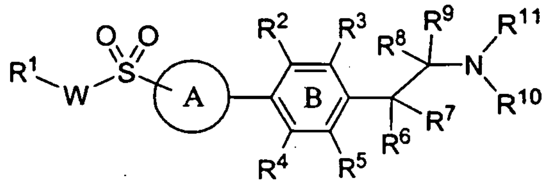
**(Ia)**

or a pharmaceutically acceptable salt, hydrate or solvate thereof;
wherein $R^1$, $R^2$, $R^3$, $R^4$, $R^5$, $R^6$, $R^7$, $R^8$, $R^9$, $R^{10}$, $R^{11}$, W and Ring A have the same definitions as described herein,

*supra* and *infra.*

In some embodiments, the present invention pertains to compounds, as described herein, provided that Ring B and the sulfur of the $R^1$-W-$S(O)_2$- group are not bonded to adjacent ring atoms of Ring A.

In some embodiments, the present invention pertains to compounds, as described herein, provided that if Ring A is 1,3-phenylene or 1,4-phenylene, and W is $C_3$-$C_7$ heterocyclylene, then the ring atom of W that is directly bonded to the sulfur of the $R^1$-W-$S(O)_2$- group is other than nitrogen.

In some embodiments, the present invention pertains to compounds as described herein provided that if W is $C_3$-$C_7$ heterocyclylene, then the ring atom of W that is directly bonded to the sulfur of the -$S(O)_2$- is other than nitrogen.

In some embodiments, the present invention pertains to compounds, as described herein, other than:

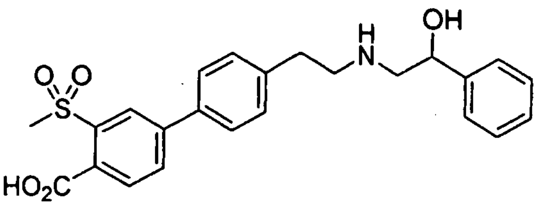

with the chemical name: 4'-[2-(2-hydroxy-2-phenyl-ethylamino)-ethyl]-3-methanesulfonyl-biphenyl-4-carboxylic acid.

In some embodiments, the present invention pertains to compounds, as described herein, other than:

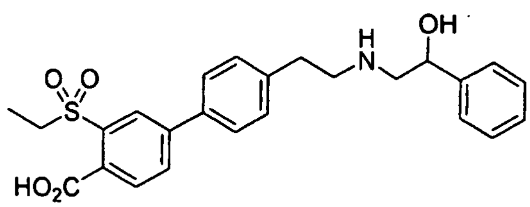

with the chemical name: 3-ethanesulfonyl-4'-[2-(2-hydroxy-2-phenyl-ethylamino)-ethyl]-biphenyl-4-carboxylic acid.

In some embodiments, the present invention pertains to compounds, as described herein, other than:

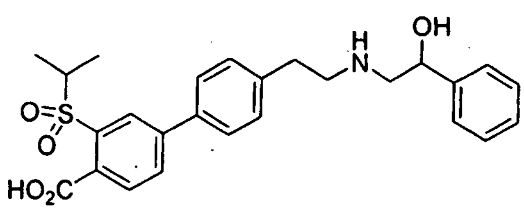

with the chemical name: 4'-[2-(2-hydroxy-2-phenyl-ethylamino)-ethyl]-3-(propane-2-sulfonyl)-biphenyl-4-carboxylic acid.

In some embodiments, the present invention pertains to compounds of Formula (**Ia**), as described herein, that are isolated.

In some embodiments, the present invention pertains to compounds of Formula (**Ia**), as described herein, that are isolated outside the body of an individual.

In some embodiments, isolated compounds of Formula (**Ia**) have a purity of greater than about 0.1%, about 1%, about 5%, about 10%, about 15%, about 20%, about 25%, about 30%, about 40%, about 45%, about 50%, about 55%, about 60%, about 65%, about 70%, about 75%, about 80%, about 85%, about 90%, about 95%, about 98%, or about 99%.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination. All combinations of the embodiments pertaining to the chemical groups represented by the variables (e.g., $R^1$, $R^2$, $R^3$, $R^4$, $R^5$, $R^6$, R', $R^8$, $R^9$, $R^{10}$, $R^{11}$, W, Ring A etc.) contained within the generic chemical formulae described herein [e.g. (**Ia**), (**Ic**), (**Ie**), (**Ig**), (**Ii**), (**Ik**), (**Im**), (**Io**), (**Iq**), (**Is**), etc.] are specifically embraced by the present

invention just as if they were explicitly disclosed, to the extent that such combinations embrace compounds that are stable compounds (i.e., compounds that can be isolated, characterized and tested for biological activity). In addition, all subcombinations of the chemical groups listed in the embodiments describing such variables, as well as all subcombinations of uses and medical indications described herein, are also specifically embraced by the present invention just as if each of such subcombination of chemical groups and subcombination of uses and medical indications were explicitly disclosed herein.

As used herein, "substituted" indicates that at least one hydrogen atom of the chemical group is replaced by a non-hydrogen substituent or group, the non-hydrogen substituent or group can be monovalent or divalent. When the substituent or group is divalent, then it is understood that this group is further substituted with another substituent or group. When a chemical group herein is "substituted" it may have up to the full valance of substitution; for example, a methyl group can be substituted by 1, 2, or 3 substituents, a methylene group can be substituted by 1 or 2 substituents, a phenyl group can be substituted by 1, 2, 3, 4, or 5 substituents, a naphthyl group can be substituted by 1, 2, 3, 4, 5, 6, or 7 substituents and the like. Likewise, "substituted with one or more substituents" refers to the substitution of a group with one substituent up to the total number of substituents physically allowed by the group. Further, when a group is substituted with more than one group they can be identical or they can be different.

Compounds of the invention can also include tautomeric forms, such as keto-enol tautomers, and the like. Tautomeric forms can be in equilibrium or sterically locked into one form by appropriate substitution. It is understood that the various tautomeric forms are within the scope of the compounds of the present invention. By way of illustration, when W is 3,5-disubstituted-1,2,4-triazolyl then there can be three possible tautomers and although only one formula may be shown it is understood that all possible tautomers are embraced by the formula, the possible tautomers are shown below:

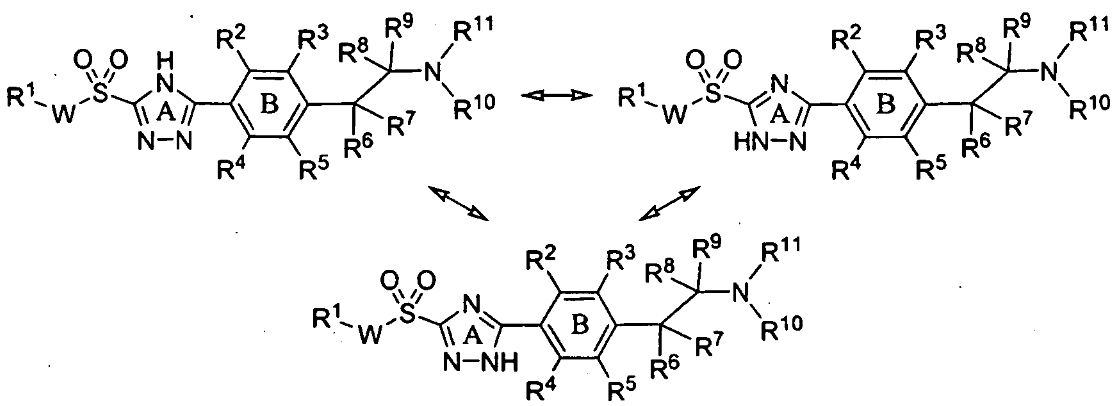

It is understood that tautomeric forms can also have corresponding nomenclature for each tautomer. Therefore, the present invention includes all tautomers and the various nomenclature designations for all tautomers.

Compounds of the invention can also include all isotopes of atoms occurring in the intermediates and/or final compounds. Isotopes include those atoms having the same atomic number but different mass numbers. For example, isotopes of hydrogen include deuterium and tritium.

It is understood and appreciated that compounds of the present invention may have one or more chiral centers, and therefore can exist as enantiomers and/or diastereomers. The invention is understood to extend to and embrace all such enantiomers, diastereomers and mixtures thereof, including but not limited, to racemates. Accordingly, some embodiments of the present invention pertain to compounds of the present invention that are *R* enantiomers. Further, some embodiments of the present invention pertain to compounds of the present invention that are *S* enantiomers. In examples where more than one chiral center is present, then, some embodiments of the present invention include compounds that are *RS* or *SR* enantiomers. In further embodiments, compounds of the present invention are *RR* or *SS* enantiomers. It is understood that compounds of the present invention are intended to represent all possible individual enantiomers and mixtures thereof just as if each had been individually named with the structure provided, unless stated or shown otherwise.

One aspect of the present invention pertains to certain compounds as shown in Formula (**Ia**):

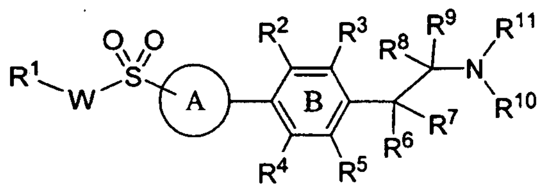

(Ia)

or a pharmaceutically acceptable salt, hydrate or solvate thereof;
wherein
$R^1$ is selected from the group consisting of H, $C_1$-$C_6$ acyl, $C_1$-$C_6$ acyloxy, $C_2$-$C_8$ alkenyl, $C_1$-$C_6$ alkoxy, $C_1$-$C_8$ alkyl, $C_1$-$C_8$ alkylcarboxamide, $C_2$-$C_8$ alkynyl, $C_1$-$C_8$ alkylsulfonamide, $C_1$-$C_8$ alkylsulfinyl, $C_1$-$C_8$ alkylsulfonyl, $C_1$-$C_8$ alkylthio, $C_1$-$C_8$ alkylureyl, amino, $C_1$-$C_8$ alkylamino, $C_2$-$C_8$ dialkylamino, carbo-$C_1$-$C_6$-alkoxy, carboxamide, carboxy, cyano, $C_3$-$C_7$ cycloalkyl, $C_2$-$C_8$ dialkylcarboxamide, $C_2$-$C_8$ dialkylsulfonamide, halogen, $C_1$-$C_6$ haloalkoxy, $C_1$-$C_6$ haloalkyl, $C_1$-$C_6$ haloalkylsulfinyl, $C_1$-$C_6$ haloalkylsulfonyl, $C_1$-$C_6$ haloalkylthio, $C_3$-$C_7$ heterocyclyl, hydroxyl, thiol, nitro, phenyl and sulfonamide, and each is optionally substituted with 1, 2, 3, 4 or 5 substituents selected independently from the group consisting of $C_1$-$C_6$ acyl, $C_1$-$C_6$ acyloxy, $C_2$-$C_8$ alkenyl, $C_1$-$C_6$ alkoxy, $C_1$-$C_8$ alkyl, $C_1$-$C_8$ alkylcarboxamide, $C_2$-$C_8$ alkynyl, $C_1$-$C_8$ alkylsulfonamide, $C_1$-$C_8$ alkylsulfinyl, $C_1$-$C_8$ alkylsulfonyl, $C_1$-$C_8$ alkylthio, $C_1$-$C_8$ alkylureyl, amino, $C_1$-$C_8$ alkylamino, $C_2$-$C_8$ dialkylamino, carbo-$C_1$-$C_6$-alkoxy, carboxamide, carboxy, cyano, $C_3$-$C_7$ cycloalkyl, $C_2$-$C_8$ dialkylcarboxamide, $C_2$-$C_8$ dialkylsulfonamide, halogen, $C_1$-$C_6$ haloalkoxy, $C_1$-$C_6$ haloalkyl, $C_1$-$C_6$ haloalkylsulfinyl, $C_1$-$C_6$ haloalkylsulfonyl, $C_1$-$C_6$ haloalkylthio, hydroxyl, thiol, nitro and sulfonamide;
or
R' together with the W-$SO_2$ group and the ring atom to which the W-$SO_2$ group is bonded form a $C_5$-$C_7$ heterocyclic ring with Ring A whereby said $C_5$-$C_7$ heterocyclic ring and Ring A share two adjacent ring atoms, and said $C_5$-$C_7$ heterocyclic ring is optionally substituted with 1, 2, 3 or 4 substituents selected independently from the group consisting of $C_1$-$C_6$ acyl, $C_1$-$C_6$ acyloxy, $C_2$-$C_8$ alkenyl, $C_1$-$C_6$ alkoxy, $C_1$-$C_8$ alkyl, $C_1$-$C_8$ alkylcarboxamide, $C_1$-$C_8$ alkynyl, $C_1$-$C_8$ alkylsulfonamide, $C_1$-$C_8$ alkylsulfinyl, $C_1$-$C_8$ alkylsulfonyl, $C_1$-$C_8$ alkylthio, $C_1$-$C_8$ alkylureyl, amino, $C_1$-$C_8$ alkylamino, $C_2$-$C_8$ dialkylamino, carbo-$C_1$-$C_6$-alkoxy, carboxamide, carboxy, cyano, $C_3$-$C_7$ cycloalkyl, $C_2$-$C_8$ dialkylcarboxamide, $C_2$-$C_8$ dialkylsulfonamide, halogen, $C_1$-$C_6$ haloalkoxy, $C_1$-$C_6$ haloalkyl, $C_1$-$C_6$ haloalkylsulfinyl, $C_1$-$C_6$ haloalkylsulfonyl, $C_1$-$C_6$ haloalkylthio, hydroxyl, thiol, nitro, oxo and sulfonamide;
W is $C_1$-$C_4$ alkylene, $C_1$-$C_4$ alkenylene, $C_3$-$C_7$ cycloalkylene, $C_3$-$C_7$ heterocyclylene or phenylene, each optionally substituted with 1, 2, 3, 4, 5, 6, 7 or 8 substituents selected independently from the group consisting of $C_1$-$C_3$ alkyl, $C_1$-$C_4$ alkoxy, carboxy, cyano, $C_1$-$C_3$ haloalkyl, halogen, hydroxyl and oxo;
Ring A is 1,3-phenylene or 1,4-phenylene, each substituted with $R^{12}$, $R^{13}$, $R^{14}$ and $R^{15}$, wherein $R^{12}$, $R^{13}$, $R^{14}$ and $R^{15}$ are each selected independently from the group consisting of H, $C_1$-$C_6$ acyl, $C_1$-$C_6$ acyloxy, $C_2$-$C_8$ alkenyl, $C_1$-$C_6$ alkoxy, $C_1$-$C_8$ alkyl, $C_1$-$C_8$ alkylcarboxamide, $C_2$-$C_8$ alkynyl, $C_1$-$C_8$ alkylsulfonamide, $C_1$-$C_8$ alkylsulfinyl, $C_1$-$C_8$ alkylsulfonyl, $C_1$-$C_8$ alkylthio, $C_1$-$C_8$ alkylureyl, amino, $C_1$-$C_8$ alkylamino, $C_2$-$C_8$ dialkylamino, carbo-$C_1$-$C_6$-alkoxy, carboxamide, carboxy, cyano, $C_3$-$C_7$ cycloalkyl, $C_2$-$C_8$ dialkylcarboxamide, $C_2$-$C_8$ dialkylsulfonamide, halogen, $C_1$-$C_6$ haloalkoxy, $C_1$-$C_6$ haloalkyl, $C_1$-$C_6$ haloalkylsulfinyl, $C_1$-$C_6$ haloalkylsulfonyl, $C_1$-$C_6$ haloalkylthio, hydroxyl, thiol, nitro and sulfonamide; or
Ring A is a 6-membered heteroarylene or a 5-membered heteroarylene, each optionally substituted with $R^{16}$, $R^{17}$ and $R^{18}$, wherein $R^{16}$, $R^{17}$ and $R^{18}$ are each selected independently from the group consisting of $C_1$-$C_6$ acyl, $C_1$-$C_6$ acyloxy, $C_2$-$C_8$ alkenyl, $C_1$-$C_6$ alkoxy, $C_1$-$C_8$ alkyl, $C_1$-$C_8$ alkylcarboxamide, $C_2$-$C_8$ alkynyl, $C_1$-$C_8$ alkylsulfonamide, $C_1$-$C_8$ alkylsulfinyl, $C_1$-$C_8$ alkylsulfonyl, $C_1$-$C_8$ alkylthio, $C_1$-$C_8$ alkylureyl, amino, $C_1$-$C_8$ alkylamino, $C_2$-$C_8$ dialkylamino, carbo-$C_1$-$C_6$-alkoxy, carboxamide, carboxy, cyano, $C_3$-$C_7$ cycloalkyl, $C_2$-$C_8$ dialkylcarboxamide, $C_2$-$C_8$ dialkylsulfonamide, halogen, $C_1$-$C_6$ haloalkoxy, $C_1$-$C_6$ haloalkyl, $C_1$-$C_6$ haloalkylsulfinyl, $C_1$-$C_6$ haloalkylsulfonyl, $C_1$-$C_6$ haloalkylthio, hydroxyl, thiol, nitro and sulfonamide;
$R^2$, $R^3$, $R^4$ and $R^5$ are each selected independently from the group consisting of H, $C_1$-$C_6$ acyl, $C_1$-$C_6$ acyloxy, $C_2$-$C_8$ alkenyl, $C_1$-$C_6$ alkoxy, $C_1$-$C_8$ alkyl, $C_1$-$C_8$ alkylcarboxamide, $C_2$-$C_8$ alkynyl, $C_1$-$C_8$ alkylsulfonamide, $C_1$-$C_8$ alkylsulfinyl, $C_1$-$C_8$ alkylsulfonyl, $C_1$-$C_8$ alkylthio, $C_1$-$C_8$ alkylureyl, amino, $C_1$-$C_8$ alkylamino, $C_2$-$C_8$ dialkylamino, carbo-$C_1$-$C_6$-alkoxy, carboxamide, carboxy, cyano, $C_3$-$C_7$ cycloalkyl, $C_2$-$C_8$ dialkylcarboxamide, $C_2$-$C_8$ dialkylsulfonamide, halogen, $C_1$-$C_6$ haloalkoxy, $C_1$-$C_6$ haloalkyl, $C_1$-$C_6$ haloalkylsulfinyl, $C_1$-$C_6$ haloalkylsulfonyl, $C_1$-$C_6$ haloalkylthio, hydroxyl, thiol, nitro and sulfonamide;
$R^6$, $R^7$, $R^8$ and $R^9$ are each selected independently from the group consisting of H, $C_1$-$C_3$ alkyl, $C_1$-$C_4$ alkoxy, carboxy, cyano, $C_1$-$C_3$ haloalkyl, halogen and hydroxyl;
and
$R^{10}$ and $R^{11}$ are each selected independently from the group consisting of H, $C_1$-$C_8$ alkyl, $C_2$-$C_8$ alkenyl, $C_2$-$C_8$ alkynyl,

$C_3$-$C_7$ cycloalkyl, aryl, heterocyclyl, heteroaryl, aryl-$C_1$-$C_4$-alkylenyl and heteroaryl-$C_1$-$C_4$-alkylenyl and each $R^{10}$ and $R^{11}$ is optionally substituted with 1, 2, 3, 4 or 5 substituents selected independently from the group consisting of $C_1$-$C_6$ acyl, $C_1$-$C_6$ acyloxy. $C_2$-$C_8$ alkenyl, $C_1$-$C_6$ alkoxy, $C_1$-$C_8$ alkyl, $C_1$-$C_8$ alkylcarboxamide, $C_2$-$C_8$ alkynyl, $C_1$-$C_8$ alkylsulfonamide, $C_1$-$C_8$ alkylsulfinyl, $C_1$-$C_8$ alkylsulfonyl, $C_1$-$C_8$ alkylthio, $C_1$-$C_8$ alkylureyl, amino, $C_1$-$C_8$ alkylamino, $C_2$-$C_8$ dialkylamino, carbo-$C_1$-$C_6$-alkoxy, carboxamide, carboxy, cyano, $C_3$-$C_7$ cycloalkyl, $C_2$-$C_8$ dialkylcarboxamide, $C_2$-$C_8$ dialkylsulfonamide, halogen, $C_1$-$C_6$ haloalkoxy, $C_1$-$C_6$ haloalkyl, $C_1$-$C_6$ haloalkylsulfinyl, $C_1$-$C_6$ haloalkylsulfonyl, $C_1$-$C_6$ haloalkylthio, hydroxyl, thiol, nitro and sulfonamide;
or
$R^{10}$ and $R^{11}$ together with the nitrogen atom to which they are both bonded form a $C_3$-$C_7$ heterocyclyl, optionally substituted with 1, 2, 3, 4, 5 or 6 substituents selected independently from the group consisting of $C_1$-$C_6$ acyl, $C_1$-$C_6$ acyloxy, $C_2$-$C_8$ alkenyl, $C_1$-$C_6$ alkoxy, $C_1$-$C_8$ alkyl, $C_1$-$C_8$ alkylcarboxamide, $C_2$-$C_8$ alkynyl, $C_1$-$C_8$ alkylsulfonamide, $C_1$-$C_8$ alkylsulfinyl, $C_1$-$C_8$ alkylsulfonyl, $C_1$-$C_8$ alkylthio, $C_1$-$C_8$ alkylureyl, amino, $C_1$-$C_8$ alkylamino, $C_2$-$C_8$ dialkylamino, carbo-$C_1$-$C_6$-alkoxy, carboxamide, carboxy, cyano, $C_3$-$C_7$ cycloalkyl, $C_2$-$C_8$ dialkylcarboxamide, $C_2$-$C_8$ dialkylsulfonamide, halogen, $C_1$-$C_6$ haloalkoxy, $C_1$-$C_6$ haloalkyl, $C_1$-$C_6$ haloalkylsulfinyl, $C_1$-$C_6$ haloalkylsulfonyl, $C_1$-$C_6$ haloalkylthio, hydroxyl, thiol, nitro, oxo and sulfonamide, and $C_1$-$C_8$ alkyl is optionally substituted with $C_1$-$C_6$ alkoxy or hydroxyl;
provided:

1) that Ring B and the sulfur of the $R^1$-W-$S(O)_2$- group are not bonded to adjacent ring atoms of Ring A;
2) if Ring A is 1,3-phenylene or 1,4-phenylene, and W is $C_3$-$C_7$ heterocyclylene, then the ring atom of W that is directly bonded to the sulfur of the $R^1$-W-$S(O)_2$- group is other than nitrogen;
and
3) said compound is other than:

4'-[2-(2-hydroxy-2-phenyl-ethylamino)-ethyl]-3-methanesulfonyl-biphenyl-4-carboxylic acid;
3-ethanesulfonyl-4'-[2-(2-hydroxy-2-phenyl-ethylamino)-ethyl]-biphenyl-4-carboxylic acid; or
4'-[2-(2-hydroxy-2-phenyl-ethylamino)-ethyl]-3-(propane-2-sulfonyl)-biphenyl-4-carboxylic acid.

Some embodiments of the present invention pertain to certain compounds as shown in Formula (**Ic**):

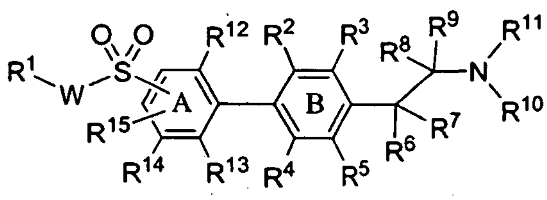

**(Ic)**

wherein each variable in Formula (**Ic**) has the same meaning as described herein, *supra* and *infra.*

In some embodiments, the present invention pertains to compounds as described herein provided that if $R^{12}$, $R^{13}$, $R^{14}$ are all H, then $R^{15}$ is other than carboxy.

Some embodiments of the present invention pertain to certain compounds wherein Ring A is 1,3-phenylene.

Some embodiments of the present invention pertain to certain compounds as shown in Formula (**Ie**):

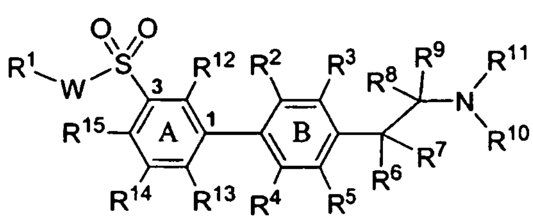

**(Ie)**

wherein each variable in Formula (**Ie**) has the same meaning as described herein, *supra* and *infra.*

In some embodiments, the present invention pertains to compounds as described herein provided that if the $R^1$-W-$S(O)_2$- group and Ring B are bonded to Ring A at ring atom 1 and ring atom 3, for example as shown in Formula

(**Ie**), and three of the $R^{12}$, $R^{13}$, $R^{14}$ and $R^{15}$ groups are all hydrogens, then the fourth $R^{12}$, $R^{13}$, $R^{14}$ and $R^{15}$ group is other than carboxy. It is understood that the numerical designation for ring atom 1 and ring atom 3 refers to a 1,3-substitution pattern of Ring A and may or may not correspond to the actual numerical designations in the chemical name.

In some embodiments, the present invention pertains to compounds as described herein provided that $R^{15}$ is other than carboxy.

Some embodiments of the present invention pertain to certain compounds wherein Ring A is 1,4-phenylene.

Some embodiments of the present invention pertain to certain compounds as shown in Formula (**Ig**):

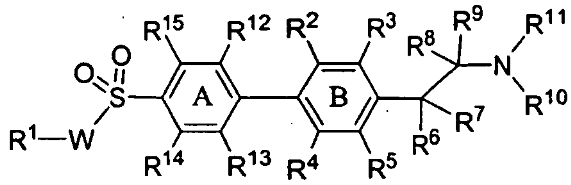

**(Ig)**

wherein each variable in Formula (**Ig**) has the same meaning as described herein, *supra* and *infra.*

In some embodiments, $R^{12}$, $R^{13}$, $R^{14}$ and $R^{15}$ are each selected independently from the group consisting of H, $C_1$-$C_8$ alkyl, carboxy and halogen.

In some embodiments, $R^{12}$, $R^{13}$, $R^{14}$ and $R^{15}$ are each selected independently from the group consisting of H, $-CH_3$, carboxy, Cl and Br.

In some embodiments, $R^{12}$, $R^{13}$, $R^{14}$ and $R^{15}$ are each **H**.

Some embodiments of the present invention pertain to certain compounds wherein Ring A is a 6-membered heteroarylene.

Some embodiments of the present invention pertain to certain compounds as shown in Formula (**Ii**):

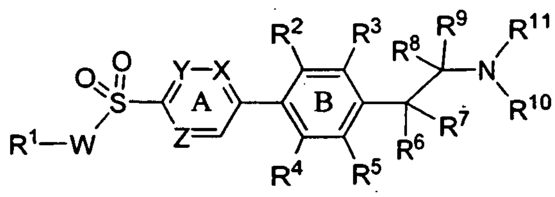

**(Ii)**

wherein, X is N or CH; Y is N or CH; and Z is N or CH; provided that at least one X, Y and Z is N; and each remaining variable in Formula (**Ii**) has the same meaning as described herein, *supra* and *infra.*

Some embodiments of the present invention pertain to certain compounds, wherein Ring A is a 5-membered heteroarylene.

Some embodiments of the present invention pertain to certain compounds as shown in Formula (**Ik**):

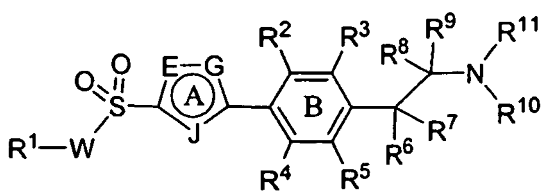

**(Ik)**

wherein, **J** is N or NH; and

E and G are each independently selected from N or S, provided that at least one E and G is N; and each remaining variable in Formula (**Ik**) has the same meaning as described herein, *supra* and *infra.*

In some embodiments, $R^1$ is selected from the group consisting of H, $C_1$-$C_6$ alkoxy, amino, carbo-$C_1$-$C_6$-alkoxy, carboxamide, carboxy, $C_3$-$C_7$ heterocyclyl, hydroxyl and phenyl, and each is optionally substituted with cyano or $C_3$-$C_7$

cycloalkyl; or

$R^1$ together with the W-$SO_2$ group and the ring atom to which the W-$SO_2$ group is bonded form a $C_5$-$C_7$ heterocyclic ring with Ring A whereby the $C_5$-$C_7$ heterocyclic ring and Ring A share two adjacent ring atoms, and the $C_5$-$C_7$ heterocyclic ring is optionally substituted with oxo.

In some embodiments, $R^1$ is selected from the group consisting of H, $C_1$-$C_6$ alkoxy, carbo-$C_1$-$C_6$-alkoxy, hydroxyl and phenyl; or

$R^1$ together with the W-$SO_2$ group and the ring atom to which the W-$SO_2$ group is bonded form a $C_5$-$C_7$ heterocyclic ring with Ring A whereby the $C_5$-$C_7$ heterocyclic ring and Ring A share two adjacent ring atoms, and the $C_5$-$C_7$ heterocyclic ring is optionally substituted with oxo.

In some embodiments, $R^1$ is selected from the group consisting of H, $C_1$-$C_6$ alkoxy, carbo-$C_1$-$C_6$-alkoxy, hydroxyl and phenyl.

In some embodiments, $R^1$ is H or $C_1$-$C_6$ alkoxy.

In some embodiments, $R^1$ is H.

In some embodiments, $R^1$ is $C_1$-$C_6$ alkoxy.

In some embodiments, $R^1$ is selected from the group consisting of H, $-OCH_3$, $-OCH_2CH_3$, $-C(=O)OCH_2CH_3$, $-C(=O)OC(CH_3)_3$, hydroxyl and phenyl.

In some embodiments, W is $C_1$-$C_4$ alkylene, $C_1$-$C_4$ alkenylene, $C_3$-$C_7$ cycloalkylene or phenylene, each optionally substituted with $C_1$-$C_3$ alkyl.

In some embodiments, W is $C_1$-$C_4$ alkylene or $C_2$-$C_4$ alkenylene, each optionally substituted with $C_1$-$C_3$ alkyl.

In some embodiments, W is selected from the group consisting of $-CH_2-$, $-CH_2CH_2-$, $-CH(CH_3)CH_2-$, $-CH_2CH_2CH_2-$, $-CH_2CH_2CH(CH_3)-$, -HC=CH-, 1,3-cyclopentylene, $-C(CH_3)_2CH_2-$, $-CH_2C(CH_3)_2CH_2-$, 4-tetrahydropyran-2-yl, 3-tetrahydropyran-5-yl and 1,4-phenylene. It is understood that 4-tetrahydropyran-2-yl and 3-tetrahydropyran-5-yl refer to the following formulae:

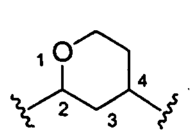
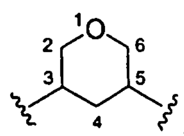

In some embodiments, W is selected from the group consisting of $-CH_2-$, $-CH_2CH_2-$, $-CH(CH_3)CH_2-$, $-CH_2CH_2CH_2-$, $-CH_2CH_2CH(CH_3)-$, -HC=CH-, and 1,3-cyclopentylene.

In some embodiments, $R^1$ and W together form a group selected from the following or any subcombination thereof:

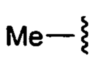, 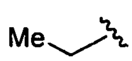, 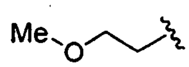, 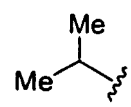, 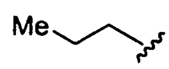,

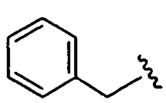, 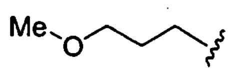, 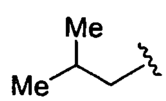, 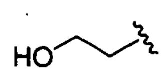,

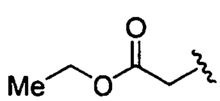, 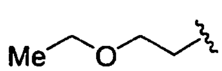, 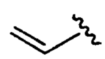, 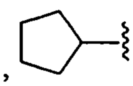, 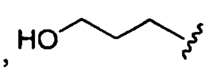,

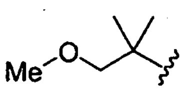, 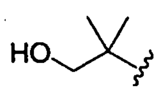, 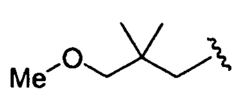, 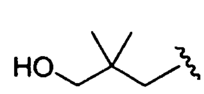,

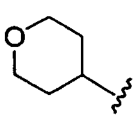, 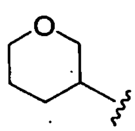, 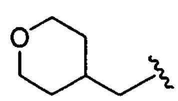 and 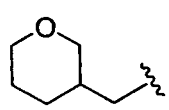.

In some embodiments, W is selected from the group consisting of $-CH_2CH_2-$ and -HC=CH-.

In some embodiments, W is $-CH_2CH_2-$.

In some embodiments, W is -HC=CH-.

In some embodiments, the present invention pertains to compounds as described herein provided that $R^1$ and

W together form a group other than $-CH_3$ (i.e. $R^1$ and W together is not methyl).

In some embodiments, the present invention pertains to compounds as described herein provided that $R^1$ and W together form a group other than $-CH_2CH_3$ (i.e. $R^1$ and W together is not ethyl).

In some embodiments, the present invention pertains to compounds as described herein provided that $R^1$ and W together form a group other than $-CH(CH_3)_2$ (i.e. $R^1$ and W together is not isopropyl).

In some embodiments, $R^2$, $R^3$, $R^4$ and $R^5$ are each H.

In some embodiments, $R^6$, $R^7$, $R^8$ and $R^9$ are each H.

In some embodiments, the present invention pertains to compounds as described herein provided that if one $R^{10}$ and $R^{11}$ group is aryl-$C_1$-$C_4$-alkylenyl, then the aryl-$C_1$-$C_4$-alkylenyl group is optionally substituted with 1, 2, 3, 4 or 5 substituents other than hydroxyl.

In some embodiments, the present invention pertains to compounds wherein $R^{10}$ and $R^{11}$ are each selected independently from the group consisting of H, $C_1$-$C_8$ alkyl, $C_2$-$C_8$ alkenyl, $C_2$-$C_8$ alkynyl, $C_3$-$C_7$ cycloalkyl, aryl, heterocyclyl, heteroaryl, aryl-$C_1$-$C_4$-alkylenyl and heteroaryl-$C_1$-$C_4$-alkylenyl and each $R^{10}$ and $R^{11}$ is optionally substituted with 1, 2, 3, 4 or 5 substituents selected independently from the group consisting of $C_1$-$C_6$ acyl, $C_1$-$C_6$ acyloxy, $C_2$-$C_8$ alkenyl, $C_1$-$C_6$ alkoxy, $C_1$-$C_8$ alkyl, $C_1$-$C_8$ alkylcarboxamide, $C_2$-$C_8$ alkynyl, $C_1$-$C_8$ alkylsulfonamide, $C_1$-$C_8$ alkylsulfinyl, $C_1$-$C_8$ alkylsulfonyl, $C_1$-$C_8$ alkylthio, $C_1$-$C_8$ alkylureyl, amino, $C_1$-$C_8$ alkylamino, $C_2$-$C_8$ dialkylamino, carbo-$C_1$-$C_6$-alkoxy, carboxamide, carboxy, cyano, $C_3$-$C_7$ cycloalkyl, $C_2$-$C_8$ dialkylcarboxamide, $C_2$-$C_8$ dialkylsulfonamide, halogen, $C_1$-$C_6$ haloalkoxy, $C_1$-$C_6$ haloalkyl, $C_1$-$C_6$ haloalkylsulfinyl, $C_1$-$C_6$ haloalkylsulfonyl, $C_1$-$C_6$ haloalkylthio, thiol, nitro and sulfonamide.

In some embodiments, $R^{10}$ and $R^{11}$ are each selected independently from the group consisting of H, $C_1$-$C_8$ alkyl, aryl-$C_1$-$C_4$-alkylenyl and heteroaryl-$C_1$-$C_4$-alkylenyl;

or

$R^{10}$ and $R^{11}$ together with the nitrogen atom to which they are both bonded form a $C_3$-$C_7$ heterocyclyl optionally substituted with 1 or 2 substituents selected independently from the group consisting of $C_1$-$C_8$ alkyl, halogen and hydroxyl, and $C_1$-$C_8$ alkyl is optionally substituted with $C_1$-$C_6$ alkoxy or hydroxyl.

In some embodiments, $R^{10}$ and $R^{11}$ are each selected independently from the group consisting of H, $C_1$-$C_8$ alkyl, aryl-$C_1$-$C_4$-alkylenyl and heteroaryl-$C_1$-$C_4$-alkylenyl.

In some embodiments, $R^{10}$ and $R^{11}$ together with the nitrogen atom to which they are both bonded form a $C_3$-$C_7$ heterocyclyl optionally substituted with 1 or 2 substituents selected independently from the group consisting of $C_1$-$C_8$ alkyl and halogen.

In some embodiments, $R^{10}$ and $R^{11}$ are each selected independently from the group consisting of H, $-CH_3$, $-CH_2CH_3$, $-CH(CH_3)_2$ and $-CH_2$-phenyl.

In some embodiments, $R^{10}$ and $R^{11}$ together with the nitrogen atom to which they are both bonded form a $C_3$-$C_7$ heterocyclyl selected from the group consisting of pyrrolidin-1-yl, 2-methyl-pyrrolidin-1-yl, 2,5-dimethyl-pyrrolidin-1-yl, 3-hydroxy-pyrrolidin-1-yl, 3,3-difluoro-pyrrolidin-1-yl, 3-hydroxymethyl-pyrrolidin-1-yl, piperidin-1-yl, morpholin-4-yl, thiomorpholin-4-yl, piperazin-1-yl and 4-methyl-piperazin-1-yl.

In some embodiments, $R^{10}$ and $R^{11}$ together with the nitrogen atom to which they are both bonded form 2-methyl-pyrrolidin-1-yl.

In some embodiments, $R^{10}$ and $R^{11}$ together with the nitrogen atom to which they are both bonded form (R)-2-methyl-pyrrolidin-1-yl.

Some embodiments of the present invention pertain to certain compounds as shown in Formula (Im):

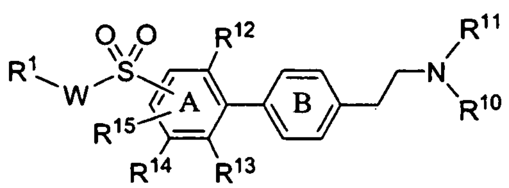

**(Im)**

or a pharmaceutically acceptable salt, hydrate or solvate thereof;
wherein:

$R^{12}$, $R^{13}$, $R^{14}$ and $R^{15}$ are each selected independently from the group consisting of H, $C_1$-$C_8$ alkyl, carboxy and halogen;

$R^1$ is selected from the group consisting of H, $C_1$-$C_6$ alkoxy, amino, carbo-$C_1$-$C_6$-alkoxy, carboxamide, carboxy,

$C_3$-$C_7$ heterocyclyl, hydroxyl and phenyl, and each is optionally substituted with cyano or $C_3$-$C_7$ cycloalkyl; or R' together with the W-$SO_2$ group and the ring atom to which the W-$SO_2$ group is bonded form a $C_5$-$C_7$ heterocyclic ring with Ring A whereby the $C_5$-$C_7$ heterocyclic ring and Ring A share two adjacent ring atoms, and the $C_5$-$C_7$ heterocyclic ring is optionally substituted with oxo;

W is $C_1$-$C_4$ alkylene, $C_2$-$C_4$ alkenylene, $C_3$-$C_7$ cycloalkylene or phenylene, each optionally substituted with $C_1$-$C_3$ alkyl; and

$R^{10}$ and $R^{11}$ are each selected independently from the group consisting of H, $C_1$-$C_8$ alkyl, aryl-$C_1$-$C_4$-alkylenyl and heteroaryl-$C_1$-$C_4$-alkylenyl;

or

$R^{10}$ and $R^{11}$ together with the nitrogen atom to which they are both bonded form a $C_3$-$C_7$ heterocyclyl optionally substituted with 1 or 2 substituents selected independently from the group consisting of $C_1$-$C_8$ alkyl, halogen and hydroxyl, and $C_1$-$C_8$ alkyl is optionally substituted with $C_1$-$C_6$ alkoxy or hydroxyl.

Some embodiments of the present invention pertain to certain compounds as shown in Formula (Im):

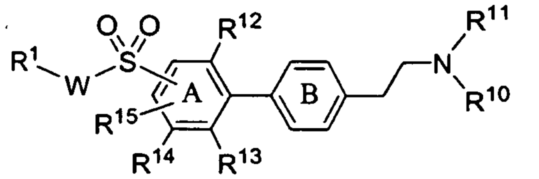

**(Im)**

or a pharmaceutically acceptable salt, hydrate or solvate thereof;
wherein:

$R^{12}$, $R^{13}$, $R^{14}$ and $R^{15}$ are each selected independently from the group consisting of **H**, $C_1$-$C_8$ alkyl, carboxy and halogen;

$R^1$ is selected from the group consisting of H, $C_1$-$C_6$ alkoxy, amino, carbo-$C_1$-$C_6$-alkoxy, carboxamide, carboxy, $C_3$-$C_7$ heterocyclyl, hydroxyl and phenyl, and each is optionally substituted with cyano or $C_3$-$C_7$ cycloalkyl; or R' together with the W-$SO_2$ group and the ring atom to which the W-$SO_2$ group is bonded form a $C_5$-$C_7$ heterocyclic ring with Ring A whereby said $C_5$-$C_7$ heterocyclic ring and Ring A share two adjacent ring atoms, and said $C_5$-$C_7$ heterocyclic ring is optionally substituted with oxo;

W is $C_1$-$C_4$ alkylene, $C_2$-$C_4$ alkenylene, $C_3$-$C_7$ cycloalkylene or phenylene, each optionally substituted with $C_1$-$C_3$ alkyl; and

$R^{10}$ and $R^{11}$ together with the nitrogen atom to which they are both bonded form 2-methyl-pyrrolidin-1-yl.

Some embodiments of the present invention pertain to certain compounds as shown in Formula (**Im**):

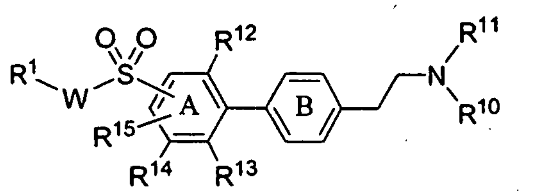

**(Im)**

or a pharmaceutically acceptable salt, hydrate or solvate thereof;
wherein:

$R^{12}$, $R^{13}$, $R^{14}$ and $R^{15}$ are each selected independently from the group consisting of H, -$CH_3$, carboxy, Cl and Br;

$R^1$ is selected from the group consisting of H, $C_1$-$C_6$ alkoxy, carbo-$C_1$-$C_6$-alkoxy, hydroxyl and phenyl; or $R^1$ together with the W-$SO_2$ group and the ring atom to which the W-$SO_2$ group is bonded form a $C_5$-$C_7$ heterocyclic ring with Ring A whereby the $C_5$-$C_7$ heterocyclic ring and Ring A share two adjacent ring atoms, and the $C_5$-$C_7$

heterocyclic ring is optionally substituted with oxo;
W is selected from the group consisting of $-CH_2-$, $-CH_2CH_2-$, $-CH(CH_3)CH_2-$, $-CH_2CH_2CH_2-$, $-CH_2CH_2CH(CH_3)-$, -HC=CH-, 1,3-cyclopentylene, $-C(CH_3)_2CH_2-$, $-CH_2C(CH_3)_2CH_2-$, 4-tetrahydropyran-2-yl, 3-tetrahydropyran-5-yl and 1,4-phenylene; and
$R^{10}$ and $R^{11}$ together with the nitrogen atom to which they are both bonded form a $C_3$-$C_7$ heterocyclyl selected from the group consisting of pyrrolidin-1-yl, 2-methyl-pyrrolidin-1-yl, 2,5-dimethyl-pyrrolidin-1-yl, 3-hydroxy-pyrrolidin-1-yl, 3,3-difluoro-pyrrolidin-1-yl, 3-hydroxymethyl-pyrrolidin-1-yl, piperidin-1-yl, morpholin-4-yl, thiomorpholin-4-yl, piperazin-1-yl and 4-methyl-piperazin-1-yl.

Some embodiments of the present invention pertain to certain compounds as shown in Formula (**Im**):

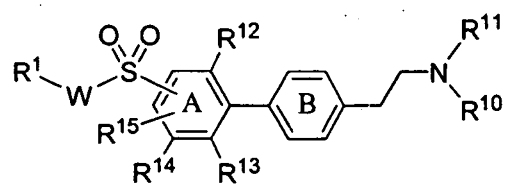

**(Im)**

or a pharmaceutically acceptable salt, hydrate or solvate thereof;
wherein:

$R^{12}$, $R^{11}$, $R^{14}$ and $R^{15}$ are each selected independently from the group consisting of H, $-CH_3$, carboxy, Cl and Br;
$R^1$ is selected from the group consisting of H, $C_1$-$C_6$ alkoxy, carbo-$C_1$-$C_6$-alkoxy, hydroxyl and phenyl; or
$R^1$ together with the W-$SO_2$ group and the ring atom to which the W-$SO_2$ group is bonded form a $C_5$-$C_7$ heterocyclic ring with Ring A whereby said $C_5$-$C_7$ heterocyclic ring and Ring A share two adjacent ring atoms, and said $C_5$-$C_7$ heterocyclic ring is optionally substituted with oxo;
W is selected from the group consisting of $-CH_2-$, $-CH_2CH_2-$, $-CH(CH_3)CH_2-$, $-CH_2CH_2CH_2-$, $-CH_2CH_2CH(CH_3)-$, -HC=CH-, 1,3-cyclopentylene, $-C(CH_3)_2CH_2-$, $-CH_2C(CH_3)_2CH_2-$, 4-tetrahydropyran-2-yl, 3-tetrahydropyran-5-yl and 1,4-phenylene; and
$R^{10}$ and $R^{11}$ together with the nitrogen atom to which they are both bonded form 2-methyl-pyrrolidin-1-yl.

Some embodiments of the present invention pertain to certain compounds as shown in Formula (**Im**):

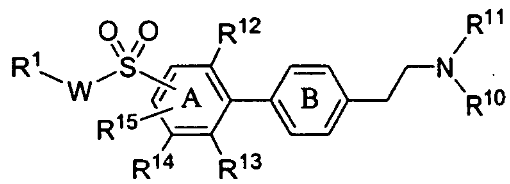

**(Im)**

or a pharmaceutically acceptable salt, hydrate or solvate thereof;
wherein:

$R^{12}$, $R^{13}$, $R^{14}$ and $R^{15}$ are each H;
$R^1$ is selected from the group consisting of H, $-OCH_3$, $-OCH_2CH_3$, $-C(=O)OCH_2CH_3$, $-C(=O)OC(CH_3)_3$, hydroxyl and phenyl;
W is selected from the group consisting of $-CH_2CH_2-$ and -HC=CH-; and
$R^{10}$ and $R^{11}$ together with the nitrogen atom to which they are both bonded form 2-methyl-pyrrolidin-1-yl.

Some embodiments of the present invention pertain to certain compounds as shown in Formula (**Io**):

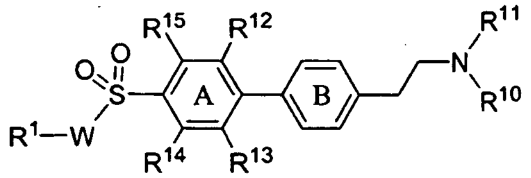

(Io)

or a pharmaceutically acceptable salt, hydrate or solvate thereof;
wherein:

$R^{12}$, $R^{13}$, $R^{14}$ and $R^{15}$ are each H;
$R^1$ is selected from the group consisting of H, $-OCH_3$, $-OCH_2CH_3$, $-C(=O)OCH_2CH_3$, $-C(=O)OC(CH_3)_3$, hydroxyl and phenyl;
W is selected from the group consisting of $-CH_2CH_2-$ and -HC=CH-; and
$R^{10}$ and $R^{11}$ together with the nitrogen atom to which they are both bonded form 2-methyl-pyrrolidin-1-yl.

Some embodiments of the present invention pertain to certain compounds as shown in Formula (**Iq**):

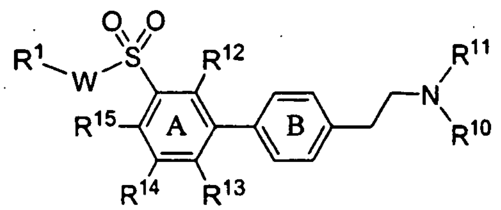

(Iq)

or a pharmaceutically acceptable salt, hydrate or solvate thereof;
wherein:

$R^{12}$ $R^{13}$, $R^{14}$ and $R^{15}$ are each H;
$R^1$ is selected from the group consisting of H, $-OCH_3$, $-OCH_2CH_3$, $-C(=O)OCH_2CH_3$, $-C(=O)OC(CH_3)_3$, hydroxyl and phenyl;
W is selected from the group consisting of $-CH_2CH_2-$ and -HC=CH-; and
$R^{10}$ and $R^{11}$ together with the nitrogen atom to which they are both bonded form 2-methyl-pyrrolidin-1-yl.

Some embodiments of the present invention pertain to certain compounds as shown in Formula (**Is**):

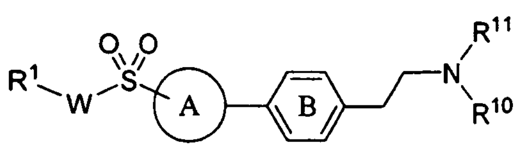

(Is)

or a pharmaceutically acceptable salt, hydrate or solvate thereof;
wherein:

Ring A is selected from:

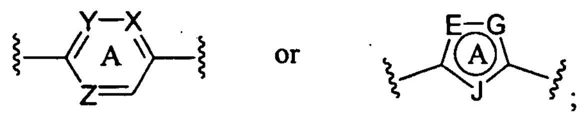

X is N or CH; Y is N or CH; and Z is N or CH; provided that at least one X, Y and Z is N;
J is N or NH; and E and G are each independently selected from N or S, provided that at least one E and G is N;
$R^1$ is selected from the group consisting of H, $C_1$-$C_6$ alkoxy, carbo-$C_1$-$C_6$-alkoxy, hydroxyl and phenyl; or
$R^1$ together with the W-$SO_2$ group and the ring atom to which the W-$SO_2$ group is bonded form a $C_5$-$C_7$ heterocyclic ring with Ring A whereby the $C_5$-$C_7$ heterocyclic ring and Ring A share two adjacent ring atoms;
W is selected from the group consisting of $-CH_2-$, $-CH_2CH_2-$, $-CH(CH_3)CH_2-$, $-CH_2CH_2CH_2-$, $-CH_2CH_2CH(CH_3)-$, -HC=CH-, 1,3-cyclopentylene, $-C(CH_3)_2CH_2-$, $-CH_2C(CH_3)_2CH_2-$, 4-tetrahydropyran-2-yl, 3-tetrahydropyran-5-yl and 1,4-phenylene; and
$R^{10}$ and $R^{11}$ together with the nitrogen atom to which they are both bonded form a $C_3$-$C_7$ heterocyclyl selected from the group consisting of pyrrolidin-1-yl, 2-methyl-pyrrolidin-1-yl, 2,5-dimethyl-pyrrolidin-1-yl, 3-hydroxy-pyrtolidin-1-yl, 3,3-difluoro-pyrrolidin-1-yl, 3-hydroxymethyl-pyrrolidin-1-yl, piperidin-1-yl, morpholin-4-yl, thiomorpholin-4-yl, piperazin-1-yl and 4-methyl-piperazin-1-yl.

Some embodiments of the present invention pertain to certain compounds as shown in Formula (**Is**):

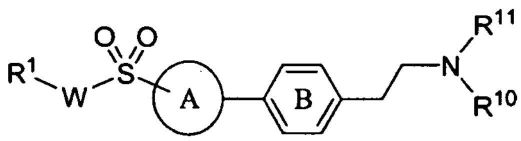

(Is)

or a pharmaceutically acceptable salt, hydrate or solvate thereof;
wherein:

Ring A is selected from:

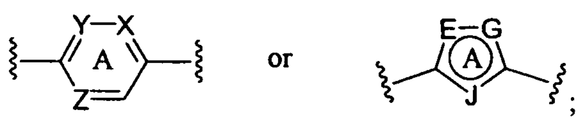

X is N or CH; Y is N or CH; and Z is N or CH; provided that at least one X, Y and Z is N;
J is N or NH; and E and G are each independently selected from N or S, provided that at least one E and G is N;
$R^1$ is selected from the group consisting of H, $-OCH_3$, $-OCH_2CH_3$, $-C(=O)OCH_2CH_3$, $-C(=O)OC(CH_3)_3$, hydroxyl and phenyl;
W is selected from the group consisting of $-CH_2CH_2-$ and -HC=CH-; and
$R^{10}$ and $R^{11}$ together with the nitrogen atom to which they are both bonded form 2-methyl-pyrrolidin-1-yl.

Some embodiments of the present invention pertain to certain compounds as shown in Formula (**Is**):

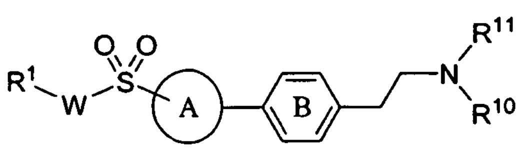

(Is)

or a pharmaceutically acceptable salt, hydrate or solvate thereof;

wherein:

Ring A is selected from the group consisting of 1,4-phenylene, 1,3-phenylene, 4-carboxy-1,3-phenylene, 4-methyl-1,3-phenylene, pyridin-2,5-ylene, pyrimidin-2,5-ylene and 1,2,4-thiadiazol-3,5-ylene;
$R^1$ is selected from the group consisting of H, $-OCH_3$, $-OCH_2CH_3$, $-C(=O)OCH_3$, $-C(=O)OCH_2CH_3$, $-C(=O)OC(CH_3)_3$, hydroxyl, phenyl, morpholin-4-yl, tetrahydro-pyran-4-yl, carboxy, 4-cyanopiperidin-1-yl, amino, cyclohexylamino, methylamino, tetrahydro-pyran-2-yl; or
W is selected from the group consisting of $-CH_2-$, $-CH_2CH_2-$, $-CH(CH_3)CH_2-$, $-CH_2CH_2CH_2-$, -HC=CH-, 1,3-cyclopentylene, $-CH_2C(CH_3)_2CH_2-$, 4-tetrahydropyran-2-yl, $-CH_2HC=CH-$, $-CH_2CH_2C(=O)-$, $-CH_2CH(CH_3)CH_2-$, $-CH_2CH(CH_3)-$ and piperidin-2,4-ylene; and
$R^{10}$ and $R^{11}$ together with the nitrogen atom to which they are both bonded form 2-methyl-pyrrolidin-1-yl.

In some embodiments, $R^1$ and W together form a group selected from the following or any subcombination thereof:

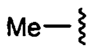, 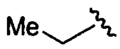, 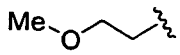, 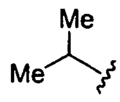, 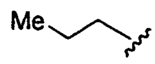,

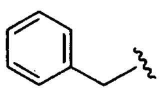, 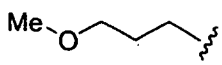, 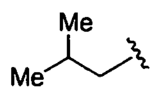, 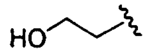,

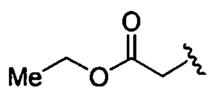, 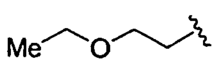, 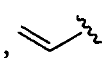, 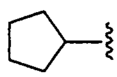, 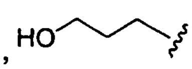,

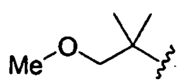, 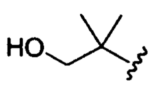, 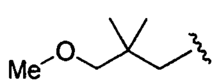, 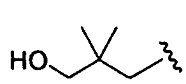,

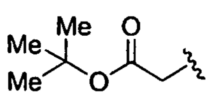, 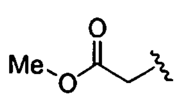, 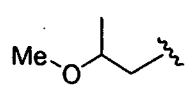,

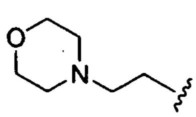, 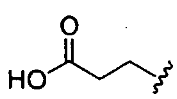, 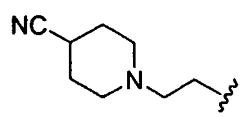,

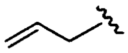, 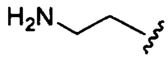, 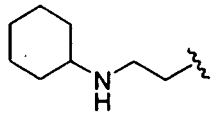, 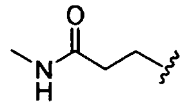,

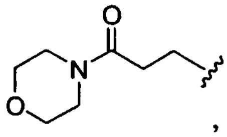, 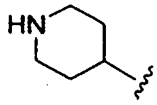, 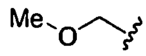, 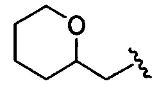,

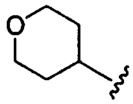, 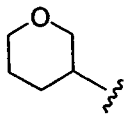, 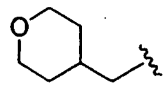 and 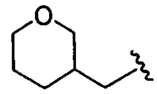.

Some embodiments of the present invention include every combination of one or more compounds selected from the following group shown in TABLE A.

**TABLE A**

| Cmpd No. | Chemical Structure | Chemical Name |
|---|---|---|
| 1 | 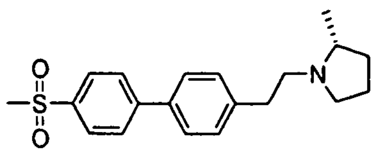 | (R)-1-[2-(4'-Methanesulfonyl-biphenyl-4-yl)-ethyl]-2-methylpyrrolidine |
| 2 | 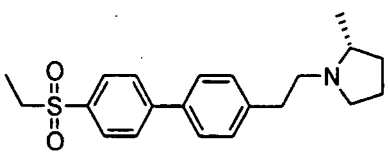 | (*R*)-1-[2-(4'-Ethanesulfonyl-biphenyl-4-yl)-ethyl]-2-methylpyrrolidine |
| 3 | 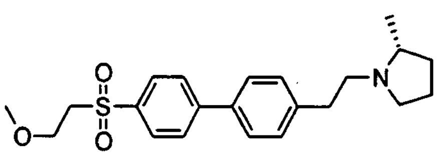 | (*R*)-1-{2-[4'-(2-Methoxy-ethanesulfonyl)-biphenyl-4-yl]- ethyl}-2-methyl-pyrrolidine |
| 4 | 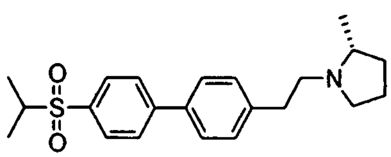 | (*R*)-2-Methyl-1-{2-[4'-(propane-2-sulfonyl)-biphenyl-4-yl]-ethyl}-pyrrolidine |
| 5 | 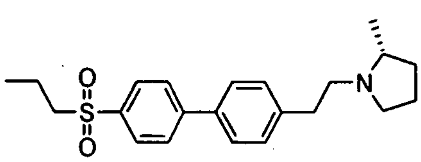 | (*R*)-2-Methyl-1- {2-[4'-(propane-1-sulfonyl)-biphenyl-4-yl]-ethyl}-pyrrolidine |
| 6 | 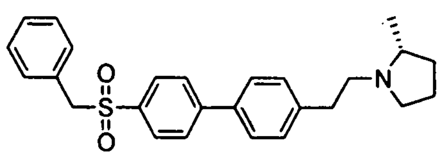 | (*R*)-2-Methyl-1-[2-(4'-phenylmethanesulfonyl-biphenyl-4-yl)-ethyl]-pyrrolidine |

(continued)

| Cmpd No. | Chemical Structure | Chemical Name |
|---|---|---|
| 7 | 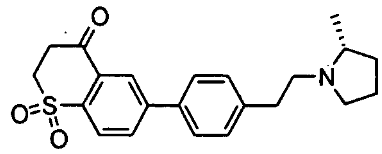 | 6- {4-[2-((*R*)-2-Methyl-pyrrolidin-1-yl)-ethyl]-phenyl}-1,1-dioxo-1 $\lambda^6$-thiochroman-4-one |
| 8 | 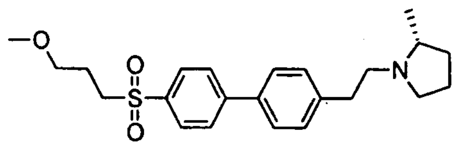 | (*R*)-1-{2-[4'-(3-Methoxy-propane-1-sulfonyl)-biphenyl-4-yl]-ethyl}-2-methyl-pyrrolidine |
| 9 | 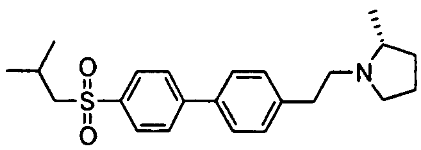 | (*R*)-2-Methyl-1-{2-[4'-(2-methyl-propane-1-sulfonyl)-biphenyl-4-yl]-ethyl}-pyrrolidine |
| 10 | 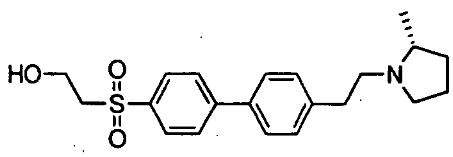 | 2-{4'-[2-((*R*)-2-Methyl-pyrrolidin-1-yl)-ethyl]-biphenyl-4-sulfonyl}-ethanol |
| 11 | 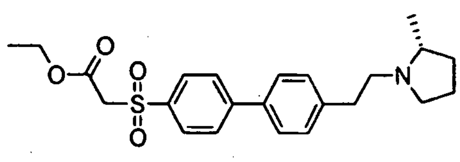 | {4'-[2-((*R*)-2-Methyl-pyrrolidin-1-yl)-ethyl]-biphenyl-4-sulfonyl}-acetic acid ethyl ester |
| 12 | 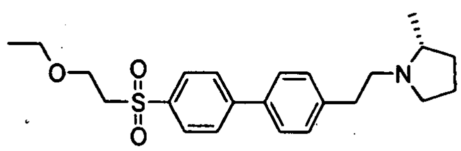 | (*R*)-1-{2-[4'-(2-Ethoxy-ethanesulfonyl)-biphenyl-4-yl]-ethyl}-2-methyl-pyrrolidine |
| 13 | 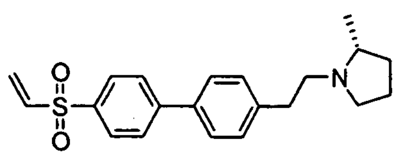 | (*R*)-1-[2-(4'-Ethenesulfonyl-biphenyl-4-yl)-ethyl]-2-methyl-pyrrolidine |
| 14 | 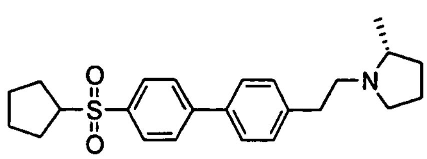 | (*R*)-1-[2-(4'-Cyclopentanesulfonyl-biphenyl-4-yl)-ethyl]-2-methyl-pyrrolidine |
| 15 | 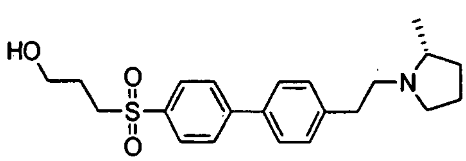 | 3- {4'-[2-((*R*)-2-Methyl-pyrrolidin-1-yl)-ethyl]-biphenyl-4-sulfonyl}-propan-1-ol |

(continued)

| Cmpd No. | Chemical Structure | Chemical Name |
|---|---|---|
| 16 | 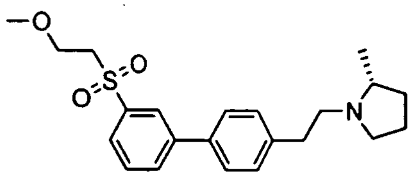 | (*R*)-1-{2-[3'-(2-Methoxy-ethanesulfonyl)-biphenyl-4-yl]-ethyl}-2-methyl-pyrrolidine |
| 17 | 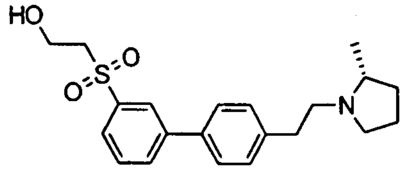 | 2-{4'-[2-((*R*)-2-Methyl-pyrrolidin-1-yl)-ethyl]-biphenyl-3-sulfonyl}-ethanol |
| 18 | 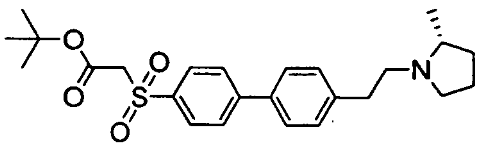 | {4'-[2-((*R*)-2-Methyl-pynolidin-1-yl)-ethyl]-biphenyl-4-sulfonyl}-acetic acid tert-butyl ester |
| 19 | 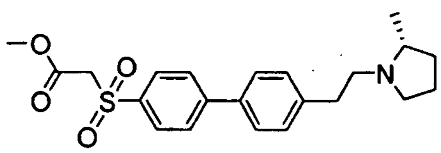 | {4'-[2-((*R*)-2-Methyl-pyrrolidin-1-yl)-ethyl]-biphenyl-4-sulfonyl}-acetic Acid Methyl Ester |

Some embodiments of the present invention include every combination of one or more compounds selected from the following group shown in TABLE B.

**TABLE B**

| Cmpd No. | Chemical Structure | Chemical Name |
|---|---|---|
| 20 | 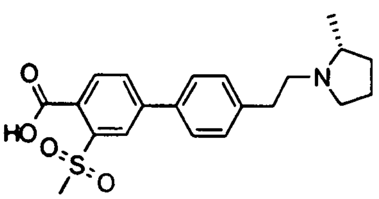 | 3-Methanesulfonyl-4'-[2-((*R*)-2-methyl-pyrrolidin-1-yl)-ethyl]-biphenyl-4-carboxylic acid |
| 21 | 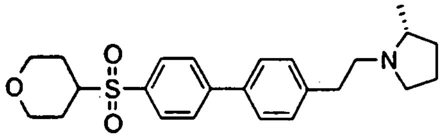 | (*R*)-2-Methyl-1-{2-[4'-(tetrahydro-pyran-4-sulfonyl)-biphenyl-4-yl]-ethyl -pyrrolidine |
| 22 | 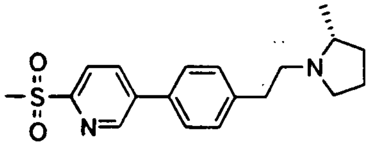 | 2-Methanesulfonyl-5- {4-[2-((*R*)-2-methyl-pyrrolidin-1-yl)-ethyl]-phenyl}-pyridine |

(continued)

| Cmpd No. | Chemical Structure | Chemical Name |
|---|---|---|
| 23 | 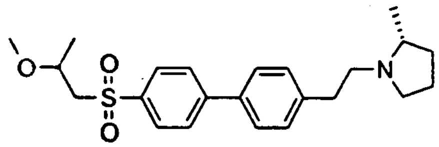 | (*R*)-1-{2-[4'-(2-Methoxy-propane-1-sulfonyl)-biphenyl-4-yl]-ethyl}-2-methyl-pyrrolidine |
| 24 | 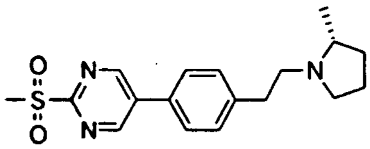 | 2-Methanesulfonyl-5-{4-[2-((*R*)-2-methyl-pyrrolidin-1-yl)-ethyl]-phenyl}-pyrimidine |
| 25 | 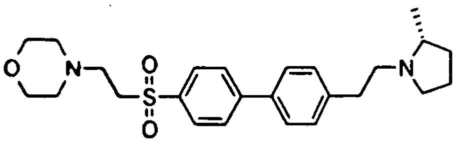 | 4-(2-{4'-[2-((*R*)-2-Methyl-pyrrolidin-1-yl)-ethyl]-biphenyl-4-sulfonyl}-ethyl)-morpholine |
| 26 | 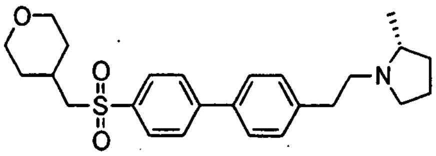 | (*R*)-2-Methyl-1-{2-[4'-(tetrahydro-pyran-4-ylmethanesulfonyl)-biphenyl-4-yl]-ethyl}-pyrrolidine |
| 27 | 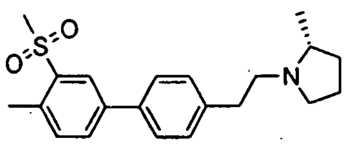 | (*R*)-1-[2-(3'-Methanesulfonyl-4'-methyl-biphenyl-4-yl)-ethyl]-2-methyl-pyrrolidine |
| 28 | 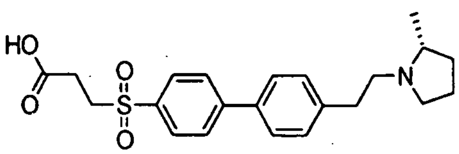 | 3-{4'-[2-((*R*)-2-Methyl-pyrrolidin-1-yl)-ethyl]-biphenyl-4-sulfonyl}-propionic acid |
| 29 | 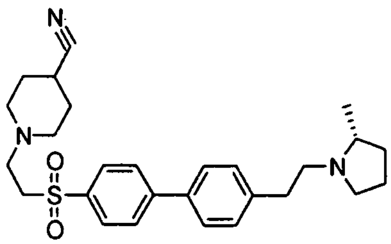 | 1-(2-{4'-[2-((*R*)-2-Methyl-pyrrolidin-1-yl)-ethyl]-biphenyl-4-sulfonyl}-ethyl)-piperidine-4-carbonitrile |
| 30 | 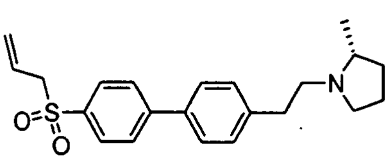 | (*R*)-2-Methyl-1-{2-[4'-(prop-2-ene-1-sulfonyl)-biphenyl-4-yl]-ethyl}-pyrrolidine |

(continued)

| Cmpd No. | Chemical Structure | Chemical Name |
|---|---|---|
| 31 | 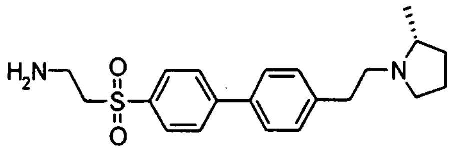 | 2-{4'-[2-((*R*)-2-Methyl-pyrrolidin-1-yl)-ethyl]-biphenyl-4-sulfonyl}-ethylamine |
| 32 | 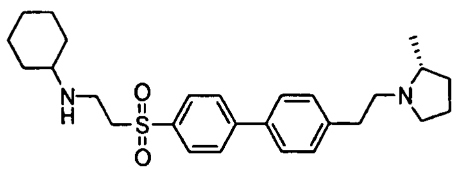 | Cyclohexyl-(2-{4'-[2-((*R*)-2-methyl-pyrrolidin-1-yl)-ethyl]-biphenyl-4-sulfonyl}-ethyl)-amine |
| 33 | 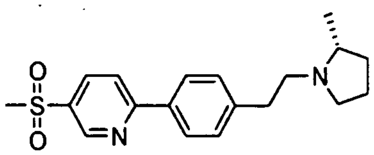 | 5-Methanesulfonyl-2-{4-[2-((*R*)-2-methyl-pyrrolidin-1-yl)-ethyl]-phenyl}-pyridine |
| 34 | 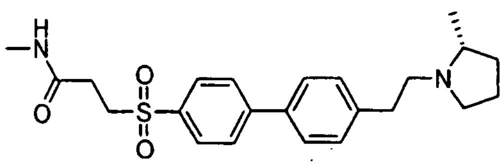 | (*R*)-*N*-methyl-3-(4'-(2-(2-methylpyrrolidin-1-yl)ethyl)biphenyl-4-ylsulfonyl)propanamide |
| 35 | 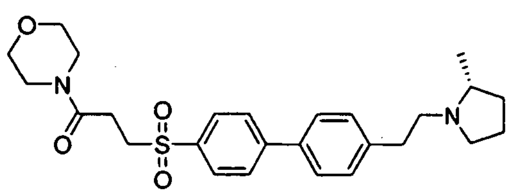 | (*R*)-3-(4'-(2-(2-methylpyrrolidin-1-yl)ethyl) biphenyl-4-ylsulfonyl)-1-morpholinopropan-1-one |
| 36 | 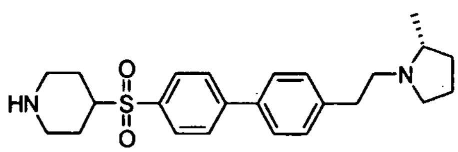 | (*R*)-4-(4'-(2-(2-Methylpyrrolidin-1-yl)ethyl) biphenyl-4-ylsulfonyl)piperidine |
| 37 | 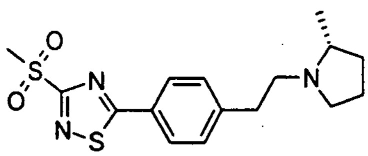 | (*R*)-5-(4-(2-(2-Methylpyrrolidin-1-yl)ethyl) phenyl)-3-(methylsulfonyl)-1,2,4-thiadiazole |
| 38 | 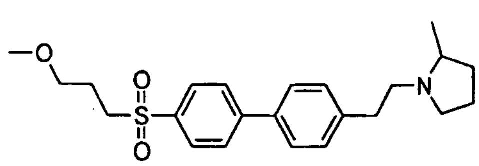 | 1-{2-[4'-(3-Methoxy-propane-1-sulfonyl)-biphenyl-4-yl]-ethyl}-2-methyl-pyrrolidine |

(continued)

| Cmpd No. | Chemical Structure | Chemical Name |
|---|---|---|
| 39 | 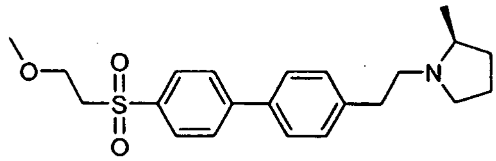 | (*S*)-1-(2-(4'-(2-, Methoxyethylsulfonyl) biphenyl-4-yl)ethyl)-2-methylpyrrolidine |
| 40 | 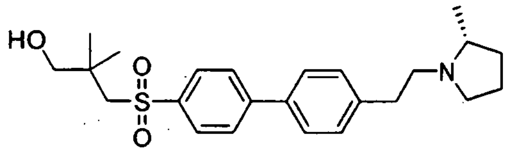 | (*R*)-2,2-Dimethyl-3-(4'-(2-(2-methylpyrrolidin-1-yl)ethyl)biphenyl-4-ylsulfonyl)propan-1-ol |
| 41 | 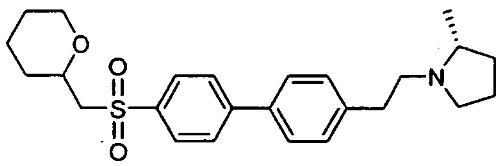 | (2*R*)-2-Methyl-1-(2-(4'-((tetrahydro-2H-pyran-2-yl)methylsulfonyl)biphenyl-4-yl)ethyl) pyrrolidine |
| 42 | 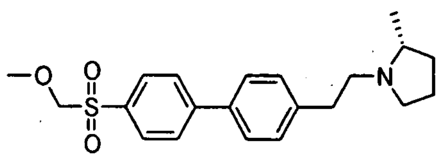 | (*R*)-1-(2-(4'-(Methoxymethylsulfonyl) biphenyl-4-yl)ethyl)-2-methylpyrrolidine |

Additionally, individual compounds and chemical genera of the present invention, for example those compounds found in TABLE A including diastereomers and enantiomers thereof, encompass all pharmaceutically acceptable salts, solvates, and particularly hydrates, thereof.

Some embodiments of the present invention relate to processes and intermediates useful in the preparation of novel compounds of Formula (**Ia**). General processes for the preparation of compounds of the invention are shown in **Figures 1** to **7** and exemplary reagents and procedures for these reactions appear hereinafter in the working Examples. Protection and deprotection may be carried out by procedures generally known in the art (see, for example, Greene, T. W. and Wuts, P. G. M., Protecting Groups in Organic Synthesis, 3rd Edition, 1999 [Wiley]).

It is understood that the present invention embraces each diastereomer, each enantiomer and mixtures thereof of each compound and generic formulae disclosed herein just as if they were each individually disclosed with the specific stereochemical designation for each chiral carbon. Separation of the individual isomers (such as, chiral HPLC, recrystallization of diastereomeric mixtures, and the like) or selective synthesis (such as, enantiomeric selective syntheses, and the like) of the individual isomers is accomplished by application of various methods, which are well known to practitioners in the art. Representative examples are shown herein.

## INDICATIONS AND METHODS OF PROPHYLAXIS AND/OR TREATMENT

Histamine [2-(imidazol-4-yl)ethylamine] exerts its physiological effects through four distinct G-protein coupled receptors (GPCRs), termed H1, H2, H3 and H4. The histamine H3-receptor was first identified in 1983, when it was determined that the H3-receptor acted as an autoreceptor controlling both the synthesis and release of histamine (see: Arrang et al. Nature 1983, 302, 832-7). At least four human and three rat splice variants have proven functional activity in pharmacological assays (Passani et al., Trends in Pharmacol. Sci. 2004, 25, 618-625). Rat and human histamine H3-receptors also show constitutive activity which means that they can transduce a signal even in the absence of a ligand. Histamine H3-receptors also function as heteroceptors, modulating the release of a number of other transmitter substances including serotonin, acetylcholine, dopamine and noradrenaline (see: Brown et al. Prog. Neurobiol. 2001, 63, 637-672). Thus, there are a number of therapeutic applications for ligands which target the histamine H3-receptor, where the ligand functions as either an antagonist or inverse agonist (for reviews see: Leurs et al. Nat. Rev. Drug. Discov. 2005, 4, 107-120; Passani et al. Trends Pharmacol. Sci. 2004, 25, 618-625).

Accordingly, preclinical studies have identified a number of indications which are amenable to treatment with

histamine H3-receptor antagonists and inverse agonists, such as compounds of the present invention. The compounds disclosed herein are believed to be useful in the treatment and/or prevention of several diseases and disorders, and in the amelioration of symptoms thereof. These compounds can be used alone or in combination with other compounds for the treatment and/or prevention of diseases and disorders. Without limitation, these diseases and disorders include the following.

Histamine H3-receptor antagonists have been shown to increase wakefulness (e.g. Lin J. S. et al. Brain Research 1990, 523, 325-330). This effect demonstrates that H3-receptor antagonists can be useful for disorders of sleep and wakefulness (Parmentier et al. J. Neurosci. 2002, 22, 7695-7711; Ligneau et al. J. Pharmacol. Exp. Ther. 1998, 287, 658-666). For example, histamine H3-receptor antagonists and inverse agonists can be used to treat the somnolence syndrome associated with different pathological conditions, for example, sleep apnea and Parkinson's disease or circumstances associated with lifestyle, for example, daytime somnolence from sleep deprivation as a result of nocturnal jobs, overwork, or jet-lag (see Passani et al., Trends Pharmacol. Sci. 2004, 25, 618-625). Somnolence is one of the major problems of public health because of its high prevalence (19-37% of the general population) and risk for causing work and traffic accidents.

Sleep apnea (alternatively sleep apnoea) is a common sleep disorder characterized by brief interruptions of breathing during sleep. These episodes, called apneas, last 10 seconds or more and occur repeatedly throughout the night. People with sleep apnea partially awaken as they struggle to breathe, but in the morning they may not be aware of the disturbances in their sleep. The most common type of sleep apnea is obstructive sleep apnea (OSA), caused by relaxation of soft tissue in the back of the throat that blocks the passage of air. Central sleep apnea (CSA) is caused by irregularities in the brain's normal signals to breathe. The hallmark symptom of the disorder is excessive daytime sleepiness. Additional symptoms of sleep apnea include restless sleep, loud snoring (with periods of silence followed by gasps), falling asleep during the day, morning headaches, trouble concentrating, irritability, forgetfulness, mood or behaviour changes, weight gain, increased heart rate, anxiety, and depression.

Few drug-based treatments of obstructive sleep apnea are known despite over two decades of research and tests. Oral administration of the methylxanthine theophylline (chemically similar to caffeine) can reduce the number of episodes of apnea, but can also produce side effects such as palpitations and insomnia. Theophylline is generally ineffective in adults with OSA, but is sometimes used to treat CSA, and infants and children with apnea. In 2003 and 2004, some neuroactive drugs, particularly modem-generation antidepressants including mirtazapine, have been reported to reduce incidences of obstructive sleep apnea. When other treatments do not completely treat the OSA, drugs are sometimes prescribed to treat a patient's daytime sleepiness or somnolence. These range from stimulants such as amphetamines to modem anti-narcoleptic medicines. The drug modafinil is seeing increased use in this role as of 2004.

In addition, for example, histamine H3-receptor antagonists and inverse agonists can be used to treat narcolepsy (Tedford et al. Soc. Neurosci. Abstr. 1999, 25, 460.3). Narcolepsy is a neurological condition most often characterized by Excessive Daytime Sleepiness (EDS), episodes of sleep and disorder of REM or rapid eye movement sleep. The main characteristic of narcolepsy is overwhelming Excessive Daytime Sleepiness (EDS), even after adequate nighttime sleep. A person with narcolepsy is likely to become drowsy or to fall asleep, often at inappropriate times and places. In addition, nighttime sleep may be fragmented with frequent wakenings. Classic symptoms of narcolepsy include, for example, cataplexy which is sudden episodes of loss of muscle function, ranging from slight weakness (such as limpness at the neck or knees, sagging facial muscles, or inability to speak clearly) to complete body collapse. Episodes may be triggered by sudden emotional reactions such as laughter, anger, surprise, or fear, and may last from a few seconds to several minutes. Another symptom of narcolepsy is sleep paralysis, which is the temporary inability to talk or move when waking up. Other symptoms include, for example, hypnagogic hallucinations which are vivid, often frightening, dreamlike experiences that occur while dozing, falling asleep and/or while awakening, and automatic behaviour which occurs when a person continues to function (talking, putting things away, etc.) during sleep episodes, but awakens with no memory of performing such activities. Daytime sleepiness, sleep paralysis, and hypnagogic hallucinations also occur in people who do not have narcolepsy, such as in people who are suffering from extreme lack of sleep. Cataplexy is generally considered unique to narcolepsy.

Currently the treatments available for narcolepsy treat the symptoms, but not the underlying cause. For cataplexy and REM-sleep symptoms, antidepressant medications and other drugs that suppress REM sleep are prescribed. The drowsiness is normally treated using stimulants such as methylphenidate (Ritalin), amphetamines (Adderall), dextroamphetamine (Dexedrine), methamphetamine (Desoxyn), modafinil (Provigil), etc. Other medications used are codeine and selegiline. The cataplexy is treated using clomipramine, imipramine, or protriptyline but this need only be done in severe cases. The drug gamma-hydroxybutyrate (GHB) (Xyrem) is approved in the USA by the Food and Drug Administration to treat both the cataplexy and excessive daytime sleepiness associated with narcolepsy.

Interestingly, modafinil (Provigil) has recently been shown to increase hypothalamic histamine release (Ishizuka et al. Neurosci. Lett. 2003, 339, 143-146).

In addition, recent studies using the classic Doberman model of narcolepsy with a non-imidazole histamine H3-receptor antagonist showed that a histamine H3-receptor antagonist can reduce the number of cataplectic attacks

and the duration of the attacks (Carruthers Ann. Meet. Eur. Histamine Res. Soc. 2004, Abs. p31).

In summary, histamine H3-receptor antagonists and inverse agonists can be used for the treatment and/or prevention of conditions associated with excessive daytime sleepiness such as hypersomnia, narcolepsy, sleep apnea, time zone change disorder, and other disorders which are associated with excessive daytime sleepiness such as fibromyalgia, and multiple sclerosis (Parmentier et al., J. Neurosci. 2002, 22, 7695-7711; Ligneau et al. J. Pharmacol. Exp. Ther. 1998, 287, 658-666). Other conditions include excessive sleepiness due to shift work, medical disorders, psychiatric disorders, narcolepsy, primary hypersomnia, and the like. Histamine H3-receptor antagonists and inverse agonists can also be used occasionally to promote wakefulness or vigilance in shift workers, sleep deprivation, post anesthesia grogginess, drowsiness as a side effect from a medication, military use and the like.

In addition, wakefulness is a prerequisite for several brain functions including attention, learning, and memory and is required for appropriate behaviours in response to environmental challenges. Histamine H3-receptor antagonists and inverse agonists have been shown to improve cognitive performance in various animal models (Hancock and Fox in Milestones in Drug Therapy, ed. Buccafusco, 2003). These compounds can be used as pro-cognitive agents and can increase vigilance. Therefore, histamine H3-receptor antagonists and inverse agonists can be used in aging or degenerative disorders in which vigilance, attention and memory are impaired, for example, as in Alzheimer's disease or other dementias.

Alzheimer's disease (AD), a neurodegenerative disorder, is the most common cause of dementia. It is characterized clinically by progressive cognitive deterioration together with neuropsychiatric symptoms and behavioural changes. The most striking early symptom is memory loss, which usually manifests as minor forgetfulness that becomes steadily more pronounced with illness progression, with relative preservation of older memories. As the disorder progresses, cognitive (intellectual) impairment extends to the domains of language, skilled movements, recognition and functions closely related to the frontal and temporal lobes of the brain such as decision-making and planning. There is currently no cure for AD, although there are drugs which offer symptomatic benefit, specifically with respect to short-term memory impairment. These drugs include acetylcholinesterase inhibitors such as donepezil (Aricept), galantamine (Razadyne) and rivastigmine (Exelon) and NMDA antagonists such as memantine.

Histamine H3-receptor antagonists and inverse agonists can be used to treat or prevent cognitive disorders (Passani et al. Trends Pharmacol. Sci. 2004, 25, 618-625), epilepsy (Vohora et al. Pharmacol. Biochem. Behav. 2001, 68, 735-741), depression (Perez-Garcia et al. Psychopharmacol. 1999, 142, 215-220), attention deficit hyperactivity disorder (ADHD), (Fox et al. Behav. Brain Res. 2002, 131, 151-61), and schizophrenia (Fox et al. J. Pharmacol. Exp. Ther. 2005, 313, 176-190). These indications are described briefly below. For additional information, see reviews by Leurs et al., Nat. Rev. Drug. Discov. 2005, 4, 107-120, and Vohora Investigational Drugs 2004, 7, 667-673). Histamine H3-receptor antagonists or inverse agonists can also be used as a novel therapeutic approach to restore cortical activation in comatose or brain-traumatized patients (Passani et al., Trends in Pharmacol. Sci. 2004, 25, 618-625).

As stated above, histamine H3-receptor antagonists and inverse agonists can be used to treat or prevent epilepsy. Epilepsy (often referred to as a seizure disorder) is a chronic neurological condition characterized by recurrent unprovoked seizures. In terms of their pattern of activity, seizures may be described as either partial (focal) or generalized. Partial seizures only involve a localized part of the brain, whereas generalized seizures involve the entire cortex. There are many different epilepsy syndromes, each presenting with its own unique combination of seizure type, typical age of onset, EEG findings, treatment, and prognosis. Some common seizure syndromes include, for example, infantile spasms (West syndrome), childhood absence epilepsy, and benign focal epilepsy of childhood (Benign Rolandic epilepsy), juvenile myoclonic epilepsy, temporal lobe epilepsy, frontal lobe epilepsy and Lennox-Gastaut syndrome.

Compounds of the present invention can be used in combination with various known drugs. For example, compounds of the present invention can be used with one or more drugs that prevent seizures or reduce seizure frequency: these include carbamazepine (common brand name Tegretol), clobazam (Frisium), clonazepam (Klonopin), ethosuximide (Zarontin), felbamate (Felbatol), fosphenytoin (Cerebyx), flurazepam (Dalmane), gabapentin (Neurontin), lamotrigine (Lamictal), levetiracetam (Keppra), oxcarbazepine (Trileptal), mephenytoin (Mesantoin), phcnobarbital (Luminal), phenytoin (Dilantin), pregabalin (Lyrica), primidone (Mysoline), sodium valproate (Epilim), tiagabine (Gabitril), topiramate (Topamax), valproate semisodium (Depakote), valproic acid (Depakene, Convulex), and vigabatrin (Sabril). Other drugs arc commonly used to abort an active seizure or interrupt a seizure flurry; these include diazepam (Valium) and lorazepam (Ativan). Drugs used only in the treatment of refractory status epilepticus include paraldehyde (Paral) and pentobarbital (Nembutal).

As stated above, a histamine H3-receptor antagonist or inverse agonist can be used as the sole agent of treatment or can be used in combination with other agents. For example, Vohora et al. show that a histamine H3-receptor antagonist can work as an anti-epilepsy, anti-seizure drug and also showed effect with sub-effective doses of the H3-receptor antagonist in combination with sub-effective doses of known anti-epileptic drugs (Vohora et al. Pharmacol. Biochem Behav 2001, 68, 735-741).

Perez-Garcia et al. (Psychopharmacol. 1999, 142, 215-220) tested the ability of a histamine H3-receptor agonist and antagonist on experimental mouse models of anxiety (elevated plus-maze) and depression (forced swimming test).

They found that while the compounds did not have a significant effect on the model of anxiety, a H3-receptor antagonist did have a significant dose-dependent effect in the model of depression. Thus, histamine H3-receptor antagonists or inverse agonists can have antidepressant effects.

Clinical depression is a state of sadness or melancholia that has advanced to the point of being disruptive to an individual's social functioning and/or activities of daily living. Clinical depression affects about 16% of the population on at least one occasion in their lives. Clinical depression is currently the leading cause of disability in the U.S. as well as other countries, and is expected to become the second leading cause of disability worldwide (after heart disease) by the year 2020, according to the World Health Organization.

Compounds of the present invention can be used in combination with various known drugs. For example, compounds of the present invention can be used with one or more of the drugs currently available that can relieve the symptoms of depression. They include, for example, monoamine oxidase inhibitors (MAOIs) such as Nardil or Moclobemide (Manerix), tricyclic antidepressants, selective serotonin reuptake inhibitors (SSRIs) such as fluoxetine (Prozac), paroxetine (Paxil), escitalopram (Lexapro), and sertraline (Zoloft), norepinephrine reuptake inhibitors such as reboxetine (Edronax), and serotonin-norepinephrine reuptake inhibitors (SNRIs) such as venlafaxine (Effexor) and duloxetine (Cymbalta).

As stated above, histamine H3-receptor antagonists and inverse agonists can be used to treat or prevent attention deficit hyperactivity disorder (ADHD). According to the Diagnostic and Statistical Manual of Mental Disorders-IV-TR, ADHD is a developmental disorder that arises in childhood, in most cases before the age of 7 years, is characterized by developmentally inappropriate levels of inattention and/or hyperactive-impulsive behavior, and results in impairment in one or more major life activities, such as family, peer, educational, occupational, social, or adaptive functioning. ADHD can also be diagnosed in adulthood.

The first-line medications used to treat ADHD are mostly stimulants, which work by stimulating the areas of the brain responsible for focus, attention, and impulse control. The use of stimulants to treat a syndrome often characterized by hyperactivity is sometimes referred to as a paradoxical effect, but there is no real paradox in that stimulants activate brain inhibitory and self-organizing mechanisms permitting the individual to have greater self regulation. The stimulants used include, for example, methylphenidate (sold as Ritalin, Ritalin SR and Ritalin LA), Metadate, Metadate ER, Metadate CD, Concerta, Focalin, Focalin XR or Methylin. The stimulants also include, for example, amphetamines such dextroamphetamine, sold as Dexedrine, Dexedrine Spansules, Adderall, and Adderall XR, a trade name for a mixture of dextroamphetamine and laevoamphetamine salts, methamphetamine sold as Desoxyn, bupropion, a dopamine and norepinephrine reuptake inhibitor, marketed under the brand name Wellbutrin. A non-stimulant medication to treat ADHD is Atomoxetine (sold as Strattera) a norepinephrine retiptake inhibitor. Other drugs sometimes used for ADHD include, for example, benzphetamine, Provigil/Alertec/modafinil and clonidine. Recently it has been reported that in a rat pup model for ADHD, a histamine H3-receptor antagonist was at least as effective as methylphenidate (Ritalin) (Hancock and Fox in Milestones in Drug Therapy, ed. Buccafusco, 2003). Compounds of the present invention can be used in combination with various known drugs. For example, compounds of the present invention can be used with one or more of the drugs used to treat ADHD and related disorders.

As stated above, histamine H3-receptor antagonists and inverse agonists can be used to treat or prevent schizophrenia. Schizophrenia is a psychiatric diagnosis that describes a mental disorder characterized by impairments in the perception or expression of reality and by significant social or occupational dysfunction. A person experiencing untreated schizophrenia is typically characterized as demonstrating disorganized thinking, and as experiencing delusions or auditory hallucinations. Although the disorder is primarily thought to affect cognition, it can also contribute to chronic problems with behavior and emotion. Schizophrenia is often described in terms of "positive" and "negative" symptoms. Positive symptoms include delusions, auditory hallucinations and thought disorder, and are typically regarded as manifestations of psychosis. Negative symptoms are so named because they are considered to be the loss or absence of normal traits or abilities, and include features such as flat, blunted or constricted affect and emotion, poverty of speech and lack of motivation. Some models of schizophrenia include formal thought disorder and planning difficulties in a third group, a "disorganization syndrome."

The first line pharmacological therapy for schizophrenia is usually the use of antipsychotic medication. Antipsychotic drugs are only thought to provide symptomatic relief from the positive symptoms of psychosis. The newer atypical antipsychotic medications (such as clozapine, risperidone, olanzapine, quetiapine, ziprasidone and aripiprazole) are usually preferred over older typical antipsychotic medications (such as chlorpromazine and haloperidol) due to their favorable side-effect profile. While the atypical antipsychotics are associated with less extra pyramidal side-effects and tardive dyskinesia than the conventional antipsychotics, some of the agents in this class (especially olanzapine and clozapine) appear to be associated with metabolic side effects such as weight gain, hyperglycemia and hypertriglyceridemia that must be considered when choosing appropriate pharmacotherapy.

Histamine H3-receptor antagonists or inverse agonists can be used to treat obesity (Hancock, Curr. Opin. Investig. Drugs 2003, 4, 1190-1197). The role of neuronal histamine in food intake has been established for many years and neuronal histamine release and/or signalling has been implicated in the anorectic actions of known mediators in the feeding cycle

such as leptin, amylin and bombesin. In the brain, the H3-receptor is implicated in the regulation of histamine release in the hypothalamus. Moreover, *in situ* hybridization studies have revealed histamine H3-receptor mRNA expression in rat brown adipose tissue, indicating a role in the regulation of thermogenesis (Karlstedt et al., Mol. Cell. Neurosci. 2003, 24, 614-622). Furthermore, histamine H3-receptor antagonists have been investigated in various preclinical models of obesity and have shown to be effective in reducing food intake, reducing weight, and decreasing total body fat in mice (Hancock, et al. Eur. J. Pharmacol. 2004, 487, 183-197). The most common drugs used for the treatment of obesity are sibutramine (Meridia) and orlistat (Xenical), both of which have limited effectiveness and significant side effects. Therefore, novel anti-obesity agents, such as histamine H3-receptor antagonists or inverse agonists, are needed.

Histamine H3-receptor antagonists or inverse agonists can also be used to treat upper airway allergic responses (U.S. Pat. Nos. 5,217,986; 5,352,707 and 5,869,479) including allergic rhinitis and nasal congestion. Allergic rhinitis is a frequently occurring chronic disease that affects a large number of people. Recent analysis of histamine H3-receptor expression in the periphery by quantitative PCR revealed that H3-receptor mRNA is abundantly expressed in human nasal mucosa (Varty et al. Eur. J. Pharmacol. 2004, 484, 83-89). In addition, in a cat model of nasal decongestion, a combination of histamine H3-receptor antagonists with the H1 receptor antagonist chlorpheniramine resulted in significant nasal decongestion without the hypertensive effect seen with adrenergic agonists. (McLeod et al. Am. J. Rhinol. 1999, 13, 391 399). Thus, histamine H3-receptor antagonists or inverse agonists can be used alone or in combination with H1 receptor blockage for the treatment of allergic rhinitis and nasal congestion.

Histamine H3-receptor antagonists or inverse agonists have therapeutic potential for the treatment of pain (Medhurst et al. Biochemical Pharmacology (2007), 73(8), 1182-1194).

## PHARMACEUTICAL COMPOSITIONS

A further aspect of the present invention pertains to pharmaceutical compositions comprising one or more compounds as described herein and one or more pharmaceutically acceptable carriers. Some embodiments pertain to pharmaceutical compositions comprising a compound of the present invention and a pharmaceutically acceptable carrier.

Some embodiments of the present invention include a method of producing a pharmaceutical composition comprising admixing at least one compound according to any of the compound embodiments disclosed herein and a pharmaceutically acceptable carrier.

Formulations may be prepared by any suitable method, typically by uniformly mixing the active compound(s) with liquids or finely divided solid carriers, or both, in the required proportions, and then, if necessary, forming the resulting mixture into a desired shape.

Conventional excipients, such as binding agents, fillers, acceptable wetting agents, tabletting lubricants, and disintegrants may be used in tablets and capsules for oral administration. Liquid preparations for oral administration may be in the form of solutions, emulsions, aqueous or oily suspensions, and syrups. Alternatively, the oral preparations may be in the form of dry powder that can be reconstituted with water or another suitable liquid vehicle before use. Additional additives such as suspending or emulsifying agents, non-aqueous vehicles (including edible oils), preservatives, and flavorings and colorants may be added to the liquid preparations. Parenteral dosage forms may be prepared by dissolving the compound of the invention in a suitable liquid vehicle and filter sterilizing the solution before filling and sealing an appropriate vial or ampule. These are just a few examples of the many appropriate methods well known in the art for preparing dosage forms.

A compound of the present invention can be formulated into pharmaceutical compositions using techniques well known to those in the art. Suitable pharmaceutically-acceptable carriers, outside those mentioned herein, are known in the art; for example, see Remington, The Science and Practice of Pharmacy, 20th Edition, 2000, Lippincott Williams & Wilkins, (Editors: Gennaro et al.)..

While it is possible that, for use in the prophylaxis or treatment, a compound of the invention may, in an alternative use, be administered as a raw or pure chemical, it is preferable however to present the compound or active ingredient as a pharmaceutical formulation or composition further comprising a pharmaceutically acceptable carrier.

The invention thus further provides pharmaceutical formulations comprising a compound of the invention or a pharmaceutically acceptable salt or derivative thereof together with one or more pharmaceutically acceptable carriers thereof and/or prophylactic ingredients. The carrier(s) must be "acceptable" in the sense of being compatible with the other ingredients of the formulation and not overly deleterious to the recipient thereof.

Pharmaceutical formulations include those suitable for oral, rectal, nasal, topical (including buccal and sub-lingual), vaginal or parenteral (including intramuscular, subcutaneous and intravenous) administration or in a form suitable for administration by inhalation, insufflation or by a transdermal patch. Transdermal patches dispense a drug at a controlled rate by presenting the drug for absorption in an efficient manner with a minimum of degradation of the drug. Typically, transdermal patches comprise an impermeable backing layer, a single pressure sensitive adhesive and a removable protective layer with a release liner. One of ordinary skill in the art will understand and appreciate the techniques appropriate for manufacturing a desired efficacious transdermal patch based upon the needs of the artisan.

The compounds of the invention, together with a conventional adjuvant, carrier, or diluent, may thus be placed into the form of pharmaceutical formulations and unit dosages thereof, and in such form may be employed as solids, such as tablets or filled capsules, or liquids such as solutions, suspensions, emulsions, elixirs, gels or capsules filled with the same, all for oral use, in the form of suppositories for rectal administration; or in the form of sterile injectable solutions for parenteral (including subcutaneous) use. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

For oral administration, the pharmaceutical composition may be in the form of, for example, a tablet, capsule, suspension or liquid. The pharmaceutical composition is preferably made in the form of a dosage unit containing a particular amount of the active ingredient. Examples of such dosage units are capsules, tablets, powders, granules or a suspension, with conventional additives such as lactose, mannitol, corn starch or potato starch; with binders such as crystalline cellulose, cellulose derivatives, acacia, corn starch or gelatins; with disintegrators such as corn starch, potato starch or sodium carboxymethyl-cellulose; and with lubricants such as talc or magnesium stearate. The active ingredient may also be administered by injection as a composition wherein, for example, saline, dextrose or water may be used as a suitable pharmaceutically acceptable carrier.

Compounds of the present invention or a solvate or physiologically functional derivative thereof can be used as active ingredients in pharmaceutical compositions, specifically as histamine H3-receptor modulators. By the term "active ingredient" is defined in the context of a "pharmaceutical composition" and is intended to mean a component of a pharmaceutical composition that provides the primary pharmacological effect, as opposed to an "inactive ingredient" which would generally be recognized as providing no pharmaceutical benefit.

The dose when using the compounds of the present invention can vary within wide limits, and as is customary and is known to the physician, it is to be tailored to the individual conditions in each individual case. It depends, for example, on the nature and severity of the illness to be treated, on the condition of the patient, on the compound employed or on whether an acute or chronic disease state is treated or prophylaxis is conducted or on whether further active compounds are administered in addition to the compounds of the present invention. Representative doses of the present invention include, but are not limited to, about 0.001 mg to about 5000 mg, about 0.001 mg to about 2500 mg, about 0.001 mg to about 1000 mg, 0.001 mg to about 500 mg, 0.001 mg to about 250 mg, about 0.001 mg to 100 mg, about 0.001 mg to about 50 mg, and about 0.001 mg to about 25 mg. Multiple doses may be administered during the day, especially when relatively large amounts are deemed to be needed, for example 2, 3 or 4, doses. Depending on the individual and as deemed appropriate from the patient's physician or care-giver it may be necessary to deviate upward or downward from the doses described herein.

The amount of active ingredient, or an active salt or derivative thereof, required for use in treatment will vary not only with the particular salt selected but also with the route of administration, the nature of the condition being treated and the age and condition of the patient and will ultimately be at the discretion of the attendant physician or clinician. In general, one skilled in the art understands how to extrapolate *in vivo* data obtained in a model system, typically an animal model, to another, such as a human. In some circumstances, these extrapolations may merely be based on the weight of the animal model in comparison to another, such as a mammal, preferably a human, however, more often, these extrapolations are not simply based on weights, but rather incorporate a variety of factors. Representative factors include the type, age, weight, sex, diet and medical condition of the patient, the severity of the disease, the route of administration, pharmacological considerations such as the activity, efficacy, pharmacokinetic and toxicology profiles of the particular compound employed, whether a drug delivery system is utilized, on whether an acute or chronic disease state is being treated or prophylaxis is conducted or on whether further active compounds are administered in addition to the compounds of the present invention and as part of a drug combination. The dosage regimen for treating a disease condition with the compounds and/or compositions of this invention is selected in accordance with a variety factors as cited above. Thus, the actual dosage regimen employed may vary widely and therefore may deviate from a preferred dosage regimen and one skilled in the art will recognize that dosage and dosage regimen outside these typical ranges can be tested and, where appropriate, may be used in the methods of this invention.

The desired dose may conveniently be presented in a single dose or as divided doses administered at appropriate intervals, for example, as two, three, four or more sub-doses per day. The sub-dose itself may be further divided, e.g., into a number of discrete loosely spaced administrations. The daily dose can be divided, especially when relatively large amounts are administered as deemed appropriate, into several, for example 2, 3 or 4, part administrations. If appropriate, depending on individual behavior, it may be necessary to deviate upward or downward from the daily dose indicated.

The compounds of the present invention can be administrated in a wide variety of oral and parenteral dosage forms. It will be obvious to those skilled in the art that the following dosage forms may comprise, as the active component, either a compound of the invention or a pharmaceutically acceptable salt of a compound of the invention.

For preparing pharmaceutical compositions from the compounds of the present invention, the selection of a

suitable pharmaceutically acceptable carrier can be either solid, liquid or a mixture of both. Solid form preparations include powders, tablets, pills, capsules, cachets, suppositories, and dispersible granules. A solid carrier can be one or more substances, which may also act as diluents, flavoring agents, solubilizers, lubricants, suspending agents, binders, preservatives, tablet disintegrating agents, or an encapsulating material.

In powders, the carrier is a finely divided solid, which is in a mixture with the finely divided active component.

In tablets, the active component is mixed with the carrier having the necessary binding capacity in suitable proportions and compacted to the desire shape and size.

The powders and tablets may contain varying percentage amounts of the active compound. A representative amount in a powder or tablet may contain from 0.5 to about 90 percent of the active compound; however, an artisan would know when amounts outside of this range are necessary. Suitable carriers for powders and tablets are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as an admixture of fatty acid glycerides or cocoa butter, is first melted and the active component is dispersed homogeneously therein, as by stirring. The molten homogenous mixture is then poured into convenient sized molds, allowed to cool, and thereby to solidify.

Formulations suitable for vaginal administration may be presented as pessaries, tampons, creams, gels, pastes, foams or sprays containing in addition to the active ingredient such carriers as are known in the art to be appropriate.

Liquid form preparations include solutions, suspensions, and emulsions, for example, water or water-propylene glycol solutions. For example, parenteral injection liquid preparations can be formulated as solutions in aqueous polyethylene glycol solution. Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose, any bland fixed oil may be employed including synthetic mono- or diglycerides. In addition, fatty acids such as oleic acid find use in the preparation of injectables.

The compounds according to the present invention may thus be formulated for parenteral administration (e.g. by injection, for example bolus injection or continuous infusion) and may be presented in unit dose form in ampoules, pre-filled syringes, small volume infusion or in multi-dose containers with an added preservative. The pharmaceutical compositions may take such forms as suspensions, solutions, or emulsions in oily or aqueous vehicles, and may contain formulatory agents such as suspending, stabilizing and/or dispersing agents. Alternatively, the active ingredient may be in powder form, obtained by aseptic isolation of sterile solid or by lyophilization from solution, for constitution with a suitable vehicle, e.g. sterile, pyrogen-free water, before use.

Aqueous formulations suitable for oral use can be prepared by dissolving or suspending the active component in water and adding suitable colorants, flavors, stabilizing and thickening agents, as desired.

Aqueous suspensions suitable for oral use can be made by dispersing the finely divided active component in water with viscous material, such as natural or synthetic gums, resins, methylcellulose, sodium carboxymethylcellulose, or other well-known suspending agents.

Also included are solid form preparations, which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

For topical administration to the epidermis, the compounds according to the invention may be formulated as ointments, creams or lotions, or as a transdermal patch.

Ointments and creams may, for example, be formulated with an aqueous or oily base with the addition of suitable thickening and/or gelling agents. Lotions may be formulated with an aqueous or oily base and will in general also contain one or more emulsifying agents, stabilizing agents, dispersing agents, suspending agents, thickening agents, or coloring agents.

Formulations suitable for topical administration in the mouth include lozenges comprising active agent in a flavored base, usually sucrose and acacia or tragacanth; pastilles comprising the active ingredient in an inert base such as gelatin and glycerin or sucrose and acacia; and mouthwashes comprising the active ingredient in a suitable liquid carrier.

Solutions or suspensions are applied directly to the nasal cavity by conventional means, for example with a dropper, pipette or spray. The formulations may be provided in single or multi-dose form. In the latter case of a dropper

or pipette, this may be achieved by the patient administering an appropriate, predetermined volume of the solution or suspension. In the case of a spray, this may be achieved for example by means of a metering atomizing spray pump.

Administration to the respiratory tract may also be achieved by means of an aerosol formulation in which the active ingredient is provided in a pressurized pack with a suitable propellant. If the compounds of the present invention or pharmaceutical compositions comprising them are administered as aerosols, for example as nasal aerosols or by inhalation, this can be carried out, for example, using a spray, a nebulizer, a pump nebulizer, an inhalation apparatus, a metered inhaler or a dry powder inhaler. Pharmaceutical forms for administration of the compounds of the present invention as an aerosol can be prepared by processes well-known to the person skilled in the art. For their preparation, for example, solutions or dispersions of the compounds of the present invention in water, water/alcohol mixtures or suitable saline solutions can be employed using customary additives, for example benzyl alcohol or other suitable preservatives, absorption enhancers for increasing the bioavailability, solubilizers, dispersants and others, and, if appropriate, customary propellants, for example include carbon dioxide, CFCs, such as, dichlorodifluoromethane, trichlorofluoromethane, or dichlorotetrafluoroethane; and the like. The aerosol may conveniently also contain a surfactant such as lecithin. The dose of drug may be controlled by provision of a metered valve.

In formulations intended for administration to the respiratory tract, including intranasal formulations, the compound will generally have a small particle size for example of the order of 10 microns or less. Such a particle size may be obtained by means known in the art, for example by micronization. When desired, formulations adapted to give sustained release of the active ingredient may be employed.

Alternatively the active ingredients may be provided in the form of a dry powder, for example, a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, e.g., gelatin, or blister packs from which the powder may be administered by means of an inhaler.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

Tablets or capsules for oral administration and liquids for intravenous administration are preferred compositions.

The compounds according to the invention may optionally exist as pharmaceutically acceptable salts including pharmaceutically acceptable acid addition salts prepared from pharmaceutically acceptable non-toxic acids including inorganic and organic acids. Representative acids include, but are not limited to, acetic, benzenesulfonic, benzoic, camphorsulfonic, citric, ethenesulfonic, dichloroacetic, formic, fumaric, gluconic, glutamic, hippuric, hydrobromic, hydrochloric, isethionic, lactic, maleic, malic, mandelic, methanesulfonic, mucic, nitric, oxalic, pamoic, pantothenic, phosphoric, succinic, sulfiric, tartaric, oxalic, p-toluenesulfonic and the like, such as those pharmaceutically acceptable salts listed in Journal of Pharmaceutical Sciences, 66:1-19 (1977).

The acid addition salts may be obtained as the direct products of compound synthesis. In the alternative, the free base may be dissolved in a suitable solvent containing the appropriate acid, and the salt isolated by evaporating the solvent or otherwise separating the salt and solvent. The compounds of this invention may form solvates with standard low molecular weight solvents using methods known to the skilled artisan, such as those described in Polymorphism in Pharmaceutical solids, edited by Harry G. Brittain, Marcel Dekker, New York, 1999.

Compounds of the present invention can be converted to "pro-drugs." The term "pro-drugs" refers to compounds that have been modified with specific chemical groups known in the art and when administered into an individual these groups undergo biotransformation to give the parent compound. Pro-drugs can thus be viewed as compounds of the invention containing one or more specialized non-toxic protective groups used in a transient manner to alter or to eliminate a property of the compound. In one general aspect, the "pro-drug" approach is utilized to facilitate oral absorption. A thorough discussion is provided in T. Higuchi and V. Stella, Pro-drugs as Novel Delivery Systems Vol. 14 of the A.C.S. Symposium Series; and in Bioreversible Carriers in Drug Design, ed. Edward B. Roche, American Pharmaceutical Association and Pergamon Press, 1987.

Some embodiments of the present invention include a method of producing a pharmaceutical composition for "combination-therapy" comprising admixing at least one compound according to any of the compound embodiments disclosed herein, together with at least one known pharmaceutical agent as described herein and a pharmaceutically acceptable carrier.

It is noted that when the histamine H3-receptor modulators are utilized as active ingredients in a pharmaceutical composition, these are not intended for use only in humans, but in other non-human mammals as well. Indeed, recent advances in the area of animal health-care mandate that consideration be given for the use of active agents, such as histamine H3-receptor modulators, for the treatment of an H3-associated disease or disorder in domestic animals (e.g., cats and dogs) and in other domestic animals (e.g., cows, chickens, fish, etc.). Those of ordinary skill in the art are

readily credited with understanding the utility of such compounds in such settings.

## OTHER UTILITIES

Another object of the present invention relates to radio-labeled compounds of the present invention that would be useful not only in radio-imaging but also in assays, both *in vitro* and *in vivo,* for localizing and quantitating the histamine H3-receptor in tissue samples, including human, and for identifying histamine H3-receptor ligands by inhibition binding of a radio-labeled compound. It is a further object of this invention to develop novel H3-receptor assays of which comprise such radio-labeled compounds.

The present invention embraces isotopically-labeled compounds of the present invention. An "isotopically" or "radio-labeled" compounds are those that are identical to compounds disclosed herein, but for the fact that one or more atoms are replaced or substituted by an atom having an atomic mass or mass number different from the atomic mass or mass number most commonly found in nature. Suitable radionuclides that may be incorporated in compounds of the present invention include but are not limited to $^{2}H$ (also written as D for deuterium), $^{3}H$ (also written as T for tritium) $^{11}C$, $^{13}C$, $^{14}C$, $^{13}N$, $^{15}N$, $^{15}O$, $^{17}O$, $^{18}O$, $^{18}F$, $^{35}S$, $^{36}Cl$, $^{82}Br$, $^{75}Br$, $^{76}Br$, $^{77}Br$, $^{123}I$, $^{124}I$, $^{125}I$ and $^{131}I$. The radionuclide that is incorporated in the instant radio-labeled compounds will depend on the specific application of that radio-labeled compound. For example, for *in vitro* histamine H3-receptor labeling and competition assays, compounds that incorporate $^{3}H$, $^{14}C$, $^{82}Br$, $^{125}I$, $^{131}I$ or $^{35}S$ will generally be most useful. For radio-imaging applications $^{11}C$, $^{18}F$, $^{125}I$, $^{123}I$, $^{124}I$, $^{131}I$, $^{75}Br$ , $^{76}Br$ or $^{77}Br$ will generally be most useful.

It is understood that a "radio-labeled "or "labeled compound" is a compound of Formula (Ia) that has incorporated at least one radionuclide; in some embodiments the radionuclide is selected from the group consisting of $^{3}H$, $^{14}C$, $^{125}I$, $^{35}S$ and $^{82}$ Br. Certain isotopically-labeled compounds of the present invention are useful in compound and/or substrate tissue distribution assays. In some embodiments the radionuclide $^{3}H$ and/or $^{14}C$ isotopes are useful in these studies. Further, substitution with heavier isotopes such as deuterium (i.e., $^{2}H$) may afford certain therapeutic advantages resulting from greater metabolic stability (e.g., increased *in vivo* half-life or reduced dosage requirements) and hence may be preferred in some circumstances. Isotopically labeled compounds of the present invention can generally be prepared by following procedures analogous to those disclosed in the Drawings and Examples *infra,* by substituting an isotopically labeled reagent for a non-isotopically labeled reagent. Other synthetic methods that are useful are discussed *infra.* Moreover, it should be understood that all of the atoms represented in the compounds of the invention can be either the most commonly occurring isotope of such atoms or the more scarce radio-isotope or nonradioactive isotope.

Synthetic methods for incorporating radio-isotopes into organic compounds are applicable to compounds of the invention and are well known in the art. These synthetic methods, for example, incorporating activity levels of tritium into target molecules, are as follows:

A. Catalytic Reduction with Tritium Gas - This procedure normally yields high specific activity products and requires halogenated or unsaturated precursors.
B. Reduction with Sodium Borohydride [$^{3}H$] - This procedure is rather inexpensive and requires precursors containing reducible functional groups such as aldehydes, ketones, lactones, esters, and the like.
C. Reduction with Lithium Aluminum Hydride [$^{3}H$] - This procedure offers products at almost theoretical specific activities. It also requires precursors containing reducible functional groups such as aldehydes, ketones, lactones, esters, and the like.
D. Tritium Gas Exposure Labeling - This procedure involves exposing precursors containing exchangeable protons to tritium gas in the presence of a suitable catalyst.
E. *N*-Methylation using Methyl Iodide [$^{3}H$] - This procedure is usually employed to prepare O-methyl or *N*-methyl ($^{3}H$) products by treating appropriate precursors with high specific activity methyl iodide ($^{3}H$). This method in general allows for higher specific activity, such as for example, about 70-90 Ci/mmol.

Synthetic methods for incorporating activity levels of $^{125}I$ into target molecules include:

A. Sandmeyer and like reactions - This procedure transforms an aryl or heteroaryl amine into a diazonium salt, such as a tetrafluoroborate salt, and subsequently to $^{125}I$ labeled compound using Na $^{125}I$. A represented procedure was reported by Zhu, G-D. and co-workers in J. Org. Chem., 2002, 67, 943-948.
B. Ortho $^{121}$Iodination of phenols - This procedure allows for the incorporation of $^{12}I$ at the ortho position of a phenol as reported by Collier, T. L. and co-workers in J. Labelled Compd. Radiopharm., 1999, 42, S264-S266.
C. Aryl and heteroaryl bromide exchange with $^{125}I$ - This method is generally a two step process. The first step is the conversion of the aryl or heteroaryl bromide to the corresponding tri-alkyltin intermediate using for example, a Pd catalyzed reaction [i.e. $Pd(Ph_3P)_4$] or through an aryl or heteroaryl lithium, in the presence of a tri-alkyltinhalide or hexaalkyldltin [e.g., $(CH_3)_3SnSn(CH_3)_3$]. A representative procedure was reported by Le Bas, M.-D. and co-workers in J. Labelled Compd. Radiopharm. 2001, 44, S280-S282.

A radiolabeled histamine H3-receptor compound of Formula (**Ia**) can be used in a screening assay to identify/evaluate compounds. In general terms, a newly synthesized or identified compound (i.e., test compound) can be evaluated for its ability to reduce binding of the "radio-labeled compound of Formula (**Ia**)" to the H3-receptor. Accordingly, the ability of a test compound to compete with the "radio-labeled compound of Formula (**Ia**)" for the binding to the histamine H3-receptor directly correlates to its binding affinity.

The labeled compounds of the present invention bind to the histamine H3-receptor. In one embodiment the labeled compound has an $IC_{50}$ less than about 500 μM, in another embodiment the labeled compound has an $IC_{50}$ less than about 100 μM, in yet another embodiment the labeled compound has an $IC_{50}$ less than about 10 μM, in yet another embodiment the labeled compound has an $IC_{50}$ less than about I μM, and in still yet another embodiment the labeled inhibitor has an $IC_{50}$ less than about 0.1 μM.

Other uses of the disclosed receptors and methods will become apparent to those in the art based upon, *inter alia,* a review of this disclosure.

As will be recognized, the steps of the methods of the present invention need not be performed any particular number of times or in any particular sequence. Additional objects, advantages, and novel features of this invention will become apparent to those skilled in the art upon examination of the following examples thereof, which are intended to be illustrative and not intended to be limiting.

## EXAMPLES

### EXAMPLE 1: Syntheses of compounds of the present invention.

Illustrated syntheses for compounds of the present invention are shown in Figures 1 through 7 where the symbols have the same definitions as used throughout this disclosure.

The compounds of the invention and their synthesis are further illustrated by the following examples. The following examples are provided to further define the invention without, however, limiting the invention to the particulars of these examples. The compounds described herein, *supra* and *infra,* are named according to the CS ChemDraw Ultra Version 7.0.1, AutoNom version 2.2. In certain instances common names are used and it is understood that these common names would be recognized by those skilled in the art.

**Chemistry:** Proton nuclear magnetic resonance ($^1H$ NMR) spectra were recorded on a Varian Mercury Vx-400 equipped with a 4 nucleus auto switchable probe and z-gradient or a Bruker Advance-400 equipped with a QNP (Quad Nucleus Probe) or a BBI (Broad Band Inverse) and z-gradient. Chemical shifts are given in parts per million (ppm) with the residual solvent signal used as reference. NMR abbreviations are used as follows: s = singlet, d = doublet, t = triplet, q = quartet, m = multiplet, br = broad, dt = doublet of triplet, td = triplet of doublet, dd = doublet of doublet, ddd = doublet of doublet of doublets. Microwave irradiations were carried out using a Smith Synthesizer™ or an Emrys Optimizer™ (Personal Chemistry). Thin-layer chromatography (TLC) was performed on silica gel 60 $F_{254}$ (Merck), preparatory thin-layer chromatography (prep TLC) was preformed on PK6F silica gel 60 A I mm plates (Whatman), and column chromatography was carried out on a silica gel column using Kieselgel 60, 0.063-0.200 mm (Merck). Evaporation was done under reduced pressure on a Büchi rotary evaporator. Celite 545 ® was used during palladium filtrations.

LCMS specs: HPLC-pumps: LC-10AD *VP*, Shimadzu Inc.; HPLC system controller: SCL-10A *VP*, Shimadzu Inc; UV-Detector: SPD-10A *VP*, Shimadzu Inc; Autosampler: CTC HTS, PAL, Leap Scientific; Mass spectrometer: API 150EX with Turbo Ion Spray source, AB/MDS Sciex; Software: Analyst 1.2.

### Example 1.1: Preparation of Intermediate (*R*)-4-(2-(2-Methylpyrrolidin-1-yl)ethyl)phenylboronic Acid.

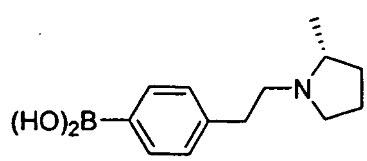

### Step A: Preparation of Intermediate 4-Bromophenethyl Methanesulfonate.

4-Bromophenethyl alcohol (38.9 g, 193 mmol) was dissolved in DCM (193 mL). Triethylamine (40.4 mL, 290

mmol) was added and the mixture was cooled in an ice bath. Methanesulfonyl chloride (18 mL, 232 mmol) was added dropwise via an addition funnel. The ice bath was removed and the mixture was stirred for 30 min. The reaction mixture was diluted with DCM (200 mL), washed with 1 M HCl twice (100 mL each), followed by brine, saturated sodium bicarbonate, and brine. The organic phase was dried with sodium sulfate and filtered. The solvent was removed under reduced pressure to give the title compound (54.0 g) in quantitative yield. $^1$H NMR (400 MHz, $CDCl_3$) δ ppm 2.89 (s, 3 H), 3.02 (t, *J*= 6.82 Hz, 2 H), 4.40 (t, *J* = 6.82 Hz, 2 H), 7.03 - 7.17 (m, 2 H), 7.43 - 7.47 (m, 2 H).

**Step B: Preparation of Intermediate (*R*)-1-(4-Bromophenethyl)-2-methylpyrrolidine.**

4-Bromophenethyl methanesulfonate (12.2 g, 43.8 mmol) was dissolved in acetonitrile (88 mL). Sodium carbonate (6.04 g, 57.0 mmol) was added, followed by (*R*)-(-)-2-methylpyrrolidme (4.48 g, 52.6 mmol). The reaction mixture was warmed to 80˚C and stirred overnight. The sodium carbonate was filtered and the solvent was removed under reduced pressure. The crude residue was re-suspended in ethyl acetate (~200 mL), extracted with 1 M HCl (75 mL). The ethyl acetate was extracted an additional three times with 1 M HCl (30 mL each). HCl layers were combined and made basic (pH~10) by addition of sodium carbonate. The basic aqueous layer was extracted with DCM (100 mL). 1 mL of 50% sodium hydroxide was added to the aqueous layer which was then extracted three times with DCM (50 mL each). DCM layers were combined, dried over $Na_2SO_4$ and filtered. The solvent was removed under reduced pressure to give a yellow oil (10.2 g, 87% crude yield). The crude oil was further purified by silica column chromatography eluting with ethyl acetate followed by 0-10% MeOH in ethyl acetate to give the title compound (8.85 g, 75%) as a pale yellow oil. Exact mass calculated for $C_{45}H_{18}BrN$: 267.1, Found: LCMS *m/z* = 268.0 ($M+H^+$); $^1$H NMR (400 MHz, Methanol-$d_4$) δ ppm 1.15 (d, *J*= 6.06 Hz, 3 H), 1.37 - 1.53 (m, 1H), 1.73 - 1.86 (m, 2H), 1.94 - 2.07 (m, 1H), 2.21 - 2.35 (m, 2 H), 2.35 - 2.48 (m, 1H), 2.68 - 2.91 (m, 2 H), 2.98 - 3.11 (m, 1 H). 3.18 - 329 (m, 1H), 7.14 - 7.20 (m, 2 H), 7.38-7.48 (m, 2 H).

**Step C: Preparation of Intermediate (*R*)-4-(2-(2-Methylpyrrolidin-1-yl)ethyl)phenylboronic Acid.**

(*R*)-1-(4-Bromophenethyl)-2-methylpyrrolidine (2.16 g, 8.04 mmol) was dissolved in THF (20 mL) under argon. The reaction mixture was cooled to -78˚C and *n*-butyl lithium (1.6 M in hexanes, 6.53 mL, 10.4 mmol) was added slowly. After 90 min of stirring, triisopropylborate (7.42 mL, 32.1 mmol) was added. The reaction was kept at -78˚C for 2 hours. It was allowed to warm to room temperature and stirred for 1.5 h. The cloudy reaction mixture was quenched with 40 mL of 1 M HCl. THF was removed under reduced pressure. The remainmg aqueous solution was made basic (pH~8) with 50% sodium hydroxide and extracted twice with ethyl acetate (50 mL each), plus three times with DCM (50 mL each). The combined organics were dried over $MgSO_4$, filtered, and concentrated to give 1.70 g of a yellow foam. The foam was triturated with $Et_2O$ (2 × 20 mL), and dried under high vacuum to give the title compound (1.19 g, 64% yield) as a pale yellow solid. Exact mass calculated for $C_{13}H_{20}BNO_2$: 233.2, Found: LCMS *m/z* = 234.2 ($M+H^+$); $^1$H NMR (400 MHz, Methanol-$d_4$) δ ppm 1.25 (d, *J* = 6.32 Hz, 3 H), 1.49 - 1.61 (m, 1H), 1.80 - 1.97 (m, 2 H), 2.04 - 2.18 (m, 1H), 2.61 - 2.74 (m, 2 H), 2.76 - 2.98 (m, 3 H), 3.19 - 3.45 (m, 2 H), 7.16 (d, 2 H), 7.48 - 7.62 (m, 2 H).

**Example 1.2: Preparation of {4'-[2-((*R*)-2-Methyl-pyrrolidin-1-yl)-ethyl]-biphenyl-4-sulfonyl}-acetic Acid Methyl Ester (Compound 19).**

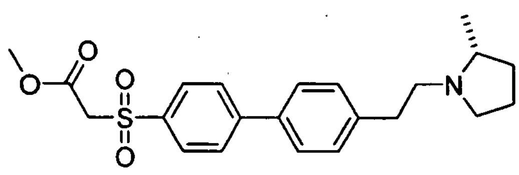

**Step A: Preparation of Intermediate 2-Biphenyl-4-yl-ethanol.**

To a 1000 mL round-bottomed flask containing 4-biphenylacetic acid (20.0 g, 94.2 mmol) in THF (250 mL) at 0 ˚C was added borane THF complex (240 mL, 240 mmol) via an addition funnel. The reaction was then heated to 65 ˚C for 3 h and then cooled to 0 ˚C. The reaction was slowly quenched with MeOH (250 mL) and concentrated. The product was diluted with EtOAc (500 mL) and washed with 1 M HCl (200 mL), saturated $NaHCO_3$ (200 mL) and brine (200 mL). The organics were dried with $MgSO_4$, filtered and concentrated to afford the title compound (18.0 g, 96% yield) as a white solid. $^1$H NMR (400 MHz, $CDCl_3$) δ ppm 2.92 (t, *J* = 6.44 Hz, 2 H), 3.91 (t, *J* = 6.57 Hz, 2 H), 7.29 - 7.37 (m, 3 H), 7.40 - 7.47 (m, 2 H), 7.52 - 7.61 (m, 4 H).

**Step B: Preparation of Intermediate Methanesulfonic Acid 2-Biphenyl-4-yl-ethyl Ester.**

To a 250 mL round-bottomed flask containing 2-biphenyl-4-yl-ethanol (5.00 g, 25.2 mmol) in DCM (50 mL) was added triethylamine (3.52 mL, 25.2 mmol) and mesyl chloride (2.16 mL, 27.7 mmol). This was stirred at -30˚C and allowed to warm to 25˚C over 2 h. The reaction was filtered and diluted with EtOAc (100 mL). This was washed with water (50 mL), 1M HCl (50 mL), saturated $NaHCO_3$ (50 mL) and brine (50 mL). The organics were dried with $MgSO_4$, filtered and concentrated to afford the title compound (5.93 g, 85% yield) as a cream colored solid. $^1$H NMR (400 MHz, $CDCl_3$) δ ppm 2.89 (s, 3 H), 3.11 (t, *J* = 6.82 Hz, 2 H), 4.46 (t, *J*= 6.95 Hz, 2 H), 7.29 - 7.34 (m, 2 H), 7.34 - 7.38 (m, 1H), 7.41 - 7.47 (m, 2 H), 7.54 - 7.60 (m, 4 H).

**Step C: Preparation of Intermediate (*R*)-1-(2-Biphenyl-4-yl-ethyl)-2-methyl-pyrrolidine.**

To a 250 mL three-neck round-bottomed flask equipped with a condenser was charged sodium carbonate (14.6 g, 137.4 mol) and (R)-2-methyl-pyrrolidine hydrochloride (5.57g, 45.8 mmol) in acetonitrile (30 mL). The mixture was allowed to stir at 25 ˚C for 10 minutes, and methanesulfonic acid 2-biphenyl-4-yl-ethyl ester (14.5 g, 52.7 mmol) in acetonitrile (50 mL) was added. The mixture was then heated to reflux and stirred for 16 h. The reaction was filtered and concentrated to yield a dark brown oil. This was dissolved in EtOAc (125 mL) and extracted with 1 M HCl (4 × 25 mL). The aqueous layers were combined and made basic by addition of 50% NaOH (20 mL). The aqueous layer was extracted with DCM (7 × 25 mL), the organic layers were combined, dried over $Na_2SO_4$, filtered and concentrated. The crude reaction mixture was purified by column chromatography (Biotage column 65M, 2-20%MeOH/DCM) to afford the title compound (10.1 g, 82%) as an orange oil. Exact mass calculated for $C_{19}H_{23}N$: 265.2, Found: LCMS *m/z* = 266.1 ($M+H^+$); $^1$H NMR (400 MHz, $CDCl_3$) δ ppm 1.13 (d, *J* = 6.06 Hz, 3 H), 1.40 - 1.51 (m, 1H), 1.66 - 1.78 (m, 1H), 1.78 - 1.88 (m, 1H), 1.88 - 1.99 (m, 1H), 2.21 (q, *J* = 8.84 Hz, 1H), 2.28 - 2.39 (m, 2 H), 2.79 - 2.94 (m, 2 H), 3.07 (td, *J* = 11.24, 6.06 Hz, 1H), 3.27 (dt, $J_1$ = 8.65, $J_2$ = 2.65 Hz, 1H), 7.29 (d, *J* = 8.34 Hz, 2 H), 7.31 - 7.35 (m, 1H), 7.38 - 7.46 (m, 2 H), 7.49 - 7.54 (m, 2 H), 7.55 - 7.62 (m, 2 H).

**Step D: Preparation of Intermediate 4'-[2-((*R*)-2-Methyl-pyrrolidin-1-yl)-ethyl]-biphenyl-4-sulfonyl chloride.**

(*R*)-1-(2-Biphenyl-4-yl-ethyl)-2-methyl-pyrrolidine (0.703 g, 2.65 mmol) in a 100 mL round-bottomed flask was dissolved in DCM (10 mL) and cooled to 0 ˚C. Chlorosulfonic acid (1.76 mL, 26.5 mmol) in DCM (10 mL) was added dropwise over 10 min. The reaction was allowed to stir for 3 h while slowly warming to 25˚C. The reaction was quenched by dropwise addition into an ice-cold solution of 50% saturated $NaHCO_3$ (10 mL) adding additional ice to keep the solution cold. This was diluted with DCM (20 mL) and the layers were separated. The aqueous layer was extracted with DCM (2 × 40 mL). The combined organics, were dried over $Na_2SO_4$, filtered and concentrated to afford the title compound (0.74 g, 74%) as a yellow solid. Exact mass calculated for $C_{19}H_{22}NO_2S$: 363.1, Found: LCMS *m/z* = 364.1 ($M+H^+$); $^1$H NMR (400 MHz, DMSO-$d_6$) δ ppm 1.21 (d, *J* = 7.07 Hz, 0.3 H), 1.40 (d, *J* = 6.57 Hz, 2.7 H), 1.62 (s, I H), 1.96 (s, 2 H), 2.14 - 2.27 (m, 1H), 2.98 - 3.12 (m, 2 H), 3.14 - 3.28 (m, 2 H), 3.44 (s, 1H), 3.49 - 3.59 (m, I H), 3.59 - 3.69 (m, 1H), 7.42 (d, *J* = 8.34 Hz, 2 H), 7.59 - 7.64 (m, 2 H), 7.64-7.70 (m, 4 H).

**Step E: Preparation of Intermediate 4'-[2-((*R*)-2-Methyl-pyrrolidin-1-yl)-ethyl]-biphenyl-4-sulfinic acid, Sodium salt.**

A 20 mL scintillation vial was charged with (*R*)-4'-[2-(2-methyl-pyrrolidin-1-yl)-ethyl]-biphenyl-4-sulfonyl chloride (1.00 g, 2.50 mmol), sodium sulfite (0.598 g, 4.75 mmol), and sodium bicarbonate (0.629 g, 7.49 mmol). Then water (9 mL) was added and the resulting mixture was stirred at 80˚C for 3 h. The reaction was filtered and washed with water (3 mL) and hexane (10 mL). The cream colored solid was dried overnight under vacuum to afford the title compound (0.660 g, 40%).

**Step F: Preparation of {4'-[2-((*R*)-2-Methyl-pyrrolidin-1-yl)-ethyl]-biphenyl-4-sulfonyl}-acetic Acid Methyl Ester.**

To a 20 mL scintillation vial was added (*R*)-4'-[2-(2-Methyl-pyrrolidin-1-yl)-ethyl]-biphenyl-4-sulfinic acid, sodium salt (125 mg, 356 μmol) DMSO (0.5 mL) and methyl 2-bromoacetate (43.9 μl, 462 μmol). This was stirred at 25˚C for 7 days. To this was added water (20 mL) and DCM (20 mL). The resulting mixture was filtered, the layers separated and the aqueous layer was extracted with DCM (20 mL). The combined organic layers were washed with water (5 mL), dried with $Na_2SO_4$, filtered and concentrated. This was recrystallized from DCM/$Et_2O$, filtered and dried under vacuum. This was dissolved in DCM (2 mL) and 1 M HCl in $Et_2O$ (1.0 mL) was added. This was filtered and dried under vacuum to afford the title compound (0.082 g, 50% yield) as a pale yellow solid. Exact mass calculated for $C_{22}H_{27}NO_4S$: 401.2, Found: LCMS *m/z* = 402.2 ($M+H^+$); $^1$H NMR (400 MHz, Methanol-$d_4$) δ ppm 1.33 (d, *J* = 6.82 Hz, 1H), 1.49 (d, *J*= 6.32

Hz, 2 H), 1.71 - 1.82 (m, 1H), 2.01 - 2.25 (m, 2 H), 2.30-2.42 (m, 1H), 3.06 - 3.24 (m, 2 H), 3.24 - 3.37 (m, 2 H), 3.53 - 3.60 (m, I H), 3.61 - 3.72 (m, 4 H), 3.73 - 3.81 (m, 1H), 4.40 (s, 2 H), 7.49 *(d,* $J$ = 8.34 Hz, 2 H), 7.72 (d, $J$ = 8.34 Hz, 2 H), 7.88 (d, $J$ = 8.34 Hz, 2 H), 8.00 (d, $J$ = 8.59 Hz, 2 H).

**Example 1.3: Preparation of 14'-[2-((*R*)-2-Methyl-pyrrolidin-1-yl)-ethyl]-biphenyl-4-sulfonyl}-acetic Acid *tert*-Butyl Ester (Compound 18).**

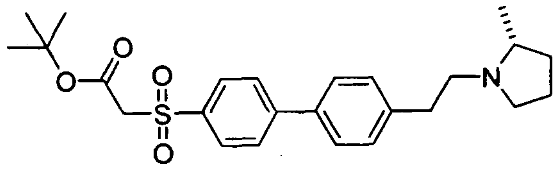

To a 20 mL scintillation vial was added (*R*)-4'-[2-(2-methyl-pyrrolidin-1-yl)-ethyl]-biphenyl-4-sulfinic acid, sodium salt (200 mg, 569 μmol), DMSO (2.0 mL) and *tert*-butyl 2-bromoacetate (109 μl, 740 μmol). This was stirred at 25°C for 3 days. To this was added water (20 mL) and dichloromethane (20 mL). The resulting mixture was filtered, the layers separated and the aqueous layer was extracted with DCM (20 mL). The combined organic layers were washed with water (5 mL), dried with $Na_2SO_4$, filtered and concentrated. The crude mixture was purified by prep HPLC (0.1% TFA in acetonitrile/0.1% TFA in water). The fractions were neutralized with saturated potassium carbonate (10 mL) and extracted with DCM (5 x 20 mL). The organics were dried with $Na_2SO_4$, filtered and concentrated to afford the title compound (326 mg, 40% yield) as a white waxy solid. Exact mass calculated for $C_{25}H_{33}NO_4S$: 443.6, Found: LCMS $m/z$ = 444.6 ($M+H^+$).

**Example 1.4: Preparation of 6-{4-[2-((*R*)-2-Methyl-pyrrolidin-1-yl)-ethyl]-phenyl}-1,1-dioxo-1$\lambda^6$-thiochroman-4-one (Compound 7).**

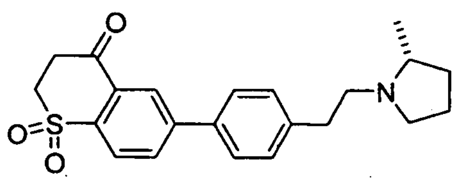

To a 5 mL Smith vial containing (*R*)-4-[2-(2-methylpyrrolidin-1-yl)ethyl]phenylboronic acid (0.182 g, 0.78 mmol) in THF (4 mL) was added 6-chloro-1,1-dioxo-1$\lambda^6$-thiochroman-4-one (0.150 g, 0.650 mmol), potassium acetate (0.414 g, 1.95 mmol), 2-dicyclohexylphosphino-2',4',6'-tri-*iso*-propyl-biphenyl (X-Phos) (0.016 g, 0.033 mmol) and palladium (II)acetate (0.0029 g, 0.013 mmol). This was heated to 120 °C using a Smith Synthesizer (microwave) for 45 min. The reaction was filtered through celite, rinsing with EtOAc (10 mL). The crude was concentrated and purified by preparative HPLC (0.1% TFA in acetonitrile/0.1% TFA in water). The fractions were lyophilized to afford the title compound (0.189 g, 61% yield) as an oil. Exact mass calculated for $C_{22}H_{25}NO_3S$: 383.2, Found: LCMS $m/z$ = 384.2 ($M+H^+$); $^1$H NMR (400 MHz, Methanol-$d_4$) δ ppm 1.33 (d, $J$ = 6.82 Hz, 1H), 1.49 (d, $J$ = 6.57 Hz, 2 H), 1.70 - 1.83 (m, 1H), 2.01 - 2.23 (m, 2 H), 2.29 - 2.41 (m, 1H), 3.05 - 3.23 (m, 2 H), 3.23 - 3.30 (m, 1 H), 3.30 - 3.33 (m, 2 H), 3.37 (t, $J$ = 3.37 Hz, 2 H), 3.48 - 3.59 (m, 1 H), 3.61 - 3.69 (m, I H), 3.73 - 3.83 (m, 1H), 3.86 (t, $J$ = 6.01 Hz, 2 H), 7.47 (d, $J$ = 8.34 Hz, 2 H), 7.67 (d, $J$ = 8.34 Hz, 2 H), 7.99 (d, $J$ = 8.18 Hz, 1H), 8.08 (dd, $J_1$ = 8.17 Hz, $J_2$ = 1.93 Hz, 1H), 8.25 (d, $J$ = 2.02 Hz, 1H).

**Example 1.5: Preparation of (*R*)-1-{2-[4'-(2-Methoxy-ethanesulfonyl)-biphenyl-4-yl]-ethyl}-2-methyl-pyrrolidine (Compound 3).**

**Step A: Preparation of Intermediate (4-Bromophenyl)(2-methoxyethyl)sulfane.**

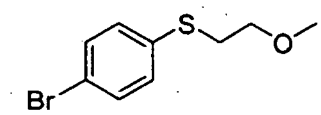

To a solution of 4-bromobenzenethiol (2.50 g, 12.6 mmol) in DMF (15 mL) was added sodium hydride (60% dispersion in mineral oil) (0.754 g, 18.8 mmol) followed by 1-bromo-2-methoxyethane (2.62 g, 18.8 mmol). The resulting mixture was stirred for 18 hours at room temperature. The reaction was diluted with water and extracted twice with EtOAc. Purification by flash chromatography on silica gel (0-5% EtOAc in hexane) yielded the title compound (2.58 g, 83% yield) as a clear oil. $^1$H NMR (400 MHz, Methanol-$d_4$) δ ppm 3.11 (t, *J*= 6.44 Hz, 2 H), 3.28 - 3.31 (m, 3 H), 3.56 (t, *J* = 6.44 Hz, 2 H), 7.28 (d, *J* = 8.59 Hz, 2 H), 7.43 (d, *J* = 8.59 Hz, 2 H).

**Step B: Preparation of Intermediate 1-Bromo-4-(2-methoxyethylsulfonyl)benzene.**

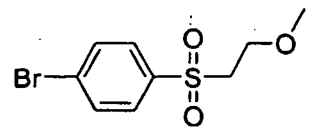

To a solution of (4-bromophenyl)(2-methoxyethyl)sulfane (2.58 g, 10.4 mmol) in DCM (25 mL) was added 3-chloroperoxybenzoic acid (77% max.) (4.91 g, 21.9 mmol). The resulting mixture was stirred for 4 hours at room temperature. The reaction was basified with 1 N NaOH and extracted 3 times with EtOAc. The organics were dried over $MgSO_4$, filtered, and concentrated to afford the title compound (2.91 g, 100% yield) as a clear oil. $^1$H NMR (400 MHz, Methanol-$d_4$) δ ppm 3.17 (s, 3 H), 3.49 (t, *J*= 5.81 Hz, 2 H), 3.71 (t, *J* = 5.81 Hz, 2 H), 7.73 - 7.87 (m, 4H).

**Step C: Preparation of (*R*)-1-{2-[4'-(2-Methoxy-ethanesulfonyl)-biphenyl-4-yl]-ethyl}-2-methyl-pyrrolidine.**

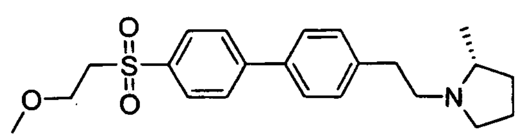

To a heavy-walled vial was added 1-bromo-4-(2-methoxyethylsulfonyl)benzene (320 mg, 1.15 mmol), (*R*)-4-[2-(2-methylpyrrolidin-1-yl)ethyl]phenylboronic acid (267 mg, 1.15 mmol), aq. $Na_2CO_3$ (2 M solution, 1.15 mL, 2.29 mmol), and tetrakis(triphenylphosphine)palladium(0) (33.1 mg, 0.029 mmol) in a mixture of EtOH (0.75 mL) and benzene (2.25 mL). The resulting reaction mixture was heated under microwave irradiation at 100˚C for 60 minutes. The reaction mixture was diluted with water and the organics separated. The aqueous layer was extracted with EtOAc. The combined organics were concentrated, dissolved in DMSO, and purified by HPLC (0.1% TFA in acetonitrile/0.1% TFA in water). The combined fractions were basified with 1 N NaOH and extracted 3 times with EtOAc. The combined organics were dried over $MgSO_4$, filtered, and concentrated. The residue was dissolved in MeOH (2 mL). Then, HCl (1M in $Et_2O$ 0.72 mL) was added followed by EtOAc (5 mL). The resulting mixture was concentrated to afford the hydrochloride salt of the title compound (303 mg, 62% yield) as a white solid. Exact mass calculated for $C_{22}H_{29}NO_3S$: 387.2, Found: LCMS *m*/*z* = 388.4 (M+H$^+$); $^1$H NMR (400 MHz, Methanol-$d_4$) δ ppm 1.33 (d, *J* = 6.82 Hz, 0.3 H), 1.50 (d, *J* = 6.32 Hz, 2.7 H), 1.70 - 1.86 (m, 1H), 2.00 - 2.25 (m, 2 H), 2.28 - 2.43 (m, 1H), 3.04 - 3.29 (m, 7 H), 3.49 - 3.58 (m, 3 H), 3.58 - 3.70 (m, 1H), 3.70 - 3.84 (m, 3 H), 7.48 (d, *J* = 7.83 Hz, 2 H), 7.70 (d, *J* = 7.07 Hz, 2 H), 7.86 (d, *J* = 7.83 Hz, 2 H), 7.97 (d, *J*= 7.83 Hz, 2H).

**Example 1.6: Preparation of (*R*)-2-Methyl-1-{2-[4'-(propane-1-sulfonyl)-biphenyl-4-yl]-ethyl}-pyrrolidine (Compound 5).**

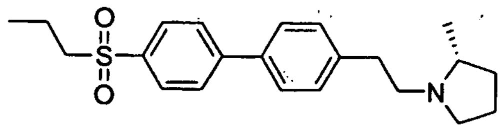

The title compound was prepared in a similar manner as described in **Example 1.5, Step C**, using 1-bromo-4-(propylsulfonyl)benzene (274 mg, 1.04 mmol) and (*R*)-4-[2-(2-methylpyrrolidin-1-yl)ethyl]phenylboronic acid (243 mg, 1.04 mmol) as starting materials to give a white solid in 52% yield. Exact mass calculated for $C_{22}H_{29}NO_2S$: 371.2, Found: LCMS *m/z* = 372.3 (M+H$^+$); $^1$H NMR (400 MHz, Methanol-$d_4$) δ ppm 1.01 (t, *J*= 7.45 Hz, 3 H), 1.33 (d, *J* = 6.82 Hz, 0.3 H), 1.49 (d, *J*= 6.57 Hz, 2.7 H), 1.65 - 1.82 (m, 3 H), 2.02 - 2.22 (m, 2 H), 2.28 - 2.41 (m, 1H), 3.03 - 3.30 (m, 6 H), 3.49 - 3.58 (m, 1H), 3.59 - 3.71 (m, I H), 3.71 - 3.83 (m, 1H), 7.38 - 7.54 (m, 2 H), 7.72 (d, *J*= 8.08 Hz, 2 H), 7.81 - 7.93 (m, 2 H), 7.93 - 8.05 (m, 2 H).

**Example 1.7: Preparation of (*R*)-2-Methyl-1-[2-(4'-phenylmethanesulfonyl-biphenyl-4-yl)-ethyl]-pyrrolidine (Compound 6).**

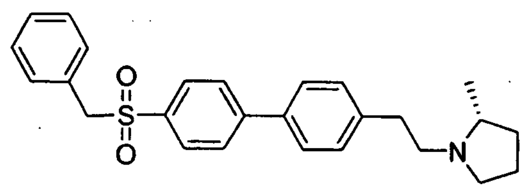

The title compound was prepared in a similar manner as described in **Example 1.5, Step C,** using 1-(benzylsulfonyl)-4-bromobenzene (115 mg, 0.370 mmol) and (*R*)-4-[2-(2-methylpyrrolidin-1-yl)ethyl]phenylboronic acid (86.1 mg, 0.370 mmol) as starting materials to give a yellow solid in 50% yield. Exact mass calculated for $C_{26}H_{29}NO_2S$: 419.2, Found: LCMS *m/z* = 420.4 (M+H$^+$); $^1$H NMR (400 MHz, Methanol-$d_4$) δ ppm 1.33 (d, *J*= 7.07 Hz, 0.3 H), 1.48 (d, *J* = 6.32 Hz, 2.7 H), 1.68 - 1.82 (m, 1H), 2.00 - 2.25 (m, 2 H), 2.28 - 2.41 (m, 1H), 3.04 - 3.28 (m, 4 H), 3.46 - 3.59 (m, 1H), 3.58 - 3.69 (m, I H), 3.70 - 3.82 (m, I H), 4.53 (s, 2 H), 7.07 - 7.20 (m, 2 H), 7.21 - 7.37 (m, 3 H), 7.46 (d, *J*= 8.08 Hz, 2 H), 7.59 - 7.82 (m, 6 H).

**Example 1.8: Preparation of (*R*)-1-{2-[4'-(3-Methoxy-propane-1-sulfonyl)-biphenyl-4-yl]-ethyl}-2-methyl-pyrrolidine (Compound 8).**

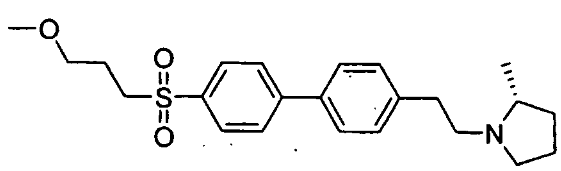

The title compound was prepared in a similar manner as described in **Example 1.5**, **Step C**, using 1-bromo-4-(3-methoxypropylsulfonyl)benzene (81.0 mg, 0.276 mmol) and (*R*)-4-[2-(2-methylpyrrolidin-1-yl)ethyl]phenylboronic acid (64.4 mg, 0.276 mmol) as starting materials to give a clear solid in 53% yield. Exact mass calculated for $C_{23}H_{31}NO_3S$: 401.2, Found: LCMS *m/z* = 402.2 (M+H$^+$); $^1$H NMR (400 MHz, Methanol-$d_4$) δ ppm 1.45 (d, *J*= 6.32 Hz, 3 H), 1.67 - 1.83 (m, 1H), 1.83 - 1.98 (m, 2 H), 2.01 - 2.18 (m, 2 H), 2.23 - 2.40 (m, 1H), 2.99 - 3.29 (m, 9 H), 3.38 - 3.53 (m, 3 H), 3.53

- 3.63 (m, 1H), 3.64 - 3.76 (m, 1H), 7.47 (d, *J*= 8.34 Hz, 2 H), 7.71 (d, *J*= 8.08 Hz, 2 H), 7.89 (d, *J*= 8.34 Hz, 2 H), 7.94 - 8.02 (m, 2 H).

**Example 1.9: Preparation of (*R*)-2-Methyl-1-{2-[4'-(2-methyl-propane-1-sulfonyl)-biphenyl-4-yl]-ethyl}-pyrrolidine (Compound 9).**

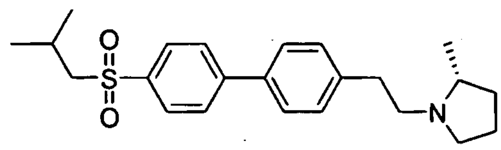

The title compound was prepared in a similar manner as described in **Example 1.5**, **Step C**, using 1-bromo-4-(isobutylsulfonyl)benzene (189 mg, 0.682 mmol) and (*R*)-4-[2-(2-methylpyrrolidin-1-yl)ethyl]phenylboronic acid (159 mg, 0.682 mmol) as starting materials to give a yellow oil in 41% yield. Exact mass calculated for $C_{23}H_{31}NO_2S$: 385.2, Found: LCMS *m/z* = 386.1 ($M+H^+$); $^1H$ NMR (400 MHz, Methanol-$d_4$) δ ppm 1.06 (d, *J*= 6.82 Hz, 6 H), 1.48 (d, *J* = 6.57 Hz, 3 H), 1.70 - 1.86 (m, 1H), 2.03 - 2.23 (m, 3 H), 2.27 - 2.41 (m, 1H), 3.07 - 3.28 (m, 6 H), 3.52 - 3.68 (m, 2 H), 3.67 - 3.81 (m, 1H), 7.47 (d, *J*= 8.08 Hz, 2 H), 7.70 (d, *J*= 8.08 Hz, 2 H), 7.87 (d, *J* = 8.34 Hz, 2 H), 7.93 - 8.03 (m, 2 H).

**Example 1.10: Preparation of 2-{4'-[2-((*R*)-2-Methyl-pyrrolidin-1-yl)-ethyl]-biphenyl-4-sulfonyl}-ethanol (Compound 10).**

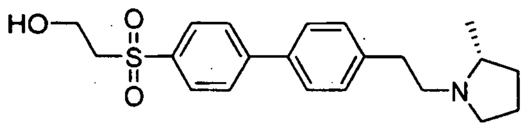

The title compound was prepared in a similar manner as described in **Example 1.5**, **Step C**, using 2-(4-bromophenylsulfonyl)ethanol (238 mg, 0.898 mmol) and (*R*)-4-[2-(2-methylpyrrolin-1-yl)ethyl]phenylboronic acid (209 mg, 0.898 mmol) as starting materials to give a white solid in 34% yield. Exact mass calculated for $C_{21}H_{27}NO_3S$: 373.2, Found: LCMS *m/z* = 374.2 ($M+H^+$); $^1H$ NMR (400 MHz, Methanol-$d_4$) δ ppm 1.33 (d, 0.3 H), 1.49 (d, *J*= 6.32 Hz, 2.7 H). 1.70 - 1.85 (m, 1H), 2.02 - 2.20 (m, 2 H), 2.28 - 2.43 (m, 1H), 3.05 - 3.28 (m, 4 H), 3.45 (t. *J* = 6.19 Hz, 2 H), 3.50 - 3.59 (m, 1H), 3.59 - 3.68 (m, I H), 3.73 - 3.82 (m, 1H), 3.89 (t, *J* = 6.19 Hz, 2H), 7.47 (d, *J*= 8.08 Hz, 2 H), 7.70 (d, *J*= 8.08 Hz, 2 H), 7.87 (d, *J*= 8.34 Hz, 2 H), 7.99 (d, *J* = 8.34 Hz, 2 H).

**Example 1.11: Preparation of {4'-[2-((*R*)-2-Methyl-pyrrolidin-1-yl)-ethyl]-biphenyl-4-sulfonyl}-acetic Acid Ethyl Ester (Compound 11).**

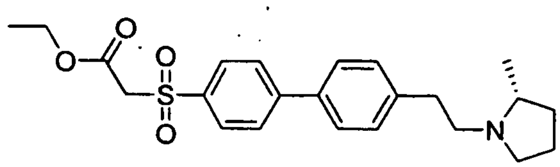

The title compound was prepared in a similar manner as described in **Example 1.5**, **Step C**, using ethyl 2-(4-bromophenylsulfonyl)acetate (207 mg, 0.674 mmol) and (*R*)-4-[2-(2-methylpyrrolidin-1-yl)ethyl]phenylboronic acid (157 mg, 0.674 mmol) as starting materials to give a clear solid in 6% yield. Exact mass calculated for $C_{23}H_{29}NO_4S$: 415.2, Found: LCMS *m/z* = 416.7 ($M+H^+$); $^1H$ NMR (400 MHz, Methanol-$d_4$) δ ppm 1.15 (m, 3 H), 1.33 (d, *J* = 6.82 Hz, 0.3 H), 1.50 (d, *J*= 6.57 Hz, 2.7 H), 1.70 - 1.84 (m, 1H), 2.00 - 2.23 (m, 2H), 2.29 - 2.43 (m, 1 H), 3.05 - 3.28 (m, 4 H), 3.50 -

3.59 (m, 1H), 3.59 - 3.70 (m, 1H), 3.71 - 3.82 (m; 1H), 4.11 (q, *J* = 7.07 Hz, 2 H), 4.38 (s, 2 H), 7.48 (d, *J*= 8.34 Hz, 2 H), 7.71 (d, *J* = 8.34 Hz, 2 H), 7.88 (d, *J* = 8.59 Hz, 2 H), 8.00 (d, *J* = 8.59 Hz, 2 H).

**Example 1.12: Preparation of (*R*)-1-[2-(4'-Cyclopentanesulfonyl-biphenyl-4-yl)-ethyl]-2-methyl-pyrrolidine (Compound 14).**

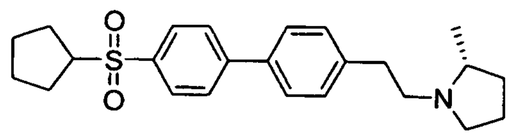

The title compound was prepared in a similar manner as described in **Example 1.5**, **Step C**, using 1-bromo-4-(cyclopentylsulfonyl)benzene (250 mg, 0.864 mmol) and (*R*)-4-[2-(2-methylpyrrolidin-1-yl)ethyl]phenylboronic acid (202 mg, 0.864 mmol) as starting materials to give a white solid in 40% yield. Exact mass calculated for $C_{24}H_{31}NO_2S$: 397.2, Found: LCMS m/z = 398.4 ($M+H^+$); $^1H$ NMR (400 MHz, Methanol-$d_4$) δ ppm 1.48 (d, *J* = 6.57 Hz, 3 H), 1.59 - 1.80 (m, 5 H), 1.84 - 1.96 (m, 2 H), 1.96 - 2.20 (m, 4 H), 2.27 - 2.42 (m, 1H), 2.99 - 3.29 (m, 3 H), 3.42 - 3.59 (m, 2 H), 3.59 - 3.81 (m, 3 H), 7.48 (d, *J* = 8.08 Hz, 2 H), 7.73 (d, *J* = 8.34 Hz, 2 H), 7.84 - 7.92 (m, 2 H), 7.93 - 8.04 (m, 2 H).

**Example 1.13: Preparation of (*R*)-1-[2-(4'-Methanesulfonyl-biphenyl-4-yl)-ethyl]-2-methyl-pyrrolidine (Compound 1).**

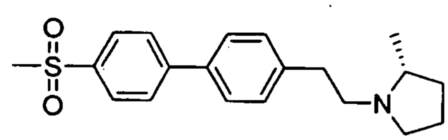

To a heavy-walled vial was added (*R*)-1-(2-(4-bromo-phenyl)-ethyl]-2-methyl-pyrrolidine (200 mg, 0.746 mmol), 4-(methylsulfonyl)phenylboronic acid (194 mg, 0.969 mmol), aq. $Na_2CO_3$ (0.746 mL, 1.49 mmol, 2 M solution), and tetrakis(triphenylphosphine)palladium(0) (21.5 mg, 0.019 mmol) in a mixture of EtOH (0.75 mL) and benzene (2.25 mL). The resulting reaction mixture was heated under microwave conditions at 100 °C for 30 minutes. The reaction mixture was diluted with water and the organic phase was separated. The aqueous layer was extracted with EtOAc. The combined organics were concentrated, dissolved in DMSO, and purified by HPLC (0.1% TFA in acetonitrile/0.1% TFA in water). The combined fractions were basified with 1 N NaOH and extracted 3 times with EtOAc. The combined organics were dried over $MgSO_4$ filtered, and concentrated. The residue was dissolved in MeOH (2 mL). Then, HCl (1 M in $Et_2O$ 0.72 mL) was added followed by EtOAc (5 mL). The resulting mixture was concentrated to afford the hydrochloride salt of the title compound (70.0 mg, 25% yield) as a white solid. Exact mass calculated for $C_{20}H_{25}NO_2S$: 343.2, Found: LCMS *m*/*z* = 344.2 ($M+H^+$); $^1H$ NMR (400 MHz, Methanol-$d_4$) δ ppm 1.33 (d, *J*= 6.82 Hz, 0.3 H), 1.48 (d, *J*= 6.57 Hz, 2.7 H), 1.70 - 1.82 (m, 1 H), 2.02 - 2.20 (m, 2 H), 2.30 - 2.40 (m, 1H), 3.06 - 3.22 (m, 4 H), 3.23 - 3.33 (m, 3 H), 3.46 - 3.57 (m, 1H), 3.59 - 3.69 (m, 1H), 3.71 - 3.84 (m, 1H), 7.48 (d, *J*= 8.34 Hz, 2 H), 7.70 (d, *J*= 8.34 Hz, 2 H), 7.87 (d, *J*= 8.59 Hz, 2 H), 8.01 (d, *J*= 8.59 Hz, 2 H).

**Example 1.14: Preparation of (*R*)-2-Methyl-1-{2-[4'-(propane-2-sulfonyl)-biphenyl-4-yl]-ethyl}-pyrrolidine (Compound 4).**

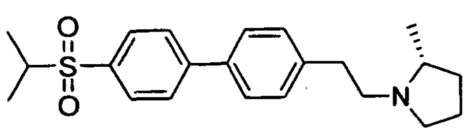

The title compound was prepared in a similar manner as described in **Example 1.13**, using (*R*)-1-[2-(4-bromo-phenyl)-ethyl]-2-methyl-pyrrolidine (200 mg, 0.746 mmol) and 4-(isopropylsulfonyl)phenylboronic acid (170 mg, 0.746 mmol) as starting materials to give a clear solid in 58% yield. Exact mass calculated for $C_{22}H_{29}NO_2S$: 371.2, Found: LCMS *m/z* = 372.3 (M+H$^+$); $^1$H NMR (400 MHz, Methanol-$d_4$) δ ppm 1.20 - 1.33 (m, 6 H), 1.48 (d, *J* = 6.06 Hz, 3 H), 1.70 - 1.87 (m, 1H), 2.04 - 2.22 (m, 2 H), 2.30 - 2.42 (m, 1H), 3.01 - 3.29 (m, 3 H), 3.32 - 3.46 (m, 2 H), 3.48 - 3.82 (m, 3 H), 7.48 (d, *J*= 7.83 Hz, 2 H), 7.70 (d, *J* = 7.83 Hz, 2 H), 7.80 - 8.05 (m, 4 H).

**Example 1.15: Preparation of (*R*)-1-[2-(4'-Ethanesulfonyl-biphenyl-4-yl)-ethyl]-2-methyl-pyrrolidine (Compound 2).**

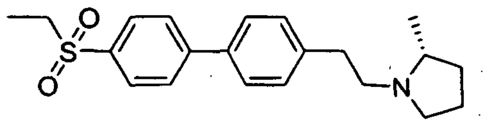

The title compound was prepared in a similar manner as described in **Example 1.5**, **Step C,** using 1-bromo-4-(ethylsulfonyl)benzene (365 mg, 1.465 mmol) and (*R*)-4-(2-(2-methylpyrrolidin-1-yl)ethyl)phenylboronic acid (342mg, 1.465 mmol) as starting materials to give a clear solid in 48% yield. Exact mass calculated for $C_{21}H_{27}NO_2S$: 357.2, Found: LCMS *m/z* = 358.1 (M-H$^+$); $^1$H NMR (400 MHz, Methanol-$d_4$) δ ppm 1.27 (t, *J*= 7.33 Hz, 3 H), 1.35 (d, *J* = 7.07 Hz, 0.3 H), 1.50 (d, *J* = 6.57 Hz, 2.7 H), 1.72 - 1.83 (m, I H), 2.03 - 2.23 (m, 2 H), 2.33 - 2.43 (m, 1H), 3.07 - 3.23 (m, 2 H), 3.23 - 3.32 (m, 4 H), 3.51 - 3.61 (m, I H), 3.63 - 3.72 (m, 1H), 3.75 - 3.83 (m, 1H), 7.49 (d, *J*= 8.34 Hz, 2 H), 7.73 (d, *J*= 8.34 Hz, 2 H), 7.88 - 7.92 (m, 2 H), 7.97 - 8.01 (m, 2 H).

**Example 1.16: Preparation of (*R*)-1-{2-[4'-(2-Ethoxy-ethanesulfonyl)-biphenyl-4-yl]-ethyl}-2-methyl-pyrrolidine (Compound 12).**

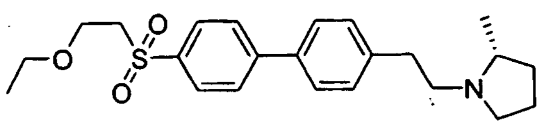

The title compound was prepared in a similar manner as described in **Example 1.5**, **Step C**, using 1-bromo-4-(2-ethoxyethylsulfonyl)benzene (210 mg, 0.716 mmol) and (*R*)-4-(2-(2-methylpyrrolidin-1-yl)ethyl)phenylboronic acid (167 mg, 0.716 mmol) as starting materials to give a clear solid in 13% yield. Exact mass calculated for $C_{23}H_{31}NO_3S$: 401.2, Found: LCMS *m/z* = 402.2 (M+H$^+$); $^1$H NMR (400 MHz, Methanol-$d_4$) δ ppm 0.95 (t, *J* = 7.07, 3 H), 1.33 (d, *J* = 6.57, 0.3 H), 1.49 (d, *J* = 6.57, 2.7 H), 1.71 - 1.82 (m, I H), 2.03 - 2.20 (m, 2 H), 2.31 - 2.40 (m, 1H), 3.07 - 3.23 (m, 2 H), 3.24 - 3.29 (m, I H), 3.32 - 3.37 (m, 3 H), 3.49 - 3.60 (m, 3 H), 3.60 - 3.70 (m, 1H), 3.73 - 3.82 (m, 3 H), 7.48 (d, *J* = 8.34 Hz, 2 H), 7.70 (d, *J* = 8.34 Hz, 2 H), 7.85 (d, *J* = 8.59 Hz, 2 H), 7.98 (m, 2 H).

**Example 1.17: Preparation of (*R*)-1-{2-[3'-(2-Methoxy-ethanesulfonyl)-biphenyl-4-yl]-ethyl}-2-methyl-pyrrolidine (Compound 16).**

**Step A: Preparation of Intermediate (3-Bromophenyl)(2-methoxyethyl)sulfane.**

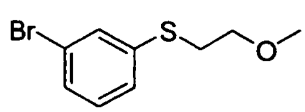

To a suspension of sodium hydride (96.6 mg, 2.42 mmol) in DMF (4.2 mL) was added 3-bromobenzenethiol

(0.22 mL, 2.132 mmol). After stirring at room temperature for 30 min, 1-bromo-2-methoxyethane (0.22 mL, 2.341 mmol) was added to the reaction mixture. After stirring for 17 h, the reaction mixture was quenched with $H_2O$ and extracted with EtOAc. The combined organic layers were washed with $H_2O$, brine, dried over $MgSO_4$ and concentrated. The residue was purified through a silica gel column with 15% EtOAc/ 85% hexanes to afford the title compound (425.1 mg, 81%) as a colorless oil. $^1H$ NMR (400 MHz, Methanol-$d_4$) δ ppm 3.13 (t, *J* = 6.5 Hz, 2 H), 3.33 (s, 3 H), 3.57 (t, *J* = 6.5 Hz, 2 H), 7.20 (dd, *J* = 7.9, 7.9 Hz, 1H), 7.30 - 7.35 (m, 2 H), 7.50 - 7.52 (m, I H).

**Step B: Preparation of Intermediate 1-Bromo-3-(2-methoxyethylsulfonyl)benzene.**

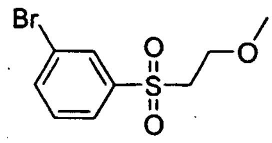

To a solution of (3-bromophenyl)(2-methoxyethyl)sulfane (425.1 mg, 1.72 mmol) in DCM (8.5 mL) was added 3-chloroperoxybenzoic acid (77% max.) (1.09 g, 4.88 mmol). The resulting reaction mixture was stirred at room temperature for 21 h. Upon completion, the reaction mixture was quenched with saturated $NaHCO_3$ and extracted with EtOAc. The combined organic layers were washed with $H_2O$, brine, dried over $MgSO_4$ and concentrated. The residue was purified through a silica gel column with 30% EtOAc/ 70% hexanes to give a product as a colorless oil (0.426 g, 89%). $^1H$ NMR (400 MHz, Methanol-$d_4$) δ ppm 3.172 (s, 3 H), 3.514 (t, *J* = 5.7 Hz, 2 H), 3.719 (t, *J* = 5.7 Hz, 2 H), 7.540 (dd, *J* = 8.0, 8.0 Hz, I H), 7.85 - 7.91 (m, 2 H), 8.06 (dd, *J* = 1.8, 1.8 Hz, 1H).

**Step C: Preparation of (*R*)-1-{2-[3'-(2-Methoxy-ethanesulfonyl)-biphenyl-4-yl]-ethyl}-2-methyl-pyrrolidine.**

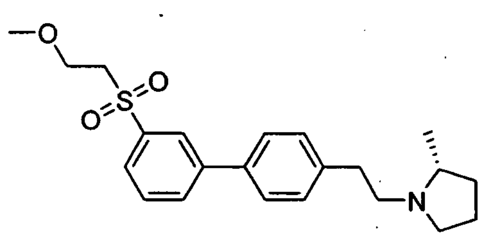

A reaction vial was charged with 1-bromo-3-(2-methoxyethylsulfonyl)benzene (200.7 mg, 0.719 mmol), (*R*)-4-(2-(2-methylpyrrolidin-1-yl)ethyl)phenylboronic acid (170.1 mg, 0.7297 mmol), sodium carbonate (0.716 mL, 1.432 mmol), and tetrakis(triphenylphosphine)palladium(0) (20.0 mg, 0.01731 mmol) in a mixture of ethanol (0.50 mL, 8.563 mmol) and benzene (1.5 mL). The resulting reaction mixture was heated at 100 °C for 1.5 h using microwave. Upon completion, the organic layer was pipetted out and the aqueous layer was extracted with EtOAc. The combined organic layers were dried over $MgSO_4$ and concentrated. The residue was purified by preparative HPLC, lyophilized, and treated with HCl to afford the hydrochloride salt of the title compound (152.3 mg). Exact mass calculated for $C_{22}H_{29}NO_3S$: 387.2, Found: LCMS *m/z* = 388.4 ($M+H^+$); $^1H$ NMR (400 MHz, Methanol-$d_4$) δ ppm 1.48 (d, *J* = 6.5 Hz, 3 H), 1.71 - 1.82 (m, 1H), 2.01 - 2.22 (m, 2 H), 2.31 - 2.41 (m, 1H), 3.06 - 3.23 (m, 5 H), 3.24 - 3.36 (m, 2 H), 3.51 - 3.58 (m, 3 H), 3.61 - 3.70 (m, I H), 3.72 - 3.80 (m, 3H), 7.48 (d, *J*= 8.3 Hz, 2 H), 7.68 - 7.73 (m, 3 H), 7.91 (ddd, *J*= 1.1, 1.6, 7.9 Hz, 1H), 7.97 (ddd, *J* = 1.1, 1.7, 7.8 Hz, 1H), 8.13 (dd, *J* = 1.7, 1.7 Hz, 1H).

**Example 1.18: Preparation of (*R*)-1-[2-(4'-Ethenesulfonyl-biphenyl-4-yl)-ethyl]-2-methyl-pyrrolidine (Compound 13).**

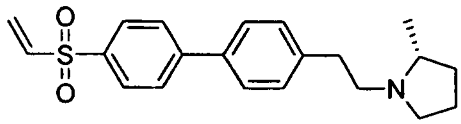

A reaction vial was charged with 2-(4-bromophenylsulfonyl)ethyl isobutyrate (120 mg, 0.357 mmol), (*R*)-4-(2-(2-methylpyrrolidin-1-y1)ethyl)phenylboronic acid (85.7 mg, 0.368 mmol), sodium carbonate (0.357 mL, 0.714 mmol), and tetrakis(triphenylphosphine)palladium(0) (11.6 mg, 10.0 $\mu$mol) in a mixture of ethanol (0.500 mL, 8.56 mmol) and benzene (1.5 mL). The resulting reaction mixture was heated at 100 °C for 1.5 h using microwave irradiation. Upon completion, the organic layer was pipetted out and the aqueous layer was extracted with EtOAc. The combined organic layers were dried over $MgSO_4$ and concentrated. The combined organic layers were dried over $MgSO_4$ and concentrated. The residue was purified by preparative HPLC, lyophilized, and treated with HCl to afford the hydrochloride salt of the title compound (16.7 mg). Exact mass calculated for $C_{21}H_{25}NO_2S$: 355.2, Found: LCMS *m/z* = 356.3 (M+H$^+$); $^1$H NMR (400 MHz, Methanol-*d*$_4$) $\delta$ ppm 1.49 (d, *J* = 6.5 Hz, 3 H), 1.71 - 1.82 (m, 1H), 2.02 - 2.21 (m, 2 H), 2.30 - 2.40 (m, 1H), 3.07 - 3.23 (m, 2 H), 3.24 - 3.35 (m, 2 H), 3.49 - 3.60 (m, 1H), 3.60 - -3.70 (m, 1H), 3.73 - 3.81 (m, 1H), 6.14 (dd, *J* = 0.0, 9.9 Hz, I H), 6.43 (dd, *J* = 0.0, 16.5 Hz, I H), 6.93 (dd, *J* = 9.9, 16.5 Hz, I H), 7.48 (d, *J* = 8.3 Hz, 2 H), 7.70 (d, *J* = 8.2 Hz, 2 H), 7.84 - 7.88 (m, 2 H), 7.93 - 7.97 (m, 2 H).

**Example 1.19: Preparation of 2-{4'-[2-((*R*)-2-Methyl-pyrrolidin-1-yl)-ethyl]-biphenyl-3-sulfonyl}-ethanol (Compound 17).**

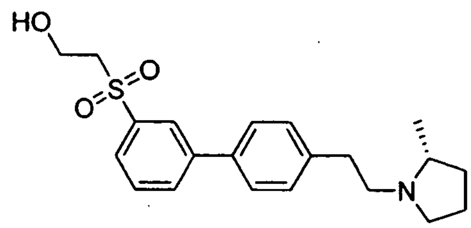

The title compound was prepared in a similar manner as described in **Example 1.17**, **Step C,** using 2-(3-bromophenylsulfonyl)ethanol (116.5 mg, 0.439 mmol) and (*R*)-4-(2-(2-methylpyrrolidin-1-yl)ethyl)phenylboronic acid (102.8 mg, 0.4410 mmol) as starting materials to give a white solid in 51% yield. Exact mass calculated for $C_{21}H_{27}NO_3S$: 373.2, Found: LCMS *m/z* = 374.2 (M+H$^+$); $^1$H NMR (400 MHz, Methanol-*d*$_4$) $\delta$ ppm 1.487 (d, *J* = 6.5 Hz, 3 H), 1.71 - 1.82 (m, 1H), 2.01 - 2.27 (m, 2 H), 2.31 - 2.41 (m, 1H), 3.06 - 3.22 (m, 2 H), 3.24 - 3.35 (m, 2 H), 3.474 (t, *J*= 6.1 Hz, 2 H), 3.49 - 3.60 (m, 1H), 3.61 - 3.70 (m, I H), 3.72 - 3.80 (m, 1H), 3.90 (t, *J* = 6.1 Hz, 2 H), 7.48 (d, *J* = 8.3 Hz, 2 H), 7.68 - 7.74 (m, 3 H), 7.90 - 7.94 (m, 1H), 7.96 - 8.00 (m, 1H), 8.15 (dd, *J* = 1.7, 1.7 Hz, 1H).

**Example 1.20: Preparation of 3-{4'-[2-((*R*)-2-Methyl-pyrrolidin-1-yl)-ethyl]-biphenyl-4-sulfonyl}-propan-1-ol (Compound 15).**

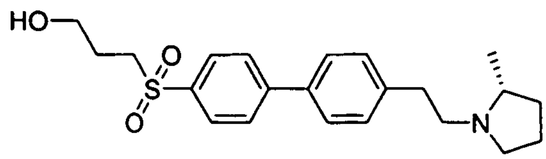

The title compound was prepared in a similar manner as described in **Example 1.5**, **Step C**, using 3-(4-bromophenylsulfonyl)propan-1-ol (200 mg, 0.716 mmol) and (*R*)-4-(2-(2-methylpyrlolidin-1-yl)ethyl)phenylboronic acid (167 mg, 0.716 mmol) as starting materials to give a clear solid in 13% yield. Exact mass calculated for $C_{22}H_{29}NO_3S$: 387.2, Found: LCMS *m/z* = 388.3 (M+H$^+$); $^1$H NMR (400 MHz, Methanol-*d*$_4$) $\delta$ ppm 1.33 (d, *J* = 6.82 Hz, 0.3 H), 1.49

(d, *J* = 6.32 Hz, 2.7 H), 1.71 - 1.82 (m, 1H), 1.84 - 1.93 (m, 2 H), 2.01 - 2.21 (m, 2 H), 2.31 - 2.41 (m, 1H), 3.06 - 3.23 (m, 2 H), 3.24 - 3.29 (m, 2 H), 3.32 - 3.36 (m, 2 H), 3.49 - 3.70 (m, 4 H), 3.73 - 3.80 (m, 1H), 7.48 (d, *J* = 8.08 Hz, 2 H), 7.72 (d, *J* = 8.34 Hz, 2 H), 7.89 (d, *J* = 8.34 Hz, 2 H), 7.97 - 8.01 (m, 2 H).

**Example 1.21: Preparation of intermediate Sodium 4'-(2-Chloroethyl)-4-biphenylsulfinate.**

**Step A: Preparation of 2-Biphenyl-4-yl=ethanol.**

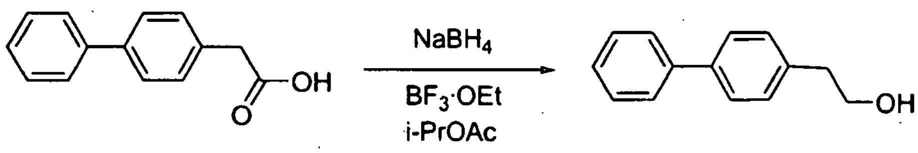


To dilute the hydrogen gas byproduct continuously vented from the reactor, a vigorous flow of nitrogen through the reactor is maintained throughout the entire preparation until the quench with aqueous sodium hydroxide is completed. To a 50 L reactor containing isopropyl acetate (16.7 L) is added sodium borohydride (0.762 Kg, 20.14 mole, 1.60 eq). The sodium borohydride is rinsed into the reactor with additional isopropyl acetate (1 L). 4-Biphenylacetic acid (2.67 Kg, 12.58 moles, 1.00 eq) is then added sufficiently slowly to maintain the stirred reactor contents at 0˚-8˚C with reactor jacket cooling. The biphenylacetic acid is rinsed into the reactor with additional isopropyl acetate (1 L). The reactor contents are then warmed to 25˚C for one hour and then cooled again to -5˚-0˚C. Borontrifluoride diethyletherate (3.584 Kg, 25.25 moles, 2.01 eq) is then added at a constant rate over a three hour period while the stirred reactor contents are maintained at 0˚-5˚C with reactor jacket cooling. The reactor contents are warmed to 20˚-25˚C, and stirring at that temperature is continued until substantially complete 4-biphenylacetic acid conversion is verified by liquid chromatography. (Substantially complete conversion is typically achieved in 1-12 hours.) A solution of sodium hydroxide (3.355 Kg, 83.9 moles, 6.67 eq) in water (15.0 L) is then added sufficiently slowly to maintain the stirred reactor contents at 10˚-20˚C with reactor jacket cooling. The reactor contents are heated to 40˚C, and stirring at that temperature is continued for one hour or until substantially all solids are dissolved. The stirred mixture is then cooled to 15˚-20˚C, and the phases are allowed to separate. The aqueous phase is drained, and the organic (upper) phase is washed with water (4 x 5.0 L). The upper organic phase is concentrated by vacuum distillation to 8 L volume at temperatures rising to no more than 60˚C. The reactor contents are cooled to 20˚C, and heptane (20 L) is added. The stirred reactor contents are concentrated by vacuum distillation to 16 L volume at temperatures rising to no more than 40˚C. The stirred reactor contents are cooled to 20˚C, stirred at that temperature for at least 2 hours, and then filtered. The filtered solid is washed with heptane (5 L) and vacuum dried at 25˚C to provide the title compound 2-biphenyl-4-yl-ethanol. The yield is about 2.148 Kg (10.83 moles, 86.1%).

**Step B: Preparation of 4'-(2-Chloro-ethyl)-biphenyl-4-sulfonic Acid.**

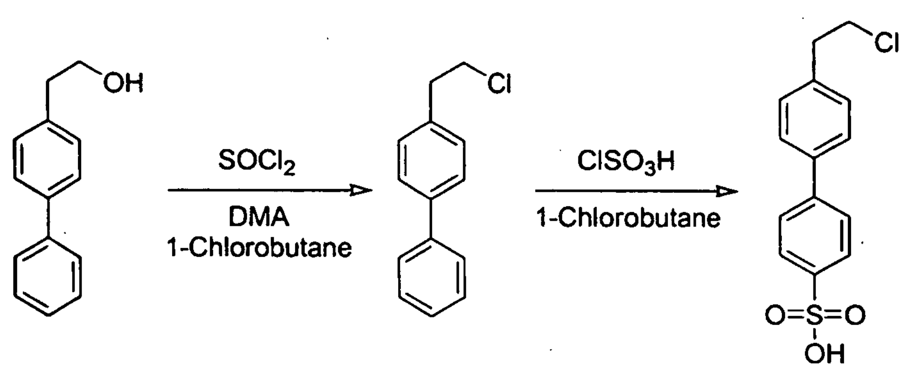


To a 30 L reactor containing 1-chlorobutane (11.4 L) stirred under nitrogen and vented to an aqueous sodium hydroxide scrubber was added 2-biphenyl-4-yl-ethanol (1.9323 Kg, 9.75 moles, 1.00 eq) and N,N-dimethylacetamide

(DMA, 84.3 g, 0.968 mole, 0.099 eq). Thionyl chloride (1.468 Kg, 12.34 mole, 1.27 eq) was then added sufficiently slowly to maintain the stirred reactor contents at 12°-20°C with reactor jacket cooling. The thionyl chloride was rinsed into the reactor with additional 1-chlorobutane (50 mL). The reactor contents were heated to 55°C, and stirring at that temperature was continued for 11 hours until conversion of 2-biphenyl-4-yl-ethanol to 4-(2-chloro-ethyl)-biphenyl was verified to be substantially complete by liquid chromatography. More 1-chlorobutane (3.9 L) was added, and the reactor contents were cooled to -5°C. Chlorosulfonic acid (1.648 Kg, 14.14 moles, 1.45 eq) was then added sufficiently slowly to maintain the stirred reactor contents at -5° to 1°C with reactor jacket cooling. The chlorosulfonic acid was rinsed into the reactor with additional 1-chlorobutane (50 mL). The reactor contents were warmed to 20°-23°C, and stirring at that temperature was continued for 17 hours until conversion of 4-(2-chloro-ethyl)-biphenyl to 4'-(2-chloro-ethyl)-biphenyl-4-sulfonic acid was verified to be substantially complete by liquid chromatography. The reaction mixture was filtered, and the filtered solids were washed with 1-chlorobutane (11.5 L) and vacuum dried at 55°C to constant weight to provide 4'-(2-chloro-ethyl)-biphenyl-4-sulfonic acid (2.674 Kg, 9.01 moles, 92.4% yield), which, if allowed to remain in contact with air, will absorb a considerable amount of water.

**Step C: Preparation of 4'-(2-Chloro-ethyl)-biphenyl-4-sulfonyl chloride.**

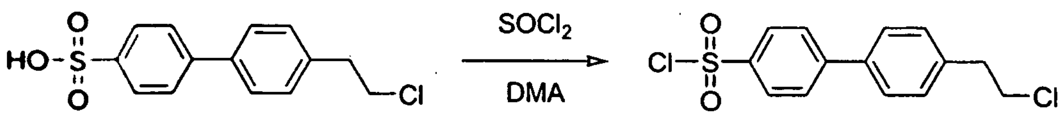


To a reactor containing thionyl chloride (3.82 Kg, 32.1 moles, 11.5 eq) stirred under nitrogen and vented to an aqueous sodium hydroxide scrubber was added 4'-(2-chloro-ethyl)-biphenyl-4-sulfonic acid (0.831 Kg after correction for 12.5 wt % water content, 2.80 moles, 1.00 eq) sufficiently slowly to maintain the stirred reactor contents at 5°-20°C with reactor jacket cooling. N,N-dimethylacetamide (28.1 g, 0.323 moles, 0.115 eq) was then added sufficiently slowly to maintain the stirred reactor contents at -1° to 5°C with reactor jacket cooling. The reactor contents were heated to 65°C, and stirring at that temperature was continued for 6.5 hours until conversion of 4'-(2-chloro-ethyl)-biphenyl-4-sulfonic acid to 4'-(2-chloro-ethyl)-biphenyl-4-sulfonyl chloride was verified to be substantially complete by liquid chromatography. After the reactor contents had been cooled to 23°C, heptane (4.75 L) was added, and stirring at 23°C was continued for 1.5 hours. The reaction mixture was then filtered. The filtered solid was washed with heptane (1 L and then 3 L) and then water (5.4 L) and vacuum dried at 40°C to constant weight to provide 4'-(2-chloro-ethyl)-biphenyl-4-sulfonyl chloride (0.6955 Kg, 2.21 moles, 78.8% yield).

**Step D: Preparation of Sodium 4'-(2-Chloroethyl)-4-biphenylsulfinate.**

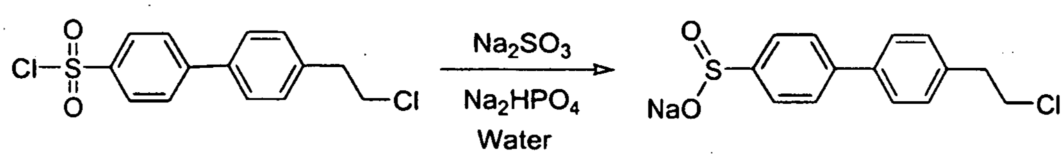


A mixture of sodium sulfite (600.3 g, 4.76 moles, 5.00 eq), $Na_2HPO_4$ (135.0 g, 0.951 mol, 1.00 eq), benzyltriethylammonium chloride (11.71 g, 47.6 mmol, 0.0500 eq), and water (2500 mL) was stirred and heated under nitrogen to 37°C. To the resulting solution was added 4'-(2-chloro-ethyl)-biphenyl-4-sulfonyl chloride (300.2 g, 0.952 mole, 1.00 eq) while maintaining stirring under nitrogen. The temperature of the brown reaction slurry was 48°C at the end of the addition and was then increased to 60°C. After the reaction mixture had been stirred at that temperature for 13.5 hours, LC/MS analysis revealed complete conversion of starting material, and the stirred mixture was allowed to cool to room temperature. The product mixture was stirred at 24°-29°C for two hours and then filtered. The filtered solid was washed with water (600 mL, then 400 mL) and acetonitrile (2 x 600 mL) and then dried under vacuum at 60°C to provide crude white sodium 4'-(2-chloroethyl)-4-biphenylsulfinate (243.8 g, 0.805 mole, 84.6% yield) containing the sulfonic acid (8.8 LC/MS area %) and sodium sulfite as impurities.

Unless the sodium sulfite impurity was removed, subsequent alkylation of sodium 4'-(2-chloroethyl)-4-biphenylsulfinate did not proceed to complete conversion. Therefore, the sodium sulfite impurity was removed by stirring the crude sodium 4'-(2-chloroethyl)-4-biphenylsulfinate (231.17 g) in water (2.0 L) under nitrogen at 40˚C for 15 minutes and then recovering the purified sodium 4'-(2-chloroethyl)-4-biphenylsulfinate by vacuum filtration, washing with water (2 x 1.0 L) and acetonitrile (2 x 500 mL), and vacuum drying at 60˚C.

**Example 1.22: Preparation of (*R*)-1-{2-14'-(2-Methoxy-ethanesulfonyl)-biphenyl-4-yl]-ethyl}-2-methyl-pyrrolidine Hydrochloride (Compound 3, as HCl salt).**

**Step A: Preparation of 4-(2-Chloro-ethyl)-4'-(2-methoxy-ethanesulfonyl)-biphenyl.**

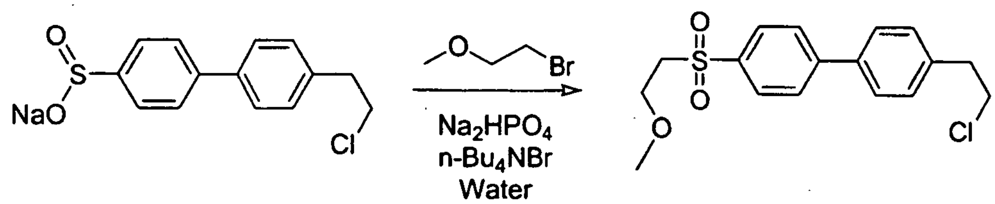


To a mixture of $Na_2HPO_4$ (468.9 mg, 3.303 mmol, 1.00 eq), tetra-n-butylammonium bromide (106.5 mg, 0.3303 mmol, 0.100 eq), sodium 4'-(2-chloroethyl)-4-biphenylsulfinate (1.00 g, 3.303 mmol, 1.00 eq) and water (5.0 mL) stirred under nitrogen was added 2-methoxyethyl bromide (0.326 mL, 3.468 mmole, 1.05 eq). The resulting mixture was stirred in an 80˚C oil bath for 3.5 hours until conversion of sodium 4'-(2-chloroethyl)-4-biphenyl sulfinate to 4-(2-chloro-ethyl)-4'-(2-methoxy-ethanesulfonyl)-biphenyl was verified to be substantially complete by LC/MS. Heating was discontinued, and methanol (10.0 mL) was added when the reactor contents were at 55˚-60˚C. After the reaction mixture had been stirred at room temperature for 4.5 hours, it was filtered. The filtered white solid was washed with water and vacuum dried at 45˚C to provide 4-(2-chloro-ethyl)-4'-(2-methoxy-ethanesulfonyl)-biphenyl (0.989 g, 2.92 mmole, 88.4% yield).

**Step B: Preparation of (R)-1-{2-[4'-(2-Methoxy-ethanesulfonyl)-biphenyl-4-yl]-ethyl}-2-methyl-pyrrolidine Hydrochloride (Compound 3, as HCl salt).**

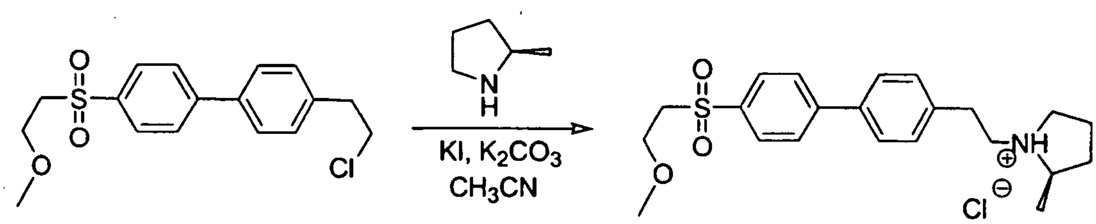


To a mixture of 4-(2-chloro-ethyl)-4'-(2-methoxy-ethanesulfonyl)-biphenyl (98 mg, 0.289 mmole, 1.00 eq) and acetonitrile (1.0 mL) stirred under nitrogen was added potassium carbonate (47.9 mg, 0.347 mmole, 1.20 eq) and potassium iodide (48 mg, 0.289 mmole, 1.00 eq). After the resulting mixture was stirred at room temperature for 15 minutes, (R)-(-)-2-methylpyrrolidine (24.6 mg, 0.289 mmole, 1.00 eq) was added. Conversion of 4-(2-chloroethyl)-4'-(2-methoxy-ethanesulfonyl)-biphenyl to the free base of (*R*)-1-{2-[4'-(2-methoxy-ethanesulfonyl)-biphenyl-4-yl]-ethyl}-2-methyl-pyrrolidine was about 50% after 16 hours of stirring at 60˚C. After addition of more (R)-(-)-2-methylpyrrolidine (9.8 mg, 0.115 mmole, 0.40 eq) and continued stirring at 80˚C for 40 hours, conversion of 4-(2-chloro-ethyl)-4'-(2-methoxy-ethanesulfonyl)-biphenyl to the free base of (*R*)-1-{2-[4'-(2-methoxy-ethanesulfonyl)-biphenyl-4-yl]-ethyl}-2-methyl-pyrrolidine was substantially complete by LC/MS analysis. The reaction mixture was cooled to room temperature and filtered to remove potassium salts. The filtrate was evaporated and dissolved in ethyl acetate. The resulting solution was extracted with 5 wt. % aqueous HCl. Sodium carbonate was added to the aqueous extract to convert the product to its free base, which was extracted into ethyl acetate. Treatment of the ethyl acetate extract with HCl gas provides (*R*)-1-{2-[4'-(2-methoxy-ethanesulfonyl)-biphenyl-4-yl]-ethyl}-2-methyl-pyrrolidine as the HCl salt.

**Example 1.23: Preparation of (*R*)-1-{2-[4'-(3-Methoxy-propane-1-sulfonyl)-biphenyl-4-yl]-ethyl}-2-methyl-pyrrolidine (Compound 8).**

**Step A: Preparation of 4-(2-Chloro-ethyl)-4'-(3-methoxy-propane-1-sulfonyl)-biphenyl.**

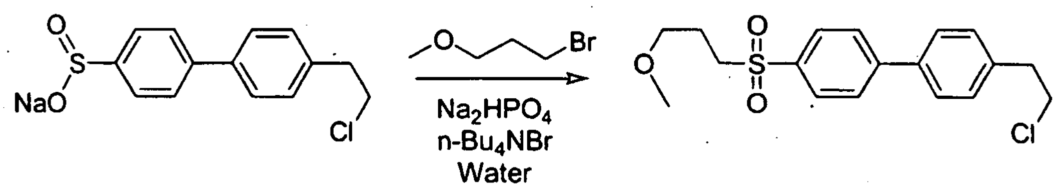


To a mixture of $Na_2HPO_4$ (15.84 g, 111.6 mmol, 1.00 eq), tetra-*n*-butylammonium bromide (3.60 g, 11.2 mmol, 0.100 eq), potassium bromide (13.28 g, 111.6 mmole, 1.00 eq), sodium 4'-(2-chloroethyl)-4-biphenylsulfinate (33.78 g, 111.6 mmol, 1.00 eq) and water (169 mL) stirred under nitrogen is added 2-methoxypropyl bromide (18.78 g, 122.7 mmole, 1.10 eq). The resulting mixture is stirred in an 80°C oil bath until conversion of sodium 4'-(2-chloroethyl)-4-biphenylsulfinate to 4-(2-chloro-ethyl)-4'-(3-methoxy-propane-1-sulfonyl)-biphenyl is verified to be substantially complete by chromatographic analysis. The reaction mixture is cooled and extracted with ethyl acetate. The ethyl acetate extract is filtered through a silica gel plug to remove residual tetra-*n*-butylammonium bromide and is then evaporated to a residue of 4-(2-chloro-ethyl)-4'-(3-methoxy-propane-1-sulfonyl)-biphenyl, which solidifies on standing.

**Step B: Preparation of (R)-1-{2-[4'-(3-Methoxy-propane-1-sulfonyl)-biphenyl-4-yl]-ethyl}-2-methyl-pyrrolidine.**

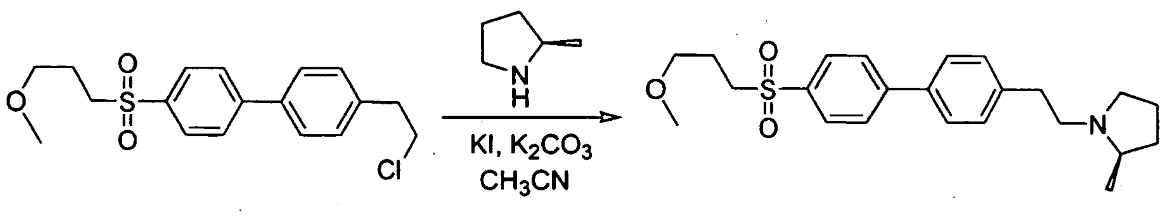


To a mixture of 4-(2-Chloro-ethyl)-4'-(3-methoxy-propane-1-sulfonyl)-biphenyl (101.9 mg, 0.289 mmole, 1.00 eq) and acetonitrile (1.0 mL) stirred under nitrogen is added potassium carbonate (47.9 mg, 0.347 mmole, 1.20 eq) and potassium iodide (48 mg, 0.289 mmole, 1.00 eq). After the resulting mixture is stirred at room temperature for 15 minutes, (*R*)-(-)-2-methylpyrrolidine (24.6 mg, 0.289 mmole, 1.00 eq) is added. After 16 hours of stirring at 60°C, more (*R*)-(-)-2-methylpyrrolidine (9.8 mg,-0.115 mmole, 0.40 eq) is added, and stirring is continued at 80°C until conversion of 4-(2-chloro-ethyl)-4'-(3-methoxy-propane-1-sulfonyl)-biphenyl to (*R*)-1-{2-[4'-(3-methoxy-propane-1-sulfonyl)-biphenyl-4-yl]-ethyl}-2-methyl-pyrrolidine is substantially complete by chromatographic analysis. The reaction mixture is cooled to room temperature and filtered to remove potassium salts. The filtrate is evaporated and dissolved in ethyl acetate. The resulting solution is extracted with 5 wt. % aqueous HCl. Sodium carbonate is added to the aqueous extract to convert the hydrochloride salt of the title compound to the free base, which is extracted into ethyl acetate. Evaporation of the ethyl acetate extract provides (*R*)-1- -{2-[4'-(3-methoxy-propane-1-sulfonyl)-biphenyl-4-yl]-ethyl}-2-methyl-pyrrolidine.

**Example 1.24: Preparation of (*R*)-2-Methyl-1-{2-(4'-(tetrahydro-pyran-4-ylmethanesulfonyl)-biphenyl-4-yl]-ethyil-pyrrolidine (Compound 26).**

**Step A: Preparation of Intermediate 4-((4-Bromophenylthio)methyl)-tetrahydro-2*H*-pyran.**

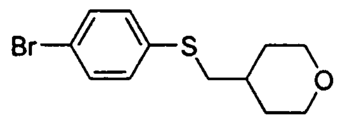

The title compound was prepared in a similar manner as described in **Example 1.5**, Step A, using 4-(bromomethyl)-tetrahydro-2*H*-pyran (0.663 g, 3.702 mmol). Purification by flash chromatography on silica gel (10% EtOAc in Hexane) yielded the title compound (0.467 g, 87.8%) as a clear oil. $^1$H NMR (400 MHz, $CDCl_3$) δ ppm 1.29 - 1.42 (m, 2 H), 1.68 - 1.82 (m, 3 H), 2.83 (d, *J*= 6.57 Hz, 2 H), 3.35 (t, *J* = 11.62 Hz, 2 H), 3.97 (dd, *J* = 11.37, 3.79 Hz, 2 H), 7.16-7.21 (m, 2 H), 7.38-7.43 (m, 2 H).

**Step B: Preparation of Intermediate 4-((4-Bromophenylsulfonyl)methyl)-tetrahydro-2*H*-pyran.**

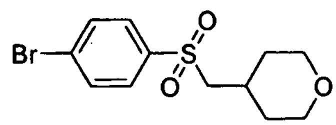

To a solution of 4-((4-bromophenylthio)methyl)-tetrahydro-2*H*-pyran (0.465 g, 1.619 mmol) in THF (2 mL) was added Oxone® (2.19 g, 3.562 mmol) in water (3 mL). The resulting mixture was stirred for 4 h at room temperature. The reaction was diluted with water and extracted twice with EtOAc. The organic phase was dried over $MgSO_4$, filtered, and concentrated to afford the title compound (0.432 g, 83.5%) as a clear oil. $^1$H NMR (400 MHz, $CDCl_3$) δ ppm 1.38 - 1.51 (m, 2 H), 1.81 (dd, *J* = 13.14, 1.77 Hz, 2 H), 2.20 - 2.33 (m, 1H), 3.01 (d, *J* = 6.32 Hz, 2 H), 3.37 - 3.45 (m, 2 H), 3.93 (dd, *J* = 11.37, 3.54 Hz, 2 H), 7.71 - 7.81 (m, 4 H).

**Step C: Preparation of (*R*)-2-Methyl-1-{2-[4'-(tetrahydro-pyran-4-ylmethanesulfonyl)-biphenyl-4-yl]-ethyl}-pyrrolidine (Compound 26).**

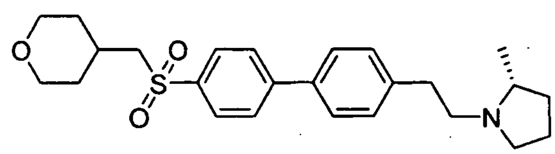

The title compound was prepared in a similar manner as described in **Example 1.5,** Step C, using 4-((4-bromophenylsulfonyl)methyl)-tetrahydro-2*H*-pyran (385 mg, 1.206 mmol) and (*R*)-4-(2-(2-methylpyrrolidin-1-yl)ethyl) phenylboronic acid (385 mg, 1.206 mmol) as starting materials to give a white solid in 60% yield. Exact mass calculated for $C_{25}H_{33}NO_3S$: 427.2, Found: LCMS *m*/*z* = 428.3 ($M+H^+$); $^1$H NMR (400 MHz, Methanol-*d$_4$*) δ ppm 1.33 (d, *J* = 6.82 Hz, 0.3 H), 1.37-1.51 (m, 4.7 H), 1.71 - 1.83 (m, 3 H), 2.01 - 2.22 (m, 3 H), 2.30 - 2.40 (m, 1H), 3.07 - 3.20 (m, 2 H), 3.20 - 3.24 (m, 2 H), 3.24 - 3.29 (m, 2 H), 3.35 - 3.44 (m, 2 H), 3.49 - 3.59 (m, 1H), 3.61 - 3.69 (m, 1H), 3.73 - 3.81 (m, 1H), 3.88 (dd, *J* = 11.62, 2.27 Hz, 2 H), 7.48 (d, *J* = 8.08 Hz, 2 H), 7.71 (d, *J* = 8.08 Hz, 2 H), 7.89 (d, *J* = 8.34 Hz, 2 H), 7.97 - 8.01 (m, 2 H).

**Example 1.25: Preparation of 2-Methanesulfonyl-5-{4-[2-((R)-2-methyl-pyrrolidin-1-yl)-ethyl]-phenyl}-pyridine (Compound 22).**

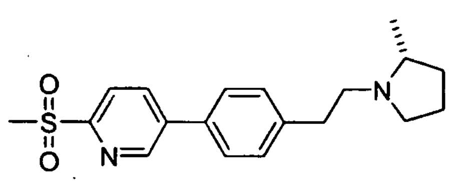

The title compound was prepared in a similar manner as described in **Example 1.5, Step C**, using 5-bromo-2-(methylsulfonyl)pyridine (150 mg, 0.635 mmol) and (*R*)-4-[2-(2-methylpyrrolidin-1-yl)ethyl]phenylboronic acid (163 mg, 0.699 mmol) as starting materials to give a white solid (HCl salt) in 16% yield. Exact mass calculated for $C_{19}H_{24}N_2O_2S$: 344.2, Found: LCMS *m/z* = 345.2 (M+H$^+$); $^1$H NMR (400 MHz, Methanol-d$_4$) δ ppm 1.33 (d, *J* = 6.82 Hz, 0.3 H), 1.44 - 1.54 (m, 2.7 H), 1.80 (s, 1H), 2.12 (d, *J* = 13.89 Hz, 2 H), 2.35 (s, 1H), 3.08 - 3.22 (m, 2 H), 3.22 - 3.35 (m, 5 H), 3.49 - 3.71 (m, 2 H), 3.72 - 3.83 (m, 1H), 7.53 (d, *J* = 8.08 Hz, 2 H), 7.76 *(d, J* = 8.08 Hz, 2 H), 8.15 (d, *J* = 8.08 Hz, I H), 8.33 (dd, *J* = 8.21, 2.15 Hz, 1 H), 9.00 (d, *J* = 1.77 Hz, 1H).

**Example 1.26: Preparation of 5-Methanesulfonyl-2-{4-[2-((*R*)-2-methyl-pyrrolidin-1-yl)-ethyl]-phenyl}-pyridine (Compound 33).**

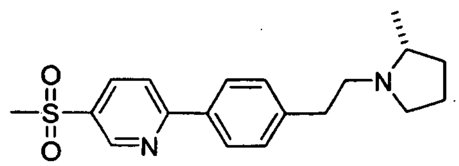

The title compound was prepared in a similar manner as described in **Example 1.5**, **Step C**, using 2-bromo-5-(methylsulfonyl)pyridine (150 mg, 0.635 mmol) and (*R*)-4-[2-(2-methylpyrrolidin-1-yl)ethyl]phenylboronic acid (163 mg, 0.699 mmol) as starting materials to give a yellow oil (HCl salt) in 14% yield. Exact mass calculated for $C_{19}H_{24}N_2O_2S$: 344.2, Found: LCMS *m/z* = 345.1 (M+H$^+$); $^1$H NMR (400 MHz, Methanol-*d$_4$*) δ ppm 1.33 (d, *J* = 6.57 Hz, 0.3 H), 1.50 (d, *J* = 6.32 Hz, 2.7 H), 1.70 - 1.85 (m, 1H),2.01 - 2.22 (m, 2 H), 2.28 - 2.42 (m, 1H), 3.11 - 3.38 (m, 7 H), 3.49 - 3.61 (m, 1H), 3.61 - 3.72 (m, 1H), 3.72 - 3.84 (m, 1H), 7.55 *(d, J* = 7.83 Hz, 2 H), 8.12 (d, *J* = 7.83 Hz, 2 H), 8.19 *(d, J* = 8.59 Hz, I H), 8.47 *(dd,J* = 8.34, 2.02 Hz, 1H), 9.14 (d, *J* = 2.02 Hz, 1H).

**Example 1.27: Preparation of (R)-1-[2-(3'-Methanesulfonyl-4'-methyl-biphenyl-4-yl)-ethyl]-2-methyl-pyrrolidine (Compound 27).**

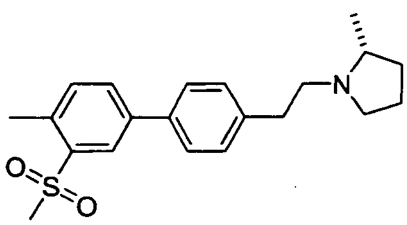

The title compound was prepared in a similar manner as described in **Example 1.5**, Step C, using 4-bromo-1-methyl-2-(methylsulfonyl)benzene (200 mg, 0.803 mmol) and (R)-4-[2-(2-methylpyrrolidin-1-yl)ethyl]phenylboronic acid (206 mg, 0.883 mmol) as starting materials to give a yellow oil (HCl salt) in 67% yield. Exact mass calculated for $C_{21}H_{27}NO_2S$: 357.2, Found: LCMS *m/z* = 358.4 (M+H$^+$); $^1$H NMR (400 MHz, Methanol-*d$_4$*) δ ppm 1.32 (dd, *J* = 6.82, 1.77 Hz, 0.3 H), 1.49 (dd, *J* = 6.44, 1.89 Hz, 2.7 H), 1.70 - 1.84 (m, 1H), 1.99 - 2.23 (m, 2 H), 2.33 (d, *J* = 6.82 Hz, I H), 2.69 - 2.77 (m, 3 H), 3.07 - 3.37 (m, 7 H), 3.48 - 3.70 (m, 2 H), 3.71 - 3.83 (m, 1H), 7.42 - 7.55 (m, 3 H), 7.64 (d, *J* = 6.32 Hz, 2 H), 7.84 (d, *J* = 7.83 Hz, I H), 8.19 (s, 1H).

**Example 1.28: Preparation of 3-Methanesulfonyl-4'-[2-((R)-2-methyl-pyrrolidin-1-yl)-ethyl]-biphenyl-4-carboxylic acid (Compound 20).**

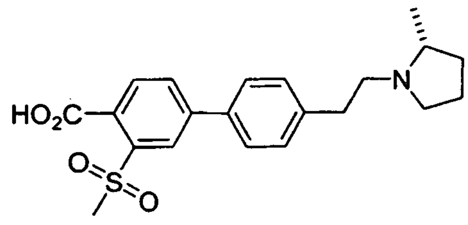

To a vial was added 4-bromo-2-(methylsulfonyl)benzoic acid (200 mg, 0.717 mmol), (*R*)-4-[2-(2-methylpyrrolidin-1-yl)ethyl]phenylboronic acid (184 mg, 0.788 mmol), aq. $Na_2CO_3$ (2 M solution, 0.717 mL, 1.43 mmol), and tetrakis (triphenylphosphine)palladium(0) (25 mg, 0.022 mmol) in a mixture of EtOH (1 mL) and benzene (3 mL). The resulting reaction mixture was heated at 100 ˚C under microwave irradiation for 60 min. Next, the organic layer was pipetted out and the aqueous layer was extracted with EtOAc. The crude mixture was concentrated and purified by HPLC (0.1% TFA in acetonitrile/0.1% TFA in water). Fractions were lyophilized to afford the title compound as a white solid (TFA salt) in 9% yield. Exact mass calculated for $C_{21}H_{25}NO_4S$: 387.2; Found: LCMS *m/z* = 388.3 (M+H$^+$); $^1$H NMR (400 MHz, Methanol-$d_4$) δ ppm 1.32 (d, *J* =5.81 Hz, 0.3 H), 1.48 (d, *J* = 5.81 Hz, 2.7 H), 1.68 - 1.82 (m, 1H), 1.99 - 2.24 (m, 2 H), 2.28 - 2.41 (m, 1H), 3.02 - 3.35 (m, 4 H), 3.42 - 3.48 (m, 3 H), 3.48 - 3.59 (m, 1H), 3.59 - 3.71 (m, 1H), 3.71 - 3.82 (m, 1H), 7.48 (d, *J* = 8.08 Hz, 2 H), 7.72 (d, *J* = 8.34 Hz, 2 H), 7.89 (d, *J* = 7.83 Hz, 1H), 8.02 (dd, *J* = 7.96, 1.89 Hz, 1H), 8.31 (d, *J* = 1.77 Hz, 1H).

**Example 1.29: Preparation of 2-{4'-[2-((R)-2-Methyl-pyrrolidin-1-yl)-ethyl]-biphenyl-4-sulfonyl}-ethylamine (Compound 31).**

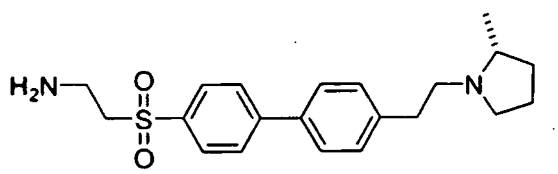

The title compound was prepared in a similar manner as described in **Example 1.28**, using 2-(4-chlorophenylsulfonyl)ethanamine (125 mg, 0.569 mmol) and (*R*)-4-[2-(2-methylpyrrolidin-1-yl)ethyl]phenylboronic acid (146 mg, 0.626 mmol) as starting materials to give a white solid (TFA salt) in 25% yield. Exact mass calculated for $C_{21}H_{28}N_2O_2S$: 372.2, Found: LCMS *m/z* = 373.1 (M+H$^+$); $^1$H NMR (400 MHz, Methanol-$d_4$) δ ppm 1.26 - 1.35 (m, 0.3 H). 1.42 - 1.50 (m, 2.7 H), 1.67 - 1.82 (m, I H), 1.97 - 2.20 (m, 2 H), 2.27 - 2.40 (m, I H), 2.95 - 3.38 (m. 4 H), 3.44 - 3.67 (m, 6 H), 3.68 - 3.82 (m, 1H), 7.24 - 7.38 (m, 3 H), 7.69 - 7.75 (m. 4 H), 7.95 - 8.01 (m, 4 H).

**Example 1.30: Preparation of 3-{4'-[2-((*R)*-2-Methyl-pyrrolidin-1-yl)-ethyl]-biphenyl-4-sulfonyl{-propionic acid (Compound 28).**

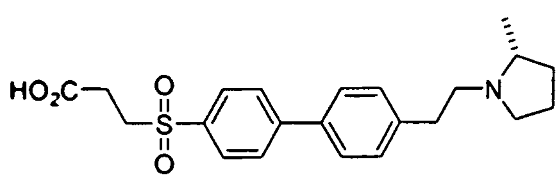

The title compound was prepared in a similar manner as described in **Example 1.28**, using 3-(4-chlorophenylsulfonyl)propanoic acid (200 mg, 0.804 mmol) and (*R*)-4-[2-(2-methylpyrrolidm-1-yl)thyl]phenylboronic acid (206 mg, 0.885 mmol) as starting materials to give a yellow oil (TFA salt) in 6% yield. Exact mass calculated for $C_{22}H_{27}NO_4S$: 401.2, Found: LCMS ml: - 402.2 (M+H$^+$); $^1$H NMR (400 MHz, Methanol-$d_4$) δ ppm 1.47 (d, *J* = 6.57 Hz, 3 H), 1.67 - 1.81 (m, 1H), 1.98 - 2.25 (m, 2 H), 2.36 (m, 1H), 2.69 (t, *J* = 7.45 Hz, 2 H), 3.03 - 3.37 (m, 4 H), 3.53 (t,*J* = 7.33 Hz, 3 H), 3.60 - 3.82 (m, 2 H), 7.48 (d, *J* = 8.08 Hz, 2 H), 7.73 (d, *J* = 8.34 Hz, 2 H), 7.90 (d, *J* = 8.34 Hz, 2 H), 8.00 (d, *J* = 8.33 Hz, 2 H).

**Example 1.31: Preparation of 2-Methanesulfonyl-5-{4-[2-((*R*)-2-methyl-pyrrolidin-1-yl)-ethyl]-phenyl}-pyrimidine (Compound 24).**

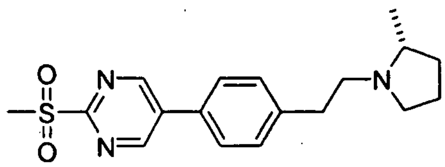

The title compound was prepared in a similar manner as described in **Example 1.28,** using 5-chloro-2-(methylsulfonyl)pyrimidine (100 mg, 0.519 mmol) and (*R*)-4-[2-(2-methylpyrrolidin-1-yl)ethyl]phenylboronic acid (133 mg, 0.571 mmol) as starting materials to give a yellow oil (TFA salt) in 5% yield. Exact mass calculated for $C_{18}H_{23}N_3O_2S$: 345.2, Found: LCMS *m/z* = 346.4 ($M+H^+$); $^1$H NMR (400 MHz, Methanol-$d_4$) δ ppm 1.24 - 1.35 (m, 0.3 H), 1.40 - 1.53 (m, 2.7 H), 1.68 - 1.83 (m, 1H), 1.98 - 2.23 (m, 2 H), 2.25 - 2.42 (m, I H), 2.93 - 3.35 (m, 5 H), 3.37 - 3.45 (m, 2 H), 3.45 - 3.83 (m, 3 H), 7.24 - 7.44 (m, 3 H), 7.51 - 7.64 (m, 1 H), 7.82 (d, *J*=8.08 Hz, 1H), 9.26 (s, 1H).

**Example 1.32: Preparation of Cyclohexyl-(2-{4'-[2-((R)-2-methyl-pyrrolidin-1-yl)-ethyl]-biphenyl-4-sulfonyl}-ethyl)-amine (Compound 32).**

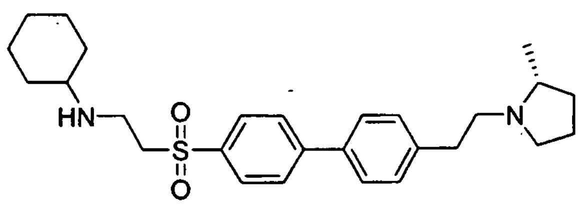

The title compound was prepared in a similar manner as described in **Example 1.28**, using N-(2-(4-chlorophenylsulfonyl)ethyl)cyclohexanamine (100 mg, 0.331 mmol) and (R)-4-[2-(2-methylpyrrolidin-1-yl)ethyl]phenylboronic acid (85 mg, 0.364 mmol) as starting materials to give a brown oil (TFA salt) in 71% yield. Exact mass calculated for $C_{27}H_{38}N_2O_2S$: 454.3, Found: LCMS *m/z* = 455.4 ($M+H^+$); $^1$H NMR (400 MHz, Methanol-$d_4$) δ ppm 1.14 -1.43 (m, 6 H), 1.48 (d, *J* = 6.32 Hz, 3 H), 1.60 - 1.94 (m, 4 H), 1.99 - 2.22 (m, 4 H), 2.27 - 2.41 (m, 1H), 3.04 - 3.35 (m, 5 H), 3.36 - 3.48 (m, 2 H), 3.47 - 3.58 (m, 1H), 3.58 - 3.71 (m, 3 H), 3.72 - 3.84 (m, 1 H), 7.49 (d, *J* = 8.08 Hz, 2 H), 7.73 (d, *J* = 8.34 Hz, 2 H), 7.94 (d, *J* = 8.34 Hz, 2 H), 8.02 - 8.10 (d, *J* = 8.59 Hz, 2 H).

**Example 1.33: Preparation of 1-(2-{4'-[2-((*R*)-2-Methyl-pyrrolidin-1-yl)-ethyl]-biphenyl-4-sulfonyl}-ethyl)-piperidine-4-carbonitrile (Compound 29).**

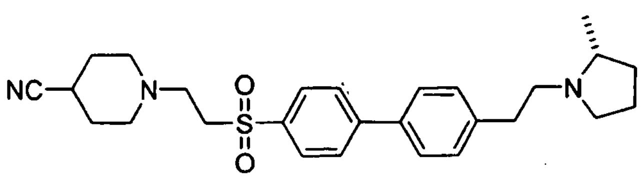

The title compound was prepared in a similar manner as described in **Example 1.28**, using 1-(2-(4-chlorophenylsulfonyl)ethyl)piperidine-4-carbonitrile (100 mg, 0.320 mmol) and (*R*)-4-[2-(2-methylpyrrolidin-1-yl)ethyl]phenylboronic acid (82 mg, 0.352 mmol) as starting materials to give a yellow oil (TFA salt) in 73% yield. Exact mass calculated for $C_{27}H_{35}N_3O_2S$: 465.2, Found: LCMS *m/z* = 466.4 ($M+H^+$); $^1$H NMR (400 MHz, Methanol-$d_4$) δ ppm 1.33 (d, *J* = 6.82 Hz, 0.3 H), 1.48 (d, *J* = 6.57 Hz, 2.7 H), 1.67 - 1.85 (m, 1 H), 1.99 - 2.42 (m, 7 H), 3.02 - 3.36 (m, 7 H), 3.36 - 3.72 (m, 6 H), 3.72 - 3.91 (m, 3 H), 7.48 (d, *J* = 8.08 Hz, 2 H), 7.72 (d, *J* = 8.08 Hz, 2 H), 7.93 (d, *J* = 8.34 Hz, 2 H), 8.05 (d, *J* =

8.34 Hz, 2 H).

**Example1.34:Preparationof4-(2-{4'-[2-((*R*)-2-Methyl-pyrrolidin-1-yl)-ethyl]-biphenyl-4-sulfonyl}-ethyl)-morpholine (Compound 25).**

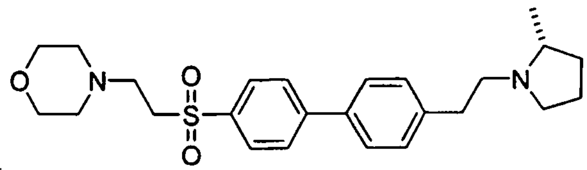

The title compound was prepared in a similar manner as described in **Example 1.28,** using 4-(2-(4-chlorophenylsulfonyl)ethyl)morpholine (100 mg, 0.345 mmol) and (*R*)-4-[2-(2-methylpyrrolidin-1-yl)ethyl]phenylboronic acid (89 mg, 0.380 mmol) as starting materials to give a yellow oil (TFA salt) in 83% yield. Exact mass calculated for $C_{25}H_{34}N_2O_3S$: 442.2, Found: LCMS *m/z* = 443.3 ($M+H^+$);$^1H$ NMR (400 MHz, Methanol-$d_4$) δ ppm 1.33 (d, *J* = 6.83 Hz, 0.3 H), 1.47 (d, *J* = 6.57 Hz, 2.7 H), 1.68 - 1.84 (m, 1 H), 1.98 - 2.24 (m, 2 H), 2.27 - 2.42 (m, 1 H), 3.04 - 3.45 (m, 7 H), 3.45 - 3.71 (m, 5 H), 3.70 - 4.08 (m, 7 H), 7.48 (d, *J* = 8.34 Hz, 2 H), 7.72 (d, *J* = 8.34 Hz, 2 H), 7.93 (d, *J* = 8.84 Hz, 2 H), 8.05 (d, *J* = 8.58 Hz, 2 H).

**Example 1.35: Preparation of (*R*)-2-Methyl-1-{2-[4'-(tetrahydro-pyran-4-sulfonyl)-biphenyl-4-yl]-ethyl}-pyrrolidine (Compound 21).**

**Step A: Preparation of Intermediate 4-(4-Bromophenylthio)-tetrahydro-2H-pyran.**

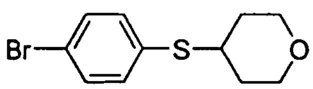

To a solution of 4-bromobenzenethiol (300 mg, 1.60 mmol) in DMF (3 mL) was added sodium hydride (60% dispersion in mineral oil) (95 mg, 2.38 mmol) and 4-bromo-tetrahydro-2*H*-pyran (458 mg, 1.75 mmol). The resulting mixture was stirred for 18 h at room temperature. The reaction was diluted with water and extracted twice with EtOAc. Purification by flash chromatography on silica gel (0-5% EtOAc in hexane) yielded the title compound (340 mg, 78%) as a clear oil. $^1H$ NMR (400 MHz, $CDCl_3$) δ ppm 1.60 - 1.76 (m, 2 H), 1.92 (dd, *J* = 11.87,1.52 Hz, 2 H), 3.20 - 3.33 (m, 1 H), 3.39 - 3.51 (m, 2 H), 3.93 - 4.05 (m, 2 H), 7.28 - 7.34 (m, 2 H), 7.42 - 7.49 (m, 2 H).

**Step B: Preparation of Intermediate 4-(4-Bromophenylsulfonyl)-tetrahydro-2H pyran.**

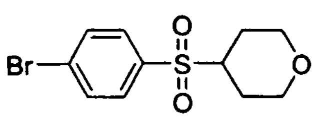

To a solution of 4-(4-bromophenylthio)-tetrahydro-2*H*-pyran (580 mg, 2.12 mmol) in THF/water (10 mL/5 mL) was added Oxone® (1.57 g, 2.55 mmol). The resulting mixture was stirred for 18 h at room temperature. The reaction was diluted with water and extracted twice with EtOAc. The combined organic layers were dried over $MgSO_4$, filtered, and concentrated to afford the title compound (620 mg, 96%) as a white solid. Exact mass calculated for $C_{11}H_{13}BrO_3S$: 304.0, Found: LCMS *m/z* (%) = 305.1 ($M+H^+$ $^{79}Br$, 100%),307.1 ($M+H^+$ $^{81}Br$, 97%); $^1H$ NMR (400 MHz, $CDCl_3$) δ ppm 1.73 - 1.88 (m, 2 H), 1.89 - 1.98 (m, 2 H), 3.10- 3.23 (m, 1H), 3.30 - 3.42 (m, 2 H), 4.04 - 4.16 (m, 2 H), 7.77 (s, 4 H).

**Step C: Preparation of (*R*)-2-Methyl-1-{2-[4'-(tetrahydro-pyran-4-sulfonyl)-biphenyl-4-yl]-ethyl}-pyrrolidine (Compound 21).**

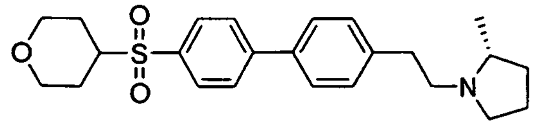

To a vial was added 4-(4-bromophenylsulfonyl)-tetrahydro-2*H*-pyran (500 mg, 1.64 mmol), (*R*)-4-[2-(2-methylpyrrolidin-1-yl)ethyl]phenylboronic acid (420 mg, 1.80 mmol), aq. $Na_2CO_3$ (2 M solution, 1.64 mL, 3.28 mmol), and tetrakis(triphenylphosphine)palladium(0) (57 mg, 0.049 mmol) in a mixture of EtOH (1 mL) and benzene (3 mL). The resulting reaction mixture was heated at 100 °C for 60 min under microwave irradiation. The reaction mixture was diluted with water and the organic phase was removed. The aqueous layer was extracted with EtOAc. The combined organic layers were concentrated, dissolved in ACN/$H_2O$ (with AcOH) and purified by HPLC (0.1% TFA in acetonitrile/0.1% TFA in water). Fractions were combined, basified with 2 M $Na_2CO_3$ and extracted three times with EtOAc. The combined organic layers were dried over $MgSO_4$, filtered, and concentrated. The residue was dissolved in MeOH (5 mL). Then, HCl (1 M in $Et_2O$, 1.65 mL) was added followed by EtOAc (5 mL). The resulting mixture was concentrated to afford the hydrochloride salt of the title compound (408 mg, 55% yield) as a white solid. Exact mass calculated for $C_{24}H_{31}NO_3S$: 413.2, Found: LCMS *m*/*z* = 414.3 (M+H$^+$); $^1$H NMR (400 MHz, $CDCl_3$) δ ppm 1.22 (d, *J* = 6.57 Hz, 0.4 H), 1.62 (d, *J* =5.56Hz,2.6H), 1.67-2.10 (m, 6 H), 2.11 - 2.31 (m, 2 H), 2.82 (s, 2 H), 3.04 - 3.22 (m, 3 H), 3.22 - 3.35 (m, 2 H), 3.49 (s, 2 H), 3.86 - 3.96 (m, 1 H), 4.00 (dd, *J* = 11.49, 3.92 Hz, 2 H), 7.33 (d, *J*= 7.58 Hz, 2 H), 7.50 (d, *J*= 7.58 Hz, 2 H), 7.67 (d, *J* = 8.08 Hz, 2 H), 7.85 (d, *J* = 8.34 Hz, 2 H).

**Example 1.36: Preparation of (R)-1-{2-[4'-(2-Methoxy-propane-1-sulfonyl)-bipbenyl-4-yl]-ethyl}-2-methyl-pyrrolidine (Compound 23).**

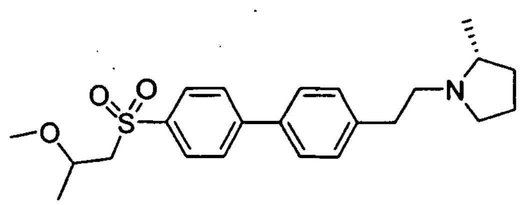

To a 4 mL vial was added (*R*)-2-Methyl-1-{2-[4'-(prop-2-ene-1-sulfonyl)-biphenyl-4-yl]-ethyl}-pyrrolidine trifluoroacetate (10 mg, 21.43 μmol), methanol (0.5 mL), and sodium methoxide (25% in MeOH) (19.60 μl, 85.74 μmol). The reaction solution was stirred at ambient temperature for approximately 20 h, at which point trifluoroacetic acid (8.3 μL, 107 μmol) was added and the resulting solution was concentrated to afford the title compound as a thick, tacky oil. Exact mass calculated for $C_{23}H_{31}NO_3S$: 401.2, Found: LCMS *m*/*z* = 402.2 (M+H$^+$); $^1$H NMR (400 MHz, Methanol-*d$_4$*) δ ppm 1.22 (d, *J* = 6.32 Hz, 3 H), 1.32 (d, *J* = 6.82 Hz, 0.3 H), 1.48 (d, *J* = 6.57 Hz, 2.7 H), 1.68 - 1.85 (m, 1 H), 1.98 - 2.20 (m, 2 H), 2.28 - 2.41 (m, 1 H), 3.05 - 3.15 (m, 2 H), 3.17 (s, 3 H), 3.19 - 3.29 (m, 2 H), 3.33 - 3.37 (m, I H), 3.49 (d, *J* = 7.58 Hz, 1 H), 3.53 (d, *J* = 7.58 Hz, 1 H), 3.58 - 3.71 (m, 1H), 3.72 - 3.90 (m, 2 H), 7.47 (d, *J* = 8.34 Hz, 2 H), 7.70 (d, *J* = 8.08 Hz, 2 H), 7.87 (d, *J* = 8.59 Hz, 2 H), 7.98 (d, *J* = 8.59 Hz, 2 H).

**Example 1.37: Preparation of (*R*)-2-Methyl-1-{2-[4'-(prop-2-ene-1-sulfonyl)-biphenyl-4-yl]-ethyl}-pyrrolidine (Compound 30).**

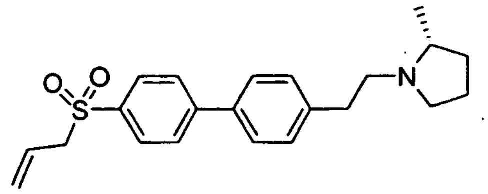

To a 4 mL vial was added (*R*)-4'-[2-(2-methyl-pyrrolidin-1-yl)-ethyl]-biphenyl-4-sulfinic acid, sodium salt ((200 mg, 569 μmol), DMSO (2.0 mL) and allyl bromide (89.5 mg, 64.0 μl, 740 μmol). The mixture was stirred at 80 °C for 6 h, at which point DCM (20 mL) was added. The resulting suspension was filtered, washed with water (10 mL), dried with $Na_2SO_4$, and concentrated. The crude mixture was purified by prep HPLC (0.1% TFA in acetonitrile/0.1% TFA in water). The fractions were combined and lyophilized to afford the title compound (77.6 mg, 29%) as a thick, tacky oil. Exact mass calculated for $C_{22}H_{27}NO_2S$: 369.2, Found: LCMS *m/z* = 370.1 (M+H$^+$); $^1$H NMR (400 MHz, $CDCl_3$) δ ppm 1.28 (d, *J* = 6.82 Hz, 0.3 H), 1.56 (d, *J* = 6.57 Hz, 2.7 H), 1.90 - 2.10 (m, 2 H), 2.19 - 2.32 (m, 2 H), 2.85 - 3.03 (m, 2 H), 3.04 - 3.16 (m, 1 H), 3.16 - 3.36 (m, 2 H), 3.57 - 3.68 (m, 1 H), 3.85 (d, *J* = 7.33 Hz, 2 H), 4.00 - 4.18 (m, 1 H), 5.20 (dd, *J* = 16.93, 1.01 Hz, 1H), 5.37 (d, *J* = 10.11 Hz, .1H), 5.76 - 5.90 (m, 1 H), 7.37 (d, *J* = 8.08 Hz, 2 H), 7.57 (d, *J* = 8.08 Hz, 2 H), 7.72 (d, *J* = 8.59 Hz, 2 H), 7.92 (d, *J* = 8.59 Hz, 2 H).

**Example 1.38: Preparation of (*R*)-5-(4-(2-(2-methylpyrrolidin-1-yl)ethyl)phenyl)-3-(methylsulfonyl)-1,2,4-thiadiazole (Compound 37).**

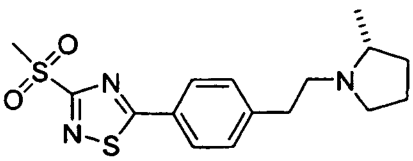

To a vial was added 5-chloro-3-(methylsulfonyl)-1,2,4-thiadiazole (125 mg, 0.629 mmol), (*R*)-4-[2-(2-methylpyrrolidin-1-yl)ethyl]phenylboronic acid (161 mg, 0.692 mmol), $K_2CO_3$ (174 mg, 1.26 mmol), and dihydrogen dichlorobis (di-tert-butylphosphinito-κP)palladate (6 mg, 0.013 mmol) in a mixture of EtOH (1 mL) and benzene (3 mL). The resulting reaction mixture was heated at 100 °C for 60 min under microwave irradiation. Upon completion, the organic layer was pipetted out and the aqueous layer was extracted with EtOAc. The crude mixture was concentrated and purified by HPLC (0.1% TFA in acetonitrile/0.1% TFA in water). The combined pure fractions were lyophilized to afford the TFA salt of the title compound (7.6 mg, 5% yield) as a yellow oil. Exact mass calculated for $C_{16}H_{21}N_3O_2S_2$: 351.1, Found: LCMS *m/z* = 352.2 (M+H$^+$); $^1$H NMR (400 MHz, $CDCl_3$) δ ppm 1.25 - 1.40 (m, 3 H), 1.64 (d, *J* = 6.32 Hz, 3 H), 1.91 - 2.43 (m, 4 H), 2.93 - 3.53 (m, 5 H), 3.70 (s, 1 H), 4.06 (s, I H), 7.37 - 7.54 (m, 2 H), 8.00 (d, *J* = 7.83 Hz, 2 H).

**Example 1.39: Preparation of (*R*)-2-Methyl-1-(2-(4'-((tetrahydro-2*H*-pyran-2-yl)methylsulfonyl)biphenyl-4-yl) ethyl)pyrrolidine (Compound 41).**

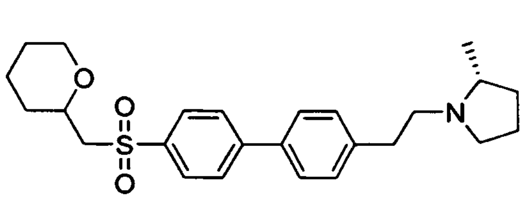

The title compound was prepared in a similar manner as described in **Example 1.35**, **Step C**, using 2-((4-bromophenylsulfonyl)methyl)tetrahydro-2*H*-pyran (185 mg, 0.580 mmol) and (*R*)-4-[2-(2-methylpyrrolidin-1-yl)ethyl]phenylboronic acid (149 mg, 0.638 mmol) as starting materials to give a clear oil (HCl salt) in 35% yield. Exact mass calculated for $C_{25}H_{33}NO_3S$: 427.2, Found: LCMS *m/z* = 428.3 (M+H$^+$); $^1$H NMR (400 MHz, DMSO-*d$_6$*) δ ppm 1.16 - 1.53 (m, 7 H),

1.55 - 1.80 (m, 3 H), 1.85 - 2.06 (m, 2 H), 2.12 - 2.26 (m, I H), 3.04 - 3.31 (m, 5 H), 3.34 - 3.58 (m, 4 H), 3.57 - 3.79 (m, 3 H), 7.47 (d, *J* = 8.08 Hz, 2 H), 7.76 (d, *J* = 8.34 Hz, 2 H), 7.88 - 7.99 (m, 4 H).

**Example 1.40: Preparation of (*R*)-2,2-Dimethyl-3-(4'-(2-(2-methylpyrrolidin-1-yl)ethyl)biphenyl-4-ylsulfonyl) propan-1-ol (Compound 40).**

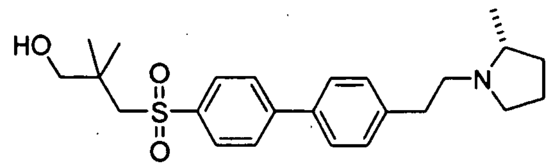

The title compound was prepared in a similar manner as described in **Example 1.35,** Step C, using 3-(4-bromophenylsulfonyl)-2,2-dimethylpropan-1-ol (149 mg, 0.485 mmol) and (*R*)-4-[2-(2-methylpyrrolidin-1-yl)ethyl]phenylboronic acid (124 mg, 0.534 mmol) as starting materials to give a clear oil (HCl salt) in 56% yield. Exact mass calculated for $C_{24}H_{33}NO_3S$: 415.2, Found: LCMS *m/z* = 416.4 ($M+H^+$);$^1$HNMR (400 MHz, $CDCl_3$) δ ppm 1.15-1.27 (m, 4 H), 1.28 - 1.40 (m, 2 H), 1.69 (d, *J* = 6.57 Hz, 3 H), 2.00 - 2.18 (m, 2 H), 2.23 - 2.42 (m, 2 H), 2.89 - 3.10 (m, 2 H), 3.11 - 3.39 (m, 4 H), 3.47 (dd, *J* = 12.63, 3.79 Hz, 1H), 3.63 (s, 1 H), 3.74 (s, 1 H), 4.06 (s, 1 H), 4.45 (s, 1 H), 7.41 (d, *J* = 8.08 Hz, 2 H), 7.60 (d, *J* = 8.08 Hz, 2 H), 7.77 (d, *J* = 8.34 Hz, 2 H), 8.00 (t, *J* = 9.09 Hz, 2 H).

**Example 1.41: Preparation of (R)-4-(4'-(2-(2-Methylpyrrolidin-1-yl)ethyl)biphenyl-4-ylsulfonyl)piperidine (Compound 36).**

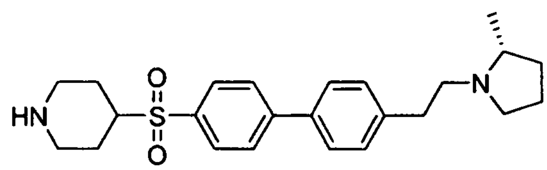

The title compound was prepared in a similar manner as described in **Example 1.28** using 4-{(4-bromophenyl) sulfonyl]piperidine hydrochloride (150 mg, 0.440 mmol) and (*R*)-4-[2-(2-methylpyrrolidin-1-yl)ethyl]phenylboronic acid (113 mg, 0.484 mmol) as starting materials to give a yellow oil (TFA salt) in 70% yield. Exact mass calculated for $C_{24}H_{32}N_2O_2S$: 412.2, Found: LCMS *m/z* = 413.2 ($M+H^+$); $^1$NMR (400 MHz, Methanol-$d_4$) δ ppm 1.32 (d, *J* = 6.82 Hz, 0.3 H), 1.48 (d, *J* = 6.32 Hz, 2.7 H), 1.70 - 1.82 (m, 1 H), 1.85 - 1.99 (m, 2 H), 2.00 - 2.19 (m, 2 H), 2.23 (d, *J* = 11.87 Hz, 2 H), 2.29 - 2.41 (m, 1 H), 2.95 - 3.36 (m, 7 H), 3.45 - 3.71 (m, 5 H), 3.72 - 3.83 (m, 1 H), 7.48 (d, *J* = 8.08 Hz, 2 H), 7.71 (d, *J* = 8.34 Hz, 2 H), 7.89 - 8.01 (m, 4 H).

**Example 1.42: Preparation of (*R*)-1-(2-(4'-(Methoxymethylsulfonyl)biphenyl-4-yl)ethyl)-2-methylpyrrolidine (Compound 42).**

**Step A: Preparation of (4-Bromophenyl)(methoxymethyl)sulfane.**

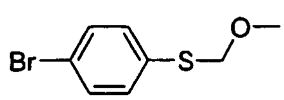

To a solution of 4-bromobenzenethiol (500 mg, 2.64 mmol) in THF (5 mL) was added triethylamine (0.737 mL, 5.29 mmol), chloromethyl methyl ether (0.301 mL, 3.97 mmol). The resulting mixture was stirred at 0 °C for 2 h in a

sealed scintillation vial. The reaction mixture was diluted with water and extracted twice with EtOAc. The organic layer were combined, dried over $MgSO_4$ filtered, and concentrated to afford the title compound (585 mg, 95% yield) as a yellow oil. $^1$H NMR (400 MHz, $CDCl_3$) δ ppm 3.47 (s, 3 H), 4.98 (s, 2 H), 7.33 - 7.41 (m, 2H), 7.41 - 7.52 (m, 2H).

**Step B: Preparation of 1-Bromo-4-(methoxymethylsulfonyl)benzene.**

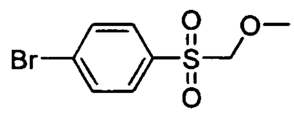

To a solution of (4-bromophenyl)(methoxymethyl)sulfane (585 mg, 2.51 mmol) in THF/water (10 mL/5 mL) was added Oxone® (1.85 g, 3.01 mmol). The resulting mixture was stirred for 18 h at room temperature. The reaction was diluted with water and extracted twice with EtOAc. The combined layers were dried over $MgSO_4$, filtered, and concentrated to afford the title compound (610 mg, 92% yield) as a white solid. $^1$H NMR (400 MHz, $CDCl_3$) δ ppm 3.70 (s, 3H), 4.54 (s, 2 H), 7.67 - 7.90 (m, 4 H).

**Step C: Preparation of (R)-1-(2-(4'-(Methoxymethylsulfonyl)biphenyl-4-yl)ethyl)-2-methylpyrrolidine (Compound 42).**

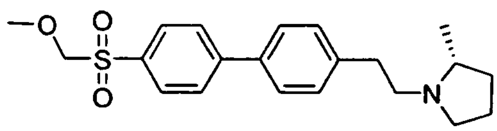

To a vial was added 1-bromo-4-(methoxymethylsulfonyl)benzene (100 mg, 0.377 mmol), (*R*)-4-[2-(2-methylpyrrolidin-1-yl)ethyl]phenylboronic acid (88 mg, 0.377 mmol), dicyclohexyl(2',4',6'-triisopropylbiphenyl-2-yl)phosphine (9.0 mg, 0.019 mmol), diacetoxypalladium (1.7 mg, 7.54 μmol) and potassium phosphate (240 mg, 1.132 mmol) in tetrahydrofuran (4 mL). The resulting reaction mixture was heated at 100 °C for 60 min under microwavc irridiation. The reaction mixture was diluted with water and the organic phase was separated The aqueous layer was extracted with EtOAc. The combined organic layers were concentrated, dissolved in ACN/$H_2O$ (with AcOH) and purified by HPLC (0.1% TFA in acetonitrile/01% TFA in water). Fractions were combined, basified with 2 M $Na_2CO_3$ and extracted three times with EtOAc. The combined organic layers were dried over $MgSO_4$, filtered, and concentrated. The residue was dissolved in MeOH (5 mL). Then, HCl (1 M in $Et_2O$, 1.65 mL) was added followed by EtOAc (5 mL). The resulting mixture was concentrated to afford the hydrochloride salt of the title compound (45.1 mg, 29% yield) as a yellow solid. Exact mass calculated for $C_{21}H_{27}NO_3S$: 373.2, Found: LCMS *m/z* = 374.2 (M+H$^+$); $^1$H NMR (400 MHz, Methanol-*d₄*) δ ppm 1.29 (brs, 0.3 H), 1.53 (brs, 2.7 H), 1.80 (brs, 1 H), 2.13 (brs, 2 H); 2.37 (brs, 1 H), 3.08 - 3.42 (m, 5 H), 3.50 - 3.70 (m, 5 H), 3.80 (brs, 1 H), 4.68 (s, 2 H), 7.50 (brs, 2 H), 7.70 (brs, 2 H), 7.88 (d, *J* = 6.57 Hz, 2 H), 7.98 (d, *J* = 6.82 Hz, 2 H).

**Example 1.43: Preparation of (*R*)-*N*-Methyl-3-(4'-(2-(2-methylpyrrolidin-1-yl)ethyl)biphenyl-4-ylsulfonyl)propanamide (Compound 34).**

**Step A: Preparation of 3-(4-Chlorophenylsulfonyl)propanoyl chloride.**

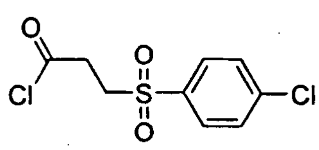

To a solution of 3-(4-chlorophenylsulfonyl)propanoic acid (431 mg, 1.73 mmol) and oxalyl chloride (330 mg, 0.228 ml, 2.60 mmol) in DCM (3 mL) was added dropwise DMF (0.3 mL). The reaction was allowed to stir for an additional 45 min. After 45 min, the reaction mixture was concentrated to afford the title compound (463 mg, 100%) as a white solid, which was used without further purification. $^1H$ NMR (400 MHz, $CDCl_3$) δ ppm 3.36 - 3.44 (m, 2 H), 3.45 - 3.54 (m, 2 H), 7.63 (d, *J* = 8.84 Hz, 2 H), 7.89 (d, *J* = 8.84 Hz, 2 H).

**Step B: Preparation of 3-(4-Chlorophenylsulfonyl)-N-methylpropanamide.**

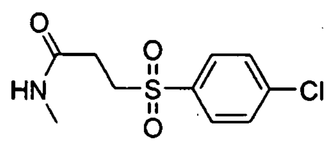

To a solution of methylamine hydrochloride (38 mg, 0.562 mmol), potassium acetate (331 mg, 3.37 mmol) dissolved in water (1 mL) was slowly added a solution of 3-(4-chlorophenylsulfonyl)propanoyl chloride (100 mg, 0.374 mmol) dissolved in THF (1 mL). The reaction mixture was allowed to stir at room temperature for 2 h. After 2 h, LCMS indicated the reaction was complete. The reaction mixture was diluted with water and the organic phase was separated. The aqueous layer was extracted with EtOAc. The combined organic layers were concentrated, dissolved in $ACN/H_2O$ (with AcOH) and purified by HPLC (0.1% TFA in acetonitrile/0.1% TFA in water). The combined fractions were basified with 2 M $Na_2CO_3$ and extracted three times with EtOAc. The combined organic layers were dried over $MgSO_4$, filtered, and concentrated to afford the title compound (26 mg, 27%) as a white solid. $^1H$ NMR (400 MHz, $CDCl_3$) δ ppm 2.55 - 2.64 (m, 2 H), 2.68 (d, *J* = 4.80 Hz, 3 H), 3.34 - 3.49 (m, 2 H), 5.92 (brs, 1 H), 7.49 (d, *J* = 8.85 Hz, 2 H), 7.78 (d, *J* = 8.59 Hz, 2 H).

**Step C: Preparation of (*R*)-*N*-Methyl-3-(4'-(2-(2-methylpyrrolidin-1-yl)ethyl)biphenyl-4-ylsulfonyl)propanamide (Compound 34).**

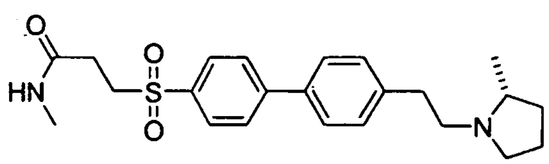

To a vial was added 3-(4-chlorophenylsulfonyl)-*N* methylpropanamide (26 mg, 0.099 mmol), (*R*)-4-[2-(2-methylpyrrolidin-1-yl)ethyl]phenylboronic acid (23 mg, 0.099 mmol), dicyclohexyl(2',4',6'-triisopropylbiphenyl-2-yl)phosphine (2.4 mg, 4.97 μmol), diacetoxypalladium (0.446 mg, 1.987 μmol) and potassium phosphate (63 mg, 0.298 mmol) in tetrahydrofuran (4 mL). The resulting reaction mixture was heated at 120 °C for 60 min under microwave irradiation. Next, the organic layer was pipetted out and the aqueous layer was extracted with EtOAc. The crude mixture was concentrated and purified by HPLC (0.1% TFA in acetonitrile/0.1% TFA in water). The combined pure fractions were lyophilized to afford the TFA salt of the title compound (29 mg, 55% yield) as a yellow oil. Exact mass calculated for $C_{23}H_{30}N_2O_3S$: 414.2, Found: LCMS *m/z* = 415.1 ($M+H^+$); $^1H$ NMR (400 MHz, Methanol-$d_4$) δ ppm 1.34 (d, *J* = 6.82 Hz, 0.3 H), 1.49 (d, *J* = 6.57 Hz, 2.7 H), 1.69 - 1.85 (m, 1 H), 2.01 - 2.25 (m, 2 H), 2.29 - 2.43 (m, 1 H), 2.53 - 2.68 (m, 6 H), 2.99 - 3.37 (m, 3 H), 3.47 - 3.61 (m, 4 H), 3.62 - 3.73 (m, 1 H), 3.73 - 3.86 (m, 1 H), 7.49 (d, *J* = 8.08 Hz, 2 H), 7.72 (d, *J* = 8.34 Hz, 2 H), 7.89 (d, *J* = 8.59 Hz, 2 H), 7.99 (d, *J* = 8.33 Hz, 2 H).

**Example 1.44: Preparation of (R)-3-(4'-(2-(2-Methylpyrrolidin-1-yl)ethyl)biphenyl-4-ylsulfonyl)-1-morpholinopropan-1-one (Compound 35).**

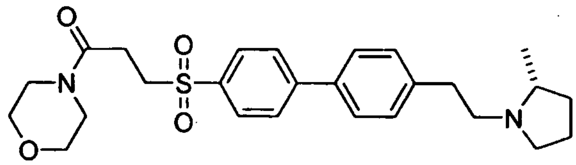

The title compound was prepared in a similar manner as described in **Example 1.43, Step C**, using 3-(4-chlorophenylsulfonyl)-1-morpholinopropan-1-one (20 mg, 0.063 mmol), (R)-4-[2-(2-methylpyrrolidin-1-yl)ethyl]phenylboronic acid (15 mg, 0.063 mmol) as starting materials to give a white solid (TFA salt) in 51% yield. Exact mass calculated for $C_{26}H_{34}N_2O_4S$: 470.2, Found: LCMS *m/z* = 471.6 (M+H$^+$); $^1$H NMR (400 MHz, Methanol-d$_4$) δ ppm 1.34 (d, *J* = 6.82 Hz, 0.3 H), 1.49 (d, *J*= 6.56 Hz, 2.7 H), 1.70 - 1.85 (m, 1 H), 2.00 - 2.25 (m, 2 H), 2.30 - 2.44 (m, 1 H), 2.85 (t, *J* = 7.33 Hz, 2 H), 3.04 - 3.38 (m, 4 H), 3.45 - 3.74 (m, 12 H), 3.73 - 3.85 (m, 1H), 7.49 (d, *J* = 8.08 Hz, 2 H), 7.74 (d, *J* = 8.08 Hz, 2 H), 7.91 (d, *J* = 8.34 Hz, 2 H), 8.02 (d, *J* = 8.34 Hz, 2 H).

**Example 1.45: Preparation of (*S*)-1-(2-(4'-(2-Methoxyethylsulfonyl)biphenyl-4-yl)ethyl)-2-methylpyrrolidine (Compound 39).**

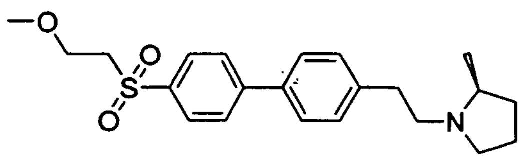

To the reaction vessel was added 4-(2-chloroethyl)-4'-(2-methoxyethylsulfonyl)biphenyl (75 mg, 0.221 mmol), sodium carbonate (70 mg, 0.664 mmol), and (*S*)-2-methylpyrrolidine (57 mg, 0.664 mmol) in acetonitrile (1 mL). The reaction mixture was refluxed for 18 h and then filtered. The filtrate was concentrated, taken up in DMSO, and purified by HPLC (0.1% TFA in acetonitrile/0.1%TFA in water). The combined fractions were lyophilized to afford the TFA salt of the title compound (9.5 mg, 8.5% yield) as a white solid. Exact mass calculated for $C_{22}H_{29}NO_3S$: 387.19, Found: LCMS *m/z* = 388.2 (M+H$^+$); $^1$H NMR (400 MHz, Methanol-*d$_4$*) δ ppm 1.33 (d, *J* = 6.82 Hz, 0.3 H), 1.48 (d, *J* = 6.57 Hz, 2.7 H), 1.70 - 1.81 (m, 1H), 2.02 - 2.19 (m, 2 H), 2.31 - 2.40 (m, 1 H), 3.04 - 3.21 (m, 5 H), 3.23 - 3.29 (m, 2 H), 3.49 - 3.57 (m, 3 H), 3.61 - 3.70 (m, 1 H), 3.71 - 3.81 (m, 3 H), 7.47 (d, *J* = 8.08 Hz, 2 H), 7.71 (d, *J* = 8.08. Hz, 2 H), 7.86 (d, *J* = 8.59 Hz, 2 H), 7.98 (d, *J* = 8.59 Hz, 2 H).

**Example 1.46: Preparation of Intermediate 2-Biphenyl-4-yl-ethanol.**

**Method 1**: To dilute and vent the hydrogen gas byproduct, the reactor was purged with nitrogen throughout the entire preparation until the quench with aqueous sodium hydroxide had been completed. To a 50 L reactor containing isopropyl acetate (16.7 L) was added sodium borohydride (0.762 kg, 20.14 mol). The sodium borohydride was rinsed into the reactor with additional isopropyl acetate (1 L), and the stirred reactor contents were cooled to 2 ˚C. 4-Biphenylacetic acid (2.67 kg, 12.58 mol) was then added sufficiently slowly to maintain the stirred reactor contents at 2-13 ˚C with reactor jacket cooling. The biphenylacetic acid was rinsed into the reactor with additional isopropyl acetate (1 L), and the stirred reactor contents were cooled to -4 ˚C. Borontrifluoride diethyletherate (3.2 L, 3.584 kg, 25.25 mol) was then added at a constant rate over two hours while the stirred reactor contents were maintained at -4 ˚C to 6 ˚C with reactor jacket cooling. The reactor contents were warmed to 20 ˚C, and LC/MS analysis revealed the 4-biphenylacetic acid conversion to be 95% after 19 min. After the reaction mixture had been stirred at 15-22 ˚C for 16.6 h, it was cooled to 1 ˚C. Six liters of a 50 wt % solution of sodium hydroxide in water was mixed with nine liters of deionized water. Eleven liters of the resulting diluted aqueous solution of sodium hydroxide was added to the stirred reaction mixture sufficiently slowly to maintain the reactor contents at 1-12 ˚C with reactor jacket cooling. The resulting mixture was stirred at 12-20 ˚C for 30 min and then heated to 40˚C. Stirring at 40-48 ˚C was continued for 31 minutes, and the mixture was then cooled to 22 ˚C. Sodium chloride (0.50 kg), deionized water (2.0 L), and methanol (500 mL) were added to the stirred mixture to facilitate phase separation. The phases were then allowed to separate. The aqueous phase was drained, and the organic (upper) phase was washed with deionized water (4 x 5.0 L). The upper organic phase was concentrated

by vacuum distillation to 11 L volume at temperatures rising to 59 ˚C. The reactor contents were cooled to 20 ˚C, and heptane (20 L) was added. The stirred reactor contents were concentrated by vacuum distillation to 16 L volume at temperatures rising to 36 ˚C. The stirred reactor contents were cooled to 20˚C, stirred at that temperature for 21.4 h, and then filtered. The filtered solid was washed with heptane (5 L) and vacuum dried at ambient temperature to provide the title compound (2.148 kg, 86.1%).

**Method 2:** Sodium borohydride (1.2 g, 31.7 mmol) was added to a flask containing tetrahydrofuran (THF) (40 mL) stirred under nitrogen. 4-Biphenylacetic acid (5.0 g, 23.6 mmol) was then added slowly over 20 min while the stirred reaction mixture was maintained at 20-30 ˚C. A THF rinse (5 mL) was then added, and the resulting mixture was stirred for 20 min. A solution of iodine (2.9 g, 11.4 mmol) in THF (10 mL) was then added slowly over about an hour while the stirred reaction mixture was maintained at 20-30 ˚C. Stirring at that temperature was continued for about an hour, at which time chromatographic analysis revealed complete conversion of 4-biphenylacetic acid. The reaction mixture was then quenched by addition of 5 wt % aqueous sodium hydroxide (26 mL). After the resulting mixture had been mixed well, the phases were allowed to separate, and the lower phase was drained from the upper phase. The lower (aqueous) phase was extracted with isopropyl acetate (2 x 25 mL). The upper (organic) phases were combined, washed with deionized water (4 x 20 mL), and evaporated at reduced pressure to provide 2-(biphenyl-4'-yl)ethanol as a light yellow crystalline residue (4.6 g, 98.5%). Alternatively, product may be crystallized by exchange of isopropyl acetate with heptane as described in Method 1 above.

**Example 1.47: Preparation of (*R*)-1-{2-[4'-(3-Methoxy-propane-1-sulfonyl)-biphenyl-4-yl]-ethyl}-2-methyl-pyrrolidine (Compound 8).**

**Step A: Preparation of Intermediate 4'-(2-Chloro-ethyl)-biphenyl-4-sulfonyl Chloride.**

To a nitrogen-purged reactor vented to an aqueous sodium hydroxide scrubber was added 4'-(2-chloro-ethyl)-biphenyl-4-sulfonic acid (2.101 kg after correction for 3.7 wt % water content, 7.08 mol) followed by thionyl chloride (5.358 L, 8.74 kg, 73.5 mol). The resulting mixture was stirred and cooled to -2.5 ˚C. *N,N*-Dimethylacetamide (68 mL, 63.7 g, 0.731 mol) was then added sufficiently slowly to maintain the stirred reactor contents at -3 to 0 ˚C with reactor jacket cooling. The reactor contents were heated to 63 ˚C, and stirring at 63-66 ˚C was continued for 6.4 h until conversion of the starting material to the product was verified to be substantially complete by LC/MS analysis. After the reactor contents had been cooled to 19 ˚C, heptane (7.62 L) was added in six equal portions over 2.5 h. Volatiles consisting mostly of thionyl chloride were then distilled off the product mixture at 30-32 ˚C and pressures falling to 109 torr. The condensate volume was 4 L. The concentrated product mixture was cooled to 22 ˚C, and stirring at 20-22 ˚C was continued for 15.4 h. The product mixture was then filtered. The filtered solid was washed with heptane (11 L) and then deionized water (11 L), both at ambient temperature, and then vacuum dried at 40 ˚C to constant weight to provide 4'-(2-chloroethyl)-biphenyl-4-sulfonyl chloride (1.664 kg, 74.6% yield, 97.9% purity by HPLC peak area).

**Step B: Preparation of Intermediate Sodium 4'-(2-Chloroethyl)-4-biphenylsulfinate.**

To a nitrogen-purged reactor containing deionized water (14.1 L) stirred at 29 ˚C was added sodium sulfite (3.3145 kg, 26.3 mol), $Na_2HPO_4$ (0.7459 kg, 5.25 mol) and benzyltriethylammonium chloride (65.1 g, 0.265 mol). Addition of the benzyltriethylammonium chloride caused the temperature of the reactor contents to rise to 37 ˚C. All the reagents dissolved upon continued stirring at 35-37 ˚C for 16 min, after which 4'-(2-chloro-ethyl)-biphenyl-4-sulfonyl chloride (1.6584 g, 5.26 mol) and a deionized water rinse (2.5 L) were added. The temperature of the stirred reactor contents was then increased to 57 ˚C, and stirring at 57-60 ˚C under nitrogen was continued for 6 h until LC/MS analysis revealed complete conversion of the starting material. The stirred mixture was cooled to 42 ˚C and then filtered. Deionized water (8.3 L) was added to the reactor and heated with stirring to 36 ˚C. The filtered solids were then added back to the reactor, and the resulting mixture was stirred at 38 ˚C overnight before being filtered. The filtered solid was washed at ambient temperature first with deionized water (3.3 L) and then twice with acetonitrile (3.3 L and then 2.8 L). The washed solids were vacuum dried at 60 ˚C to provide crude white sodium 4'-(2-chloroethyl)-4-biphenylsulfinate (1.2282 kg, 77.1 % yield, 92.6% pure by HPLC peak area) containing 4'-(2-chloro-ethyl)-biphenyl-4-sulfonic acid (7.4 HPLC area %).

**Step C: Preparation of 4-(2-Chloro-ethyl)-4'-(3-methoxy-propane-1-sulfonyl)-biphenyl and 4-(2-Bromo-ethyl) 4'-(3-methoxy-propane-1-sulfonyl)-biphenyl.**

To a stirred mixture of sodium 4'-(2-chloroethyl)-4-biphenylsulfinate (217.9 g, 719.7 mmol, ), sodium phosphate, dibasic (102.2 g, 719.7 mmol), tetrabutylammonium bromide (TBAB) (232.0 g, 719.7 mmol), potassium bromide (85.65 g, 719.7 mmol) and deionized water (809 mL) was added 1-bromo-3-methoxypropane (137.7 g, 899.9 mmol) at ambient temperature. The resulting mixture became a clear solution as it was stirred and heated under nitrogen to 80 ˚C. After

the reaction mixture had been stirred at 80 ˚C for 16 h, additional 1-bromo-3-methoxypropane (12.11 g, 79.1 mmol) was added. After another 4 h of stirring at 80 ˚C, more 1-bromo-3-methoxypropane (6.0 g, 39.2 mmol) was added. Heating at 80 ˚C was continued for two more hours (for a total of 22 h) and then discontinued. Methanol (1.09 L) was added when the mixture had cooled to about 65 ˚C, and the stirred mixture was then allowed to cool to ambient temperature overnight. The resulting white precipitate was filtered, slurry-washed with deionized water (2 x 500 mL), air-dried, and then stirred in ethyl acetate (1.0 L) for 1 h at ambient temperature. The mixture was filtered through a silica gel plug to remove TBAB, producing a clear yellow filtrate. The solvent was removed under reduced pressure, resulting in a yellowish-white solid. The solid was slurry-washed in heptane (2 x 500 mL) at ambient temperature, filtered, and air-dried, resulting in very little purification. The heptane-washed solids (294.8 g) were dissolved in anhydrous ethanol (1.0 L) at 73.4 ˚C. The stirred solution was allowed to cool to ambient temperature and was then placed in an ice-water bath for 30 min. The white solids were filtered, slurry-washed in ethanol (2 x 500 mL), and then vacuum dried first at 40 ˚C for 15 h and then at 60 ˚C for 9 h. The resulting solid (178.9 g, 66.0%) was determined to be 43.5% 4-(2-chloro-ethyl)-4'-(3-methoxy-propane-1-sulfonyl)-biphenyl and 50.6% 4-(2-bromo-ethyl)-4'-(3-methoxy-propane-1-sulfonyl)-biphenyl by HPLC peak area. Exact mass calculated for $C_{18}H_{21}ClO_3S$: 352.09, Found: LCMS *m/z* = 353.1 ($M+H^+$); Exact mass calculated for $C_{18}H_{21}BrO_3S$: 396.04, Found: LCMS *m/z* (%) = 397.2 ($M+H^{+78}Br$, 100), 399.0 ($M+H^{+80}Br$, 97); $^1$H NMR (400 MHz, DMSO-$d_6$) δppm 1.72-1.83 (m, 2H), 3.07-3.13 (Cl, t, *J*= 7.00 Hz, 2 H), 3.12-3.19 (s, 3 H), 3.16-3.24 (Br, t, *J* = 7.18, 2 H), 3.31-3.39 (m, 4 H), 3.25-3.33 (Br, t, *J* = 7.15 Hz, 2 H), 3.88-3.95 (Cl, t, *J* = 6.99 Hz, 2 H), 7.41-7.48 (d, *J* = 7.09 Hz, 2 H), 7.69-7.74 (d, *J* = 8.13 Hz, 2 H), 7.93-7.97 (m, 4 H).

**Step D: Preparation of Intermediate 4-(2-Bromo-ethyl)-4'-(3-methoxy-propane-1-sulfonyl)-biphenyl.**

A total of 161.8 g of the mixture of 4-(2-chloro-ethyl)-4'-(3-methoxy-propane-1-sulfonyl)-biphenyl and 4-(2-bromo-ethyl)-4'-(3-methoxy-propane-1-sulfonyl)-biphenyl prepared in the previous example (43.5% and 50.6% respectively by HPLC peak area) was dissolved in acetonitrile (1.0 L) at ambient temperature. TBAB (88.71 g, 275.2 mmol) and LiBr (95.84 g, 1104 mmol) were added and rinsed into the reaction flask with more acetonitrile (600 mL for a total of 1.6 L). As the resulting mixture was stirred and heated at 60-65 ˚C under nitrogen for 44 h, additional LiBr was added: 94.75 g (1091 mmol) after 5.5 h; 99.82 g (1149 mmol) after 20 h; and 64.49 g (743 mmol) after 28 h. The reaction mixture was then allowed to cool to 33 ˚C. The liquid phase of the reaction mixture was decanted from the solids, which were rinsed with acetonitrile. The rinse was added to the supernatant, and the solvent was removed under reduced pressure. Deionized water (1.5 L) was added to the evaporation residue. A white solid precipitated, and the resulting mixture was stirred for 1.0 h at ambient temperature. The solids were filtered, washed with deionized water (3 x 500 mL), and vacuum dried at 40-45 ˚C for 4 days. The dried solids were dissolved in a mixture of ethyl acetate (3.4 L) and acetonitrile (3.1 L) and, the resulting solution was filtered through a silica gel plug that was subsequently washed with acetonitrile (2 x 500 mL). The filtrate and washes were combined, and solvent was removed under reduced pressure. The evaporation residue was vacuum dried at 45 ˚C to provide a white solid (160.2 g, 99.0% recovery) found to be 10.7% 4-(2-chloro-ethyl)-4'-(3-methoxy-propane-1-sulfonyl)-biphenyl and 85.6% 4-(2-bromo-ethyl)-4'-(3-methoxy-propane-1-sulfonyl)-biphenyl by HPLC peak area. Exact mass calculated for $C_{18}H_{21}BrO_3S$: 396.04, Found: LCMS *m/z* (%) = 397.2 ($M+H^{+78}Br$, 100), 399.0 ($M+H^{+80}Br$, 97); $^1$HNMR (400 MHz, DMSO-$d_6$) δ ppm 1.72-1.83 (m, 2 H), 3.12-3.19 (s, 3 H), 3.16-3.24 (t, *J* = 7.18 Hz, 2 H), 3.31-3.39 (m, 4 H) 3.25-3.33 (t, *J* = 7.15 Hz, 2 H), 7.41-7.48 (d, *J* = 7.09 Hz, 2 H), 7.69-7.74 (d, *J* = 8.13 Hz, 2 H), 7.93-7.97 (m, 4 H).

**Step E: Preparation of (*R*)-1-{2-[4'-(3-Methoxy-propane-1-sulfonyl)-biphenyl-4-yl-ethyl}-2-methyl-pyrrolidine (Compound 8).**

4-(2-Bromo-ethyl)-4'-(3-methoxy-propane-1-sulfonyl)-biphenyl and 4-(2-chloro-ethyl)-4'-(3-methoxy-propane-1-sulfonyl)-biphenyl (85.55% and 9.31% respectively by HPLC peak area) (198.4 g, 0.499 mol, based on the major starting material) was transferred to a 5 L 3-necked round-bottomed flask fitted with a mechanical stirrer, a temperature probe, a condenser and a nitrogen inlet. (*R*)-2-Methylpyrrolidine-L-tartrate (95.9 g) was added to the reaction flask followed by acetonitrile (2 L). To this mixture, stirred under nitrogen, potassium carbonate (213.9 g, 1.548 mol) was transferred followed by acetonitrile (380 mL, with washings). The slurry was warmed to 60 ˚C, and water (119 mL) was added slowly. Heating was continued overnight at 60 ˚C. The reaction mixture was cooled to room temperature when the starting 4-(2-bromo-ethyl)-4'-(3-methoxy-propane-1-sulfonyl)-biphenyl was not observed by LC/MS. The reaction mixture was concentrated by distillation of acetonitrile under reduced pressure. The residue was diluted with water (1.2 L) and extracted with ethyl acetate (2 x 600 mL followed by 500 mL). The combined organic layers were washed with 2 N HCl (2 x 600 mL followed by 500 mL). The combined aqueous layers were cooled by an ice bath and slowly neutralized with 50% aqueous NaOH (maintaining the internal temperature within 25 ˚C) and basified further to pH 12-14. The aqueous mixture was extracted with ethyl acetate (2 x 600 mL followed by 500 mL). The combined ethyl acetate layers were washed with water (2 x 600 mL followed by 500 mL) until the washings had neutral pH, dried over $MgSO_4$, filtered

and the solvent was removed under reduced pressure. Heptane (300 mL) was added and distilled off to remove residual ethyl acetate. The oily residue was dried overnight under vacuum to afford the crude product (126 g). The product was taken up in heptane (1.6 L), heated to 80 °C and stirred for 1 h. The product was dissolved in hot heptane and the impurities remained as a sticky solid. The solution was filtered hot and heptane was removed under reduced pressure. The residue was dried overnight under vacuum to obtain the product as a pale yellow, waxy solid (113.2 g). HPLC of the product showed 97.63% purity (by peak area). This was dissolved in ethyl acetate (700 mL) and washed with 2 N HCl (500 mL, containing 15% NaCl). Additional 2 N HCl (300 mL), and water (200 mL followed by 100 mL) were required for the separation of the layers. The organic layer was washed with an additional 2 N HCl (400 mL). The combined aqueous layers were washed with ethyl acetate (3 x 600 mL). HPLC of the acidic aqueous phase showed product purity as 99.09% (by peak area). The aqueous layer was extracted with ethyl acetate (600 mL) and then neutralized by slow addition of 50% aqueous NaOH, while maintaining the temperature below 25 °C with cooling by an ice bath. The aqueous layer was then basified further to pH 12-14. The aqueous mixture was extracted with ethyl acetate (2 x 600 mL), and the ethyl acetate extracts were washed with water (700 mL) followed by 5% NaCl solution (700 mL). The combined organic phase was dried over $MgSO_4$, filtered and the solvent was removed under reduced pressure. The residue was suspended in minimum volume of heptane which was distilled off under reduced pressure. The desired product was dried under vacuum to obtain a pale yellow, waxy solid (96.7g, 48.2%). HPLC purity: 99.04% (by peak area); chiral assay, 99.3% ee. Exact mass calculated for $C_{23}H_{31}NO_3S$: 401.20, Found: LCMS *m*/*z* = 401.8 $(M+H)^+$, 316.8, 285.2, 207.1, 179.8. NMR (400 MHz, DMSO-*d*$_6$) δ ppm 1.02 (d, *J*= 6 Hz, 3 H), 1.27 (m, 1 H), 1.64 (m, 2 H), 1.81 (m, 3 H), 2.13 (m, 1 H), 2.28 (broad m, 2 H), 2.79 (m, 2 H), 3.00 (m, 1 H), 3.15 (m, 1 H), 3.17 (s, 3 H), 3.35 (m, 4 H), 7.38 (d, *J* = 8.18 Hz, 2 H), 7.68 (d, *J* = 8.24 Hz, 2 H), 7.94 (s, 4 H).

**Step F: Preparation of (*R*)-1-{2-[4'-(3-Methoxy-propane-1-sulfonyl)-biphenyl-4-yl]-ethyl}-2-methyl-pyrrolidine Di-citrate (Compound 8).**

(*R*)-1-{2-[4'-(3-Methoxy-propane-1-sulfonyl)-biphenyl-4-yl]-ethyl -2-methyl-pyrrolidine free base (58.7 g, 0.146 mol) was transferred to a 1 L 3-necked round-bottomed flask, fitted with a mechanical stirrer and a nitrogen inlet. Acetonitrile (600 mL) was added, the mixture was stirred under nitrogen until a clear solution was obtained. To a 250 mL Erlenmeyer flask containing citric acid (59 g, 0.307 mol) was added water (29.5 mL) and the slurry was heated at 60 °C to obtain a clear solution. The warm solution of citric acid was added slowly into the acetonitrile solution of (*R*)-1-{2-[4'-(3-methoxy-propane-1-sulfonyl)-biphenyl-4-yl]-ethyl}-2-methyl-pyrrolidine free base. Additional water (5 mL) was used to wash the Erlenmeyer flask, and was added to the reaction mixture. After 5 min the solution became cloudy and the mixture was stirred at room temperature for 1.5 h. The mixture was filtered and the solids were washed with acetonitrile (300 mL) and dried in a vacuum oven at 40 °C under house vacuum (~15 Torr) to obtain (*R*)-1-{2-[4'-(3-methoxy-propane-1-sulfonyl)-biphenyl-4-yl]-ethyl}-2-methyl-pyrrolidine di-citrate (104.6 g, 91%). HPLC purity, 99.15% (by peak area). Chiral assay, 99.5% ee. Exact mass calculated for $C_{23}H_{31}NO_3S$: 401.20, Found: LCMS *m*/*z* = 402.0 $(M+H)^+$, 316.8, 285.0, 242.5, 207.1, 179.9, 137.0. NMR (400 MHz, DMSO-*d*$_6$) δ ppm 1.35 (d, *J* = 6.48 Hz, 3 H), 1.61 (m, 1 H), 1.79 (m, 2 H), 1.95 (m, 2 H), 2.18 (m, 1 H), 2.61 (m, 8 H), 3.05 (m, 2 H), 3.18 (s, 3 H), 3.2 (m, 2 H), 3.35 (m, 4 H), 3.5 (m, 3 H), 7.48 (d, *J* = 8.24 Hz, 2 H), 7.66 (d, *J* = 8.24 Hz, 2 H), 7.96 (s, 4 H).

**EXAMPLE 2: Salt screen.**

Freebase stock solutions were created by dissolving compounds in appropriate solvents and aliquoting to individual vials. Counter ions were added in molar excess to the individual vials of freebase stock solutions in an effort to create a unique salt form of the compound. Any precipitated material was collected, if possible, and characterized with PXRD and DSC being the typical first tier screening.

**Example 2.1: Salt Screen of 2-{4'-(2-((*R*)-2-Methyl-pyrrolidin-1-yl)-ethyl)-biphenyl-4-sulfonyl}-ethanol (Compound 10)**

2-(4'-[2-((*R*)-2-Methyl-pyrrolidin-1-yl)-ethyl]-biphenyl-4-sulfonyl)-ethanol was screened as described above in ethyl acetate, THF, methanol and acetone using the following counter ions: hydrochloric, hydrobromic, phosphoric, sulfuric, methanesulfonic, D-gluconic, DL-lactic, acetic, citric, tartaric, malonic and malic. The citric acid salt was isolated and characterized by PXRD & DSC.

**Example 2.2: Characterization of (*R*)-1-{2-[4'(2-Methoxy-ethanesulfonyl)-biphenyl-4-yl]-ethyl}-2-methyl-pyrrolidine hydrochloride salt (Compound 3).**

(*R*)-1-(2-[4'-(2-Methoxy-ethanesulfonyl)-biphenyl-4-yl]-ethyl) -2-methyl-pyrrolidine hydrochloride salt was characterized by PXRD, DSC and TGA.

**Example 2.3: Salt Screen of (*R*)-1-{2-[4'-(3-Methoxy-propane-1-sulfonyl)-biphenyl-4-yl]-ethyl}-2-methyl-pyrrolidine (Compound 8).**

(*R*)-1-{2-[4'-(3-Methoxy-propane-1-sulfonyl)-biphenyl-4-yl]-ethyl}-2-methyl-pyrrolidine was screened as described above in ethanol, methanol, acetone, isopropyl alcohol, ethyl acetate, THF, MTBE, acetonitrile, toluene and isopropyl acetate using the following counter ions: hydrochloric, hydrobromic, phosphoric, sulfuric, methanesulfonic, ethanesulfonic, benzenesulfonic, toluenesulfonic, D-gluconic, DL-lactic, acetic, citric, tartaric and malonic. The mono-citric acid and di-citric acid salts were isolated and characterized by PXRD, DSC, vapor sorption, NMR, FTIR and HPLC.

**EXAMPLE 3: [$^3$H] *N*-Alpha-Methyl-Histamine Competitive Histamine H3-receptor Binding Assay**

The histamine receptor binding assay was conducted using standard laboratory procedures as described below. A crude membrane fraction was prepared from whole rat brain cortex using a polytron to homogenize the tissue followed by differential centrifugation in a HEPES-based buffer containing protease inhibitors. Membranes where frozen at -80˚C until needed. Frozen membranes were thawed and resuspended in ice-cold assay buffer consisting of 50 mM TRIS containing 5 mM EDTA (pH = 7.4). 50 micrograms ($\mu$g) of membrane protein was added to each well of a 96-well assay plate along with test compound and [$^3$H]-*N*-$\alpha$-methyl-histamine (1 nanomolar (nM) final assay concentration). Imetit was used as an assay positive control at varying concentrations. The plate was incubated for 30 min at room temperature. The assay was terminated by rapid filtration through a 96-well glass fiber filtration plate (GF/C) using a cell harvester (Perkin-Elmer). Captured membranes were washed three times with cold assay buffer and plates were dried at 50˚C. 35 microliters ($\mu$L) of scintillation cocktail was added to each well and membrane-bound radioactivity was recorded using a TopCount 96-well plate scintillation counter (Perkin-Elmer).

The following table shows the observed activities for certain compounds of the present invention.

| Compound No. | $K_i$ Binding Assay (nM) |
|---|---|
| Cmpd 1 | 2.4 |
| Cmpd 3 | 3.0 |
| Cmpd 6 | 1.1 |
| Cmpd 8 | 1.5 |
| Cmpd 17 | 16 |

Certain other compounds of the invention had activity values ranging from about 16 nM to about 750 pM in this assay.

**EXAMPLE 4: Rat Polysomnography Protocol**

Animals: Male Sprague-Dawley rats (225-350 g) (Harlan, San Diego, CA) were singly housed and maintained in a humidity- (30-70%) and temperature- (20-22 ˚C) controlled facility on a 12 h:12 h light/dark cycle (lights on at 6:30 A.M.) with free access to food (Harlan-Teklad Western Res., Orange, CA, Rodent Diet 8604) and water. Rats were allowed at least three days of habituation to the animal facility before surgery.

**Procedures:**

Rats were anaesthetized with a ketamine/xylazine mixture, and surgically prepared for EEG and EMG recording. After 2-3 weeks of post-surgical recovery, rats were habituated to polypropylene test cages for at least three days. On test days, the rats were placed in the test chambers and habituated overnight. At 10 am the next day, the rats were administered the test compound, connected to the recording apparatus, and placed back into the test chambers for 3 h.

Data analysis

EEG and EMG data were digitized and stored in 10 s epochs over the three hour test period. These data were then visually scored, and each 10 s epoch characterized as either a non-REM sleep, REM sleep, or waking episode. Total wake time over the three hour period was calculated for each rat after either vehicle administration or test compound.

Percent increase in wakefulness was then derived for each rat.

The following table shows the observed percent increase in wakefulness over 1 h after oral administration of a representative compound at 0.6 mg/kg.

| Cmpd No. | % Increase in wakefulness +/- s.e. |
|---|---|
| Cmpd 10 | 46 ± 15 |

**EXAMPLE 5: Human Histamine H3-Receptor Binding Assay - MDS Pharma Services** (taiwan).

Compounds of the invention were tested for their ability to bind to the human histamine H3-receptor using the MDS Pharma Services (Taiwan) assay, Catalogue No. 239810. Certain compounds of the present invention and their corresponding activity values are shown in following table.

| Compound No. | Binding Assay (Ki, nM) |
|---|---|
| Cmpd 5 | 3.7 |
| Cmpd 7 | 2.9 |

Certain other compounds of the invention had activity values ranging from about 1.7 nM to about 10.1 nM in this assay.

**EXAMPLE 6: Blockade of RAMH-Induced Drinking Assay**

When administered to rodents, H3 agonists such as R-α-methyl-histamine (RAMH) induce a drinking response that is sensitive to reversal with an H3 antagonist. Blockade of RAMH-induced drinking can therefore be utilized as an *in vivo* assay for functional H3 antagonist activity. In this assay, male Sprague Dawley rats (250-350g) were housed three per cage and maintained under a reverse 12 h light cycle (lights off at 1130 h). At 1030 h on the day of test, rats were individually housed in new cages and food was removed. 120 min later, rats were administered test article (vehicle or H3 antagonist, 0.3 mg/kg PO). 30 min later, water was removed, and RAMH (vehicle or RAMH 3 mg/kg salt SC) was administered. 10 min after administration of RAMH, weighed water bottles were placed in the cages, and drinking was allowed for 20 min. Water consumption was determined for each animal by weighing each bottle to the nearest 0.1 g. Data is expressed as percentage reduction in water intake according to the following formula:

$$[1-[(ANTAGONIST/RAMH) - (VEHICLE/RAMH) / (VEHICLE/RAMH) - (VEHICLE/VEHICLE)]]*100$$

| Compound No. | % inhibition of RAMH-induced drinking |
|---|---|
| 2 | 82.6 ± 12.9 |
| 7 | 92.5 ± 15.8 |
| 15 | 58.8 ± 16.1 |

## Claims

**1.** A compound selected from compounds of Formula (**Ia**):

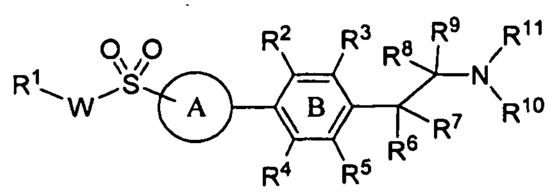

**(Ia)**

and pharmaceutically acceptable salts, hydrates and solvates thereof;
wherein:

$R^1$ is selected from H, $C_1$-$C_6$ acyl, $C_1$-$C_6$ acyloxy, $C_2$-$C_8$ alkenyl, $C_1$-$C_6$ alkoxy, $C_1$-$C_8$ alkyl, $C_1$-$C_8$ alkylcarboxamide, $C_2$-$C_8$ alkynyl, $C_1$-$C_8$ alkylsulfonamide, $C_1$-$C_8$ alkylsulfinyl, $C_1$-$C_8$ alkylsulfonyl, $C_1$-$C_8$ alkylthio, $C_1$-$C_8$ alkylureyl, amino, $C_1$-$C_8$ alkylamino, $C_2$-$C_8$ dialkylamino, carbo-$C_1$-$C_6$-alkoxy, carboxamide, carboxy, cyano, $C_3$-$C_7$ cycloalkyl, $C_2$-$C_8$ dialkylcarboxamide, $C_2$-$C_8$ dialkylsulfonamide, halogen, $C_1$-$C_6$ haloalkoxy, $C_1$-$C_6$ haloalkyl, $C_1$-$C_6$ haloalkylsulfinyl, $C_1$-$C_6$ haloalkylsulfonyl, $C_1$-$C_6$ haloalkylthio, $C_3$-$C_7$ heterocyclyl, hydroxyl, thiol, nitro, phenyl and sulfonamide, and each is optionally substituted with 1, 2, 3, 4 or 5 substituents selected independently from $C_1$-$C_6$ acyl, $C_1$-$C_6$ acyloxy, $C_2$-$C_8$ alkenyl, $C_1$-$C_6$ alkoxy, $C_1$-$C_8$ alkyl, $C_1$-$C_8$ alkylcarboxamide, $C_2$-$C_8$ alkynyl, $C_1$-$C_8$ alkylsulfonamide, $C_1$-$C_8$ alkylsulfinyl, $C_1$-$C_8$ alkylsulfonyl, $C_1$-$C_8$ alkylthio, $C_1$-$C_8$ alkylureyl, amino, $C_1$-$C_8$ alkylamino, $C_2$-$C_8$ dialkylamino, carbo-$C_1$-$C_6$-alkoxy, carboxamide, carboxy, cyano, $C_3$-$C_7$ cycloalkyl, $C_2$-$C_8$ dialkylcarboxamide, $C_2$-$C_8$ dialkylsulfonamide, halogen, $C_1$-$C_6$ haloalkoxy, $C_1$-$C_6$ haloalkyl, $C_1$-$C_6$ haloalkylsulfinyl, $C_1$-$C_6$ haloalkylsulfonyl, $C_1$-$C_6$ haloalkylthio, hydroxyl, thiol, nitro and sulfonamide; or

$R^1$ together with the W-$SO_2$ group and the ring atom to which the W-$SO_2$ group is bonded form a $C_5$-$C_7$ heterocyclic ring with Ring A whereby said $C_5$-$C_7$ heterocyclic ring and Ring A share two adjacent ring atoms, and said $C_5$-$C_7$ heterocyclic ring is optionally substituted with 1, 2, 3 or 4 substituents selected independently from $C_1$-$C_6$ acyl, $C_1$-$C_6$ acyloxy, $C_2$-$C_8$ alkenyl, $C_1$-$C_6$ alkoxy, $C_1$-$C_8$ alkyl, $C_1$-$C_8$ alkylcarboxamide, $C_2$-$C_8$ alkynyl, $C_1$-$C_8$ alkylsulfonamide, $C_1$-$C_8$ alkylsulfinyl, $C_1$-$C_8$ alkylsulfonyl, $C_1$-$C_8$ alkylthio, $C_1$-$C_8$ alkylureyl, amino, $C_1$-$C_8$ alkylamino, $C_2$-$C_8$ dialkylamino, carbo-$C_1$-$C_8$-alkoxy, carboxamide, carboxy, cyano, $C_3$-$C_7$ cycloalkyl, $C_2$-$C_8$dialkylcarboxamide, $C_2$-$C_8$ dialkylsulfonamide, halogen, $C_1$-$C_6$ haloalkoxy, $C_1$-$C_6$ haloalkyl, $C_1$-$C_6$ haloalkylsulfinyl, $C_1$-$C_6$ haloalkylsulfonyl, $C_1$-$C_6$ haloalkylthio, hydroxyl, thiol, nitro, oxo and sulfonamide;

W is $C_1$-$C_4$ alkylene, $C_2$-$C_4$ alkenylene, $C_3$-$C_7$ cycloalkylene, $C_3$-$C_7$ heterocyclylene or phenylene, each optionally substituted with 1, 2, 3, 4, 5, 6, 7 or 8 substituents selected independently from $C_1$-$C_3$ alkyl, $C_1$-$C_4$ alkoxy, carboxy, cyano, $C_1$-$C_3$ haloalkyl, halogen, hydroxyl and oxo;

Ring A is 1,3-phenylene or 1,4-phenylene, each substituted with $R^{12}$, $R^{13}$, $R^{14}$ and $R^{15}$, wherein $R^{12}$, $R^{13}$, $R^{14}$ and $R^{15}$ are each selected independently from H, $C_1$-$C_6$ acyl, $C_1$-$C_6$ acyloxy, $C_2$-$C_8$ alkenyl, $C_1$-$C_6$ alkoxy, $C_1$-$C_8$ alkyl, $C_1$-$C_8$ alkylcarboxamide, $C_2$-$C_8$ alkynyl, $C_1$-$C_8$ alkylsulfonamide, $C_1$-$C_8$ alkylsulfinyl, $C_1$-$C_8$ alkylsulfonyl, $C_1$-$C_8$ alkylthio, $C_1$-$C_8$ alkylureyl, amino, $C_1$-$C_8$ alkylamino, $C_2$-$C_8$ dialkylamino, carbo-$C_1$-$C_6$-alkoxy, carboxamide, carboxy, cyano, $C_3$-$C_7$ cycloalkyl, $C_2$-$C_8$ dialkylcarboxamide, $C_2$-$C_8$ dialkylsulfonamide, halogen, $C_{1\text{-}}C_6$ haloalkoxy, $C_1$-$C_6$ haloalkyl, $C_1$-$C_6$ haloalkylsulfinyl, $C_1$-$C_6$ haloalkylsulfonyl, $C_1$-$C_6$ haloalkylthio, hydroxyl, thiol, nitro and sulfonamide; or

Ring A is a 6-membered heteroarylene or a 5-membered heteroarylene, each optionally substituted with $R^{16}$, $R^{17}$ and $R^{18}$, wherein $R^{16}$, $R^{17}$ and $R^{18}$ are each selected independently from $C_1$-$C_6$ acyl, $C_1$-$C_6$ acyloxy, $C_2$-$C_8$alkenyl, $C_1$-$C_6$alkoxy, $C_1$-$C_8$alkyl, $C_1$-$C_8$alkylcarboxamide, $C_2$-$C_8$alkynyl, $C_1$-$C_8$ alkylsulfonamide, $C_1$-$C_8$ alkylsulfinyl, $C_1$-$C_8$ alkylsulfonyl, $C_1$-$C_8$ alkylthio, $C_1$-$C_8$ alkylureyl, amino, $C_1$-$C_8$ alkylamino, $C_2$-$C_8$ dialkylamino, carbo-$C_1$-$C_6$-alkoxy, carboxamide, carboxy, cyano, $C_3$-$C_7$ cycloalkyl, $C_2$-$C_8$ dialkylcarboxamide, $C_2$-$C_8$ dialkylsulfonamide, halogen, $C_1$-$C_6$ haloalkoxy, $C_1$-$C_6$ haloalkyl, $C_1$-$C_6$ haloalkylsulfinyl, $C_1$-$C_8$ haloalkylsulfonyl, $C_1$-$C_6$ haloalkylthio, hydroxyl, thiol, nitro and sulfonamide;

$R^2$, $R^3$, $R^4$ and $R^5$ are each selected independently from H, $C_1$-$C_8$ acyl, $C_1$-$C_8$ acyloxy, $C_2$-$C_8$ alkenyl, $C_1$-$C_6$ alkoxy, $C_1$-$C_8$ alkyl, $C_1$-$C_8$ alkylcarboxamide, $C_2$-$C_8$ alkynyl, $C_1$-$C_8$ alkylsulfonamide, $C_1$-$C_8$ alkylsulfinyl, $C_1$-$C_8$ alkylsulfonyl, $C_1$-$C_8$ alkylthio, $C_1$-$C_8$ alkylureyl, amino, $C_1$-$C_8$ alkylamino, $C_2$-$C_8$ dialkylamino, carbo-$C_1$-$C_6$-alkoxy, carboxamide, carboxy, cyano, $C_3$-$C_7$ cycloalkyl, $C_2$-$C_8$ dialkylcarboxamide, $C_2$-$C_8$ dialkylsulfonamide, halogen, $C_1$-$C_6$ haloalkoxy, $C_1$-$C_6$ haloalkyl, $C_1$-$C_6$ haloalkylsulfinyl, $C_1$-$C_6$ haloalkylsulfonyl, $C_1$-$C_6$ haloalkylthio, hydroxyl, thiol, nitro and sulfonamide;

$R^6$, $R^7$, $R^8$ and $R^9$ are each selected independently from H, $C_1$-$C_3$ alkyl, $C_1$-$C_4$ alkoxy, carboxy, cyano, $C_1$-$C_3$ haloalkyl, halogen and hydroxyl; and

$R^{10}$ and $R^{11}$ together with the nitrogen atom to which they are both bonded form 2-methyl-pyrrolidin-1-yl;

provided:

1) that Ring B and the sulfur of the $R^1$-W-S(O)$_2$- group are not bonded to adjacent ring atoms of Ring A; and
2) if Ring A is 1,3-phenylene or 1,4-phenylene, and W is $C_3$-$C_7$ heterocyclylene, then the ring atom of W that is directly bonded to the sulfur of the $R^1$-W-S(O)$_2$- group is other than nitrogen.

**2.** A compound according to claim 1, wherein Ring A is 1,3-phenylene.

**3.** A compound according to claim 1, wherein Ring A is 1,4-phenylene.

**4.** A compound according to any one of claims 1 to 3, wherein $R^{12}$, $R^{13}$, $R^{14}$ and $R^{15}$ are each selected independently from H, $C_1$-$C_8$ alkyl, carboxy and halogen.

**5.** A compound according to any one of claims 1 to 3, wherein $R^{12}$, $R^{13}$, $R^{14}$ and $R^{15}$ are each selected independently from H, -$CH_3$, carboxy, Cl and Br.

**6.** A compound according to any one of claims 1 to 3, wherein $R^{12}$ $R^{13}$, $R^{14}$ and $R^{15}$ are each H.

**7.** A compound according to claim 1, wherein Ring A is a 6-membered heteroarylene.

**8.** A compound according to claim 1, wherein Ring A is a 5-membered heteroarylene.

**9.** A compound according to any one of claims 1 to 8, wherein:

$R^1$ is selected from H, $C_1$-$C_6$ alkoxy, amino, carbo-$C_1$-$C_6$-alkoxy, carboxamide, carboxy, $C_3$-$C_7$ heterocyclyl, hydroxyl and phenyl, and each is optionally substituted with cyano or $C_3$-$C_7$ cycloalkyl; or
$R^1$ together with the W-$SO_2$ group and the ring atom to which the W-$SO_2$ group is bonded form a $C_5$-$C_7$ heterocyclic ring with Ring A whereby said $C_5$-$C_7$ heterocyclic ring and Ring A share two adjacent ring atoms, and said $C_5$-$C_7$ heterocyclic ring is optionally substituted with oxo.

**10.** A compound according to any one of claims 1 to 8, wherein:

$R^1$ is selected from H, $C_1$-$C_6$ alkoxy, carbo-$C_1$-$C_6$-alkoxy, hydroxyl and phenyl; or
$R^1$ together with the W-$SO_2$ group and the ring atom to which the W-$SO_2$ group is bonded form a $C_5$-$C_7$ heterocyclic ring with Ring A whereby said $C_5$-$C_7$ heterocyclic ring and Ring A share two adjacent ring atoms, and said $C_5$-$C_7$ heterocyclic ring is optionally substituted with oxo.

**11.** A compound according to any one of claims 1 to 8, wherein $R^1$ is selected from H, $C_1$-$C_6$ alkoxy, carbo-$C_1$-$C_6$-alkoxy, hydroxyl and phenyl.

**12.** A compound according to any one of claims 1 to 8, wherein $R^1$ is selected from H, -$OCH_3$, -$OCH_2CH_3$, -C(=O)$OCH_2CH_3$, -C(=O)OC$(CH_3)_3$, hydroxyl and phenyl.

**13.** A compound according to any one of claims 1 to 12, wherein W is $C_1$-$C_4$ alkylene, $C_1$-$C_4$ alkenylene, $C_3$-$C_7$ cycloalkylene or phenylene, each optionally substituted with $C_1$-$C_3$ alkyl.

**14.** A compound according to any one of claims 1 to 12, wherein W is $C_1$-$C_4$ alkylene or $C_2$-$C_4$ alkenylene, each optionally substituted with $C_1$-$C_3$ alkyl.

**15.** A compound according to any one of claims 1 to 12, wherein W is selected from -$CH_2$-, -$CH_2CH_2$-, -$CH(CH_3)CH_2$-, -$CH_2CH_2CH_2$-, -$CH_2CH_2CH(CH_3)$-, -HC=CH-, 1,3-cyclopentylene, -$C(CH_3)_2CH_2$-, -$CH_2C(CH_3)_2CH_2$-, 4-tetrahydropyran-2-yl, 3-tetrahydropyran-5-yl and 1,4-phenylene.

**16.** A compound according to any one of claims 1 to 12, wherein W is selected from -$CH_2$-, -$CH_2CH_2$-, -$CH(CH_3)CH_2$-, -$CH_2CH_2CH_2$-, -$CH_2CH_2CH(CH_3)$ -, -HC=CH-, and 1,3-cyclopentylene.

**17.** A compound according to any one of claims 1 to 12, wherein W is selected from -$CH_2CH_2$- and -HC=CH-.

**18.** A compound according to any one of claims 1 to 17, wherein $R^2$, $R^3$, $R^4$ and $R^5$ are each H.

**19.** A compound according to any one of claims 1 to 18, wherein $R^6$, $R^7$, $R^8$ and $R^9$ are each H.

**20.** A compound according to any one of claims 1 to 19, wherein $R^{10}$ and $R^{11}$ together with the nitrogen atom to which they are both bonded form (*R*)-2-methyl-pyrrolidin-1-yl.

**21.** A compound according to claim 1, selected from compounds of Formula (Im) and pharmaceutically acceptable salts, hydrates, and solvates thereof:

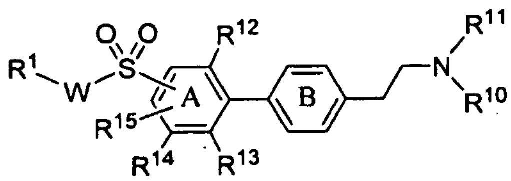

**(Im)**

wherein:

$R^{12}$, $R^{13}$, $R^{14}$ and $R^{15}$ are each selected independently from H, $C_1$-$C_8$ alkyl, carboxy and halogen;
$R^1$ is selected from H, $C_1$-$C_6$ alkoxy, amino, carbo-$C_1$-$C_6$-alkoxy, carboxamide, carboxy, $C_3$-$C_7$ heterocyclyl, hydroxyl and phenyl, and each is optionally substituted with cyano or $C_3$-$C_7$ cycloalkyl; or
$R^1$ together with the W-$SO_2$ group and the ring atom to which the W-$SO_2$ group is bonded form a $C_5$-$C_7$ heterocyclic ring with Ring A whereby said $C_5$-$C_7$ heterocyclic ring and Ring A share two adjacent ring atoms, and said $C_5$-$C_7$ heterocyclic ring is optionally substituted with oxo;
W is $C_1$-$C_4$ alkylene, $C_2$-$C_4$ alkenylene, $C_3$-$C_7$ cycloalkylene or phenylene, each optionally substituted with $C_1$-$C_3$ alkyl; and
$R^{10}$ and $R^{11}$ together with the nitrogen atom to which they are both bonded form 2-methyl-pyrrolidin-1-yl.

**22.** A compound according to claim 1, selected from compounds of Formula (Im) and pharmaceutically acceptable salts, hydrates, and solvates thereof:

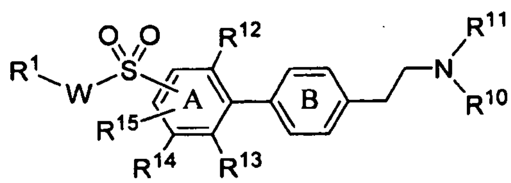

**(Im)**

wherein:

$R^{12}$, $R^{13}$, $R^{14}$ and $R^{15}$ are each selected independently from H, $-CH_3$, carboxy, Cl and Br;
$R^1$ is selected from H, $C_1$-$C_6$ alkoxy, carbo-$C_1$-$C_6$-alkoxy, hydroxyl and phenyl; or
$R^1$ together with the W-$SO_2$ group and the ring atom to which the W-$SO_2$ group is bonded form a $C_5$-$C_7$ heterocyclic ring with Ring A whereby said $C_5$-$C_7$ heterocyclic ring and Ring A share two adjacent ring atoms, and said $C_5$-$C_7$ heterocyclic ring is optionally substituted with oxo;
W is selected from $-CH_2-$, $-CH_2CH_2-$, $-CH(CH_3)CH_2-$, $-CH_2CH_2CH_2-$, $-CH_2CH_2CH(CH_3)-$, -HC=CH-, 1,3-cyclopentylene, $-C(CH_3)_2CH_2-$, $-CH_2C(CH_3)_2CH_2-$, 4-tetrahydropyran-2-yl, 3-tetrahydropyran-5-yl and 1,4-phenylene; and
$R^{10}$ and $R^{11}$ together with the nitrogen atom to which they are both bonded form 2-methyl-pyrrolidin-1-yl.

**23.** A compound according to claim 1, selected from compounds of Formula (Im) and pharmaceutically acceptable salts, hydrates, and solvates thereof:

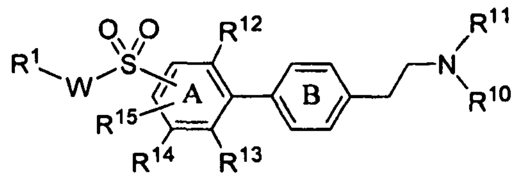

(Im)

wherein:

$R^{12}$ , $R^{13}$, $R^{14}$ and $R^{15}$ are each H;
$R^1$ is selected from H, $-OCH_3$, $-OCH_2CH_3$, $-C(=O)OCH_2CH_3$, $-C(=O)OC(CH_3)_3$, hydroxyl and phenyl;
W is selected from $-CH_2CH_2-$ and -HC=CH-; and
$R^{10}$ and $R^{11}$ together with the nitrogen atom to which they are both bonded form 2-methyl-pyrrolidin-1-yl.

**24.** A compound according to claim 1, selected from compounds of Formula (Io) and pharmaceutically acceptable salts, hydrates, and solvates thereof:

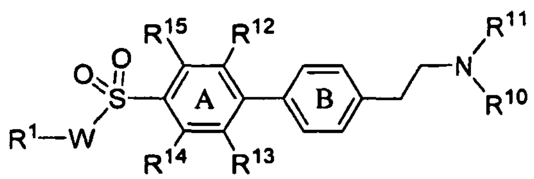

(Io)

wherein:

$R^{12}$, $R^{13}$, $R^{14}$ and $R^{15}$ are each H;
$R^1$ is selected from H, $-OCH_3$, $-OCH_2CH_3$, $-C(=O)OCH_2CH_3$, $-C(=O)OC(CH_3)_3$, hydroxyl and phenyl;
W is selected from $-CH_2CH_2-$ and -HC=CH-; and
$R^{10}$ and $R^{11}$ together with the nitrogen atom to which they are both bonded form 2-methyl-pyrrolidin-1-yl.

**25.** A compound according to claim 1, selected from compounds of Formula (Iq) and pharmaceutically acceptable salts, hydrates, and solvates thereof:

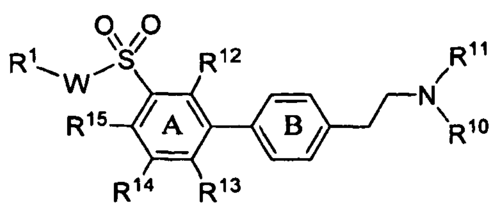

(Iq)

wherein:

$R^{12}$, $R^{13}$, $R^{14}$ and $R^{15}$ are each H;
$R^1$ is selected from H, $-OCH_3$, $-OCH_2CH_3$, $-C(=O)OCH_2CH_3$, $-C(=O)OC(CH_3)_3$, hydroxyl and phenyl;
W is selected from $-CH_2CH_2-$ and -HC=CH-; and
$R^{10}$ and $R^{11}$ together with the nitrogen atom to which they are both bonded form 2-methyl-pyrrolidin-1-yl.

**26.** A compound according to claim 1, selected from compounds of Formula (Is) and pharmaceutically acceptable salts,

hydrates, and solvates thereof:

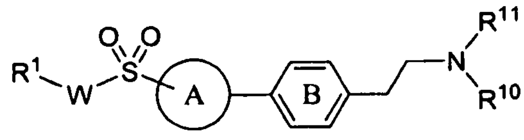

(Is)

wherein:

Ring A is:

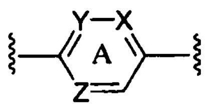

or

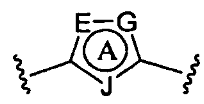

;

X is N or CH; Y is N or CH; and Z is N or CH; provided that at least one X, Y and Z is N;
J is N or NH; and E and G are each independently selected from N and S, provided that at least one of E and G is N;
$R^1$ is selected from H, $C_1$-$C_6$ alkoxy, carbo-$C_1$-$C_6$-alkoxy, hydroxyl and phenyl; or
$R^1$ together with the W-$SO_2$ group and the ring atom to which the W-$SO_2$ group is bonded form a $C_5$-$C_7$ heterocyclic ring with Ring A whereby said $C_5$-$C_7$ heterocyclic ring and Ring A share two adjacent ring atoms;
W is selected from -$CH_2$-, -$CH_2CH_2$-, -$CH(CH_3)CH_2$-, -$CH_2CH_2CH_2$-, -$CH_2CH_2CH(CH_3)$ -, -HC=CH-, 1,3-cyclopentylene, -$C(CH_3)_2CH_2$-, -$CH_2C(CH_3)_2CH_2$-, 4-tetrahydropyran-2-yl, 3-tetrahydropyran-5-yl and 1,4-phenylene; and
$R^{10}$ and $R^{11}$ together with the nitrogen atom to which they are both bonded form 2-methyl-pyrrolidin-1-yl.

**27.** A compound according to claim 1, selected from compounds of Formula (**Is**) and pharmaceutically acceptable salts, hydrates, and solvates thereof:

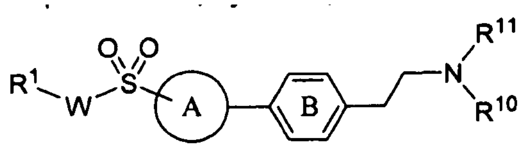

(Is)

wherein:

Ring A is:

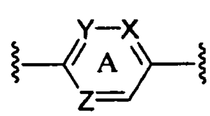

or

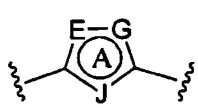

;

X is N or CH; Y is N or CH; and Z is N or CH; provided that at least one X, Y and Z is N;
J is N or NH; and E and G are each independently selected from N and S, provided that at least one of E and G is N;
$R^1$ is selected from H, -$OCH_3$, -$OCH_2CH_3$, -$C(=O)OCH_2CH_3$, -$C(=O)OC(CH_3)_3$, hydroxyl and phenyl;
W is selected from -$CH_2CH_2$- and -HC=CH-; and
$R^{10}$ and $R^{11}$ together with the nitrogen atom to which they are both bonded form 2-methyl-pyrrolidin-1-yl.

**28.** A compound according to claim 1, selected from the following compounds and pharmaceutically acceptable salts, hydrates, and solvates thereof:

1-[2-(4'-Methanesulfonyl-biphenyl-4-yl)-ethyl]-2-methyl-pyrrolidine;
1-[2-(4'-Ethanesulfonyl-biphenyl-4-yl)-ethyl]-2-methyl-pyrrolidine;
1-{2-[4'-(2-Methoxy-ethanesulfonyl)-biphenyl-4-yl]-ethyl}-2-methyl-pyrrolidine;
2-Methyl-1-{2-[4'-(propane-2-sulfonyl)-biphenyl-4-yl]-ethyl}-pyrrolidine;
2-Methyl-1-{2-[4'-(propane-1-sulfonyl)-biphenyl-4-yl]-ethyl}-pyrrolidine;
2-Methyl-1-[2-(4'-phenylmethanesulfonyl-biphenyl-4-yl)-ethyl]-pyrrolidine;
6-{4-[2-(2-Methyl-pyrrolidin-1-yl)-ethyl]-phenyl}-1,1-dioxo-1$\lambda^6$-thiochroman-4-one;
1-{2-[4'-(3-Methoxy-propane-1-sulfonyl)-biphenyl-4-yl]-ethyl}-2-methyl-pyrrolidine;
2-Methyl-1-{2-[4'-(2-methyl-propane-1-su Ifonyl)-biphenyl-4-yl]-ethyl}-pyrrolidine;
2-(4'-[2-(2-Methyl-pyrrolidin-1-yl)-ethyl]-biphenyl-4-sulfonyl)-ethanol;
{4'-[2-(2-Methyl-pyrrolidin-1-yl)-ethyl]-biphenyl-4-sulfonyl}-acetic acid ethyl ester;
1-{2-[4'-(2-Ethoxy-ethanesulfonyl)-biphenyl-4-yl]-ethyl}-2-methyl-pyrrolidine;
1-[2-(4'-Ethenesulfonyl-biphenyl-4-yl)-ethyl]-2-methyl-pyrrolidine;
1-[2-(4'-Cyclopentanesulfonyl-biphenyl-4-yl)-ethyl]-2-methyl-pyrrolidine;
3-{4'-[2-(2-Methyl-pyrrolidin-1-yl)-ethyl]-biphenyl-4-sulfonyl}-propan-1-ol;
1-{2-[3'-(2-Methoxy-ethanesulfonyl)-biphenyl-4-yl]-ethyl}-2-methyl-pyrrolidine;
2-{4'-[2-(2-Methyl-pyrrolidin-1-yl)-ethyl]-biphenyl-3-sulfonyl}-ethanol;
{4'-[2-(2-Methyl-pyrrolidin-1-yl)-ethyl]-biphenyl-4-sulfonyl}-acetic acid tert-butyl ester; and
{4'-[2-(2-Methyl-pyrrolidin-1-yl)-ethyl]-biphenyl-4-sulfonyl}-acetic acid methyl ester.

**29.** The compound according to claim 28, wherein said compound is the (R)-enantiomer:

**30.** A compound according to claim 1, selected from compounds of Formula (**Is**) and pharmaceutically acceptable salts, hydrates, and solvates thereof:

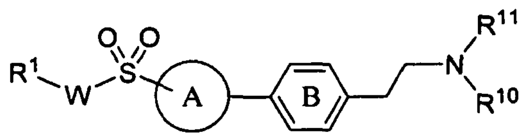

(Is)

wherein:

Ring A is selected from 1,4-phenylene, 1,3-phenylene, 4-carboxy-1,3-phenylene, 4-methyl-1,3-phenylene, pyridin-2,5-ylene, pyrimidin-2,5-ylene and 1,2,4-thiadiazol-3,5-ylene;
$R^1$ is selected from H, $-OCH_3$, $-OCH_2CH_3$, $-C(=O)OCH_3$, $-C(=O)OCH_2CH_3$, $-C(=O)OC(CH_3)_3$, hydroxyl, phenyl, morpholin-4-yl, tetrahydro-pyran-4-yl, carboxy, 4-cyanopiperidin-1-yl, amino, cyclohexylamino, methylamino, and tetrahydro-pyran-2-yl;
W is selected from $-CH_2-$, $-CH_2CH_2-$, $-CH(CH_3)CH_2-$, $-CH_2CH_2CH_2-$, -HC=CH-, 1,3-cyclopentylene, $-CH_2C(CH_3)_2CH_2-$, 4-tetrahydropyran-2-yl, $-CH_2HC=CH-$, $-CH_2CH_2C(=O)-$, $-CH_2CH(CH_3)CH_2-$, $-CH_2CH(CH_3)-$ and piperidin-2,4-ylene; and
$R^{10}$ and $R^{11}$ together with the nitrogen atom to which they are both bonded form 2-methyl-pyrrolidin-1-yl.

**31.** A compound according to claim 1, selected from the following compounds and pharmaceutically acceptable salts, hydrates, and solvates thereof:

3-Methanesulfonyl-4'-[2-(2-methyl-pyrrolidin-1-yl)-ethyl]-biphenyl-4-carboxylic acid;
2-Methyl-1-{2-[4'-(tetrahydro-pyran-4-sulfonyl)-biphenyl-4-yl]-ethyl}-pyrrolidine;
2-Methanesulfonyl-5-{4-[2-(2-methyl-pyrrolidin-1-yl)-ethyl]-phenyl}-pyridine;
1-{2-[4'-(2-Methoxy-propane-1-sulfonyl)-biphenyl-4-yl]-ethyl}-2-methyl-pyrrolidine;
2-Methanesulfonyl-5-{4-[2-(2-methyl-pyrrolidin-1-yl)-ethyl]-phenyl}-pyrimidine;
4-(2-{4'-[2-(2-Methyl-pyrrolidin-1-yl)-ethyl]-biphenyl-4-sulfonyl}-ethyl)-morpholine;
2-Methyl-1-{2-[4'-(tetrahydro-pyran-4-ylmethanesulfonyl)-biphenyl-4-yl]-ethyl}-pyrrolidine;
1-[2-(3'-Methanesulfonyl-4'-methyl-biphenyl-4-yl)-ethyl]-2-methyl-pyrrolidine;
3-{4'-[2-(2-Methyl-pyrrolidin-1-yl)-ethyl]-biphenyl-4-sulfonyl}-propionic acid;
1-(2-{4'-[2-(2-Methyl-pyrrolidin-1-yl)-ethyl]-biphenyl-4-sulfonyl}-ethyl)-piperidine-4-carbonitrile;

2-Methyl-1-{2-[4'-(prop-2-ene-1-sulfonyl)-biphenyl-4-yl]-ethyl}-pyrrolidine;
2-{4'-[2-(2-Methyl-pyrrolidin-1-yl)-ethyl]-biphenyl-4-sulfonyl}-ethylamine;
Cyclohexyl-(2-{4'-[2-(2-methyl-pyrrolidin-1-yl)-ethyl]-biphenyl-4-sulfonyl}-ethyl)-amine;
5-Methanesulfonyl-2-{4-[2-(2-methyl-pyrrolidin-1-yl)-ethyl]-phenyl}-pyridine;
*N*-Methyl-3-(4'-(2-(2-methylpyrrolidin-1-yl)ethyl)biphenyl-4-ylsulfonyl)propanamide;
3-(4'-(2-(2-Methylpyrrolidin-1-yl)ethyl)biphenyl-4-ylsulfonyl)-1-morpholinopropan-1-one;
4-(4'-(2-(2-Methylpyrrolidin-1-yl)ethyl)biphenyl-4-ylsulfonyl)piperidine;
5-(4-(2-(2-Methylpyrrolidin-1-yl)ethyl)phenyl)-3-(methylsulfonyl)-1,2,4-thiadiazole;
1-{2-[4'-(3-Methoxy-propane-1-sulfonyl)-biphenyl-4-yl]-ethyl}-2-methyl-pyrrolidine;
1-(2-(4'-(2-Methoxyethylsulfonyl)biphenyl-4-yl)ethyl)-2-methylpyrrolidine;
2,2-Dimethyl-3-(4'-(2-(2-methylpyrrolidin-1-yl)ethyl)biphenyl-4-ylsulfonyl)propan-1-ol;
2-Methyl-1-(2-(4'-((tetrahydro-2H-pyran-2-yl)methylsulfonyl)biphenyl-4-yl)ethyl)pyrrolidine; and
1-(2-(4'-(Methoxymethylsulfonyl)biphenyl-4-yl)ethyl)-2-methylpyrrolidine.

**32.** A compound according to claim 31, wherein said compound is the (*R*)-enantiomer.

**33.** A compound according to claim 1, selected from the following compound and pharmaceutically acceptable salts, hydrates, and solvates thereof:

(*R*)-1-{2-[4'-(2-Methoxy-ethanesulfonyl)-biphenyl-4-yl]-ethyl}-2-methyl-pyrrolidine.

**34.** A compound according to claim 1, selected from the following compound and pharmaceutically acceptable salts, hydrates, and solvates thereof:

(*R*)-1-{2-[4'-(3-Methoxy-propane-1-sulfonyl)-biphenyl-4-yl]-ethyl}-2-methyl-pyrrolidine.

**35.** A compound according to claim 1, selected from the following compound and pharmaceutically acceptable salts, hydrates, and solvates thereof:

{4'-[2-((*R*)-2-Methyl-pyrrolidin-1-yl)-ethyl]-biphenyl-4-sulfonyl}-acetic acid ethyl ester.

**36.** A compound according to claim 1, selected from the following compound and pharmaceutically acceptable salts, hydrates, and solvates thereof:

(*R*)-2-Methyl-1-{2-[4'-(tetrahydro-pyran-4-sulfonyl)-biphenyl-4-yl]-ethyl}-pyrrolidine.

**37.** A compound according to claim 1, selected from the following compound and pharmaceutically acceptable salts, hydrates, and solvates thereof:

(*R*)-2-Methyl-1-{2-[4'-(tetrahydro-pyran-4-ylmethanesulfonyl)-biphenyl-4-yl]-ethyl}-pyrrolidine.

**38.** A pharmaceutical composition comprising a compound according to any one of claims 1 to 37 and a pharmaceutically acceptable carrier.

**39.** A process for preparing a composition comprising admixing a compound according to any one of claims 1 to 37 and a pharmaceutically acceptable carrier.

**40.** A compound according to any one of claims 1 to 37 for use in a method of treatment of the human or animal body by therapy.

**41.** A compound according to any one of claims 1. to 37 for use in a method for the treatment of a cognitive disorder, epilepsy, brain trauma, depression, obesity, a disorder of sleep or wakefulness, narcolepsy, cataplexy, hypersomnia, somnolence syndrome, jet lag, sleep apnea, attention deficit hyperactivity disorder (ADHD), schizophrenia, an allergy, an allergic response in the upper airway, allergic rhinitis, nasal congestion, dementia, or Alzheimer's disease.

**42.** A compound according to any one of claims 1 to 37 for use in a method for the treatment of a cognitive disorder.

**43.** A compound according to any one of claims 1 to 37 for use in a method for the treatment of cataplexy.

**44.** A compound according to any one of claims 1 to 37 for use in a method of inducing wakefulness.

**45.** A compound according to any one of claims 1 to 37 for use in a method of treatment of narcolepsy.

**46.** Use of a compound according to any one of claims 1 to 37 for the production of a medicament for the treatment of a cognitive disorder, epilepsy, brain trauma, depression, obesity, a disorder of sleep or wakefulness, narcolepsy, cataplexy, hypersomnia, somnolence syndrome, jet lag, sleep apnea, attention deficit hyperactivity disorder (ADHD), schizophrenia, an allergy, an allergic response in the upper airway, allergic rhinitis, nasal congestion, dementia, or Alzheimer's disease.

**47.** Use of a compound according to any one of claims 1 to 37 for the production of a medicament for the treatment of a cognitive disorder.

**48.** Use of a compound according to any one of claims 1 to 37 for the production of a medicament for the treatment of cataplexy.

**49.** Use of a compound according to any one of claims 1 to 37 for the production of a medicament for inducing wakefulness.

**50.** Use of a compound according to any one of claims 1 to 37 for the production of a medicament for the treatment of narcolepsy.

**Patentansprüche**

**1.** Verbindung, ausgewählt aus Verbindungen der Formel (Ia):

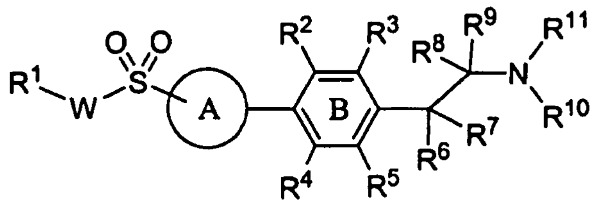

**(Ia)**

und pharmazeutisch annehmbaren Salzen, Hydraten und Solvaten davon; worin:

$R^1$ aus H, $C_1$-$C_6$-Acyl, $C_1$-$C_6$-Acyloxy, $C_2$-$C_8$-Alkenyl, $C_1$-$C_6$-Alkoxy, $C_1$-$C_8$-Alkyl, $C_1$-$C_8$-Alkylcarboxamid, $C_2$-$C_8$-Alkinyl, $C_1$-$C_8$-Alkylsulfonamid, $C_1$-$C_8$-Alkylsulfinyl, $C_1$-$C_8$-Alkylsulfonyl, $C_1$-$C_8$-Alkylthio, $C_1$-$C_8$-Alkylureyl, Amino, $C_1$-$C_8$-Alkylamino, $C_2$-$C_8$-Dialkylamino, Carbo-$C_1$-$C_6$-alkoxy, Carboxamid, Carboxy, Cyano, $C_3$-$C_7$-Cycloalkyl, $C_2$-$C_8$-Dialkylcarboxamid, $C_2$-$C_8$-Dialkylsulfonamid, Halogen, $C_1$-$C_6$-Halogenalkoxy, $C_1$-$C_6$-Halogenalkyl, $C_1$-$C_6$-Halogenalkylsulfinyl, $C_1$-$C_6$-Halogenalkylsulfonyl, $C_1$-$C_6$-Halogenalkylthio, $C_3$-$C_7$-Heterocyclyl, Hydroxyl, Thiol, Nitro, Phenyl und Sulfonamid ausgewählt ist, die jeweils gegebenenfalls mit 1, 2, 3, 4 oder 5 Substituenten substituiert sind, die unabhängig voneinander aus $C_1$-$C_6$-Acyl, $C_1$-$C_6$-Acyloxy, $C_2$-$C_8$-Alkenyl, $C_1$-$C_6$-Alkoxy, $C_1$-$C_8$-Alkyl, $C_1$-$C_8$-Alkylcarboxamid, $C_2$-$C_8$-Alkinyl, $C_1$-$C_8$-Alkylsulfonamid, $C_1$-$C_8$Alkylsulfinyl, $C_1$-$C_8$-Alkylsulfonyl, $C_1$-$C_8$-Alkylthio, $C_1$-$C_8$-Alkylureyl, Amino, $C_1$-$C_8$-Alkylamino, $C_2$-$C_8$-Dialkylamino, Carbo-$C_1$-$C_6$-alkoxy, Carboxamid, Carboxy, Cyano, $C_3$-$C_7$-Cycloalkyl, $C_2$-$C_8$-Dialkylcarboxamid, $C_2$-$C_8$-Dialkylsulfonamid, Halogen, $C_1$-$C_6$-Halogenalkoxy, $C_1$-$C_6$-Halogenalkyl, $C_1$-$C_6$-Halogenalkylsulfinyl, $C_1$-$C_6$-Halogenalkylsulfonyl, $C_1$-$C_6$-Halogenalkylthio, Hydroxyl, Thiol, Nitro und Sulfonamid ausgewählt sind; oder

$R^1$ zusammen mit der W-$SO_2$-Gruppe und dem Ringatom, an das die W-$SO_2$-Gruppe gebunden ist, einen heterozyklischen $C_5$-$C_7$-Ring mit Ring A bildet, sodass der heterozyklische $C_5$-$C_7$-Ring und der Ring A zwei gemeinsame benachbarte Ringatome aufweisen, und der heterozyklische $C_5$-$C_7$-Ring gegebenenfalls mit 1, 2, 3 oder 4 Substituenten substituiert ist, die unabhängig voneinander aus $C_1$-$C_6$-Acyl, $C_1$-$C_6$-Acyloxy, $C_2$-$C_8$-Alkenyl, $C_1$-$C_6$-Alkoxy, $C_1$-$C_8$-Alkyl, $C_1$-$C_8$-Alkylcarboxamid, $C_2$-$C_8$-Alkinyl, $C_1$-$C_8$-Alkylsulfonamid, $C_1$-$C_8$-Alkylsul-

finyl, $C_1$-$C_8$-Alkylsulfonyl, $C_1$-$C_8$-Alkylthio, $C_1$-$C_8$-Alkylureyl, Amino, $C_1$-$C_8$-Alkylamino, $C_2$-$C_8$-Dialkylamino, Carbo-$C_1$-$C_6$-alkoxy, Carboxamid, Carboxy, Cyano, $C_3$-$C_7$-Cycloalkyl, $C_2$-$C_8$-Dialkylcarboxamid, $C_2$-$C_8$-Dialkylsulfonamid, Halogen, $C_1$-$C_6$-Halogenalkoxy, $C_1$-$C_6$-Halogenalkyl, $C_1$-$C_6$-Halogenalkylsulfinyl, $C_1$-$C_6$-Halogenalkylsulfonyl, $C_1$-$C_6$-Halogenalkylthio, Hydroxyl, Thiol, Nitro, Oxo und Sulfonamid ausgewählt sind;
W $C_1$-$C_4$-Alkylen, $C_2$-$C_4$-Alkenylen, $C_3$-$C_7$-Cycloalkylen, $C_3$-$C_7$-Heterocyclylen oder Phenylen ist, die jeweils gegebenenfalls mit 1, 2, 3, 4, 5, 6, 7 oder 8 Substituenten substituiert sind, die unabhängig voneinander aus $C_1$-$C_3$-Alkyl, $C_1$-$C_4$-Alkoxy, Carboxy, Cyano, $C_1$-$C_4$-Halogenalkyl, Halogen, Hydroxyl und Oxo ausgewählt sind;
der Ring A 1,3-Phenylen oder 1,4-Phenylen ist, die jeweils mit $R^{12}$, $R^{13}$, $R^{14}$ und $R^{15}$ substituiert sind, worin $R^{12}$ , $R^{13}$, $R^{14}$ und $R^{15}$ jeweils unabhängig voneinander aus H, $C_1$-$C_6$-Acyl, $C_1$-$C_6$-Acyloxy, $C_2$-$C_8$-Alkenyl, $C_1$-$C_6$-Alkoxy, $C_1$-$C_8$-Alkyl, $C_1$-$C_8$-Alkylcarboxamid, $C_2$-$C_8$-Alkinyl, $C_1$-$C_8$-Alkylsulfonamid, $C_1$-$C_8$-Alkylsulfinyl, $C_1$-$C_8$-Alkylsulfonyl, $C_1$-$C_8$-Alkylthio, $C_1$-$C_8$-Alkylureyl, Amino, $C_1$-$C_8$-Alkylamino, $C_2$-$C_8$-Dialkylamino, Carbo-$C_1$-$C_6$-alkoxy, Carboxamid, Carboxy, Cyano, $C_3$-$C_7$-Cycloalkyl, $C_2$-$C_8$-Dialkylcarboxamid, $C_2$-$C_8$-Dialkylsulfonamid, Halogen, $C_1$-$C_6$-Halogenalkoxy, $C_1$-$C_6$-Halogenalkyl, $C_1$-$C_6$-Halogenalkylsulfinyl, $C_1$-$C_6$-Halogenalkylsulfonyl, $C_1$-$C_6$-Halogenalkylthio, Hydroxyl, Thiol, Nitro und Sulfonamid ausgewählt sind; oder
der Ring A ein 6-gliedriges Heteroarylen oder ein 5-gliedriges Heteroarylen ist, die jeweils gegebenenfalls mit $R^{16}$, $R^{17}$ und $R^{18}$ substituiert sind, worin $R^{16}$, $R^{17}$ und $R^{18}$ jeweils unabhängig voneinander aus $C_1$-$C_6$-Acyl, $C_1$-$C_6$-Acyloxy, $C_2$-$C_8$-Alkenyl, $C_1$-$C_6$-Alkoxy, $C_1$-$C_8$-Alkyl, $C_1$-$C_8$-Alkylcarboxamid, $C_2$-$C_8$-Alkinyl, $C_1$-$C_8$-Alkylsulfonamid, $C_1$-$C_8$-Alkylsulfinyl, $C_1$-$C_8$-Alkylsulfonyl, $C_1$-$C_8$-Alkylthio, $C_1$-$C_8$-Alkylureyl, Amino, $C_1$-$C_8$-Alkylamino, $C_2$-$C_8$-Dialkylamino, Carbo-$C_1$-$C_6$-alkoxy, Carboxamid, Carboxy, Cyano, $C_3$-$C_7$-Cycloalkyl, $C_2$-$C_8$-Dialkylcarboxamid, $C_2$-$C_8$-Dialkylsulfonamid, Halogen, $C_1$-$C_6$-Halogenalkoxy, $C_1$-$C_6$-Halogenalkyl, $C_1$-$C_6$-Halogenalkylsulfinyl, $C_1$-$C_6$-Halogenalkylsulfonyl, $C_1$-$C_6$-Halogenalkylthio, Hydroxyl, Thiol, Nitro und Sulfonamid ausgewählt sind;
$R^2$, $R^3$, $R^4$ und $R^5$ jeweils unabhängig voneinander aus H, $C_1$-$C_6$-Acyl, $C_1$-$C_6$-Acyloxy, $C_2$-$C_8$-Alkenyl, $C_1$-$C_6$-Alkoxy, $C_1$-$C_8$-Alkyl, $C_1$-$C_8$-Alkylcarboxamid, $C_2$-$C_8$-Alkinyl, $C_1$-$C_8$-Alkylsulfonamid, $C_1$-$C_8$-Alkylsulfinyl, $C_1$-$C_8$-Alkylsulfonyl, $C_1$-$C_8$-Alkylthio, $C_1$-$C_8$-Alkylureyl, Amino, $C_1$-$C_8$-Alkylamino, $C_2$-$C_8$-Dialkylamino, Carbo-$C_1$-$C_6$-alkoxy, Carboxamid, Carboxy, Cyano, $C_3$-$C_7$-Cycloalkyl, $C_2$-$C_8$-Dialkylcarboxamid, $C_2$-$C_8$-Dialkylsulfonamid, Halogen, $C_1$-$C_6$-Halogenalkoxy, $C_1$-$C_6$-Halogenalkyl, $C_1$-$C_6$-Halogenalkylsulfinyl, $C_1$-$C_6$-Halogenalkylsulfonyl, $C_1$-$C_6$-Halogenalkylthio, Hydroxyl, Thiol, Nitro und Sulfonamid ausgewählt sind;
$R^6$, $R^7$, $R^8$ und $R^9$ jeweils unabhängig voneinander aus H, $C_1$-$C_3$-Alkyl, $C_1$-$C_4$-Alkoxy, Carboxy, Cyano, $C_1$-$C_3$-Halogenalkyl, Halogen und Hydroxyl ausgewählt sind; und
$R^{10}$ und $R^{11}$ zusammen mit dem Stickstoffatom, an das sie gebunden sind, 2-Methylpyrrolidin-1-yl bilden;
mit der Maßgabe, dass:

1) der Ring B und der Schwefel der $R^1$-W-S(O)$_2$-Gruppe nicht an benachbarte Ringatome von Ring A gebunden sind; und
2) wenn der Ring A 1,3-Phenylen oder 1,4-Phenylen ist und W $C_3$-$C_7$-Heterocyclylen ist, das Ringatom von W, das direkt an den Schwefel der $R^1$-W-S(O)$_2$-Gruppe gebunden ist, kein Stickstoff ist.

**2.** Verbindung nach Anspruch 1, worin der Ring A 1,3-Phenylen ist.

**3.** Verbindung nach Anspruch 1, worin der Ring A 1,4-Phenylen ist.

**4.** Verbindung nach einem der Ansprüche 1 bis 3, worin $R^{12}$ , $R^{13}$, $R^{14}$ und $R^{15}$ jeweils unabhängig voneinander aus H, $C_1$-$C_8$-Alkyl, Carboxy und Halogen ausgewählt sind.

**5.** Verbindung nach einem der Ansprüche 1 bis 3, worin $R^{12}$ , $R^{13}$ , $R^{14}$ und $R^{15}$ jeweils unabhängig voneinander aus H, -$CH_3$, Carboxy, Cl und Br ausgewählt sind.

**6.** Verbindung nach einem der Ansprüche 1 bis 3, worin $R^{12}$, $R^{13}$, $R^{14}$ und $R^{15}$ jeweils H sind.

**7.** Verbindung nach Anspruch 1, worin der Ring A ein 6-gliedriges Heteroarylen ist.

**8.** Verbindung nach Anspruch 1, worin der Ring A ein 5-gliedriges Heteroarylen ist.

**9.** Verbindung nach einem der Ansprüche 1 bis 8, worin:

$R^1$ aus H, $C_1$-$C_6$-Alkoxy, Amino, Carbo-$C_1$-$C_6$-alkoxy, Carboxamid, Carboxy, $C_3$-$C_7$-Heterocyclyl, Hydroxyl und Phenyl ausgewählt ist, die jeweils gegebenenfalls mit Cyano oder $C_3$-$C_7$-Cycloalkyl substituiert sind; oder

$R^1$ zusammen mit der W-$SO_2$-Grupp und dem Ringatom, an das die W-$SO_2$-Gruppe gebunden ist, einen heterocyclischen $C_5$-$C_7$-Ring mit Ring A bildet, sodass der heterocyclische $C_5$-$C_7$-Ring und der Ring A zwei gemeinsame benachbarte Ringatome aufweisen, und der heterocyclische $C_5$-$C_7$-Ring gegebenenfalls mit Oxo substituiert ist.

**10.** Verbindung nach einem der Ansprüche 1 bis 8, worin:

$R^1$ aus H, $C_1$-$C_6$-Alkoxy, Carbo-$C_1$-$C_6$-alkoxy, Hydroxyl und Phenyl ausgewählt ist; oder
$R^1$ zusammen mit der W-$SO_2$-Gruppe und dem Ringatom, an das die W-$SO_2$-Gruppe gebunden ist, einen heterocyclischen $C_5$-$C_7$-Ring mit Ring A bildet, sodass der heterocyclische $C_5$-$C_7$-Ring und der Ring A zwei gemeinsame benachbarte Ringatome aufweisen, und der heterocyclische $C_5$-$C_7$-Ring gegebenenfalls mit Oxo substituiert ist.

**11.** Verbindung nach einem der Ansprüche 1 bis 8, worin $R^1$ aus H, $C_1$-$C_6$-Alkoxy, Carbo-$C_1$-$C_6$-alkoxy, Hydroxyl und Phenyl ausgewählt ist.

**12.** Verbindung nach einem der Ansprüche 1 bis 8, worin $R^1$ aus H, $-OCH_3$, $-OCH_2CH_3$, $-C(=O)OCH_2CH_3$, $-C(=O)OC(CH_3)_3$, Hydroxyl und Phenyl ausgewählt ist.

**13.** Verbindung nach einem der Ansprüche 1 bis 12, worin W $C_1$-$C_4$-Alkylen, $C_1$-$C_4$-Alkenylen, $C_3$-$C_7$Cycloalkylen oder Phenylen ist, die jeweils gegebenenfalls mit $C_1$-$C_3$-Alkyl substituiert sind.

**14.** Verbindung nach einem der Ansprüche 1 bis 12, worin W $C_1$-$C_4$-Alkylen oder $C_2$-$C_4$-Alkenylen ist, die jeweils gegebenenfalls mit $C_1$-$C_3$-Alkyl substituiert sind.

**15.** Verbindung nach einem der Ansprüche 1 bis 12, worin W aus $-CH_2-$, $-CH_2CH_2-$, $-CH(CH_3)CH_2-$, $-CH_2CH_2CH_2-$, $-CH_2CH_2CH(CH_3)-$, -HC=CH-, 1,3-Cyclopentylen, $-C(CH_3)_2CH_2-$, $-CH_2C(CH_3)_2CH_2-$, 4-Tetrahydropyran-2-yl, 3-Tetrahydropyran-5-yl und 1,4-Phenylen ausgewählt ist.

**16.** Verbindung nach einem der Ansprüche 2 bis 12, worin W aus $-CH_2-$, $-CH_2CH_2-$, $-CH(CH_3)CH_2-$, $-CH_2CH_2CH_2-$, $-CH_2CH_2CH(CH_3)-$, -HC=CH- und 1,3-Cyclopentylen ausgewählt ist.

**17.** Verbindung nach einem der Ansprüche 1 bis 12, worin W aus $-CH_2CH_2-$ und -HC=CH- ausgewählt ist.

**18.** Verbindung nach einem der Ansprüche 1 bis 17, worin $R^2$, $R^3$, $R^4$ und $R^5$ jeweils H sind.

**19.** Verbindung nach einem der Ansprüche 1 bis 18, worin $R^6$, $R^7$, $R^8$ und $R^9$ jeweils H sind.

**20.** Verbindung nach einem der Ansprüche 1 bis 19, worin $R^{10}$ und $R^{11}$ zusammen mit dem Stickstoffatom, an das sie gebunden sind, (R)-2-Methylpyrrolidin-1-yl bilden.

**21.** Verbindung nach Anspruch 1, ausgewählt aus Verbindungen der Formel (Im) und pharmazeutisch annehmbaren Salzen, Hydraten und Solvaten davon:

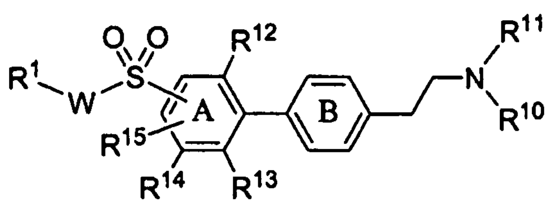

**(Im)**

worin:

$R^{12}$, $R^{13}$, $R^{14}$ und $R^{15}$ jeweils unabhängig voneinander aus H, $C_1$-$C_8$-Alkyl, Carboxy und Halogen ausgewählt

sind;

$R^1$ aus H, $C_1$-$C_6$-Alkoxy, Amino, Carbo-$C_1$-$C_6$-alkoxy, Carboxamid, Carboxy, $C_3$-$C_7$-Heterocyclyl, Hydroxyl und Phenyl ausgewählt ist, die jeweils gegebenenfalls mit Cyano oder $C_3$-$C_7$-Cycloalkyl substituiert sind; oder

$R^1$ zusammen mit der W-$SO_2$-Grupp und dem Ringatom, an das die W-$SO_2$-Gruppe gebunden ist, einen heterocyclischen $C_5$-$C_7$-Ring mit Ring A bildet, sodass der heterocyclische $C_5$-$C_7$-Ring und der Ring A zwei gemeinsame benachbarte Ringatome aufweisen, und der heterocyclische $C_5$-$C_7$-Ring gegebenenfalls mit Oxo substituiert ist;

W $C_1$-$C_4$-Alkylen, $C_2$-$C_4$-Alkenylen, $C_3$-$C_7$-Cycloalkylen oder Phenylen ist, die jeweils gegebenenfalls mit $C_1$-$C_3$-Alkyl substituiert sind; und

$R^{10}$ and $R^{11}$ zusammen mit dem Stickstoffatom, an das sie gebunden sind, 2-Methylpyrrolidin-1-yl bilden.

**22.** Verbindung nach Anspruch 1, ausgewählt aus Verbindungen der Formel (Im) und pharmazeutisch annehmbaren Salzen, Hydraten und Solvaten davon:

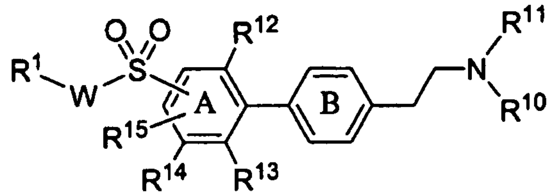

**(Im)**

worin:

$R^{12}$, $R^{13}$, $R^{14}$ und $R^{15}$ jeweils unabhängig voneinander aus H, $-CH_3$, Carboxy, Cl und Br ausgewählt sind;

$R^1$ aus H, $C_1$-$C_6$-Alkoxy, Carbo-$C_1$-$C_6$-alkoxy, Hydroxyl und Phenyl ausgewählt ist; oder

$R^1$ zusammen mit der W-$SO_2$-Grupp und dem Ringatom, an das die W-$SO_2$-Gruppe gebunden ist, einen heterocyclischen $C_5$-$C_7$-Ring bilden, sodass der heterocyclische $C_5$-$C_7$-Ring und der Ring A zwei gemeinsame benachbarte Ringatome aufweisen, und der heterocyclische $C_5$-$C_7$-Ring gegebenenfalls mit Oxo substituiert ist;

W aus $-CH_2-$, $-CH_2CH_2-$, $-CH(CH_3)CH_2-$, $-CH_2CH_2CH_2-$, $-CH_2CH_2CH(CH_3)-$, -HC=CH-, 1,3-Cyclopentylen, $-C(CH_3)_2CH_2-$, $-CH_2C(CH_3)_2CH_2-$, 4-Tetrahydropyran-2-yl, 3-Tetrahydropyran-5-yl und 1,4-Phenylen ausgewählt ist; and

$R^{10}$ und $R^{11}$ zusammen mit dem Stickstoffatom, an das sie gebunden sind, 2-Methylpyrrolidin-1-yl bilden.

**23.** Verbindung nach Anspruch 1, ausgewählt aus Verbindungen der Formel (Im) und pharmazeutisch annehmbaren Salzen, Hydraten und Solvaten davon:

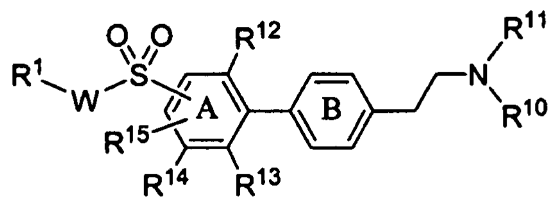

**(Im)**

worin:

$R^{12}$, $R^{13}$, $R^{14}$ und $R^{15}$ jeweils H sind; $-C(=O)OC(CH_3)_3$,

$R^1$ aus H, $-OCH_3$, $-OCH_2CH_3$, $-C(=O)OCH_2CH_3$, $-C(=O)OC(CH_3)_3$, Hydroxyl und Phenyl ausgewählt ist;

W aus $-CH_2CH_2-$ und -HC=CH- ausgewählt ist; and

$R^{10}$ und $R^{11}$ zusammen mit dem Stickstoffatom, an das sie gebunden sind, 2-Methylpyrrolidin-1-yl bilden.

**24.** Verbindung nach Anspruch 1, ausgewählt aus Verbindungen der Formel (Io) und pharmazeutisch annehmbaren Salzen, Hydraten und Solvaten davon:

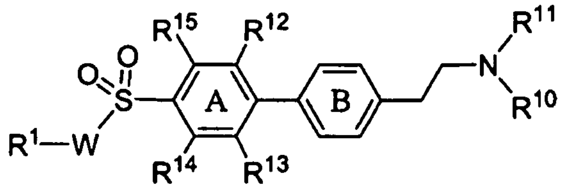

(Io)

worin:

$R^{12}$, $R^{13}$, $R^{14}$ und $R^{15}$ jeweils H sind;
$R^1$ aus H, $-OCH_3$, $-OCH_2CH_3$, $-C(=O)OCH_2CH_3$, $-C(=O)OC(CH_3)_3$, Hydroxyl und Phenyl ausgewählt ist;
W aus $-CH_2CH_2-$ und -HC=CH- ausgewählt ist; und
$R^{10}$ und $R^{11}$ zusammen mit dem Stickstoffatom, an das sie gebunden sind, 2-Methylpyrrolidin-1-yl bilden.

**25.** Verbindung nach Anspruch 1, ausgewählt aus Verbindungen der Formel (Iq) und pharmazeutisch annehmbaren Salzen, Hydraten und Solvaten davon:

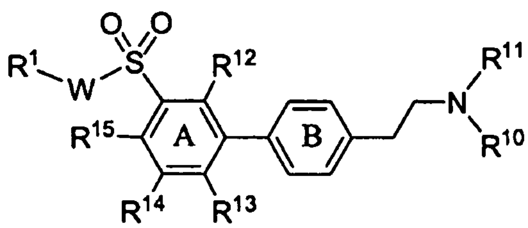

(Iq)

worin:

$R^{12}$, $R^{13}$, $R^{14}$ und $R^{15}$ jeweils H sind;
$R^1$ aus H, $-OCH_3$, $-OCH_2CH_3$, $-C(=O)OCH_2CH_3$, $-C(=O)OC(CH_3)_3$, Hydroxyl und Phenyl ausgewählt ist;
W aus $-CH_2CH_2-$ und -HC=CH- ausgewählt ist; und
$R^{10}$ und $R^{11}$ zusammen mit dem Stickstoffatom, an das sie gebunden sind, 2-Methylpyrrolidin-1-yl bilden.

**26.** Verbindung nach Anspruch 1, ausgewählt aus Verbindungen der Formel (Is) und pharmazeutisch annehmbaren Salzen, Hydraten und Solvaten davon:

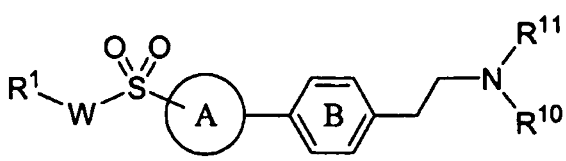

(Is)

worin:

der Ring A:

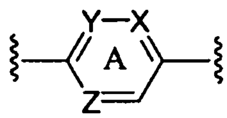
oder
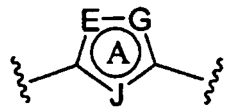

ist;
X = N oder CH ist; Y = N oder CH ist; und Z = N oder CH ist; mit der Maßgabe, dass zumindest eines aus X, Y und Z = N ist;
J = N oder NH ist; und E und G jeweils unabhängig voneinander aus N und S ausgewählt sind, mit der Maßgabe, dass zumindest eines aus E und G = N ist;
$R^1$ aus H, $C_1$-$C_6$-Alkoxy, Carbo-$C_1$-$C_6$-alkoxy, Hydroxyl und Phenyl ausgewählt ist; oder
$R^1$ zusammen mit der W-$SO_2$-Gruppe und dem Ringatom, an das die W-$SO_2$-Gruppe gebunden ist, einen heterocyclischen $C_5$-$C_7$-Ring mit Ring A bildet, sodass der heterocyclische $C_5$-$C_7$-Ring und der Ring A zwei gemeinsame benachbarte Ringatome aufweisen;
W aus $-CH_2-$, $-CH_2CH_2-$, $-CH(CH_3)CH_2-$, $-CH_2CH_2CH_2-$, $-CH_2CH_2CH(CH_3)-$, -HC=CH-, 1,3-Cyclopentylen, $-C(CH_3)_2CH_2-$, $-CH_2C(CH_3)_2CH_2-$, 4-Tetrahydropyran-2-yl, 3-Tetrahydropyran-5-yl und 1,4-Phenylen ausgewählt ist; und
$R^{10}$ and $R^{11}$ zusammen mit dem Stickstoffatom, an das sie gebunden sind, 2-Methylpyrrolidin-1-yl bilden.

**27.** Verbindung nach Anspruch 1, ausgewählt aus Verbindungen der Formel (Is) und pharmazeutisch annehmbaren Salzen, Hydraten und Solvaten davon:

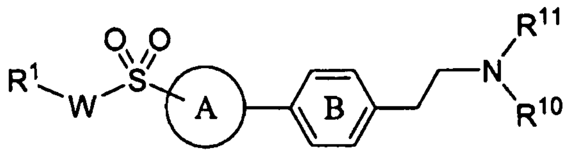

**(Is)**

worin:

der Ring A:

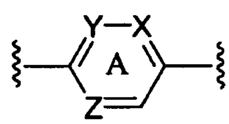
oder
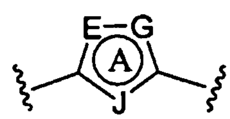

ist;
X = N oder CH ist; Y = N oder CH ist; und Z = N oder CH ist; mit der Maßgabe, dass zumindest eines aus X, Y und Z = N ist;
J = N oder NH ist; und E und G jeweils unabhängig voneinander aus N und S ausgewählt sind, mit der Maßgabe, dass zumindest eines aus E und G = N ist;
$R^1$ aus H, $-OCH_3$, $-OCH_2CH_3$, $-C(=O)OCH_2CH_3$, $-C(=O)OC(CH_3)_3$, Hydroxyl und Phenyl ausgewählt ist;
W aus $-CH_2CH_2-$ und -HC=CH- ausgewählt ist; und
$R^{10}$ und $R^{11}$ zusammen mit dem Stickstoffatom, an das sie gebunden sind, 2-Methylpyrrolidin-1-yl bilden.

**28.** Verbindung nach Anspruch 1, ausgewählt aus folgenden Verbindungen und pharmazeutisch annehmbaren Salzen, Hydraten und Solvaten davon:

1-[2-(4'-Methansulfonylbiphenyl-4-yl)ethyl]-2-methylpyrrolidin;
1-[2-(4'-Ethansulfonylbiphenyl-4-yl)ethyl]-2-methylpyrrolidin;
1-{2-[4'-(2-Methoxyethansulfonyl)biphenyl-4-yl]ethyl}-2-methylpyrrolidin;

2-Methyl-1-{2-[4'-(propan-2-sulfonyl)biphenyl-4-yl]ethyl}pyrrolidin;
2-Methyl-1-{2-[4'-(propan-1-sulfonyl)biphenyl-4-yl]ethyl}pyrrolidin;
2-Methyl-1-[2-(4'-phenylmethansulfonylbiphenyl-4-yl)ethyl]pyrrolidin;
6-{4-[2-(2-Methylpyrrolidin-1-yl)ethyl]phenyl}-1,1-dioxo-1λ$^6$-thiochroman-4-on;
1-{2-[4'-(3-Methoxypropan-1-sulfonyl)biphenyl-4-yl]ethyl}-2-methylpyrrolidin;
2-Methyl-1-{2-[4'-(2-methylpropan-1-sulfonyl)biphenyl-4-yl]ethyl}pyrrolidin;
2-{4'-[2-(2-Methylpyrrolidin-1-yl)ethyl]biphenyl-4-sulfonyl}ethanol;
{4'-[2-(2-Methylpyrrolidin-1-yl)ethyl]biphenyl-4-sulfonyl}essigsäureethylester;
1-{2-[4'-(2-Ethoxyethansulfonyl)biphenyl-4-yl]ethyl}-2-methylpyrrolidin;
1-[2-(4'-Ethensulfonylbiphenyl-4-yl)ethyl]-2-methylpyrrolidin;
1-[2-(4'-Cyclopentansulfonylbiphenyl-4-yl)ethyl]-2-methylpyrrolidin;
3-{4'-[2-(2-Methylpyrrolidin-1-yl)ethyl]biphenyl-4-sulfonyl}propan-1-ol;
1-{2-[3'-(2-Methoxyethansulfonyl)biphenyl-4-yl]ethyl}-2-methylpyrrolidin;
2-{4'-[2-(2-Methylpyrrolidin-1-yl)ethyl]biphenyl-3-sulfonyl}ethanol;
{4'-[2-(2-Methylpyrrolidin-1-yl)ethyl]biphenyl-4-sulfonyl}essigsäure-tert-butylester; und
{4'-[2-(2-Methylpyrrolidin-1-yl)ethyl]biphenyl-4-sulfonyl}essigsäuremethylester.

**29.** Verbindung nach Anspruch 28, worin die Verbindung das (R)-Enantiomer ist.

**30.** Verbindung nach Anspruch 1, ausgewählt aus Verbindungen der Formel (Is) und pharmazeutisch annehmbaren Salzen, Hydraten und Solvaten davon:

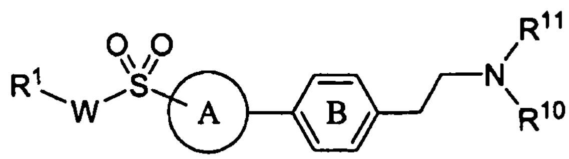

**(Is)**

worin:

der Ring A aus 1,4-Phenylen, 1,3-Phenylen, 4-Carboxy-1,3-phenylen, 4-Methyl-1,3-phenylen, Pyridin-2,5-ylen, Pyrimidin-2,5-ylen und 1,2,4-Thiadiazol-3,5-ylen ausgewählt ist;
$R^1$ aus H, $-OCH_3$, $-OCH_2CH_3$, $-C(=O)OCH_3$, $-C(=O)OCH_2CH_3$, $-C(=O)OC(CH_3)_3$, Hydroxyl, Phenyl, Morpholin-4-yl, Tetrahydropyran-4-yl, Carboxy, 4-Cyanopiperidin-1-yl, Amino, Cyclohexylamino, Methylamino und Tetrahydropyran-2-yl ausgewählt ist;
W aus $-CH_2-$, $-CH_2CH_2-$, $-CH(CH_3)CH_2-$, $-CH_2CH_2CH_2-$, -HC=CH-, 1,3-Cyclopentylen, $-CH_2C(CH_3)_2CH_2-$, 4-Tetrahydropyran-2-yl, $-CH_2HC=CH-$, $-CH_2CH_2C(=O)-$, $-CH_2CH(CH_3)CH_2-$, $-CH_2CH(CH_3)-$ und Piperidin-2,4-ylen ausgewählt ist; und
$R^{10}$ und $R^{11}$ zusammen mit dem Stickstoffatom, an das sie gebunden sind, 2-Methylpyrrolidin-1-yl bilden.

**31.** Verbindung nach Anspruch 1, ausgewählt aus folgenden Verbindungen und pharmazeutisch annehmbaren Salzen, Hydraten und Solvaten davon:

3-Methansulfonyl-4'-[2-(2-methylpyrrolidin-1-yl)ethyl]biphenyl-4-carbonsäure;
2-Methyl-1-{2-[4'-(tetrahydropyran-4-sulfonyl)biphenyl-4-yl]ethyl}pyrrolidin;
2-Methansulfonyl-5-{4-[2-(2-methylpyrrolidin-1-yl)ethyl]phenyl}pyridin;
1-{2-[4'-(2-Methoxypropan-1-sulfonyl)biphenyl-4-yl]ethyl}-2-methylpyrrolidin;
2-Methansulfonyl-5-{4-[2-(2-methylpyrrolidin-1-yl)ethyl]phenyl}pyrimidin;
4-(2-{4'-[2-(2-Methylpyrrolidin-1-yl)ethyl]biphenyl-4-sulfonyl}ethyl)morpholin;
2-Methyl-1-{2-[4'-(tetrahydropyran-4-ylmethansulfonyl)biphenyl-4-yl]ethyl}pyrrolidin;
1-[2-(3'-Methansulfonyl-4'-methylbiphenyl-4-yl)ethyl]-2-methylpyrrolidin;
3-{4'-[2-(2-Methylpyrrolidin-1-yl)ethyl]biphenyl-4-sulfonyl}propionsäure;
1-(2-{4'-[2-(2-Methylpyrrolidin-1-yl)ethyl]biphenyl-4-sulfonyl}ethyl)piperidin-4-carbonitril;
2-Methyl-1-{2-[4'-(prop-2-en-1-sulfonyl)biphenyl-4-yl]ethyl}pyrrolidin;
2-{4'-[2-(2-Methylpyrrolidin-1-yl)ethyl]biphenyl-4-sulfonyl}ethylamin;

Cyclohexyl-(2-{4'-[2-(2-methylpyrrolidin-1-yl)ethyl]biphenyl-4-sulfonyl}ethyl)amin;
5-Methansulfonyl-2-{4-[2-(2-methylpyrrolidin-1-yl)ethyl]phenyl}pyridin;
N-Methyl-3-(4'-(2-(2-methylpyrrolidin-1-yl)ethyl)biphenyl-4-ylsulfonyl)propanamid;
3-(4'-(2-(2-Methylpyrrolidin-1-yl)ethyl)biphenyl-4-ylsulfonyl)-1-morpholinopropan-1-on;
4-(4'-(2-(2-Methylpyrrolidin-1-yl)ethyl)biphenyl-4-ylsulfonyl)piperidin;
5-(4-(2-(2-Methylpyrrolidin-1-yl)ethyl)phenyl)-3-(methylsulfonyl)-1,2,4-thiadiazol;
1-{2-[4'-(3-Methoxypropan-1-sulfonyl)biphenyl-4-yl]ethyl}-2-methylpyrrolidin;
1-(2-(4'-(2-Methoxyethylsulfonyl)biphenyl-4-yl)ethyl)-2-methylpyrrolidin;
2,2-Dimethyl-3-(4'-(2-(2-methylpyrrolidin-1-yl)ethyl)biphenyl-4-ylsulfonyl)propan-1-ol;
2-Methyl-1-(2-(4'-((tetrahydro-2H-pyran-2-yl)methylsulfonyl)biphenyl-4-yl)-ethyl)pyrrolidin;
1-(2-(4'-(Methoxymethylsulfonyl)biphenyl-4-yl)ethyl)-2-methylpyrrolidin.

**32.** Verbindung nach Anspruch 31, worin die Verbindung das (R)-Enantiomer ist.

**33.** Verbindung nach Anspruch 1, ausgewählt aus folgender Verbindung und pharmazeutisch annehmbaren Salzen, Hydraten und Solvaten davon:

(R)-1-{1-[4'-(2-Methoxyethansulfonyl)biphenyl-4-yl]ethyl}-2-methylpyrrolidon.

**34.** Verbindung nach Anspruch 1, ausgewählt aus folgender Verbindung und pharmazeutisch annehmbaren Salzen, Hydraten und Solvaten davon:

(R)-1-{2-[4'-(3-Methoxypropan-1-sulfonyl)biphenyl-4-yl]ethyl}-2-methylpyrrolidin.

**35.** Verbindung nach Anspruch 1, ausgewählt aus folgender Verbindung und pharmazeutisch annehmbaren Salzen, Hydraten und Solvaten davon:

{4'-[2-((R)-2-Methylpyrrolidin-1-yl)ethyl]biphenyl-4-sulfonyl}essigsäureethylester.

**36.** Verbindung nach Anspruch 1, ausgewählt aus folgender Verbindung und pharmazeutisch annehmbaren Salzen, Hydraten und Solvaten davon:

(R)-2-Methyl-1-{2-[4'-(tetrahydropyran-4-sulfonyl)biphenyl-4-yl]ethyl}pyrrolidin.

**37.** Verbindung nach Anspruch 1, ausgewählt aus folgender Verbindung und pharmazeutisch annehmbaren Salzen, Hydraten und Solvaten davon:

(R)-2-Methyl-1-{2-[4'-(tetrahydropyran-4-ylmethansulfonyl)biphenyl-4-yl]ethyl}-pyrrolidin.

**38.** Pharmazeutische Zusammensetzung, eine Verbindung nach einem der Ansprüche 1 bis 37 und einen pharmazeutisch annehmbaren Träger umfassend.

**39.** Verfahren zur Herstellung einer Zusammensetzung, umfassend das Vermischen einer Verbindung nach einem der Ansprüche 1 bis 37 und eines pharmazeutisch annehmbaren Trägers.

**40.** Verbindung nach einem der Ansprüche 1 bis 37 zur Verwendung in einem Verfahren zur Behandlung eines menschlichen oder tierischen Körpers mittels Therapie.

**41.** Verbindung nach einem der Ansprüche 1 bis 37 zur Verwendung in einem Verfahren zur Behandlung einer kognitiven Störung, von Epilepsie, eines Hirntraumas, einer Depression, von Obesität, einer Schlaf- oder Wachheitsstörung, von Narkolepsie, von Kataplexie, von Hypersomnie, des Somnolenzsyndroms, eines Jetlags, einer Schlafapnoe, des Aufmerksamkeitsdefizitsyndroms (ADHD), von Schizophrenie, einer Allergie, einer allergischen Reaktion in den oberen Atemwegen, von allergischer Rhinitis, von Nasenverstopfung, von Demenz oder der Alzheimer-Krankheit.

**42.** Verbindung nach einem der Ansprüche 1 bis 37 zur Verwendung in einem Verfahren zur Behandlung einer kognitiven Störung.

43. Verbindung nach einem der Ansprüche 1 bis 37 zur Verwendung in einem Verfahren zur Behandlung von Kataplexie.

44. Verbindung nach einem der Ansprüche 1 bis 37 zur Verwendung in einem Verfahren zur Herbeiführung von Wachheit.

45. Verbindung nach einem der Ansprüche 1 bis 37 zur Verwendung in einem Verfahren zur Behandlung von Narkolepsie.

46. Verwendung einer Verbindung nach einem der Ansprüche 1 bis 37 zur Herstellung eines Medikaments zur Behandlung einer kognitiven Störung, von Epilepsie, eines Hirntraumas, einer Depression, von Obesität, einer Schlaf- oder Wachheitsstörung, von Narkolepsie, von Kataplexie, von Hypersomnie, des Somnolenzsyndroms, eines Jetlags, einer Schlafapnoe, des Aufmerksamkeitsdefizitsyndroms (ADHD), von Schizophrenie, einer Allergie, einer allergischen Reaktion in den oberen Atemwegen, von allergischer Rhinitis, von Nasenverstopfung, von Demenz oder der Alzheimer-Krankheit.

47. Verwendung einer Verbindung nach einem der Ansprüche 1 bis 37 zur Herstellung eines Medikaments zur Behandlung einer kognitiven Störung.

48. Verwendung einer Verbindung nach einem der Ansprüche 1 bis 37 zur Herstellung eines Medikaments zur Behandlung von Kataplexie.

49. Verwendung einer Verbindung nach einem der Ansprüche 1 bis 37 zur Herstellung eines Medikaments zur Herbeiführung von Wachheit.

50. Verwendung einer Verbindung nach einem der Ansprüche 1 bis 37 zur Herstellung eines Medikaments zur Behandlung von Narkolepsie.

## Revendications

1. Composé sélectionné parmi des composés de la Formule (Ia) :

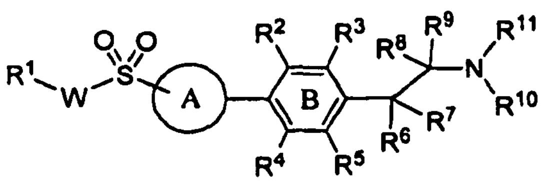

(Ia)

et de sels, hydrates et solvates pharmaceutiquement acceptables de celui-ci; où:

$R^1$ est sélectionné parmi H, acyle$C_1$-$C_6$, acyloxy$C_1$-$C_6$, alkényle$C_2$-$C_8$, alkoxy$C_1$-$C_6$, alkyle$C_1$-$C_8$, alkylcarboxamide$C_1$-$C_8$, alkynyle$C_2$-$C_8$, alkylsulfonamide$C_1$-$C_8$, alkylsulfinyle$C_1$-$C_8$, alkylsulfonyle$C_1$-$C_8$, alkylthio$C_1$-$C_8$, alkyluréyle$C_1$-$C_8$, amino, alkylamino$C_1$-$C_8$, dialkylamino$C_2$-$C_8$, carbo-alkoxy$C_1$-$C_6$, carboxamide, carboxy, cyano, cycloalkyle$C_3$-$C_7$, dialkylcarboxamide$C_2$-$C_8$, dialkylsulfonamide$C_2$-$C_8$, halogéne, haloalkoxy$C_1$-$C_6$, haloalkyle$C_1$-$C_6$, haloalkylsulfinyle$C_1$-$C_6$, haloalkylsulfonyle$C_1$-$C_6$, haloalkylthio$C_1$-$C_6$, hétérocyclyle$C_3$-$C_7$, hydroxyle, thiole, nitro, phényle et sulfonamide, et chacun est optionnellement substitué par 1, 2, 3, 4 ou 5 substituants sélectionnés indépendamment parmi acyle$C_1$-$C_6$, acyloxy$C_1$-$C_6$, alkényle$C_2$-$C_8$, alkoxy$C_1$-$C_6$, alkyle$C_1$-$C_8$, alkylcarboxamide$C_1$-$C_8$, alkynyle$C_2$-$C_8$, alkylsulfonamide$C_1$-$C_8$, alkylsulfinyle$C_1$-$C_8$, alkylsulfonyle$C_1$-$C_8$, alkylthio$C_1$-$C_8$, alkyluréyle$C_1$-$C_8$, amino, alkylamino$C_1$-$C_8$, dialkylamino$C_2$-$C_8$, carbo-alkoxy$C_1$-$C_6$, carboxamide, carboxy, cyano, cycloalkyle$C_3$-$C_7$, dialkylcarboxamide$C_2$-$C_8$, dialkylsulfonamide$C_2$-$C_8$, halogène, haloalkoxy$C_1$-$C_6$, haloalkyle$C_1$-$C_6$, haloalkylsulfinyle$C_1$-$C_6$, haloalkylsulfonyle$C_1$-$C_6$, haloalkyl-thio$C_1$-$C_6$, hydroxyle, thiol, nitro et sulfonamide; ou

$R^1$ ensemble avec le groupe W-$SO_2$ et l'atome de cycle auquel le groupe W-$SO_2$ est lié forment un cycle hétérocyclique $C_5$-$C_7$ avec le Cycle A, par quoi ledit cycle hétérocyclique $C_5$-$C_7$ et le Cycle A partagent deux atomes de cycle adjacents, et ledit cycle hétérocyclique $C_5$-$C_7$ est optionnellement substitué par 1, 2, 3 ou 4

substituants sélectionnés indépendamment parmi acyle$C_1$-$C_6$, acyloxy$C_1$-$C_6$, alkényle$C_2$-$C_8$, alkoxy$C_1$-$C_6$, alkyle$C_1$-$C_8$, alkylcarboxamide$C_1$-$C_8$, alkynyle$C_2$-$C_8$, alkylsulfonamide$C_1$-$C_8$, alkylsulfinyle$C_1$-$C_8$, alkylsulfonyle$C_1$-$C_8$, alkylthio$C_1$-$C_8$, alkyluréyle$C_1$-$C_8$, amino, alkylamino$C_1$-$C_8$, dialkylamino$C_2$-$C_8$, carboalkoxy$C_1$-$C_6$, carboxamide, carboxy, cyano, cycloalkyle$C_3$-$C_7$, dialkyl-carboxamide$C_2$-$C_8$, dialkylsulfonamide$C_2$-$C_8$, halogène, haloalkoxy$C_1$-$C_6$, haloalkyle$C_1$-$C_6$, haloalkylsulfinyle$C_1$-$C_6$, haloalkylsulfonyle$C_1$-$C_6$, haloalkylthio$C_1$-$C_6$, hydroxyle, thiol, nitro, oxo et sulfonamide;

W est alkylène$C_1$-$C_4$, alkénylène$C_2$-$C_4$, cycloalkylène$C_3$-$C_7$, hétérocyclylène$C_3$-$C_7$ ou phénylène, chacun optionnellement substitué par 1, 2, 3, 4, 5, 6, 7 ou 8 substituants sélectionnés indépendamment parmi alkyle$C_1$-$C_3$, alkoxy$C_1$-$C_4$, carboxy, cyano, haloalkyle$C_1$-$C_3$, halogène, hydroxyle et oxo;

le Cycle A est 1, 3-phénylène ou 1,4-phénylène, chacun substitué par $R^{12}$, $R^{13}$, $R^{14}$ et $R^{15}$, où $R^{12}$, $R^{13}$, $R^{14}$ et $R^{15}$ sont chacun indépendamment sélectionnés parmi H, acyle$C_1$-$C_6$, acyloxy$C_1$-$C_6$, alkényle$C_2$-$C_8$, alkoxy$C_1$-$C_6$, alkyle$C_1$-$C_8$, alkylcarboxamide$C_1$-$C_8$, alkynyle$C_2$-$C_8$, alkylsulfonamide$C_1$-$C_8$, alkylsulfinyle$C_1$-$C_8$, alkylsulfonyle$C_1$-$C_8$, alkylthio$C_1$-$C_8$, alkyluréyle$C_1$-$C_8$, amino, alkylamino$C_1$-$C_8$, dialkylamino$C_2$-$C_8$, carboalkoxy$C_1$-$C_6$, carboxamide, carboxy, cyano, cycloalkyle$C_3$-$C_7$, dialkylcarboxamide$C_2$-$C_8$, dialkylsulfonamide$C_2$-$C_8$, halogène, haloalkoxy$C_1$-$C_6$, haloalkyle$C_1$-$C_6$, haloalkylsulfinyle$C_1$-$C_6$, haloalkylsulfonyle$C_1$-$C_6$, haloalkylthio$C_1$-$C_6$, hydroxyle, thiol, nitro et sulfonamide; ou

le Cycle A est un hétéroarylène à 6 membres ou un hétéroarylène à 5 membres, 1 chacun optionnellement substitué par $R^{16}$, $R^{17}$ et $R^{18}$, où $R^{16}$, $R^{17}$ et $R^{18}$ sont chacun sélectionnés indépendamment parmi acyle$C_1$-$C_6$, acyloxy$C_1$-$C_6$, alkényle$C_2$-$C_8$, alkoxy$C_1$-$C_6$, alkyle$C_1$-$C_8$, alkylcarboxamide$C_1$-$C_8$, alkynyle$C_2$-$C_8$, alkylsulfonamide$C_1$-$C_8$, alkylsulfinyle$C_1$-$C_8$, alkylsulfonyle$C_1$-$C_8$, alkylthio$C_1$-$C_8$, alkyluréyle$C_1$-$C_8$, amino, alkylamino$C_1$-$C_8$, dialkylamino$C_2$-$C_8$, carboalkoxy $C_1$-$C_8$, carboxamide, carboxy, cyano, cycloalkyle$C_3$-$C_7$, dialkylcarboxamide$C_2$-$C_8$, dialkylsulfonamide$C_2$-$C_8$, halogène, haloalkoxy$C_1$-$C_8$, haloalkyle$C_1$-$C_6$, haloalkylsulfinyle$C_1$-$C_6$, haloalkyl-sulfonyle$C_1$-$C_6$, haloalkylthio$C_1$-$C_6$, hydroxyle, thiol, nitro et sulfonamide;

$R^2$, $R^3$, $R^4$ et $R^5$ sont chacun sélectionnés indépendamment parmi H, acyle$C_1$-$C_6$, acyloxy$C_1$-$C_6$, alkényle$C_2$-$C_8$, alkoxy$C_1$-$C_6$, alkyle$C_1$-$C_8$, alkylcarboxamide$C_1$-$C_8$, alkynyle$C_2$-$C_8$, alkylsulfonamide$C_1$-$C_8$, alkylsulfinyle$C_1$-$C_8$, alkylsulfonyle$C_1$-$C_8$, alkylthio$C_1$-$C_8$, alkyluréyle$C_1$-$C_8$, amino, alkylamino$C_1$-$C_8$, dialkylamino$C_2$-$C_8$, carboalkoxy $C_1$-$C_6$, carboxamide, carboxy, cyano, cycloalkyle$C_3$-$C_7$, dialkylcarboxamide$C_2$-$C_8$, dialkylsulfonamide$C_2$-$C_8$, halogène, haloalkoxy$C_1$-$C_6$, haloalkyle$C_1$-$C_6$, haloalkylsulfinyle$C_1$-$C_6$, haloalkylsulfonyle$C_1$-$C_6$, haloalkylthio$C_1$-$C_6$, hydroxyle, thiol, nitro et sulfonamide;

$R^6$, $R^7$, $R^8$ et $R^9$ sont chacun sélectionnés indépendamment parmi H, alkyle$C_1$-$C_3$, alkoxy$C_1$-$C_4$, carboxy, cyano, haloalkyle$C_1$-$C_3$, halogène et hydroxyle; et

$R^{10}$ et $R^{11}$ ensemble avec l'atome d'azote auquel ils sont tous les deux liés forment 2-méthyl-pyrrolidin-1-yle; à condition:

1) que le Cycle B et le soufre du groupe $R^1$-W-$S(O)_2$- ne soient pas liés aux atomes de cycle adjacents du Cycle A; et

2) si le Cycle A est 1,3-phénylène ou 1,4-phénylène, et que W est hétérocyclylène$C_3$-$C_7$, alors l'atome de cycle de W qui est directement lié au soufre du groupe $R^1$-W-$S(O)_2$- est autre qu'azote.

**2.** Composé selon la revendication 1, où le Cycle A est 1,3-phénylène.

**3.** Composé selon la revendication 1, où le Cycle A est 1,4-phénylène.

**4.** Composé selon l'une quelconque des revendications 1 à 3, où $R^{12}$, $R^{13}$, $R^{14}$ et $R^{15}$ sont chacun sélectionnés indépendamment de H, alkyle$C_1$-$C_8$, carboxy et halogène.

**5.** Composé selon l'une quelconque des revendications 1 à 3, où $R^{12}$, $R^{13}$, $R^{14}$ et $R^{15}$ sont chacun sélectionnés indépendamment de H, $-CH_3$, carboxy, Cl et Br.

**6.** Composé selon l'une quelconque des revendications 1 à 3, où $R^{12}$, $R^{13}$, $R^{14}$ et $R^{15}$ sont chacun H.

**7.** Composé selon la revendication 1, où le Cycle A est un hétéroarylène à 6 membres.

**8.** Composé selon la revendication 1, où le Cycle A est un hétéroarylène à 5 membres.

**9.** Composé selon l'une quelconque des revendications 1 à 8, dans lequel:

$R^1$ est sélectionné parmi H, alkoxy$C_1$-$C_6$, amino, carboalkoxy$C_1$-$C_6$, carboxamide, carboxy, hétérocyclyle$C_3$-$C_7$,

hydroxyle et phényle, et chacun est optionnellement substitué par cyano ou cycloalkyle$C_3$-$C_7$; ou
$R^1$ ensemble avec le groupe W-$SO_2$ et l'atome de cycle auquel le groupe W-$SO_2$ est lié forment un cycle hétérocyclique $C_5$-$C_7$ avec le Cycle A par quoi ledit cycle hétérocyclique $C_5$-$C_7$ et le Cycle A partagent deux atomes de cycle adjacents, et ledit cycle hétérocyclique $C_5$-$C_7$ est optionnellement substitué par oxo.

**10.** Composé selon l'une quelconque des revendications 1 à 8, dans lequel:

$R^1$ est sélectionné parmi H, alcoxy$C_1$-$C_6$, carboalcoxy$C_1$-$C_6$, hydroxyle et phényle;
ou
$R^1$ ensemble avec le groupe W-$SO_2$ et l'atome de cycle auquel le groupe W-$SO_2$ est lié forment un cycle hétérocyclique $C_5$-$C_7$ avec le Cycle A par quoi ledit cycle hétérocyclique $C_5$-$C_7$ et le Cycle A partagent deux atomes de cycle adjacents, et ledit cycle hétérocyclique $C_5$-$C_7$ est optionnellement substitué par oxo.

**11.** Composé selon l'une quelconque des revendications 1 à 8, où $R^1$ est sélectionné parmi H, alcoxy$C_1$-$C_6$, carboalcoxy$C_1$-$C_6$, hydroxyle et phényle.

**12.** Composé selon l'une quelconque des revendications 1 à 8, où $R^1$ est sélectionné parmi H, $-OCH_3$, $-OCH_2CH_3$, $-C(=O)OCH_2CH_3$, $-C(=O)OC(CH_3)_3$, hydroxyle et phényle.

**13.** Composé selon l'une quelconque des revendications 1 à 12, où W est alkylène$C_1$-$C_4$, alkénylène$C_1$-$C_4$, cycloalkylène$C_3$-$C_7$ ou phénylène, chacun optionnellement substitute par alkyle$C_1$-$C_3$.

**14.** Composé selon l'une quelconque des revendications 1 à 12, où W est alkylène$C_1$-$C_4$ ou alkénylène$C_2$-$C_4$, chacun optionnellement substitué par alkyle$C_1$-$C_3$.

**15.** Composé selon l'une quelconque des revendications 1 à 12, où W est sélectionné parmi $-CH_2-$, $-CH_2CH_2-$, $-CH(CH_3)CH_2-$, $-CH_2CH_2CH_2-$, $-CH_2CH_2CH(CH_3)$ -, -HC=CH-, 1,3-cyclopentylène, $-C(CH_3)_2CH_2-$, $-CH_2C(CH_3)_2CH_2-$, 4-tétrahydropyran-2-yle, 3-tétrahydropyran-5-yle et 1,4-phénylène.

**16.** Composé selon l'une quelconque des revendications 1 à 12, où W est sélectionné parmi $-CH_2-$, $-CH_2CH_2-$, $-CH(CH_3) CH_2-$, $-CH_2CH_2CH_2-$, $-CH_2CH_2CH(CH_3)$ -HC=CH- et 1,3-cyclopentylène.

**17.** Composé selon l'une quelconque des revendications 1 à 12, où W est sélectionné parmi $-CH_2CH_2-$ et -HC=CH-.

**18.** Composé selon l'une quelconque des revendications 1 à 17, où $R^2$, $R^3$, $R^4$ et $R^5$ sont chacun H.

**19.** Composé selon l'une quelconque des revendications 1 à 18, où $R^6$, $R^7$, $R^8$ et $R^9$ sont chacun H.

**20.** Composé selon l'une quelconque des revendications 1 à 19, où $R^{10}$et $R^{11}$ ensemble avec l'atome d'azote auquel ils sont tous deux liés forment (R)-2-méthyl-pyrrolidin-1-yle.

**21.** Composé selon la revendication 1, sélectionné parmi des composés de la Formule (Im) et de sels, hydrates et solvates pharmaceutiquement acceptables de celui-ci:

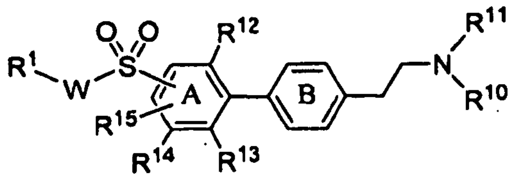

**(Im)**

où:

$R^{12}$, $R^{13}$, $R^{14}$ et $R^{15}$ sont chacun indépendamment sélectionnés parmi H, alkyle$C_1$-$C_8$, carboxy et halogène;
$R^1$ est sélectionné parmi H, alkoxy$C_1$-$C_6$, amino, carbo- alkoxy$C_1$-$C_8$, carboxamide, carboxy, hétérocyclyle$C_3$-$C_7$, hydroxyle et phényle, et chacun est optionnellement substitué par cyano ou cycloalkyle

$C_3$-$C_7$;

ou

$R^1$ ensemble avec le groupe W-$SO_2$ et l'atome de cycle auquel le groupe W-$SO_2$ est lié forment un cycle hétérocyclique $C_5$-$C_7$ avec le Cycle A par quoi ledit cycle hétérocyclique $C_5$-$C_7$ et le Cycle A partagent deux atomes de cycle adjacents, et ledit cycle hétérocyclique $C_5$-$C_7$ est optionnellement substitué par oxo;

W est alkylène$C_1$-$C_4$, alkénylène$C_2$-$C_4$, cycloalkylène$C_3$-$C_7$ ou phénylène, chacun optionnellement substitué par alkyle$C_1$-$C_3$; et

$R^{10}$ et $R^{11}$ ensemble avec l'atome d'azote auquel ils sont tous les deux liés forment 2-méthyl-pyrrolidin-1-yle.

**22.** Composé selon la revendication 1, sélectionné parmi des composés de la Formule (Im) et de sels, hydrates et solvates pharmaceutiquement acceptables de celui-ci:

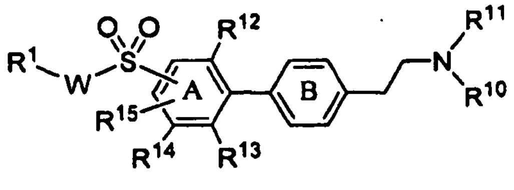

**(Im)**

où:

$R^{12}$, $R^{13}$, $R^{14}$ et $R^{15}$ sont chacun indépendamment sélectionnés parmi H, -$CH_3$, carboxy, Cl et Br;

$R^1$ est sélectionné parmi H, alkoxy$C_1$-$C_6$, carboalcoxy$C_1$-$C_6$, hydroxyle et phényle;

ou

$R^1$ ensemble avec le groupe W-$SO_2$ et l'atome de cycle auquel le groupe W-$SO_2$ est lié forment un cycle hétérocyclique $C_5$-$C_7$ avec le Cycle A par quoi ledit cycle hétérocyclique $C_5$-$C_7$ et le Cycle A partagent deux atomes de cycle adjacents, et ledit cycle hétérocyclique $C_5$-$C_7$ est optionnellement substitué par oxo;

W est sélectionné parmi -$CH_2$-, -$CH_2CH_2$-, -CH ($CH_3$) $CH_2$-, -$CH_2CH_2CH_2$-, -$CH_2CH_2CH(CH_3)$-, -HC=CH-, 1,3-cyclopentylène, -$C(CH_3)_2CH_2$-, -$CH_2C(CH_3)_2CH_2$-, 4-tétrahydropyran-2-yle, 3-tétrahydropyran-5-yle et 1,4-phénylène; et

$R^{10}$ et $R^{11}$ ensemble avec l'atome d'azote auquel ils sont tous les deux lies forment 2-méthyl-pyrrolidin-1-yle.

**23.** Composé selon la revendication 1, sélectionné parmi des composés de la Formule (Im) et de sels, hydrates et solvates pharmaceutiquement acceptables de celui-ci:

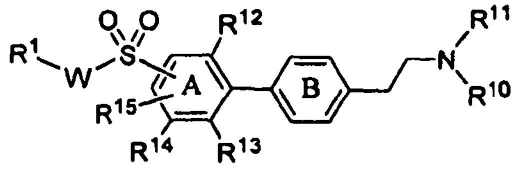

**(Im)**

où:

$R^{12}$, $R^{13}$, $R^{14}$ et $R^{15}$ sont chacun H;

$R^1$ est sélectionné parmi H, -$OCH_3$, -$OCH_2CH_3$, -C(=O)$OCH_2CH_3$, -C(=O)OC$(CH_3)_3$, hydroxyle et phényle; W est sélectionné parmi -$CH_2CH_2$- et -HC=CH-; et $R^{10}$ et $R^{11}$ forment ensemble avec l'atome d'azote auquel ils sont tous les deux lies 2-méthyl-pyrrolidin-1-yle.

**24.** Composé selon la revendication 1, sélectionné parmi des composés de la Formule (Io) et de sels, hydrates et solvates pharmaceutiquement acceptables de celui-ci:

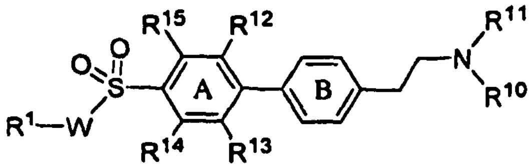

(Io)

où:

$R^{12}$, $R^{13}$, $R^{14}$ et $R^{15}$ sont chacun H;
$R^1$ est sélectionné parmi H, -OCH3, $-OCH_2CH_3$, $-C(=O)OCH_2CH_3$, $-C(=O)OC(CH_3)_3$, hydroxyle et phényle;
W est sélectionné parmi $-CH_2CH_2-$ et -HC=CH-; et
$R^{10}$ et $R^{11}$ ensemble avec l'atome d'azote auquel ils sont tous les deux liés forment 2-méthyl-pyrrolidin-1-yle.

**25.** Composé selon la revendication 1, sélectionné parmi des composés de la Formule (Iq) et de sels, hydrates et solvates pharmaceutiquement acceptables de celui-ci:

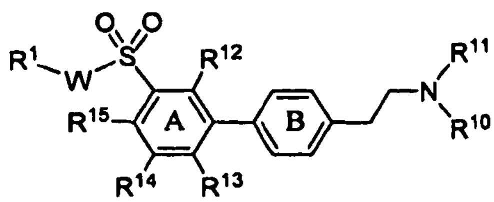

(Iq)

où:

$R^{12}$, $R^{13}$, $R^{14}$ et $R^{15}$ sont chacun H;
$R^1$ est selectionné parmi H, $-OCH_3$, $-OCH_2CH_3$, $-C(=O)OCH_2CH_3$, $-C(=O)OC(CH_3)_3$, hydroxyle et phényle;
W est sélectionné parmi $-CH_2CH_2-$ et -HC=CH-; et
$R^{10}$ et $R^{11}$ ensemble avec l'atome d'azote auquel ils sont tous les deux lies forment 2-méthyl-pyrrolidin-1-yle.

**26.** Composé selon la revendication 1, sélectionné parmi des composés de la Formule (Is) et de sels, hydrates et solvates pharmaceutiquement acceptables de celui-ci:

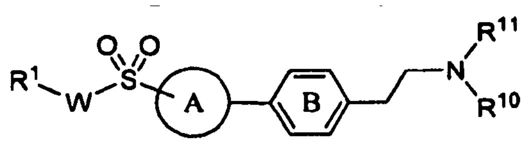

(Is)

où:

le Cycle A est:

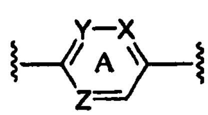

ou

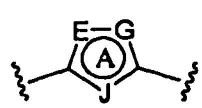

;

X est N ou CH; Y est N ou CH; et Z est N ou CH; à condition qu'au moins un de X, Y et Z soit N;

J est N ou NH; et E et G sont chacun indépendamment sélectionnés parmi N et S, à condition qu'au moins un de E et G soit N;

$R^1$ est sélectionné parmi H, alcoxy$C_1$-$C_6$, carboalcoxy$C_1$-$C_6$, hydroxyle et phényle;

ou

$R^1$ ensemble avec le groupe W-$SO_2$ et l'atome de cycle auquel le groupe W-$SO_2$ est lié forment un cycle hétérocyclique $C_5$-$C_7$ avec le Cycle A par quoi ledit cycle hétérocyclique $C_5$-$C_7$ et le Cycle A partagent deux atomes de cycle adjacents;

W est sélectionné parmi $-CH_2-$, $-CH_2CH_2-$, $-CH(CH_3)\ CH_2-$, $-CH_2CH_2CH_2-$, $-CH_2CH_2CH(CH_3)$ -HC=CH-, 1,3-cyclopentylène, $-C(CH_3)=CH_2-$, $-CH_2C(CH_3)_2CH_2-$, 4-tétrahydropyran-2-yle, 3-tétrahydropyran-5-yle et 1,4-phénylène; et

$R^{10}$ et $R^{11}$ ensemble avec l'atome d'azote auquel ils sont tous les deux liés forment 2-méthyl-pyrrolidin-1-yle.

**27.** Composé selon la revendication 1, sélectionné parmi des composés de la Formule (Is) et de sels, hydrates et solvates pharmaceutiquement acceptables de celui-ci:

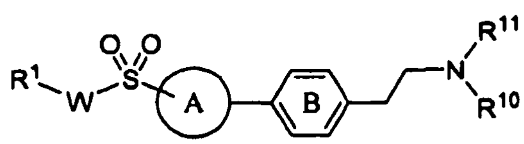

**(Is)**

où:

le Cycle A est:

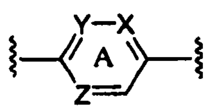

ou

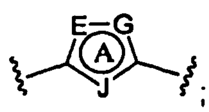

X est N ou CH; Y est N ou CH; et Z est N ou CH; à condition qu'au moins un parmi X, Y et Z soit N;

J est N ou NH; et E et G sont chacun indépendamment sélectionnés parmi N et S; à condition qu'au moins un de E et G soit N;

$R^1$ est sélectionné parmi H; $-OCH_3$, $-OCH_2CH_3$; $-C(=O)OCH_2CH_3$; $-C(=O)OC(CH_3)_3$, hydroxyle et phényle;

W est sélectionné parmi $-CH_2CH_2-$ et -HC=CH-; et

$R^{10}$ et $R^{11}$ ensemble avec l'atome d'azote auquel ils sont tous les deux liés forment 2-méthyl-pyrrolidin-1-yle.

**28.** Composé selon la revendication 1, sélectionné parmi les composés suivants et des sels, hydrates et solvates pharmaceutiquement acceptables de celui-ci:

1-[2-(4'-Méthanesulfonyl-biphényl-4-yl)-éthyl]-2-méthyl-pyrrolidine;
1-[2-(4'-Ethanesulfonyl-biphényl-4-yl)-éthyl]-2-méthyl-pyrrolidine;
1-[2-(4'-(2-Méthoxy-éthanesulfonyl)-biphényl-4-yl]-éthyl)-2-méthyl-pyrrolidine;
2-Méthyl-1-{2-[4'-(propane-2-sulfonyl)-biphényl-4-yl]-éthyl}-pyrrolidine;
2-Méthyl-1-{2-[4'-(propane-1-sulfonyl)-biphényl-4-yl]-éthyl)-pyrrolidine;
2-Méthyl-1-[2-(4'-phénylméthanesulfonyl-biphényl-4-yl)-éthyl]-pyrrolidine;
6-{4-[2-(2-Méthyl-pyrrolidin-1-yl)-éthyl]-phényl}-1,1-dioxo-1$\lambda^6$-thiochroman-4-one;
1-{2-[4'-(3-Méthoxy-propane-1-sulfonyl)-biphényl-4-yl]-éthyl}-2-méthyl-pyrrolidine;
2-Méthyl-1-{2-[4'-(2-méthyl-propane-1-sulfonyl)-biphényl-4-yl]-éthyl)-pyrrolidine;
2-{4'-[2-(2-Méthyl-pyrrolidin-1-yl)-éthyl]-biphényl-4-sulfonyl}-éthanol;
Éthyl ester de l'acide {4'-[2-(2-méthyl-pyrrolidin-1-yl)-éthyl]-biphényl-4-sulfonyl}-acétique;
1-{2-(4'-(2-Ethoxy-éthanesulfonyl)-biphényl-4-yl]-éthyl)-2-méthyl-pyrrolidine;
1-(2-(4'-Ethènesulfonyl-biphényl-4-yl)-éthyl]-2-méthyl-pyrrolidine;
1-[2-(4'-Cyclopentanesulfonyl-biphényl-4-yl)-éthyl]-2-méthyl-pyrrolidine;

3-{4'-[2-(2-Méthyl-pyrrolidin-1-yl)-éthyl] - biphényl-4-sulfonyl}-propan-1-ol;
1-{2-[3'-(2-Méthoxy-éthanesulfonyl)-biphényl-4-yl]-éthyl)-2-méthyl-pyrrolidine;
2-{4'-[2-(2-Méthyl-pyrrolidin-1-yl)-éthyl]-biphényl-3-sulfonyl}-éthanol;
Tert-butyl ester de l'acide {4'-[2-(2-méthylpyrrolidin-1-yl)-éthyl]-biphényl-4-sulfonyl}-acétique; et
Méthyl ester de l'acide {4'-[2-(2-Méthyl-pyrrolidin-1-yl)-éthyl]-biphényl-4-sulfonyl}-acétique.

**29.** Composé selon la revendication 28, où ledit composé est le (R)-énantiomère.

**30.** Composé selon la revendication 1, sélectionné parmi des composés de la Formule (Is) et des sels, hydrates et solvates pharmaceutiquement acceptables de celui-ci:

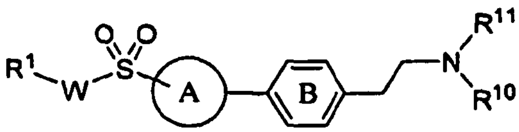

**(Is)**

où:

le Cycle A est sélectionné parmi 1,4-phénylène, 1,3-phénylène, 4-carboxy-1,3-phénylène, 4-méthyl-1,3-phénylène, pyridin-2,5-ylène, pyrimidin-2,5-ylène et 1,2,4-thiadiazol-3,5-ylène;
$R^1$ est sélectionné parmi H, $-OCH_3$, $-OCH_2CH_3$, $-C(=O)OCH_3$, $-C(=O)OCH_2CH_3$, $-C(=O)OC(CH_3)_3$, hydroxyle, phényle, morpholin-4-yle, tétrahydro-pyran-4-yle, carboxy, 4-cyanopipéridin-1-yle, amino, cyclohexylamino, méthylamino et tétrahydro-pyran-2-yle;
W est sélectionné parmi $-CH_2-$, $-CH_2CH_2-$, $-CH(CH_3)CH_2-$, $-CH_2CH_2CH_2-$, -HC=CH-, 1,3-cyclopentylène, $-CH_2C(CH_3)_2CH_2-$, 4-tétrahydropyran-2-yle, $-CH_2HC=CH-$, $-CH_2CH_2C(=O)-$, $-CH_2CH(CH_3)CH_2-$, $-CH_2CH(CH_3)-$ et pipéridin-2,4-ylène; et
$R^{10}$ et $R^{11}$ ensemble avec l'atome d'azote auquel ils sont tous les deux liés forment 2-méthyl-pyrrolidin-1-yle.

**31.** Composé selon la revendication 1, sélectionné parmi les composés suivants et des sels, hydrates et solvates pharmaceutiquement acceptables de celui-ci:

Acide 3-méthanesulfonyl-4'-[2-(2-méthyl-pyrrolidin-1-yle)-éthyll-biphényl-4-carboxylique;
2-Méthyl-1-{2-[4'-(tétrahydro-pyran-4-sulfonyl)-biphényl-4-yl]-éthyl}-pyrrolidine;
2-Méthanesulfonyl-5-{4-[2-(2-méthyl-pyrrolidin-1-yl)-éthyl]-phényl}-pyridine;
1-{2-[4'-(2-Méthoxy-propane-1-sulfonyl)-biphényl-4-yl-éthyl}-2-méthyl-pyrrolidine;
2-Méthanesulfonyl-5-{4-[2-(2-méthyl-pyrrolidin-1-yl)-éthyl]-phényl}-pyrimidine;
4-(2-{4'-[2-(2-Méthyl-pyrrolidin-1-yl)-éthyl]-biphényl-4-sulfonyl}-éthyl)-morpholine;
2-Méthyl-1-{2-[4'-(tétrahydro-pyran-4-ylméthanesulfonyl)-biphényl-4-yl]-éthyl}-pyrrolidine;
1-[2-(3'-Méthanesulfonyl-4'-méthyl-biphényl-4-yl)-éthyl]-2-méthyl-pyrrolidine;
Acide 3-{4'-[2-(2-Méthyl-pyrrolidin-1-yl)-éthyl]-biphényl-4-sulfonyl}-propionique;
1-(2-{4'-[2-(2-Méthyl-pyrrolidin-1-yl)-éthyl]-biphényl-4-sulfonyl}-éthyl)-pipéridine-4-carbonitrile;
2-Méthyl-1-{2-[4'-(prop-2-ène-1-sulfonyl)-biphényl-4-yl]-éthyl}-pyrrolidine;
2-{4'-[2-(2-Méthyl-pyrrolidin-1-yl)-éthyl]-biphényl-4-sulfonyl}-éthyl)-amine;
Cyclohexyl-(2-{4'-[2-(2-méthyl-pyrrolidin-1-yl)-éthyl]-biphényl-4-sulfonyl}-éthyl)-amine;
5-Méthanesulfonyl-2-{4-[2-(2-méthyl-pyrrolidin-1-yl)-éthyl]-phényl}-pyridine;
N-Méthyl-3-(4'-(2-(2-méthylpyrrolidin-1-yl)éthyl)biphényl-4-ylsulfonyl)propanamide;
3-(4'-(2-(2-Méthylpyrrolidin-1-yl)éthyl)biphényl-4-ylsulfonyl)-1-morpholinopropan-1-one;
4-(4'-(2-(2-Méthylpyrrolidin-1-yl)éthyl)biphényl-4-ylsulfonyl)piperidine;
5-(4-(2-(2-Méthylpyrrolidin-1-yl)éthyl)phéyl)-3-(méthylsulfonyl)-1,2,4-thiadiazole;
1-{2-[4'-(3-Méthoxy-propane-1-sulfonyl)-biphényl-4-yl]-éthyl}-2-méthyl-pyrrolidine;
1-(2-(4'-(2-Méthoxyéthylsulfonyl)biphényl-4-yl)éthyl)-2-méthylpyrrolidine;
2,2-Diméthyl-3-(4'-(2-(2-méthylpyrrolidin-1-yl)éthyl)biphenyl-4-ylsulfonyl)propan-1-ol;
2-Méthyl-1-(2-(4'-((tétrahydro-2H-pyran-2-yl)méthylsulfonyl)biphényl-4-yl)éthyl)pyrrolidine; et
1-(2-(4'-(Méthoxyméthylsulfonyl)biphényl-4-yl)éthyl)-2-méthylpyrrolidine.

**32.** Composé selon la revendication 31, où ledit composé est le (R)-énantiomère.

**33.** Composé selon la revendication 1, sélectionné parmi le composé suivant et des sels, hydrates et solvates pharmaceutiquement acceptables de celui-ci:

(R)-1-{2-[4'-(2-Méthoxy-éthanesulfonyl)-biphényl-4-yl]-éthyl}-2-méthyl-pyrrolidine.

**34.** Composé selon la revendication 1, sélectionné parmi le composé suivant et des sels, hydrates et solvates pharmaceutiquement acceptables de celui-ci:

(R)-1-{-[4'-(3-Méthoxy-propan-1-sulfonyl)-biphényl-4-yl]-éthyl}-2-méthyl-pyrrolidine.

**35.** Composé selon la revendication 1, sélectionné parmi le composé suivant et des sels, hydrates, solvates pharmaceutiquement acceptables de celui-ci:

Ethyl ester de l'acide (4'-[2-((R)-2-méthylpyrrolidin-1-yl)-ethyl]-biphényl-4-sulfonyl}-acétique.

**36.** Composé selon la revendication 1, sélectionné parmi le compose suivant et des sels, hydrates et solvates pharmaceutiquement acceptables de celui-ci:

(R)-2-Méthyl-1-(2-[4'-(tétrahydro-pyran-4-sulfonyl)-biphényl-4-yl]-éthyl)-pyrrolidine.

**37.** Composé selon la revendication 1, sélectionné parmi le composé suivant et des sels, hydrates et solvates pharmaceutiquement acceptables de celui-i:

(R)-2-Méthyl-1-(2-[4'-(tétrahydro-pyran-4-ylméthanesulfonyl)-biphényl-4-yl]-éthyl)-pyrrolidine.

**38.** Composition pharmaceutique comprenant un composé selon l'une quelconque des revendications 1 à 37 et un support pharmaceutiquement acceptable.

**39.** Procédé de préparation d'une composition comprenant le mélange d'un composé selon l'une quelconque des revendications 1 à 37 et d'un support pharmaceutiquement acceptable.

**40.** Composé selon l'une quelconque des revendications 1 à 37 pour utilization dans une méthode de traitement du corps humain ou animal par thérapie.

**41.** Composé selon l'une quelconque des revendications 1 à 37 destiné à être utilisé dans une méthode de traitement d'un trouble cognitif, épilepsie, trauma du cerveau, depression, obésité, un trouble du sommeil ou de veille, narcolepsie, cataplexie, hypersomnie, syndrome de somnolence, décalage horaire, apnée du sommeil, trouble déficitaire de l'attention avec hyperactivité (ADHD), schizophrénie, une allergie, une réponse allergique dans le conduit aérien supérieur, rhinite allergique, congestion nasale, démence ou la maladie d'Alzheimer.

**42.** Composé selon l'une quelconque des revendications 1 à 37 pour utilization d'une méthode de traitement d'un trouble cognitif.

**43.** Composé selon l'une quelconque des revendications 1 à 37 pour utilization dans une méthode de traitement de cataplexie.

**44.** Composé selon l'une quelconque des revendications 1 à 37 pour utilization dans une méthode pour induire l'état de veille.

**45.** Composé selon l'une quelconque des revendications 1 à 37 pour utilization dans une méthode de traitement de la narcolepsie.

**46.** Utilisation d'un composé selon l'une quelconque des revendications 1 à 37 pour la production d'un medicament pour le traitement d'un trouble cognitif, épilepsie, trauma du cerveau, depression, obésité, un trouble du sommeil ou de la veille, narcolepsie, cataplexie, hypersomnie, syndrome de somnolence, décalage horaire, apnée du sommeil, trouble déficitaire de l'attention avec hyperactivité (ADHD), schizophrénie, une allergie, une réponse allergique dans le conduit aérien supérieur, rhinite allergique, congestion nasale, démence ou la maladie d'Alzheimer.

**47.** Utilisation d'un composé selon l'une quelconque des revendications 1 à 37 pour la production d'un medicament pour le traitement d'un trouble cognitif.

**48.** Utilisation d'un composé selon l'une quelconque des revendications 1 à 37 pour la production d'un medicament pour le traitement de la cataplexie.

**49.** Utilisation d'un composé selon l'une quelconque des revendications 1 à 37 pour la production d'un medicament pour induire l'état de veille.

**50.** Utilisation d'un composé selon l'une quelconque des revendications 1 à 37 pour la production d'un medicament pour le traitement de la narcolepsie.

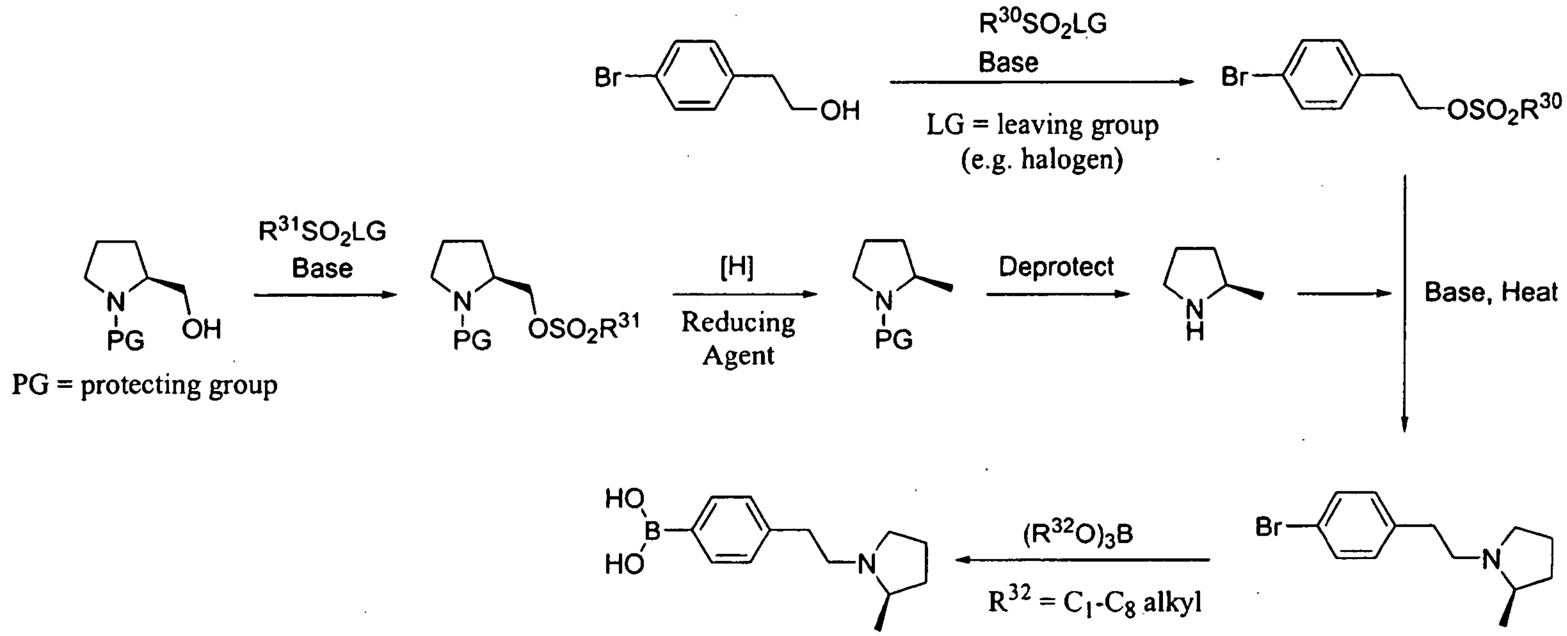


$R^{30}$ and $R^{31}$ are each independently $C_1$-$C_8$ alkyl, $C_2$-$C_8$ alkenyl, aryl-$C_1$-$C_4$-alkylenyl or aryl, and each $R^{30}$ and $R^{31}$ is optionally substituted with 1, 2, 3, 4 or 5 substituents selected independently from the group consisting of $C_1$-$C_8$ alkyl, $C_1$-$C_8$ alkoxy, halogen and nitro.

**Figure 1**

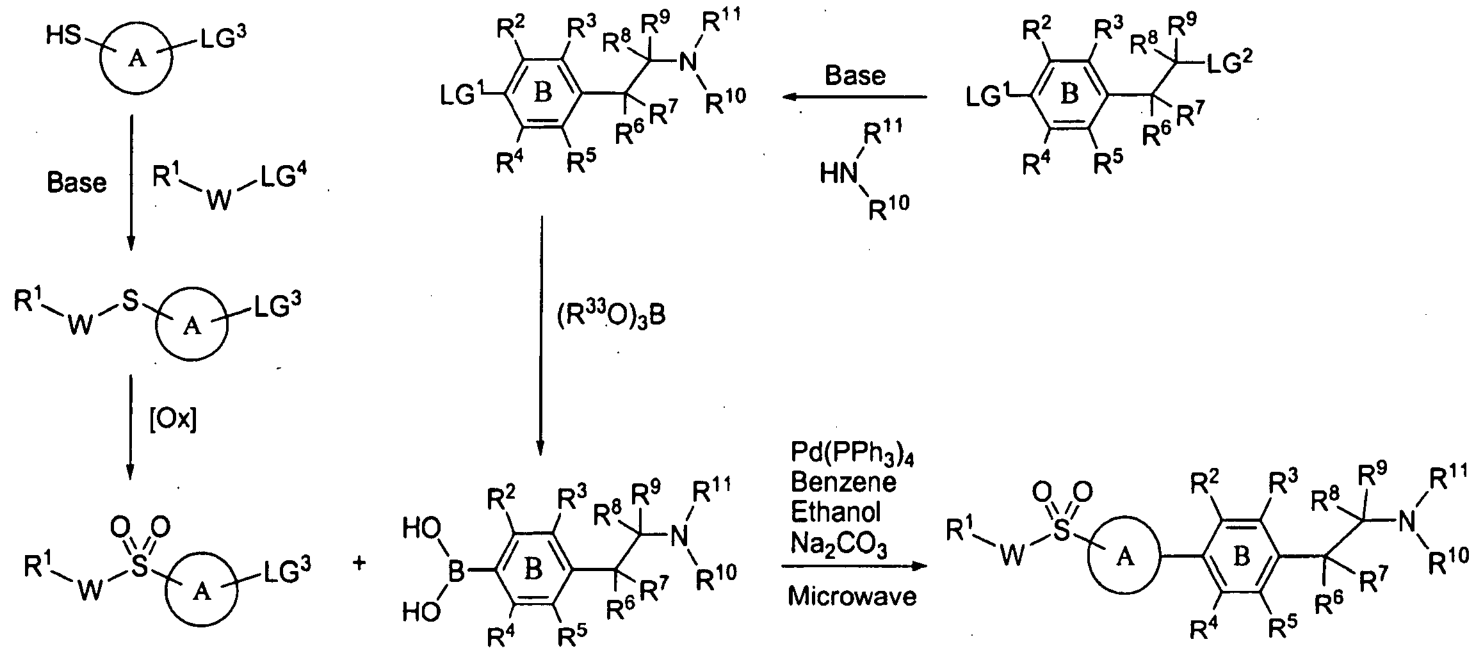


(Ia)

$LG^1$, $LG^2$, $LG^3$ and $LG^4$ are each independently a leaving group, for example halogen, triflate and the like.
$R^{33} = C_1$-$C_8$ alkyl

**Figure 2**

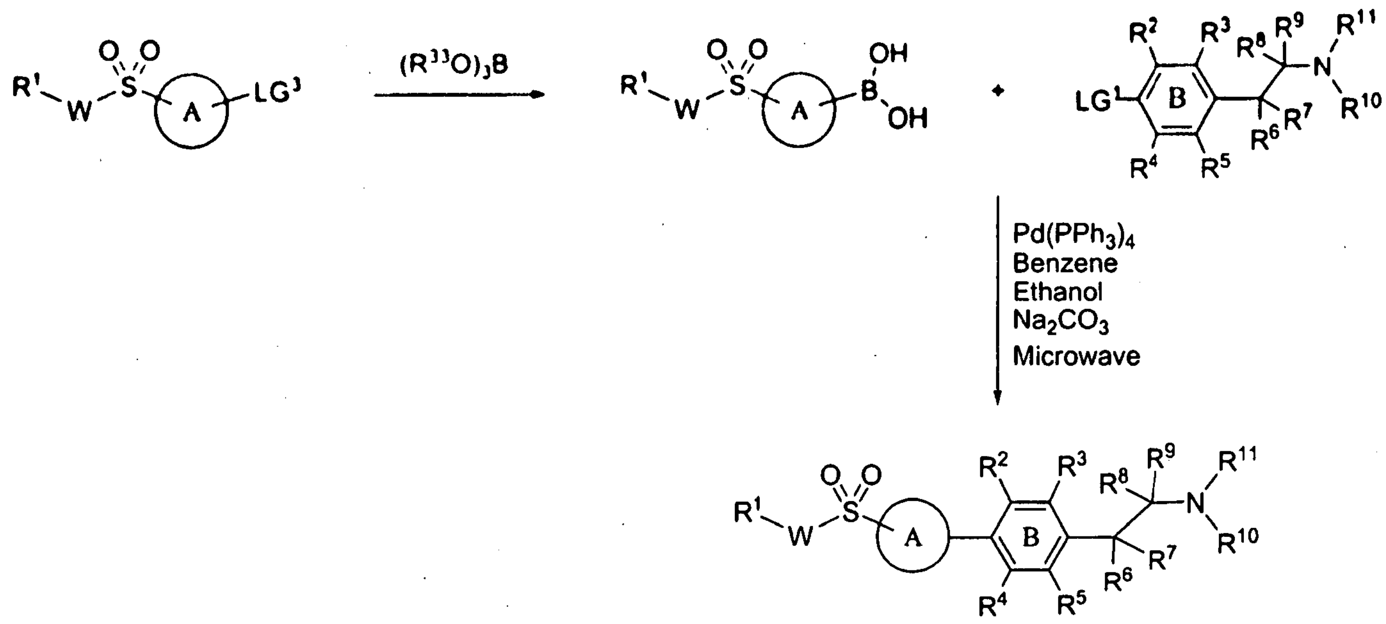


(Ia)

$LG^1$ and $LG^3$ are each a leaving group, for example halogen, triflate and the like.
$R^{33} = C_1\text{-}C_8$ alkyl

# Figure 3

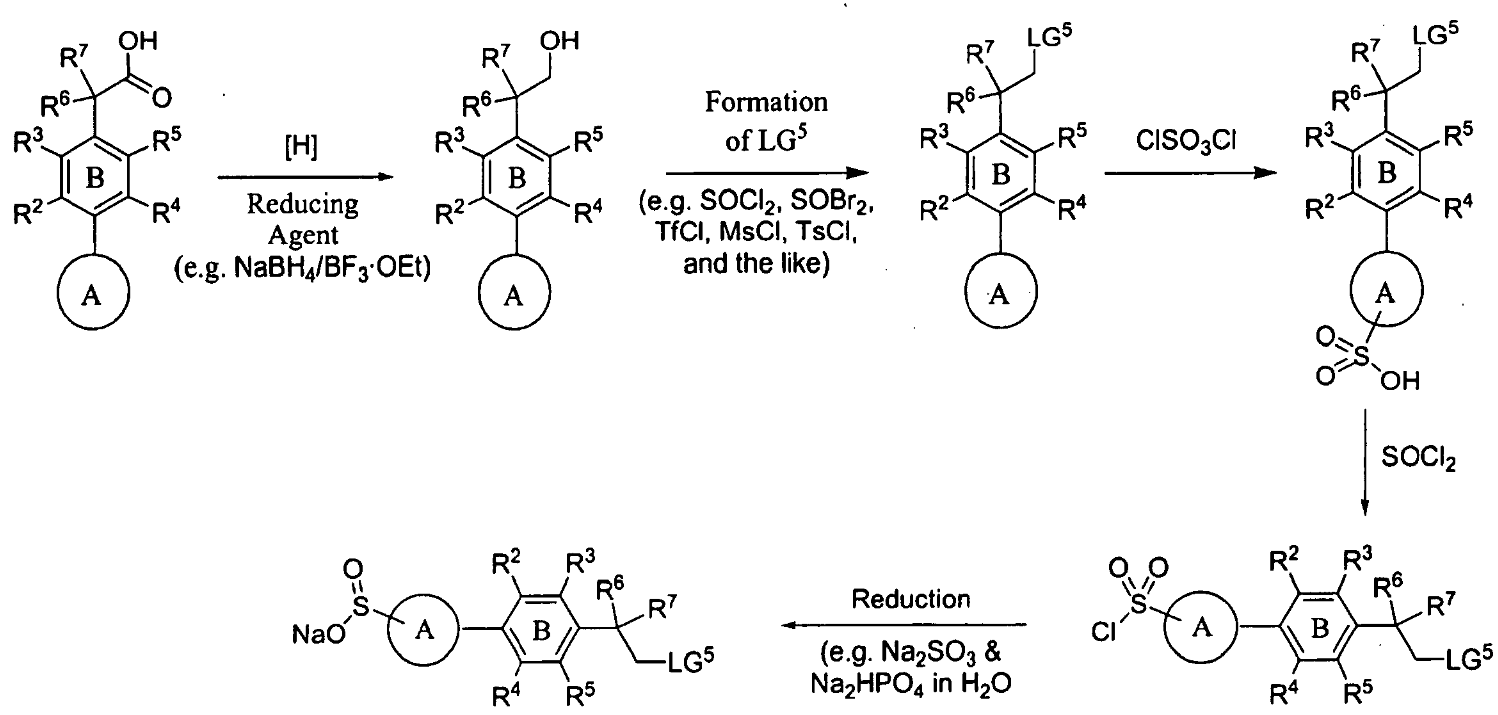


$LG^5$ is a leaving group, for example halogen, triflate and the like.

**Figure 4**

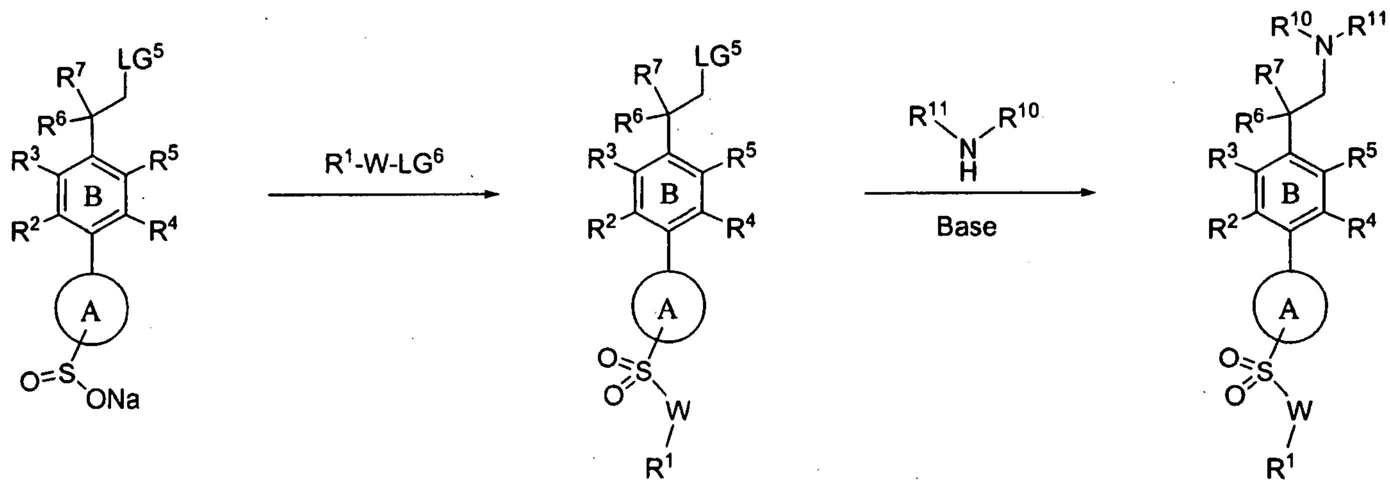


$LG^5$ and $LG^6$ are each a leaving group, for example halogen, triflate and the like.

**Figure 5**

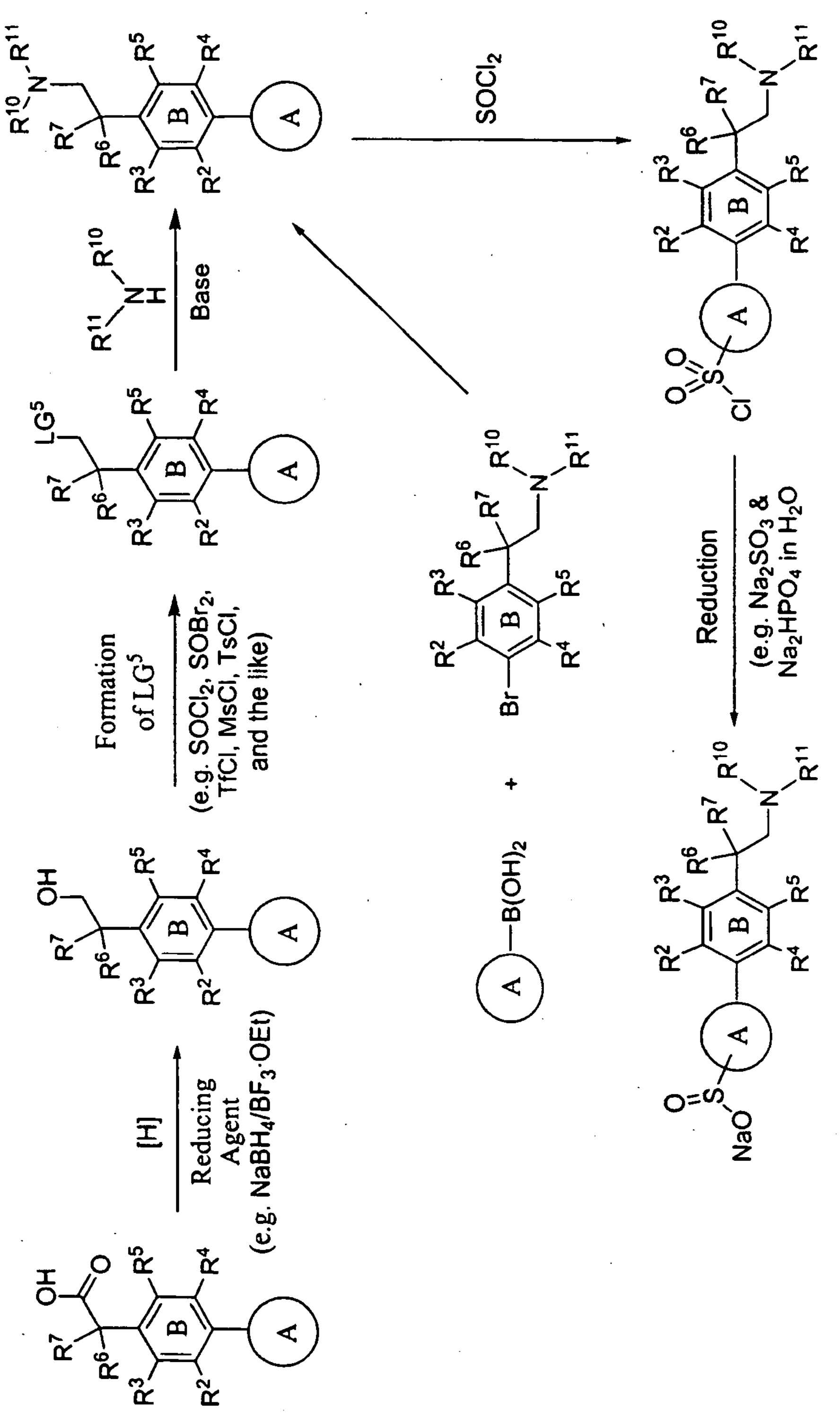


$LG^5$ is a leaving group, for example halogen, triflate and the like.

**Figure 6**

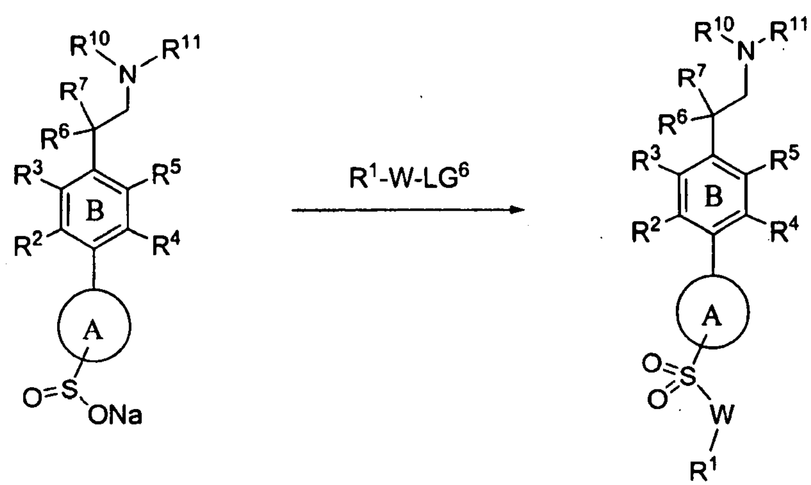


$LG^6$ is a leaving group, for example halogen, triflate and the like.

**Figure 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008005338 A1
- US 20050256127 A1
- WO 2005097740 A1
- US 5217986 A
- US 5352707 A
- US 5869479 A


### Non-patent literature cited in the description


- **Celanire et al.** *Drug Discovery Today,* December 2005, vol. 10 (23/24), 1613-1627
- **Greene, T. W. ; Wuts, P. G. M.** Protecting Groups in Organic Synthesis. Wiley, 1999
- **Arrang et al.** *Nature,* 1983, vol. 302, 832-7
- **Passani et al.** *Trends in Pharmacol. Sci.,* 2004, vol. 25, 618-625
- **Brown et al.** *Prog. Neurobiol.,* 2001, vol. 63, 637-672
- **Leurs et al.** *Nat. Rev. Drug. Discov.,* 2005, vol. 4, 107-120
- **Passani et al.** *Trends Pharmacol. Sci.,* 2004, vol. 25, 618-625
- **Lin J. S. et al.** *Brain Research,* 1990, vol. 523, 325-330
- **Parmentier et al.** *J. Neurosci.,* 2002, vol. 22, 7695-7711
- **Ligneau et al.** *J. Pharmacol. Exp. Ther.,* 1998, vol. 287, 658-666
- **Tedford et al.** *Soc. Neurosci. Abstr.,* 1999, vol. 25, 460.3
- **Ishizuka et al.** *Neurosci. Lett.,* 2003, vol. 339, 143-146
- **Carruthers.** *Ann. Meet. Eur. Histamine Res. Soc.,* 2004, 31
- **Hancock ; Fox.** Milestones in Drug Therapy. 2003
- **Vohora et al.** *Pharmacol. Biochem. Behav.,* 2001, vol. 68, 735-741
- **Perez-Garcia et al.** *Psychopharmacol.,* 1999, vol. 142, 215-220
- **Fox et al.** *Behav. Brain Res.,* 2002, vol. 131, 151-61
- **Fox et al.** *J. Pharmacol. Exp. Ther.,* 2005, vol. 313, 176-190
- **Vohora.** *Investigational Drugs,* 2004, vol. 7, 667-673
- **Vohora et al.** *Pharmacol. Biochem Behav,* 2001, vol. 68, 735-741
- **Hancock.** *Curr. Opin. Investig. Drugs,* 2003, vol. 4, 1190-1197
- **Karlstedt et al.** *Mol. Cell. Neurosci.,* 2003, vol. 24, 614-622
- **Hancock et al.** *Eur. J. Pharmacol.,* 2004, vol. 487, 183-197
- **Varty et al.** *Eur. J. Pharmacol.,* 2004, vol. 484, 83-89
- **McLeod et al.** *Am. J. Rhinol.,* 1999, vol. 13, 391 399
- **Medhurst et al.** *Biochemical Pharmacology,* 2007, vol. 73 (8), 1182-1194
- **Remington.** The Science and Practice of Pharmacy. Lippincott Williams & Wilkins, 2000
- *Journal of Pharmaceutical Sciences,* 1977, vol. 66, 1-19
- Polymorphism in Pharmaceutical solids. Marcel Dekker, 1999
- Pro-drugs as Novel Delivery Systems Vol. 14 of the A.C.S. Symposium Series. **T. Higuchi ; V. Stella.** Bioreversible Carriers in Drug Design. American Pharmaceutical Association and Pergamon Press
- **Zhu, G-D.** *J. Org. Chem.,* 2002, vol. 67, 943-948
- **Collier, T. L.** *J. Labelled Compd. Radiopharm.,* 1999, vol. 42, S264-S266
- **Le Bas, M.-D.** *J. Labelled Compd. Radiopharm.,* 2001, vol. 44, S280-S282